# EUROPEAN PATENT APPLICATION

(11) **EP 1 229 010 A1**
(43) Date of publication of application: **07.08.2002**
(21) Application number: 00962891.8
(22) Date of filing: 27.09.2000
(51) Int. Cl.: C07C 1/00

(54) **NOVEL DIARYLAMIDE DERIVATIVES AND USE THEREOF AS MEDICINES**

(30) Priority: 01.10.1999 JP 28127199; 13.10.1999 JP 29078999
(71) Applicant: Japan Energy Corporation, Tokyo-to 105-8407 (JP)
(72) Inventor: OGITA, Haruhisa Japan Energy Corporation, Toda-shi, Saitama 335-8502 (JP); ISOBE, Yoshiaki Japan Energy Corporation, Toda-shi, Saitama 335-8502 (JP); TAKAKU, Haruo Japan Energy Corporation, Toda-shi, Saitama 335-8502 (JP)
(74) Representative: Ziebig, Marlene, Dr. Dipl.-Chem.
(86) International application number: JP0006667
(87) International publication number: WO0125190

(57) **Abstract**

This invention relates to a diarylamide derivative represented by formula (1) or a salt thereof and a pharmaceutical composition comprising, as an active ingredient, the compound: 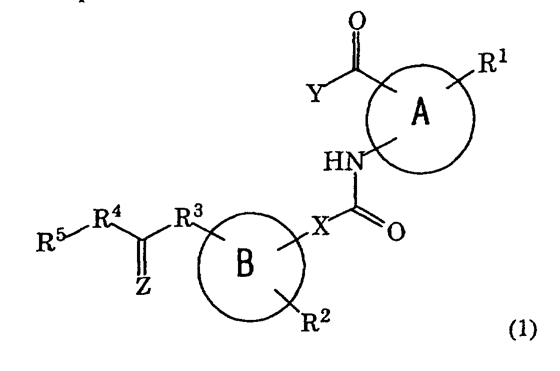 wherein,
A and B denote an aromatic ring such as a benzene ring; COY and NHCOX are adjacent to each other and these substituents are linked to carbon in the aromatic ring A; X denotes alkylene, alkyleneoxy, or a single bond; Y denotes an alkyl, alkoxy, hydroxyl, or substituted or unsubstituted amino group; R¹ denotes hydrogen, halogen, hydroxyl, alkyl or the like, provided that, when A denotes a benzene ring, R¹ does not denote hydrogen; R² denotes hydrogen, halogen, hydroxyl, alkyl or the like; R³ and R⁴ denote a substituted or unsubstituted imino group, an oxygen atom, or a single bond; R⁵ denotes alkyl, substituted or unsubstituted phenyl or the like; and Z denotes oxygen or sulfur.

## Description

### TECHNICAL FIELD

The present invention relates to diarylamide derivatives useful as pharmaceuticals, and more particularly to diarylamide derivatives having an inhibitory action against abnormal cell proliferation and pharmaceutically acceptable salts thereof.

### BACKGROUND ART

Growth factors such as insulin, epidermal growth factors, or platelet-derived growth factors (hereinafter abbreviated to PDGF) play important roles in proliferation of various cells including vascular smooth muscle cells. Especially, PDGF is known to be associated with regulation of cell proliferation/tiifferentiation as a strong cell growth factor (Cell, 46, 155 (1986)). For example, in diseases such as restenosis after percutaneous transluminal coronary angioplasty or coronary artery bypass surgery and mesangial cell proliferative nephritis, PDGF and PDGF receptors are abnormally produced in cells of pathology sites, and abnormal proliferation of cells in pathology sites is observed in these diseases.

Tranilast ((E)-2-(3,4-dimethoxycinnamoylamino)benzoic acid) inhibits PDGF-caused proliferation of vascular smooth muscle cells and prevents restenosis after percutaneous transluminal coronary angioplasty in clinical tests (Am. Heart. J, 134 (4), 712 (1997)). However, the inhibitory action of tranilast against proliferation of vascular smooth muscle cells in *in vitro* testing is weak (WO 97/09301 describes the inhibitory action against proliferation of vascular smooth muscle cells in thoracic aorta of spontaneous hypertensive rat as IC₅₀ = 231 µM), and thus, with a dose exhibiting efficacy in the clinical test, disadvantageously, hepatotoxicity frequently appears.

Mesangial cell proliferative nephritis is a disease caused by abnormal proliferation of mesangial cells in a kidney, and it is reported in Japanese Patent Laid-Open No. 306024/1998 that tranilast has inhibitory action against such proliferation.

WO 97/29744 and Br. J. Pharmacol., 122(6), 1061-1066 (1997) report that tranilast inhibits proliferation of cultured human skin microvascular endothelial cell caused by vascular endothelial growth factors and inhibits arterialization even in a mouse in vivo arterialization model in a dosage-dependent manner, thus rendering tranilast to be useful for improving neovascular diseases such as proliferative diabetic retinopathy, senile disciform macular degeneration, prematurity retinopathy, sickle cell retinopathy, occlusion of retinal vein, arterialization associated with corneal transplantation or cataract, neovascular glaucoma, rubeosis iridis, rheumatic arthritis, psoriasis, scleredema, various tumors, abnormal capillary plexus of single adventitial of atherosclerosis, and arterialization in cornea due to use of contact lens for a long period of time.

In addition, in diseases or pathologies such as leukaemia, cancer, psoriasis, glomerular disease, organ fibrous disease, articular rheumatism, arteriosclerosis, heart infarction, brain infarction, and diabetes, PDGF and PDGF receptors are abnormally produced in pathology sites. Conventional inhibitors against cell proliferation elicited by PDGF include 3-arylquinoline derivatives described in J. Med. Chem., 37, 2627 (1994), quinoxaline derivatives described in Cancer Research, 54, 6106 (1994), and bismono- and bicyclic aryl and heteroaryl derivatives described in WO 92/20642.

### DISCLOSURE OF THE INVENTION

Under the above circumstances, the object of the present invention is to provide a novel compound or a pharmaceutically acceptable salt thereof useful for prevention or treatment of cell proliferative diseases such as arteriosclerosis, vascular reocclusion disease, nephritis, diabetic retinopathy, psoriasis, and senile disciform macular degeneration by seeking a drug for inhibiting, at lower concentration, proliferation of vascular smooth muscle cells, vascular endothelial cells, epidermal cells and the like.

The present inventors, therefore, have conducted concentrated studies to attain the above object, and as a result, have found that a diarylamide derivative having a specific structure inhibits cell proliferation at low concentration. This has led to the completion of the present invention.

More specifically, the present invention includes the following.
(i) A diarylamide derivative represented by general formula (1) or a pharmaceutically acceptable salt thereof: 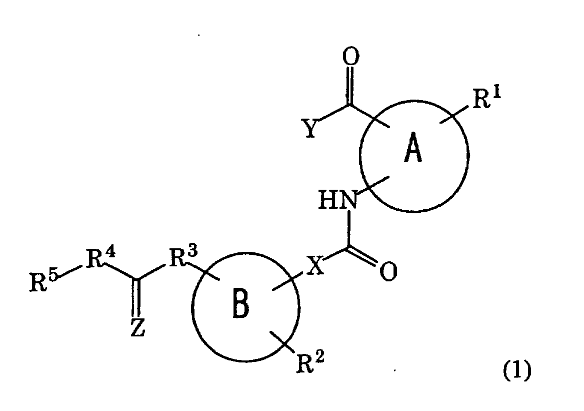 wherein,
   A is an aromatic ring selected from the group consisting of a benzene ring, a pyridine ring, a thiophene ring, a furan ring, and a naphthalene ring;
   a substituent represented by COY and a substituent represented by NHCOX are adjacent to each other and these substituents are linked to a carbon atom in the aromatic ring;
   X denotes a C₁-C₄-alkylene group, a C₁-C₄-alkyleneoxy group, or a single bond;
   Y is selected from the group consisting of a C₁-C₄-alkyl group, a C₁-C₄-alkoxy group, a hydroxyl group, and N(R⁶)(R⁷) in which each of R⁶ and R⁷, which can be the same or different, is selected from the group consisting of a hydrogen atom, a C₁-C₄-alkyl group, a C₁-C₄-alkoxy group, a C₃-C₉-cycloalkyl group, a C₄-C₉-cycloalkyl-alkyl group, a C₅-C₈-morpholino-N-alkoxy group, a C₃-C₉-alkenyl group, a phenyl group, a pyridyl group, and an aralkyl group, wherein the phenyl group and the pyridyl group and the aromatic ring of the aralkyl group are optionally substituted with 1 to 3 substituents selected from the group consisting of a C₁-C₄-alkyl group, a C₁-C₄-alkoxy group, and a halogen atom;
   R¹ is selected from the group consisting of a hydrogen atom, a halogen atom, a hydroxyl group, a C₁-C₄-alkyl group, a C₃-C₉-cycloalkyl group, a C₄-C₉-cycloalkyl-alkyl group, a C₁-C₄-alkoxy group, a C₃-C₉-cycloalkyloxy group, a C₄-C₉-cycloalkyl-alkoxy group, an aralkyloxy group, a C₁-C₄-acyl group, and a nitro group, and 1 to 4 R¹s are present at a desired position in A, each of which can be the same or different, and when two R¹s are present, they may together form a C₁-C₄-alkylenedioxy group, provided that, when A is a benzene ring, R¹ does not denote a hydrogen atom;
   B denotes a benzene, pyridine, or thiophene ring;
   R² is a substituent selected from the group consisting of a hydrogen atom, a halogen atom, a hydroxyl group, a C₁-C₄-alkyl group, a C₁-C₄-alkoxy group, a C₁-C₄-alkylthio group, a C₁-C₄-hydroxyalkoxy group, a C₃-C₉-cycloalkyloxy group, a C₄-C₉-cycloalkyl-alkoxy group, an aralkyloxy group, a C₁-C₄-acyl group, a cyano group, a C₅-C₈-morpholino-N-alkoxy group, and an amino group which can be monosubstituted or disubstituted with a C₁-C₄-alkyl group, and 1 to 4 R²s, each of which can be the same or different, are present at a desired position;
   R³ and R⁴ denote, when Y denotes other than a C₁-C₄-alkyl group, an oxygen atom or NR⁸ in which each R⁸ is selected from the group consisting of a hydrogen atom and a C₁-C₄-alkyl group, each of which can be the same or different, and when Y denotes a C₁-C₄-alkyl group, R³ denotes an oxygen atom or NR⁸ and R⁴ denotes an oxygen atom, NR⁸, or a single bond;
   R⁵ is selected from the group consisting of a C₁-C₈-alkyl group, a C₂-C₄-alkenyl group, a C₃-C₉-cycloalkyl group, a C₄-C₉-cycloalkyl-alkyl group, a tetrahydropyranyl group, an aralkyl group, an indanyl group, an aromatic acyl group, a phenyl group, a pyridyl group, a furyl group, and a thienyl group, wherein the aromatic rings of the aralkyl group, the indanyl group, and the aromatic acyl group, and the phenyl group, the pyridyl group, the furyl group, and the thienyl group optionally have 1 to 5 substituents selected from the group consisting of a halogen atom, a hydroxyl group, a cyano group, a C₁-C₄-alkyl group, a C₁-C₄-alkoxy group, a C₁-C₄-alkylthio group, a C₂-C₅-alkoxycarbonyl group, a carboxyl group, a C₁-C₄-acyl group, an aromatic acyl group, a C₁-C₄-acyloxy group, a trifluoromethyl group, a phenyl group, a phenoxy group, a phenylthio group, a pyridyl group, a morpholino group, an aralkyloxy group, a nitro group, a methylsulfonyl group, an aminosulfonyl group, and an amino group that is optionally monosubstituted or disubstituted with a C₁-C₄-alkyl group or a C₁-C₄-acyl group, and wherein adjacent two substituents may together form a C₁-C₄-alkylenedioxy group to form a ring; and
   Z denotes an oxygen or sulfur atom.
(ii) The compound according to (i) above wherein, in general formula (1), X denotes a C₁-C₄-alkylene group.
(iii) The compound according to (i) above wherein, in general formula (1), X denotes a single bond.
(iv) The compound according to any one of (i) to (iii) above wherein, in general formula (1), each of A and B, which can be the same or different, denotes a benzene or pyridine ring.
(v) The compound according to any one of (i) to (iv) above wherein, in general formula (1), A and B denote a benzene ring.
(vi) The compound according to any one of (i) to (v) above wherein, in general formula (1), Y denotes an unsubstituted amino group, a hydroxyl group, or a C₁-C₄-alkoxy group.
(vii) The compound according to any one of (i) to (v) above wherein, in general formula (1), Y denotes a C₁-C₄-alkyl group.
(viii) The compound according to any one of (i) to (vii) above wherein, in general formula (1), R² denotes a hydrogen atom or a C₁-C₄-alkoxy group.
(ix) The compound according to any one of (i) to (viii) above wherein, in general formula (1), R⁵ denotes a benzyl group, a phenyl group, a pyridyl group, or a pyridylmethyl group wherein the aromatic rings of the benzyl group and the pyridylmethyl group and the phenyl group and the pyridyl group optionally have 1 to 5 substituents selected from the group consisting of a halogen atom, a C₁-C₄-alkyl group, a C₁-C₄-alkoxy group, a C₂-C₅-alkoxycarbonyl group, a C₁-C₄-acyl group, a trifluoromethyl group, a C₁-C₄-alkylthio group, and an amino group which has been disubstituted with a C₁-C₄-alkyl group.
(x) The compound according to any one of (i) to (ix) above wherein, in general formula (1), R⁵ denotes a C₁-C₄-alkyl group, a C₂-C₄-alkenyl group, or a C₃-C₆-cycloalkyl group.
(xi) The compound according to any one of (i) to (x) above wherein, in general formula (1), R³ and R⁴ denote NH.
(xii) The compound according to any one of (i) to (x) above wherein, in general formula (1), R³ denotes NH and R⁴ denotes an oxygen atom.
(xiii) A pharmaceutical composition comprising, as an active ingredient, the compound or pharmaceutically acceptable salt thereof according to any one of (i) to (xii) above.
(xiv) A pharmaceutical composition comprising, as an active ingredient, the compound or pharmaceutically acceptable salt thereof according to any one of (i) to (xii) above, that is usable for prevention or treatment of diseases caused by abnormal proliferation of vascular smooth muscle cells.
(xv) A pharmaceutical composition comprising, as an active ingredient, the compound or pharmaceutically acceptable salt thereof according to any one of (i) to (xii) above, that is usable for prevention or treatment of restenosis or atherosclerosis after percutaneous transluminal coronary angioplasty or coronary artery bypass surgery.
(xvi) A pharmaceutical composition comprising, as an active ingredient, the compound or pharmaceutically acceptable salt thereof according to any one of (i) to (xii) above, that is usable for prevention or treatment of diseases caused by abnormal proliferation of mesangial cells.
(xvii) A pharmaceutical composition comprising, as an active ingredient, the compound or pharmaceutically acceptable salt thereof according to any one of (i) to (xii) above, that is usable for prevention or treatment of diseases caused by abnormal proliferation of vascular endothelial cells or epidermal cells.
(xviii) A pharmaceutical composition comprising, as an active ingredient, the compound or pharmaceutically acceptable salt thereof according to any one of (i) to (xii) above, that is usable for prevention or treatment of psoriasis, diabetic retinopathy, or senile disciform macular degeneration.

The compound of the present invention will now be described in more detail. The compound of the present invention is represented by general formula (1) in which R¹, R², R³, R⁴, R⁵, X, Y, Z, ring A, and ring B are as defined above, respectively. The following substituents described herein are described in more detail with reference to specific examples as follows.

Examples of a halogen atom include fluorine, chlorine, bromine, and iodine.

Examples of a C₁-C₄-alkyl group include methyl, ethyl, propyl, isopropyl, butyl, isobutyl, s-butyl, and t-butyl.

Examples of a C₃-C₉-cycloalkyl group include cyclopropyl, cyclobutyl, cyclopentyl, cyclohexyl, and cycloheptyl.

Examples of a C₄-C₉-cycloalkyl-alkyl group include cyclopentylmethyl, cyclohexylmethyl, cyclopentylethyl, and cyclohexylethyl.

Examples of a C₂-C₄-alkenyl group include allyl, vinyl, isopropenyl, 1-propenyl, 2-propenyl, 1-butenyl, 2-butenyl, 3-butenyl, 1-methyl-1-propenyl, 2-methyl-1-propenyl, 1-methyl-2-propenyl, and 2-methyl-2-propenyl.

Examples of a C₃-C₉-alkenyl group include allyl, isopropenyl, 1-propenyl, 2-propenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 3-pentenyl, 4-pentenyl, 3-methyl-2-butenyl, hexenyl, heptenyl, octenyl, and nonenyl.

Examples of a C₁-C₄-alkoxy group include methoxy, ethoxy, propoxy, isopropoxy, butoxy, isobutoxy, s-butoxy, and t-butoxy.

Examples of a C₃-C₉-cycloalkyloxy group include cyclopropoxy, cyclobutoxy, cyclopentoxy, cyclohexyloxy, and cycloheptyloxy.

Examples of a C₄-C₉-cycloalkyl-alkoxy group include cyclopentylmethoxy, cyclohexylmethoxy, cyclopentylethoxy, and cyclohexylethoxy.

Examples of an aralkyloxy group include benzyloxy, 1-naphthylmethoxy, 2-naphthylmethoxy, 2-phenylethoxy, 1-phenylethoxy, 3-phenylpropoxy, 4-phenylbutoxy, 5-phenylpentoxy, and 6-phenylhexyloxy.

Examples of a C₁-C₄-acyl group include formyl, acetyl, propionyl, and butyryl.

Examples of an aromatic acyl group include benzoyl, toluoyl, and naphthoyl.

Examples of an amino group monosubstituted with a C₁-C₄-alkyl group include methylamino, ethylamino, propylamino, isopropylamino, butylamino, isobutylamino, s-butylamino, and t-butylamino.

Examples of an amino group disubstituted with a C₁-C₄-alkyl group include dimethylamino, diethylamino, dipropylamino, and dibutylamino.

Examples of a C₂-C₅-alkoxycarbonyl group include methoxycarbonyl, ethoxycarbonyl, propoxycarbonyl, isopropoxycarbonyl, butoxycarbonyl, isobutoxycarbonyl, s-butoxycarbonyl, and t-butoxycarbonyl.

Examples of a C₁-C₄-alkylenedioxy group include methylenedioxy and ethylenedioxy.

Examples of a C₁-C₄-hydroxyalkoxy group include hydroxymethoxy, hydroxyethoxy, hydroxypropoxy, and hydroxybutoxy.

Examples of a C₅-C₈-morpholino-N-alkoxy group include morpholino-N-methoxy, morpholino-N-ethoxy, morpholino-N-propoxy, and morpholino-N-butoxy.

Examples of an aralkyl group (including a heteroaromatic substituted alkyl group) include benzyl, 1-naphthylmethyl, 2-naphthylmethyl, 2-phenylethyl, 1-phenylethyl, 3-phenylpropyl, 4-phenylbutyl, 5-phenylpentyl, 6-phenylhexyl, methylbenzyl, 1-methylphenethyl, dimethylbenzyl, 1-dimethylphenethyl, 1-ethylbenzyl, diethylbenzyl, thienylmethyl, thienylethyl, furylmethyl, furylethyl, pyridylmethyl, and pyridylethyl.

Examples of a C₁-C₄-alkylene group include methylene, ethylene, trimethylene, and tetramethylene.

Examples of a C₁-C₄-alkyleneoxy group include methyleneoxy, ethyleneoxy, trimethyleneoxy, and tetramethyleneoxy.

Examples of a C₁-C₄-acyloxy group include acetyloxy, propionyloxy, and butyryloxy.

Examples of a C₁-C₄-alkylthio group include methylthio, ethylthio, propylthio, isopropylthio, butylthio, isobutylthio, s-butylthio, and t-butylthio.

In the diarylamide derivative of the present invention represented by general formula (1), an aromatic ring represented by A is as described above in which a benzene ring and a pyridine ring are preferred, with the benzene ring being more preferred.

As the group represented by X, a single bond (direct bond), a methylene group and an ethylene group are preferred. The ethylene group is particularly preferred.

Substituents represented by Y are as described above in which an amino group, a hydroxyl group, a C₁-C₄-alkoxy group, and a C₁-C₄-alkyl group are preferred and an amino group, a methoxy group, an ethoxy group, and a methyl group are more preferred.

Substituents represented by R¹ are as described above in which one or two substituents selected from the group consisting of a C₁-C₄-alkoxy group, a nitro group, and a halogen atom are preferably present and these substituents are more preferably a methoxy group, a ethoxy group, a methylenedioxy group, or fluorine. A binding position of R¹ is, when a ring A is a benzene ring, preferably monosubstitution at the 4-or 5-position to the substituent represented by NHCOX or disubstitution at the 4- and 5-positions .

The ring represented by B is as described above in which a benzene ring is preferred.

R² is preferably a hydrogen atom or monosubstitution of C₁-C₄-alkoxy group.

Preferably, as R³ and R⁴, both R³ and R⁴ are NH or R³ denotes NH and R⁴ denotes an oxygen atom.

R⁵ is preferably a benzyl, phenyl, pyridyl, or pyridylmethyl group, and the aromatic rings of the benzyl group and the pyridylmethyl group and the phenyl group and the pyridyl group optionally have 1 to 5 substituents selected from the group consisting of a halogen atom, a C₁-C₄-alkyl group, a C₁-C₄-alkoxy group, a C₂-C₅-alkoxycarbonyl group, a C₁-C₄-acyl group, a trifluoromethyl group, a C₁-C₄-alkylthio group, and an amino group that is disubstituted with a C₁-C₄-alkyl group.

Z is preferably oxygen.

The compound of the present invention can be synthesized by, for example, the following processes although the process for producing the compound of the present invention is, needless to say, not limited to these processes only.

All the compounds of the present invention are novel compounds which have not been heretofore described in any literature, however, they can be produced by conventional processes described in literature or processes similar thereto. Examples of such literature include "Organic Functional Group Preparations" by S. R. Sandier et al. (Academic Press Inc., New York and London, 1968), "Synthetic Organic Chemistry" by S. R. Wagner et al. (John Wiley, 1961), "Comprehensive Organic Transformations" by R. C. Larock (1989), "Encyclopedia of Reagents for Organic Synthesis" by L. A. Paquette et al. (1995), and "Compendium of Organic Synthetic Methods" by M. B. Smith (1995). As an analogous compound of the compound according to the present invention, synthesis of a compound in which A denotes a benzene ring, R¹ denotes a hydrogen atom, and X denotes a single bond in formula (1) has been reported, and a compound can be synthesized also by a similar process. Examples of reports include Indian. J. Chem., Sect. B (1987), 26B (12), 1133-9, Japanese Patent Publication No. 24825/1990, Acta Chim. Acad. Sci. Hung. (1981), 107 (1), 57-66, Tetrahedron (1968), 24 (16), 5529-45, Acta Chim. Acad. Sci. Hung. (1966), 48 (1), 77-87, J. Org. Chem. (1967), 32 (2), 462-3, Acta Vet. (Brno) (1971), 40 (2), 209-14, J. Org. Chem. (1974), 39 (13), 1931-5, and J. Chem. Eng. Data (1968), 13 (4), 577-9. There is no description concerning physiological activities of the compound in the above-described literature. A starting compound to be used in production can be a commercially available one, or can be produced by a conventional process if necessary. Examples of production processes are described below.

### [Production process 1]

A compound in which R³ denotes NH in general formula (1) can be produced in accordance with the following reaction steps: 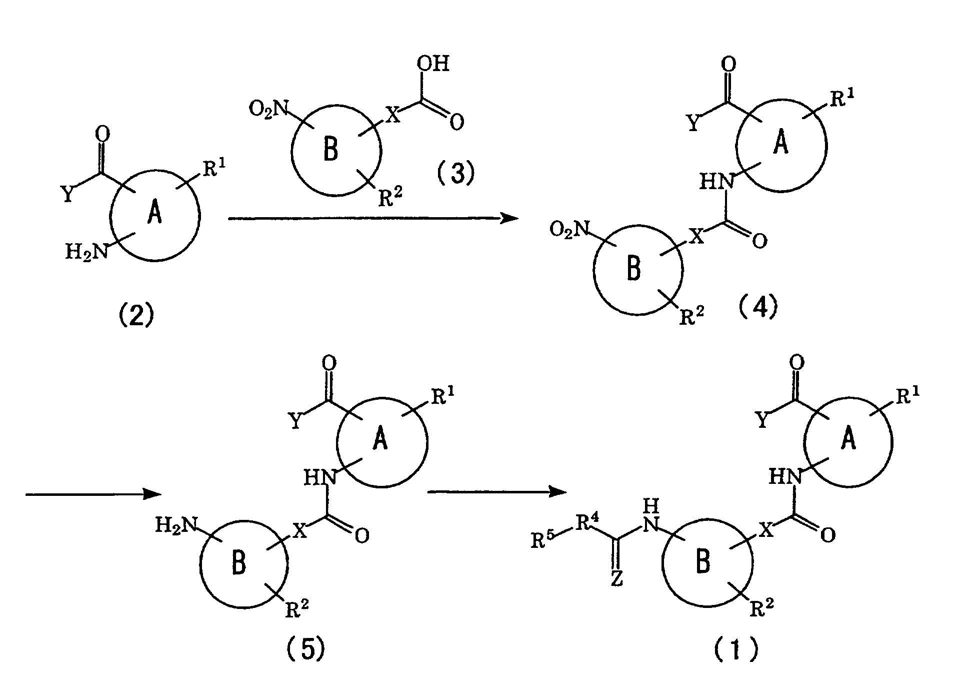 wherein,
R¹, R², R⁴, R⁵, X, Y, Z, ring A, and ring B are as defined above.

For a compound (2) that is a starting material, a commercially available product can be purchased or the compound (2) can be produced by a conventional process described in literature or a process similar thereto. For example, when ring A is a benzene ring, the compound (2) can be produced using the following compounds as starting materials.

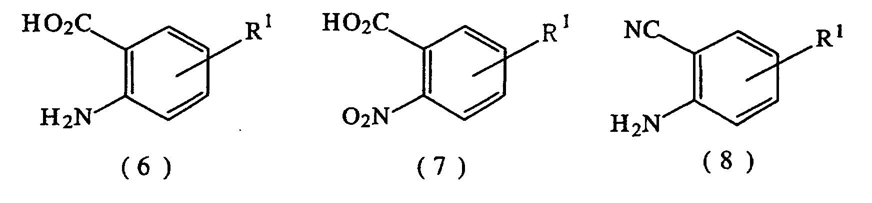

An anthranilic acid derivative represented by general formula (6) can be subjected to condensation with an amine using a carbodiimide reagent such as dicyclohexylcarbodiimide, thereby producing a compound in which Y denotes N(R⁶)(R⁷). A compound in which Y denotes a C₁-C₄-alkyl group, a C₁-C₄-alkoxy group, or N(R⁶)(R⁷) can be produced through treatment of a nitrobenzoic acid derivative represented by general formula (7) with thionyl chloride or the like followed by reaction with an alcohol or an amine in an inactive solvent in the presence of a base or through the same treatment as with general formula (6), followed by conversion of a nitro group into an amino group in accordance with a conventional process described in literature or a process similar thereto. Regarding the nitrile derivative represented by general formula (8), the nitrile group can be hydrolyzed by a conventional process described in literature or a process similar thereto to synthesize a compound in which Y denotes a hydroxyl group.

The compound represented by general formula (4) can be produced by a conventional process described in literature or a process similar thereto, that is, a condensation reaction of an amine derivative represented by general formula (2) with a carboxylic acid derivative represented by general formula (3). This condensation reaction can be carried out in the presence of various condensing reagents. Condensing reagents usable herein include, for example, a carbodiimide reagent such as dicyclohexylcarbodiimide, carbonyldiimidazole, and 2-chloro-1-methylpyridinium iodide. A condensation reaction can also be carried out by reacting the carboxylic acid compound represented by general formula (3) with a halogenizing reagent such as thionyl chloride to convert it into a corresponding acid halide or, for example, converting it into an acid anhydride as a reaction activator by means of p-toluenesulfonyl chloride or the like, and then reacting with the amine derivative represented by general formula (2). In this condensation reaction, a suitable solvent can be used, which is selected from inactive solvents, for example, ethers such as tetrahydrofuran, aromatic hydrocarbons such as toluene, hydrocarbons such as cyclohexane, halogenated hydrocarbons such as dichloromethane and chloroform, nitriles such as acetonitrile, esters such as ethyl acetate, N,N-dimethylformamide, and dimethyl sulfoxide. The reaction can be carried out at 0°C to the reflux temperature of the solvent used.

The compound represented by general formula (5) can be produced by converting a nitro group of the amide derivative represented by general formula (4) into an amino group by a conventional process described in literature or a process similar thereto. For example, the compound can be produced by performing a hydrogenation reaction in an alcoholic solvent such as methanol or ethanol in the presence of a catalyst such as palladium-carbon, iron, or tin powder. The reaction can be carried out at 0°C to the reflux temperature of the solvent used.

When R⁴ denotes NH in the compound represented by general formula (1), the compound can be produced as follows. The compound represented by general formula (5) is optionally reacted with isocyanate (R⁵NCO) or isothiocyanate (R⁵NCS) prepared by a conventional method in the presence of bases, for example, organic bases such as triethylamine, pyridine, and dimethylaminopyridine, inorganic bases such as potassium carbonate, sodium hydroxide, and sodium hydride, and metal alkoxides such as sodium methoxide and potassium t-butoxide, in adequate inactive solvents, for example, ethers such as diethyl ether, tetrahydrofuran, and 1,4-dioxane, aromatic hydrocarbons such as benzene and toluene, halogenated hydrocarbons such as dichloromethane and chloroform, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours. Alternatively, an isocyanate equivalent which is separately prepared from a corresponding amine and triphosgene or carbonyldiimidazole, is used instead of isocyanate in a reaction, and thus synthesis can be carried out.

When R⁴ denotes an oxygen atom in the compound represented by general formula (1), the compound can be produced as follows. The compound represented by general formula (5) is reacted with a carbamic acid halide (R⁵OCOX) prepared by a conventional process, if necessary, in the presence of bases, for example, organic bases such as triethylamine, pyridine, and dimethylaminopyridine, inorganic bases such as potassium carbonate, sodium hydroxide, and sodium hydride, metal alkoxides such as sodium methoxide and potassium t-butoxide, in adequate inactive solvents, for example, ethers such as diethyl ether, tetrahydrofuran, and 1,4-dioxane, aromatic hydrocarbons such as benzene and toluene, halogenated hydrocarbons such as dichloromethane, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours. Alternatively, a carbamic acid halide equivalent which is separately prepared from a corresponding alcohol and triphosgene or carbonyldiimidazole is used instead of a carbamic acid halide in a reaction, and thus synthesis can be carried out.

When R⁴ denotes NR⁸ and R⁸ denotes a C₁-C₄-alkyl group in the compound represented by general formula (1), the compound can be produced as follows. The compound represented by general formula (5) is reacted with carbamoyl chloride or thiocarbamoyl chloride represented by R⁴R⁵NCZCl prepared by a conventional method, if necessary, in the presence of bases, for example, organic bases such as triethylamine, pyridine, and dimethylaminopyridine, inorganic bases such as potassium carbonate, sodium hydroxide, and sodium hydride, and metal alkoxides such as sodium methoxide and potassium t-butoxide, in adequate inactive solvents, for example, ethers such as diethyl ether, tetrahydrofuran, and 1,4-dioxane, aromatic hydrocarbons such as benzene and toluene, halogenated hydrocarbons such as dichloromethane and chloroform, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours.

The compound represented by general formula (1), when R⁴ denotes a single bond, can be produced through a condensation reaction of the compound represented by general formula (5) with a carboxylic acid derivative represented by R⁵CO₂H by, for example, a conventional process described in literature or a process similar thereto, if necessary. This condensation reaction can be carried out in the presence of various condensing reagents. Condensing reagents usable herein include, for example, a carbodiimide reagent such as dicyclohexylcarbodiimide, carbonyldiimidazole, or 2-chloro-1-methylpyridinium iodide. The condensation reaction can also be carried out by reacting the carboxylic acid compound represented by R⁵CO₂H with a halogenizing reagent such as thionyl chloride to convert it into a corresponding acid halide or, for example, converting it into an acid anhydride as a reaction activator by means of p-toluenesulfonyl chloride or the like, followed by reaction with the amino compound represented by general formula (5). In this condensation reaction, a suitable solvent can be used, which is selected from inactive solvents, for example, ethers such as tetrahydrofuran, aromatic hydrocarbons such as toluene, hydrocarbons such as cyclohexane, halogenated hydrocarbons such as dichloromethane and chloroform, nitriles such as acetonitrile, and esters such as ethyl acetate, N,N-dimethylformamide, and dimethyl sulfoxide. The reaction can be carried out at 0°C to the reflux temperature of the solvent used.

### [Production process 2]

A compound in which R³ denotes NR⁸ in compound (1) can be produced in accordance with the following reaction steps: 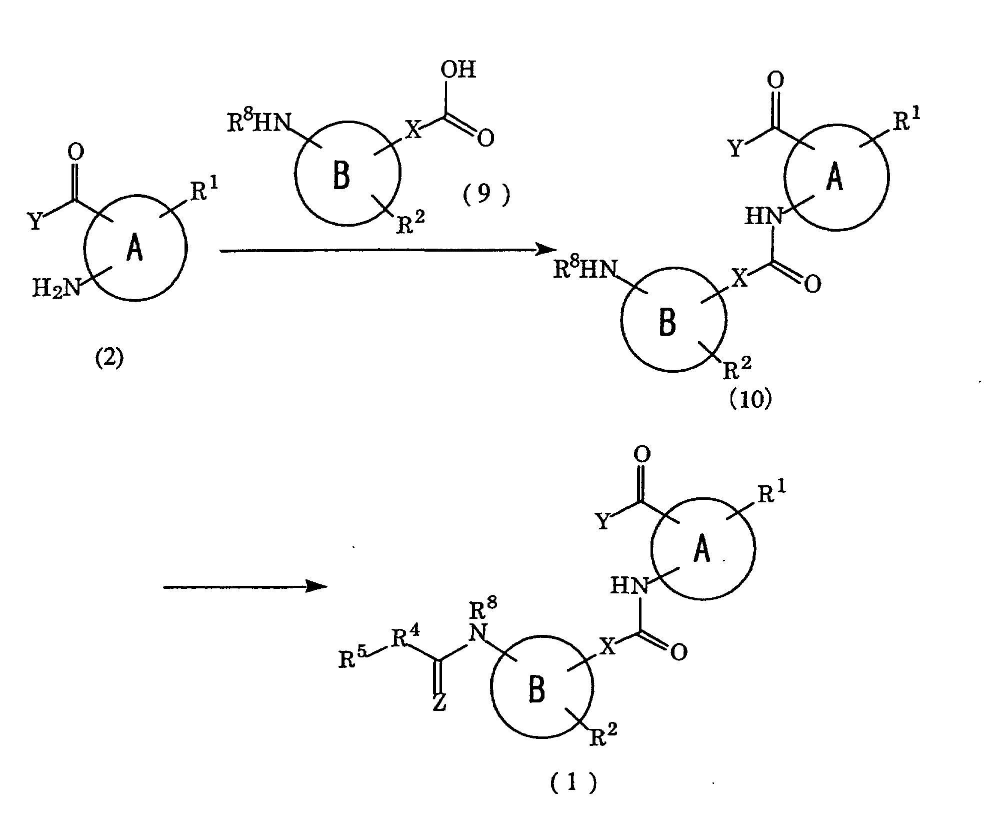 wherein,
R¹, R², R⁴, R⁵, R⁸, X, Y, Z, ring A, and ring B are as defined above.

The compound represented by general formula (10) can be produced by a conventional process described in literature or a process similar thereto, that is, a condensation reaction of the amine derivative represented by general formula (2) with the carboxylic acid derivative represented by general formula (9). This condensation reaction can be carried out in the presence of condensing reagents. Condensing reagents usable herein include, for example, a carbodiimide reagent such as dicyclohexylcarbodiimide, carbonyldiimidazole, or 2-chloro-1-methylpyridinium iodide. In this condensation reaction, a suitable solvent can be used, which is selected from inactive solvents, for example, ethers such as tetrahydrofuran, aromatic hydrocarbons such as toluene, hydrocarbons such as cyclohexane, halogenated hydrocarbons such as dichloromethane, nitriles such as acetonitrile, esters such as ethyl acetate, N,N-dimethylformamide, and dimethyl sulfoxide. The reaction can be carried out at 0°C to the reflux temperature of the solvent used.

When R⁴ denotes NH in the compound represented by general formula (1), the compound can be produced as follows. The compound represented by general formula (10) is reacted with isocyanate (R⁵NCO) or isothiocyanate (R⁵NCS) prepared by a conventional method, if necessary, in the presence of bases, for example, organic bases such as triethylamine, pyridine, and dimethylaminopyridine, inorganic bases such as potassium carbonate, sodium hydroxide, and sodium hydride, and metal alkoxides such as sodium methoxide and potassium t-butoxide, in adequate inactive solvents, for example, ethers such as diethyl ether, tetrahydrofuran, and 1,4-dioxane, aromatic hydrocarbons such as benzene and toluene, halogenated hydrocarbons such as dichloromethane and chloroform, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours.

When R⁴ denotes NR⁸ and R⁸ denotes a C₁-C₄-alkyl group in the compound represented by general formula (1), the compound can be produced as follows. The compound represented by general formula (10) is reacted with carbamoyl chloride or thiocarbamoyl chloride represented by R⁸R⁵NCZCl prepared by a conventional process, if necessary, in the presence of bases, for example, organic bases such as triethylamine, pyridine, and dimethylaminopyridine, inorganic bases such as potassium carbonate, sodium hydroxide, and sodium hydride, and metal alkoxides such as sodium methoxide and potassium t-butoxide, in adequate inactive solvents, for example, ethers, such as diethyl ether, tetrahydrofuran, and 1,4-dioxane, aromatic hydrocarbons such as benzene and toluene, halogenated hydrocarbons such as dichloromethane and chloroform, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours.

### [Production process 3]

A compound in which R³ denotes NR⁸ in compound (1) can be produced in accordance with the following reaction steps: 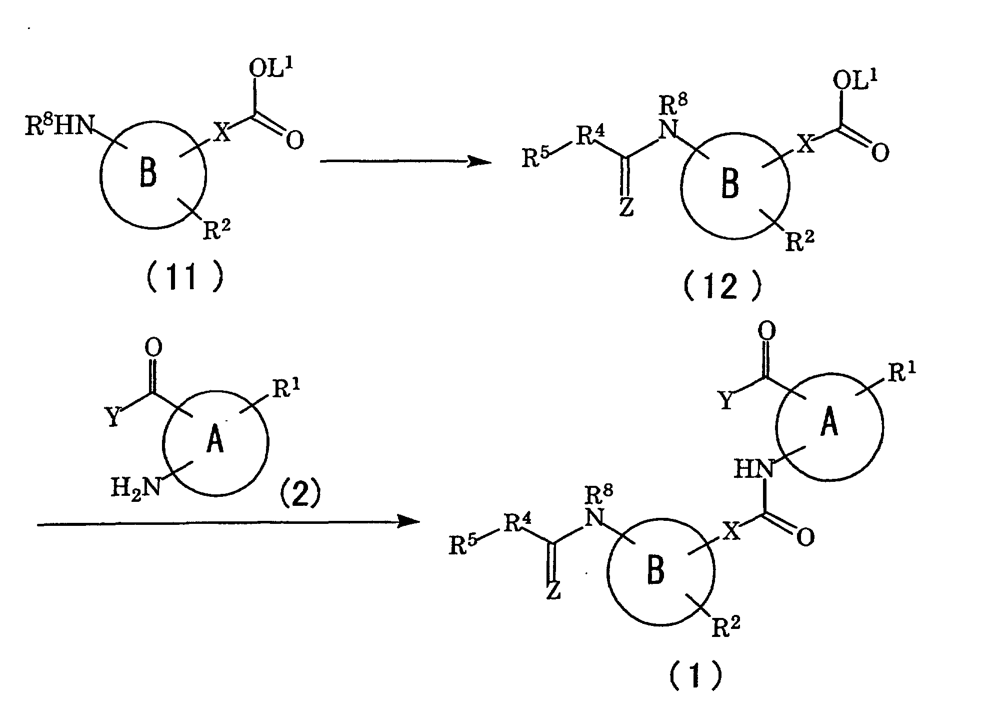 wherein,
R¹, R², R⁴, R⁵, R⁸, X, Y, Z, ring A, and ring B are as defined above and L¹ denotes a hydrogen atom or a protective group such as a benzyl group or an alkyl group.

The compound represented by general formula (12) can be produced as follows when R⁴ denotes NH. The compound represented by general formula (11) is reacted with isocyanate (R⁵NCO) or isothiocyanate (R⁵NCS) prepared by a conventional method, if necessary, in the presence of bases, for example, organic bases such as triethylamine, pyridine, and dimethylaminopyridine, inorganic bases such as potassium carbonate, sodium hydroxide, and sodium hydride, and metal alkoxides such as sodium methoxide and potassium t-butoxide, in adequate inactive solvents, for example, ethers such as diethyl ether, tetrahydrofuran, and 1,4-dioxane, aromatic hydrocarbons such as benzene and toluene, halogenated hydrocarbons such as dichloromethane and chloroform, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours.

The compound represented by general formula (12) can be produced as follows when R⁴ denotes NR⁸ and R⁸ denotes a C₁-C₄-alkyl group. The compound represented by general formula (11) is reacted with carbamoyl chloride or thiocarbamoyl chloride represented by R⁸R⁵NCZCl prepared by a conventional method, if necessary, in the presence of bases, for example, organic bases such as triethylamine, pyridine, and dimethylaminopyridine, inorganic bases such as potassium carbonate, sodium hydroxide, and sodium hydride, and metal alkoxides such as sodium methoxide and potassium t-butoxide, in adequate inactive solvents, for example, ethers, such as diethyl ether, tetrahydrofuran, and 1,4-dioxane, aromatic hydrocarbons such as benzene and toluene, halogenated hydrocarbons such as dichloromethane and chloroform, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours.

The compound represented by general formula (1) can be produced by a conventional process described in literature or a process similar thereto, that is, a condensation reaction of the amine derivative represented by general formula (2) with the compound represented by general formula (12) or the compound which was subjected to a deprotection reaction represented by general formula (12). This condensation reaction can be carried out in the presence of condensing reagents. Condensing reagents usable herein include, for example, a carbodiimide reagent such as dicyclohexylcarbodiimide, carbonyldiimidazole, or 2-chloro-1-methylpyridinium iodide. In this condensation reaction, a suitable solvent can be used, which is selected from inactive solvents, for example, ethers such as tetrahydrofuran, aromatic hydrocarbons such as toluene, hydrocarbons such as cyclohexane, halogenated hydrocarbons such as dichloromethane, nitriles such as acetonitrile, esters such as ethyl acetate, N,N-dimethylformamide, and dimethyl sulfoxide.

### [Production process 4]

A compound in which R³ denotes an oxygen atom in compound (1) can be produced in accordance with the following reaction steps: 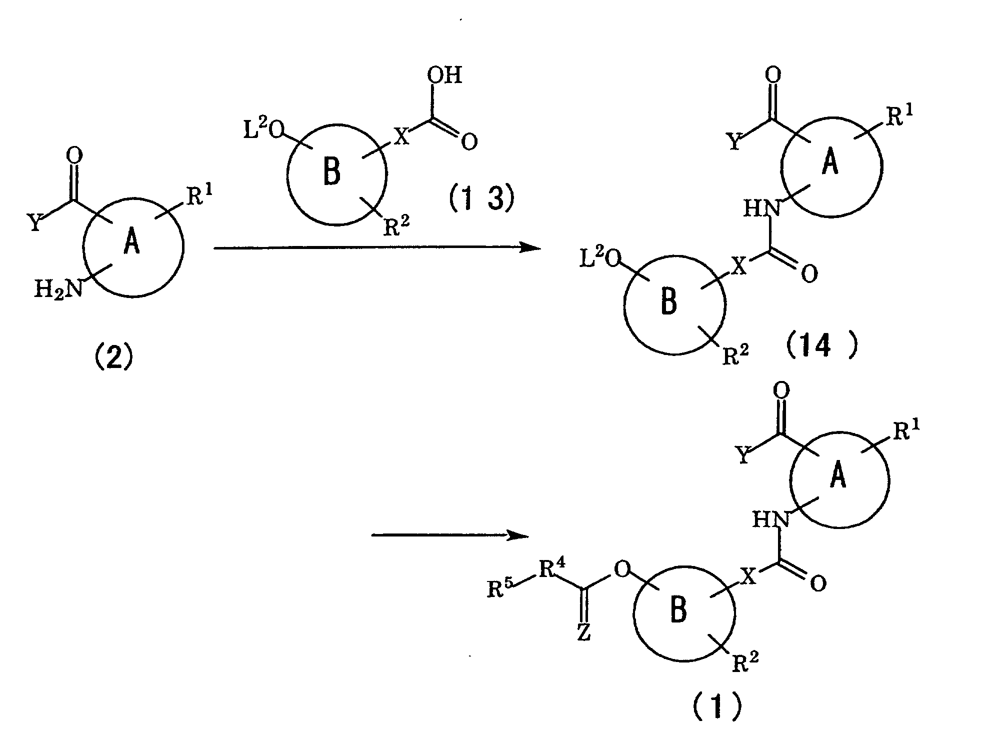 wherein,
R¹, R², R⁴, R⁵, X, Y, Z, ring A, and ring B are as defined above and L² denotes a hydrogen atom or a protective group.

The compound represented by general formula (14) can be produced by a conventional process described in literature or a process similar thereto, that is, a condensation reaction of the amine derivative represented by general formula (2) with the carboxylic acid derivative represented by general formula (13). This condensation reaction can be carried out in the presence of various condensing reagents. Condensing reagents usable herein include, for example, a carbodiimide reagent such as dicyclohexylcarbodiimide, carbonyldiimidazole, or 2-chloro-1-methylpyridinium iodide. The condensation reaction can also be carried out by reacting the carboxylic acid compound represented by general formula (13) with a halogenizing reagent such as thionyl chloride to convert it into a corresponding acid halide or, for example, converting it into an acid anhydride as a reaction activator by means of p-toluenesulfonyl chloride or the like, followed by reaction with the amino compound represented by general formula (2). In this condensation reaction, a suitable solvent can be used, which is selected from inactive solvents, for example, ethers such as tetrahydrofuran, aromatic hydrocarbons such as toluene, hydrocarbons such as cyclohexane, halogenated hydrocarbons such as dichloromethane and chloroform, nitriles such as acetonitrile, esters such as ethyl acetate, N,N-dimethylformamide, and dimethyl sulfoxide. The reaction can be carried out at 0°C to the reflux temperature of the solvent used.

The compound represented by general formula (1) can be produced as follows when R⁴ denotes NH. The compound represented by general formula (5) is reacted with isocyanate (R⁵NCO) or isothiocyanate (R⁵NCS) prepared by a conventional method, if necessary, in the presence of bases, for example, organic bases such as triethylamine, pyridine, and dimethylaminopyridine, inorganic bases such as potassium carbonate, sodium hydroxide, and sodium hydride, and metal alkoxides such as sodium methoxide and potassium t-butoxide, in adequate inactive solvents, for example, ethers such as diethyl ether, tetrahydrofuran, and 1,4-dioxane, aromatic hydrocarbons such as benzene and toluene, halogenated hydrocarbons such as dichloromethane, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours. Alternatively, an isocyanate equivalent which is separately prepared from a corresponding amine and triphosgene or carbonyldiimidazole, is used instead of isocyanate in a reaction, and thus synthesis can be carried out.

The compound represented by general formula (1) can be produced as follows when R⁴ = NR⁸ and R⁸ ≠ H. The compound represented by general formula (5) is reacted with carbamoyl chloride or thiocarbamoyl chloride represented by R⁸R⁵NCZCl prepared by a conventional method, if necessary, in the presence of bases, for example, organic bases such as triethylamine, pyridine, and dimethylaminopyridine, inorganic bases such as potassium carbonate, sodium hydroxide, and sodium hydride, and metal alkoxides such as sodium methoxide and potassium t-butoxide, in adequate inactive solvents, for example, ethers, such as diethyl ether, tetrahydrofuran, and 1,4-dioxane, aromatic hydrocarbons such as benzene and toluene, halogenated hydrocarbons such as dichloromethane and chloroform, aprotic polar solvents such as N,N-dimethylformamide, dimethyl sulfoxide, and N-methylpyrrolidone or a mixed solvent thereof, at -20°C to the boiling point of the solvent used for 10 minutes to 48 hours.

When a group defined in the production processes is changed under the conditions of a utilized process or is unsuitable for carrying out the process, a subject compound can be obtained by, for example, utilizing a method for introducing and eliminating a protective group which is commonly used in organic synthetic chemistry (for example, see Protective Groups in Organic Synthesis, by Green (John Wiley) (1981)). Some of the compounds (1) can be further led to a novel derivative (1) by adopting this as a synthetic intermediate.

An intermediate and a subject compound in the various production processes can be subjected to a purification means which is commonly used in organic synthetic chemistry such as neutralization, filtration, extraction, washing, drying, concentration, recrystallization, or various types of chromatography in order to be isolated and purified. The intermediate can be subjected to the next reaction without particular purification.

An isomer can be present in some compounds (1). In addition to these, the present invention includes all the possible isomers and mixtures thereof.

When a salt of compound (1) is to be obtained, when compound (1) can be attained in the form of salt, it can be purified in that state. When the salt can be attained in a free form, it can be dissolved or suspended in a suitable organic solvent to form a salt by a conventional method with the addition of an acid or base. Pharmaceutically acceptable salts include, for example, acid addition salts with mineral acids such as hydrochloric acid, hydrobromic acid, hydriodic acid, sulfuric acid, and phosphoric acid, acid addition salts with organic acids such as formic acid, acetic acid, methanesulfonic acid, benzenesulfonic acid, p-toluenesulfonic acid, propionic acid, citric acid, succinic acid, butyric acid, oxalic acid, malonic acid, maleic acid, lactic acid, malic acid, carbonic acid, glutamic acid, and aspartic acid, a salt with inorganic bases including sodium salt, potassium salt, and calcium salt, and a salt with organic amines including morpholine and piperidine and amino acid.

Compound (1) and a pharmaceutically acceptable salt thereof are sometimes present in the form of an adduct with water or various solvents and the present invention also includes these adducts.

Specific examples of compound (1) obtained by the above-described production processes are shown in Table 1 to Table 8. The compound of the present invention is, needless to say, not limited to these.

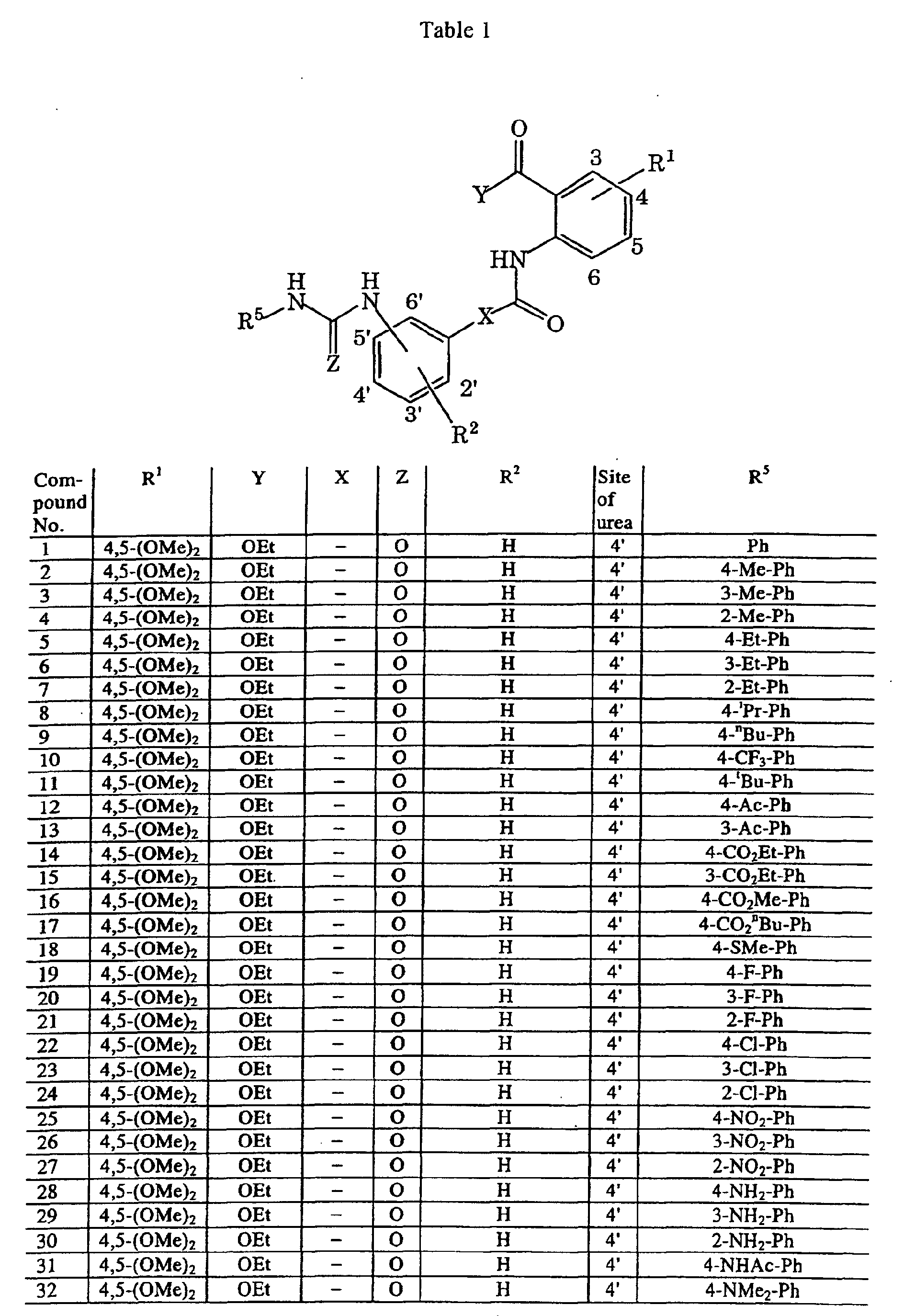

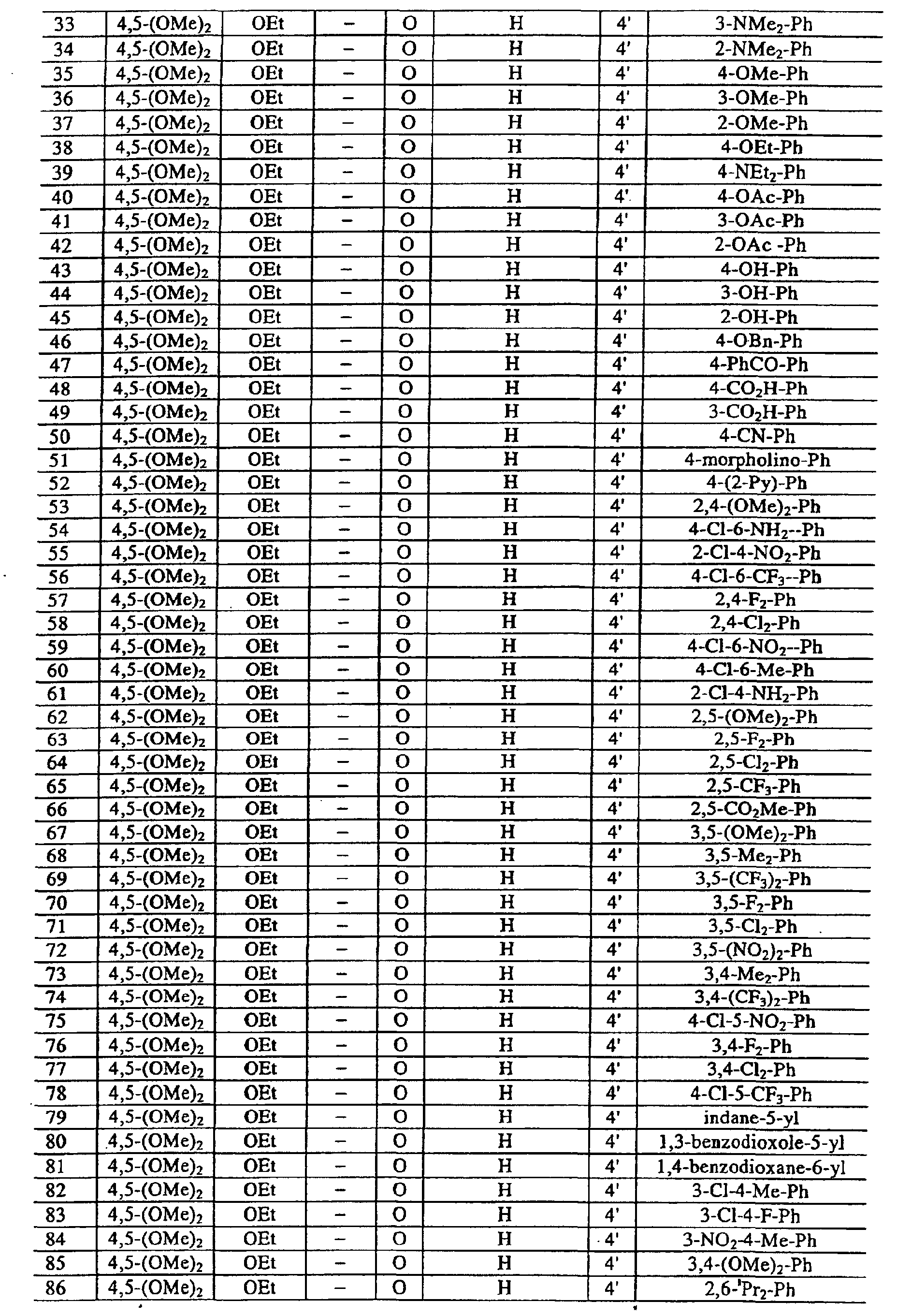

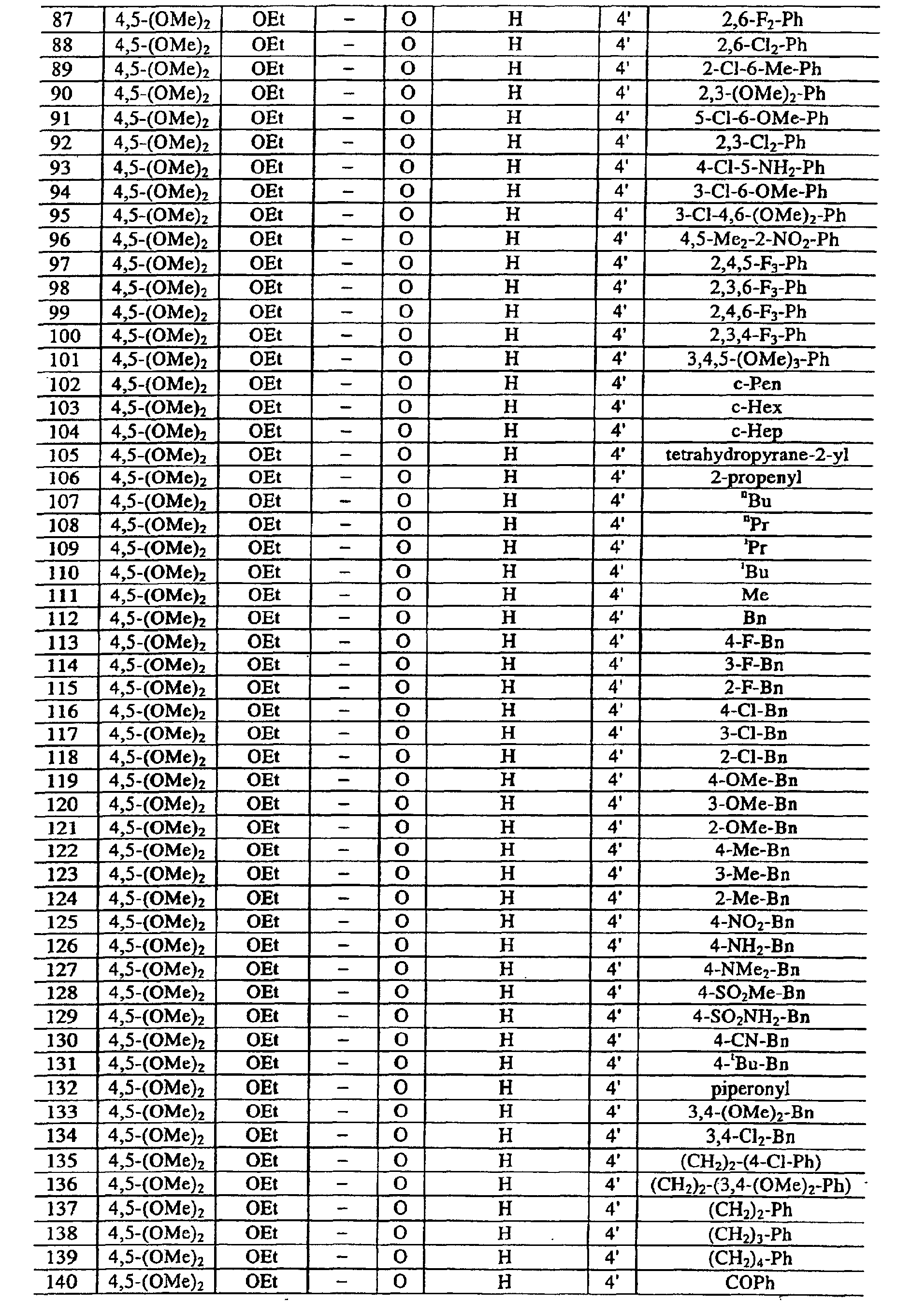

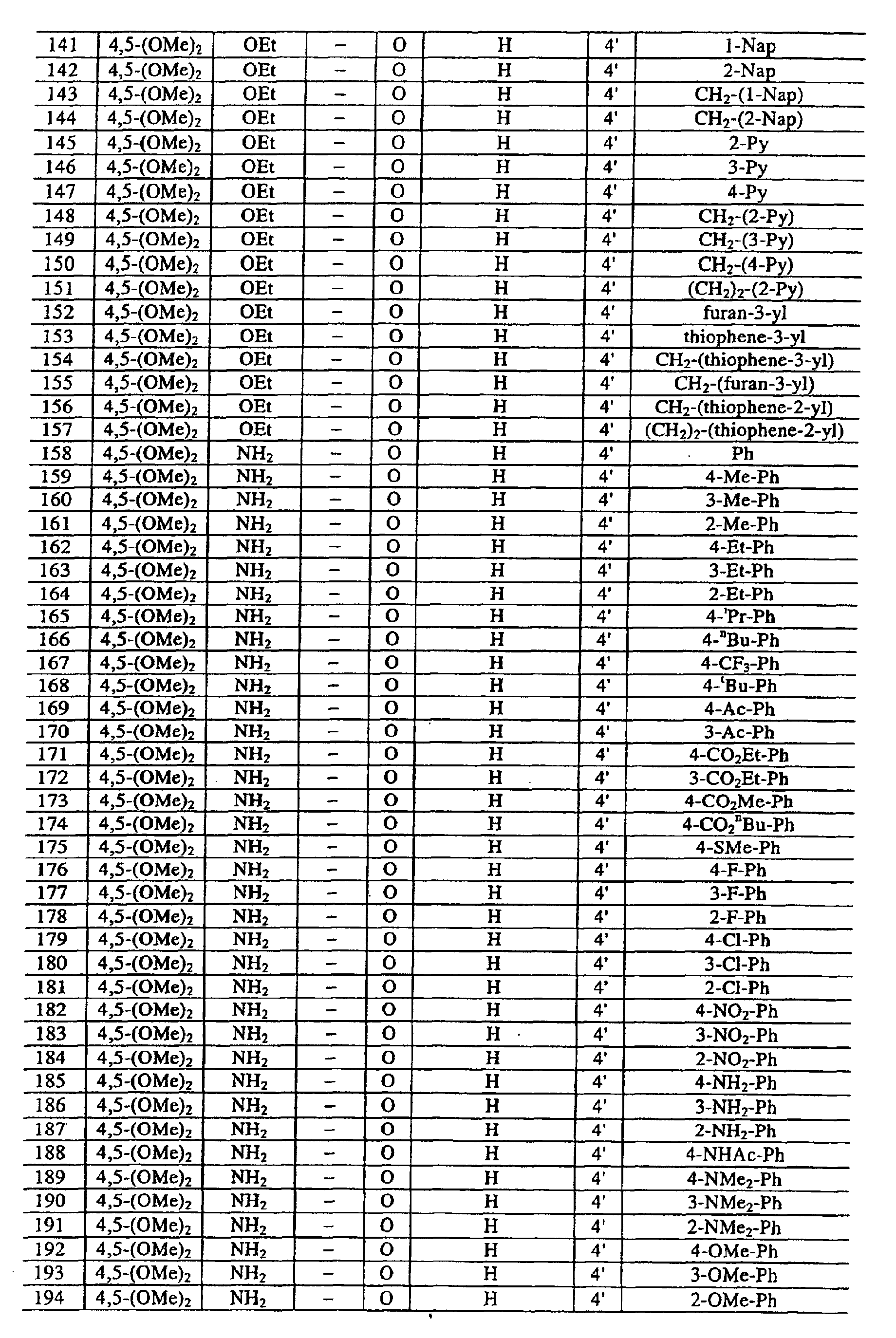

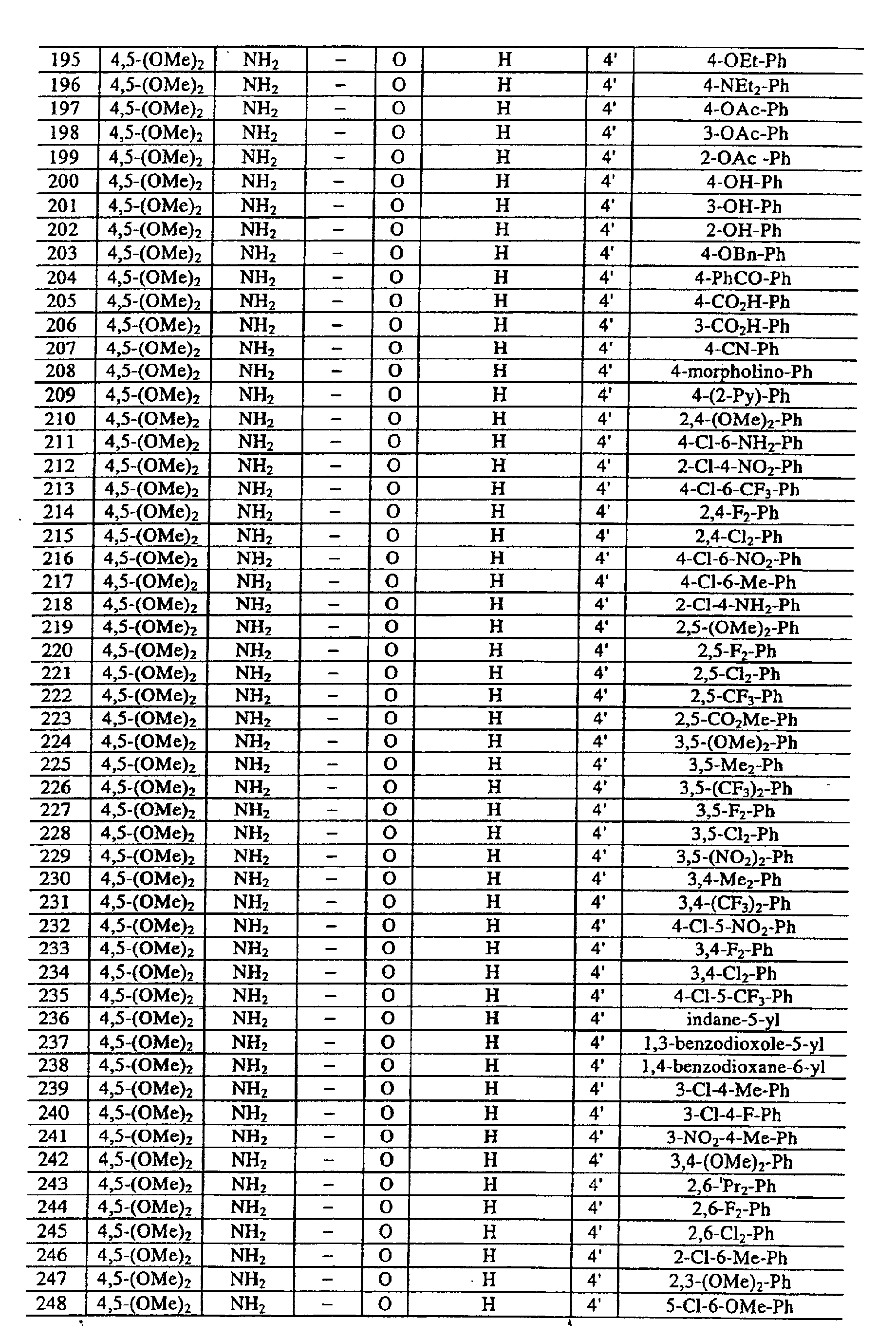

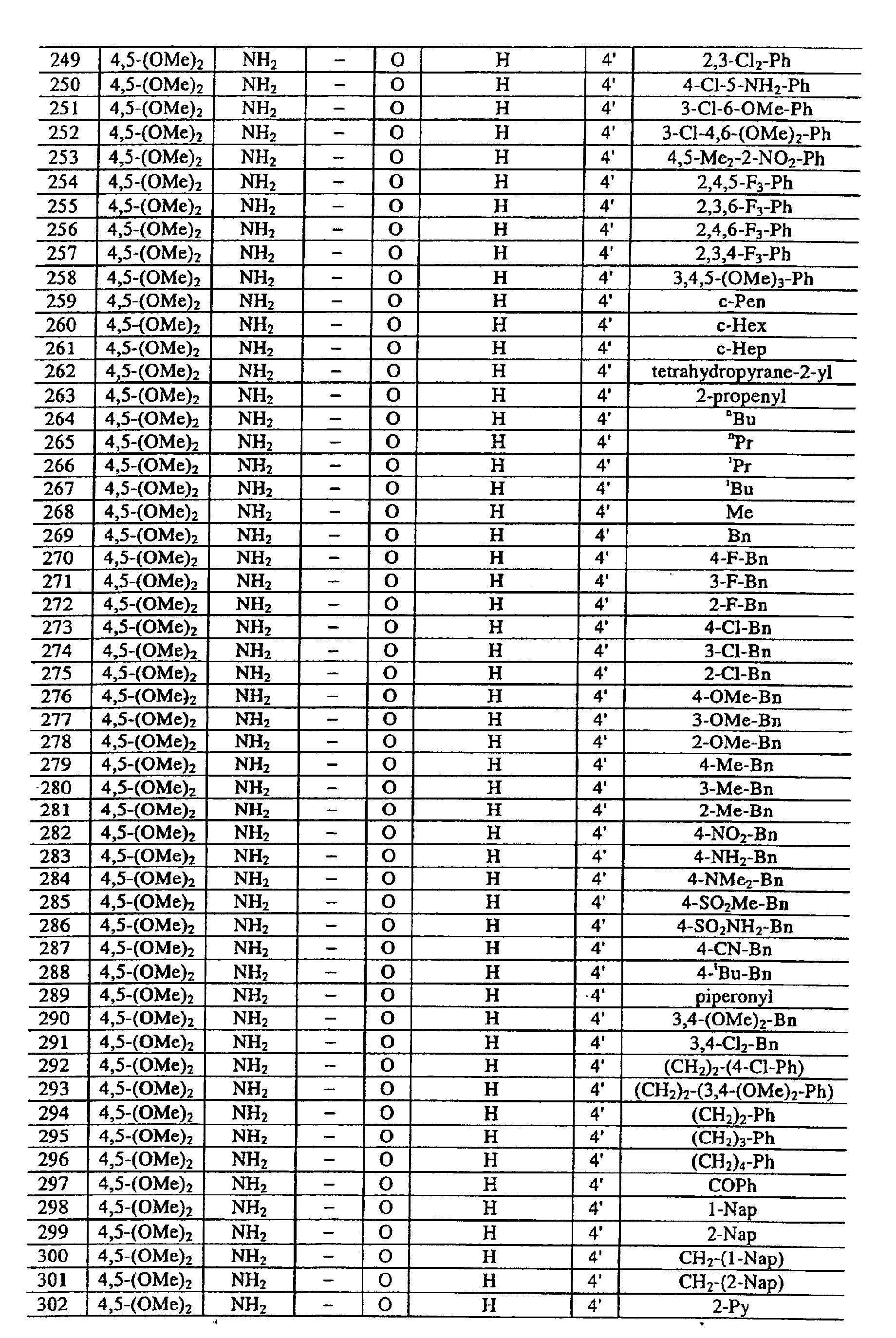

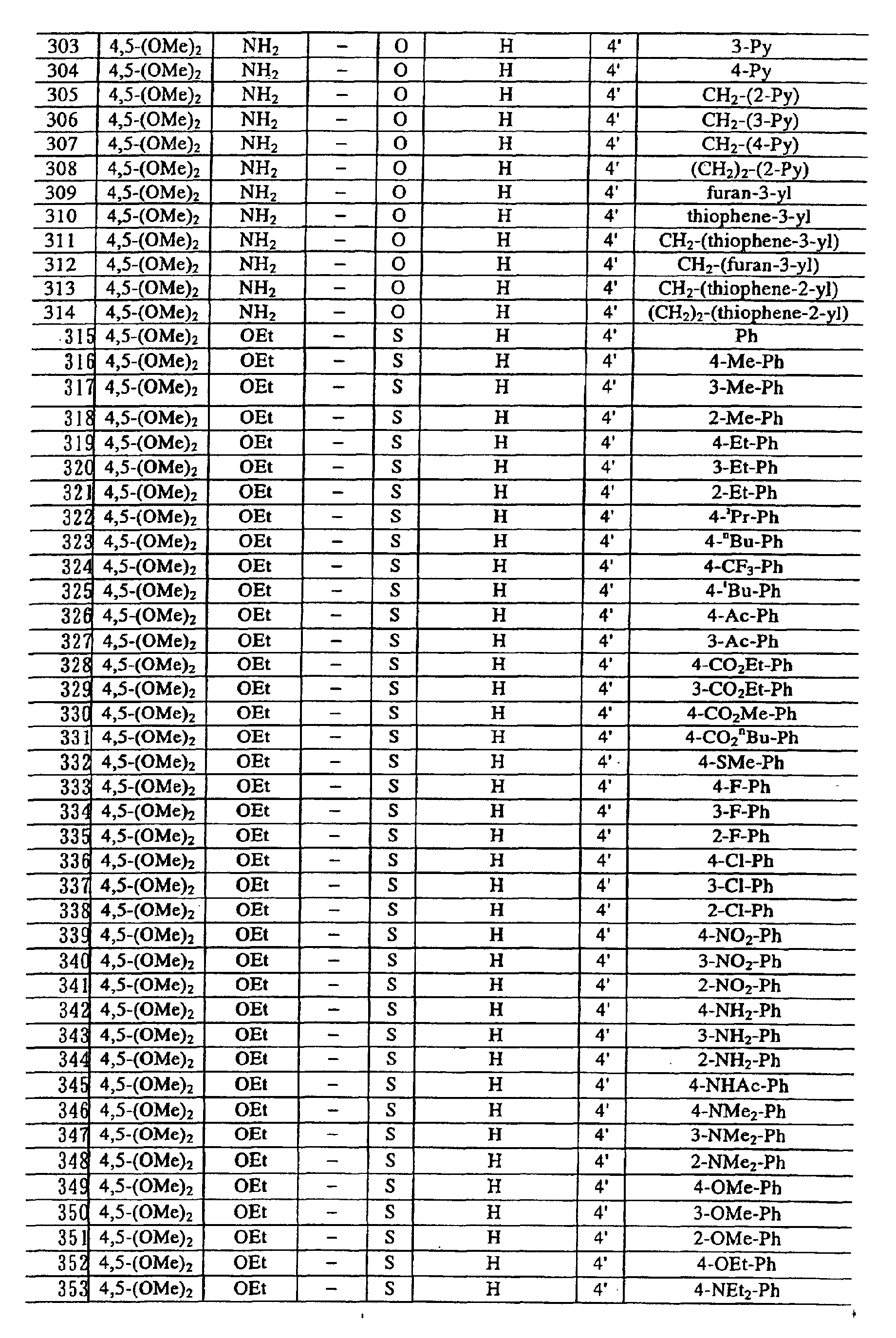

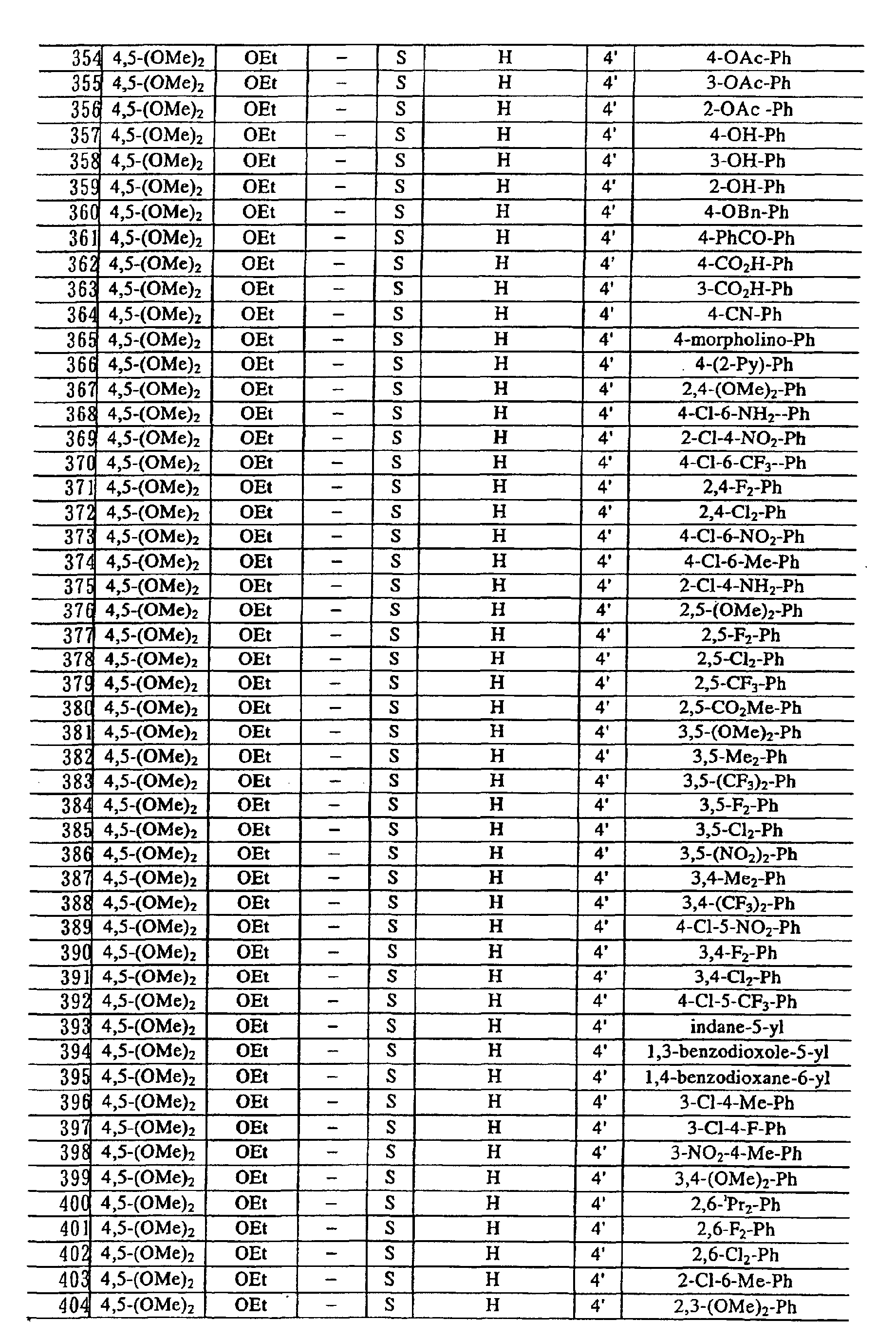

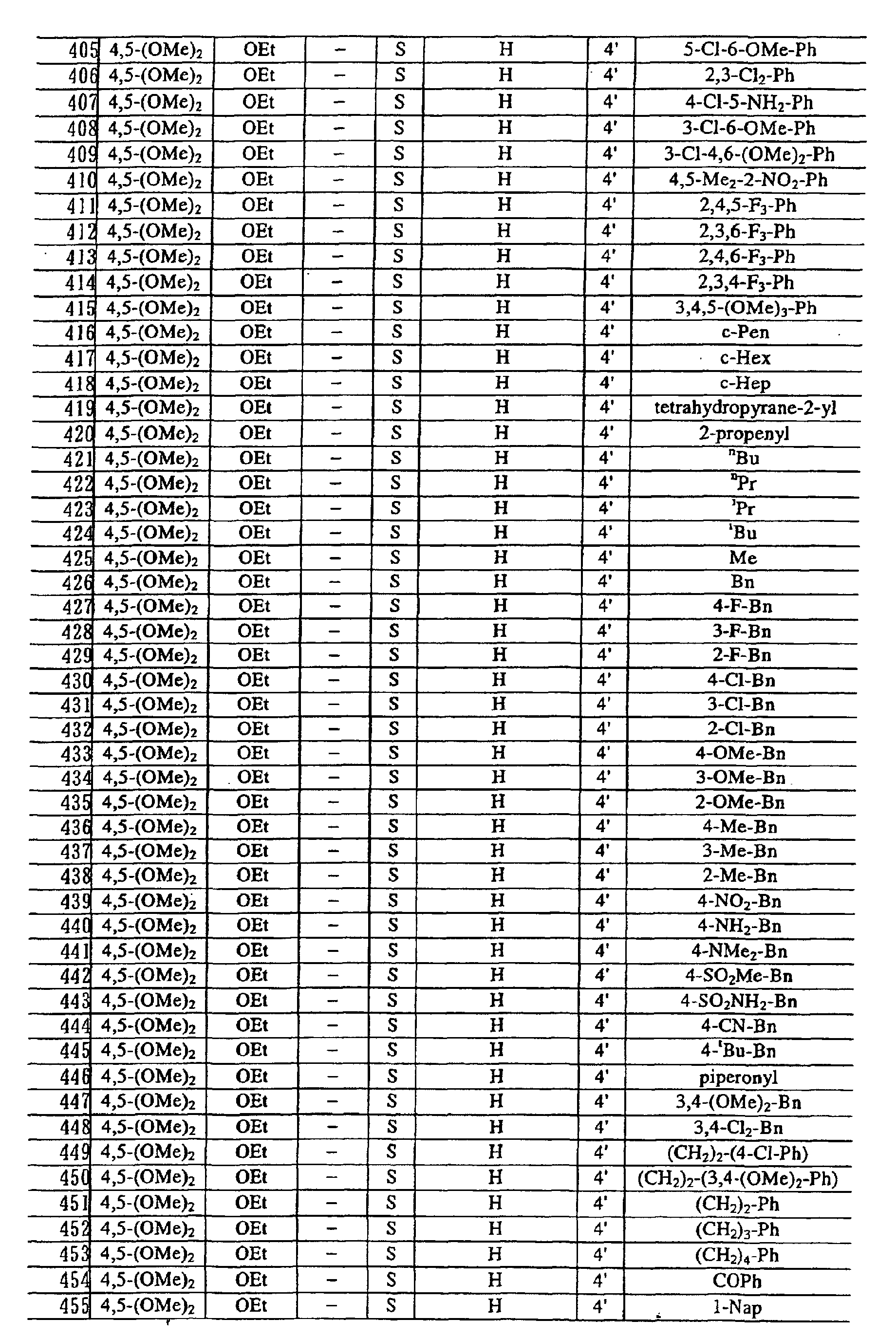

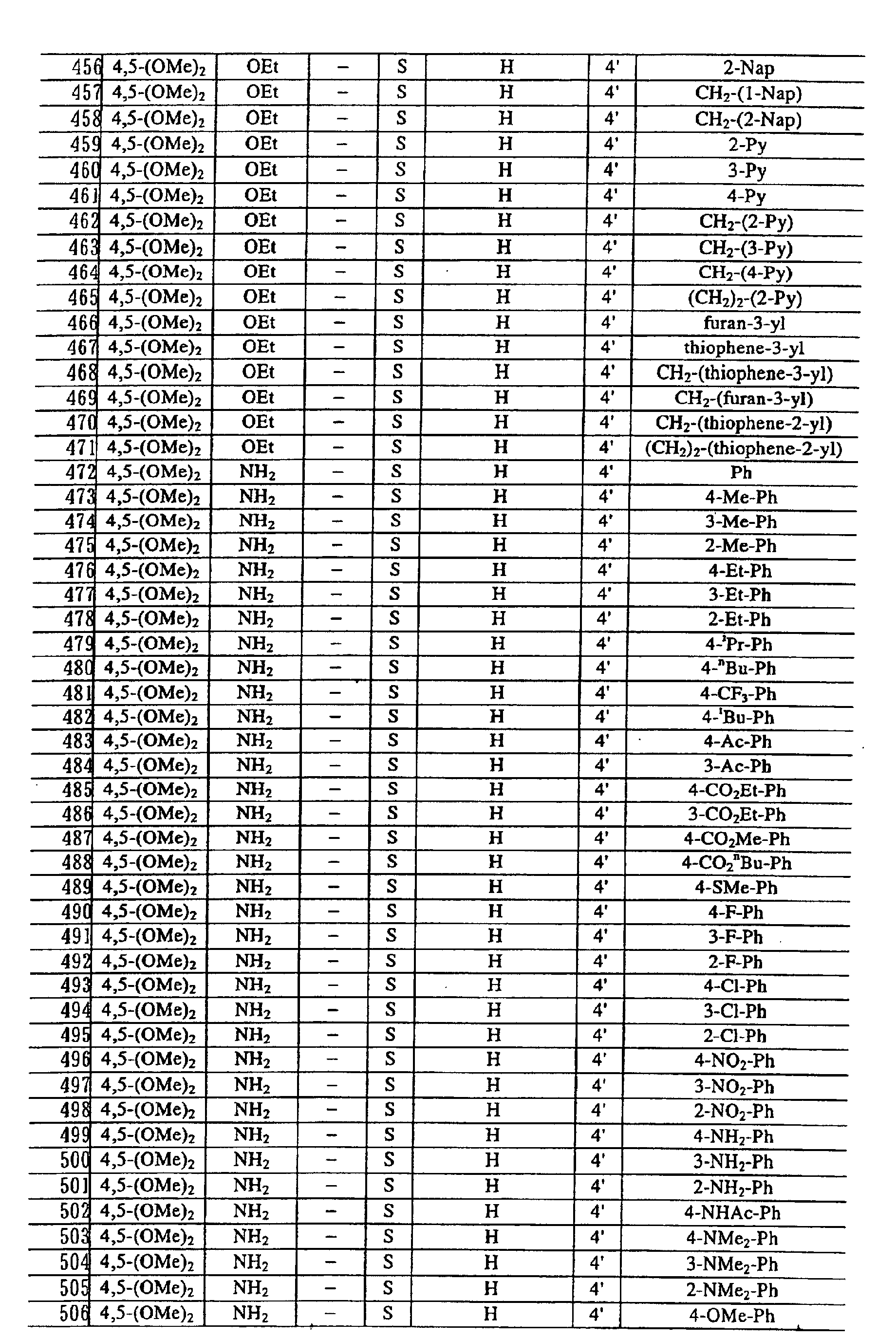

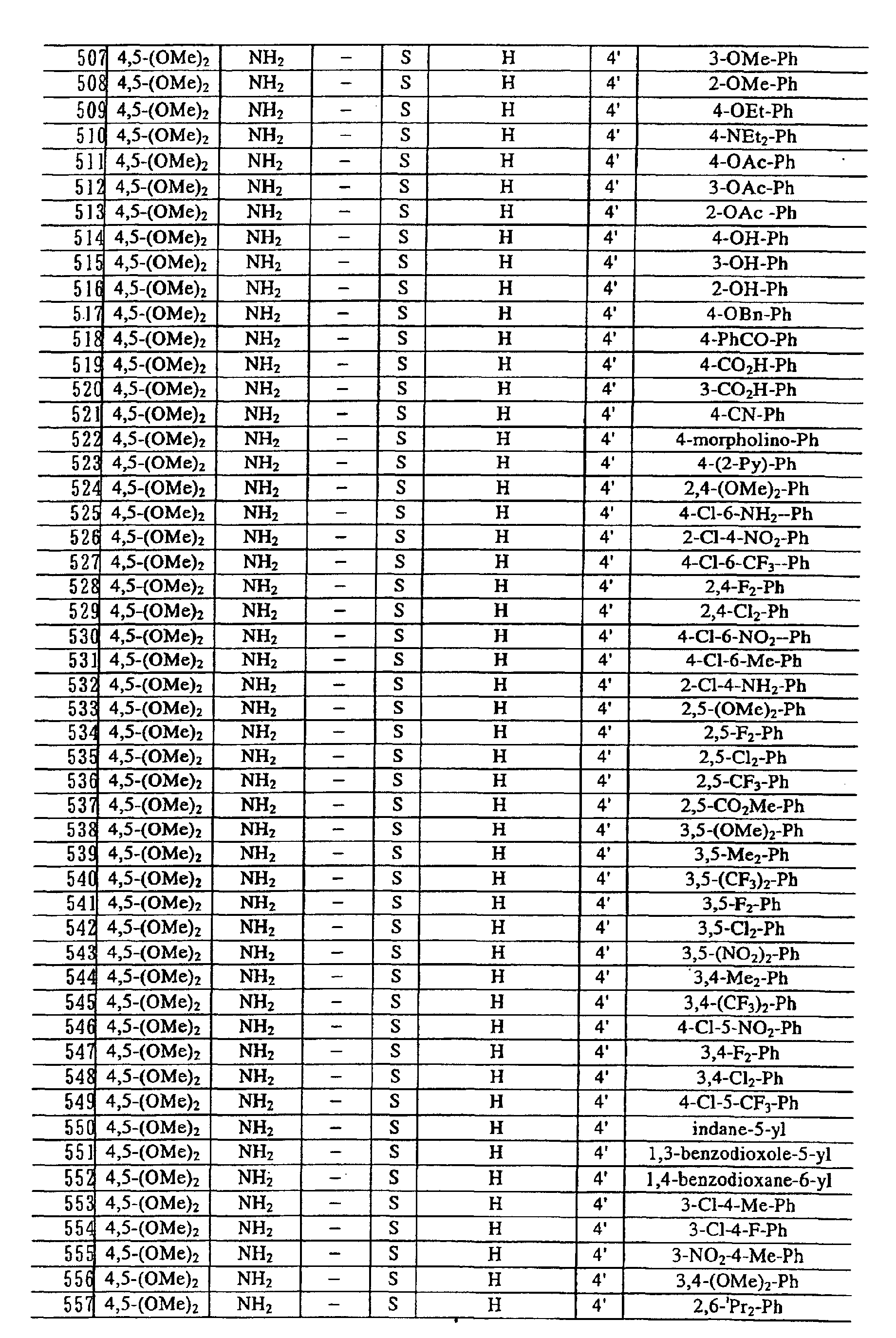

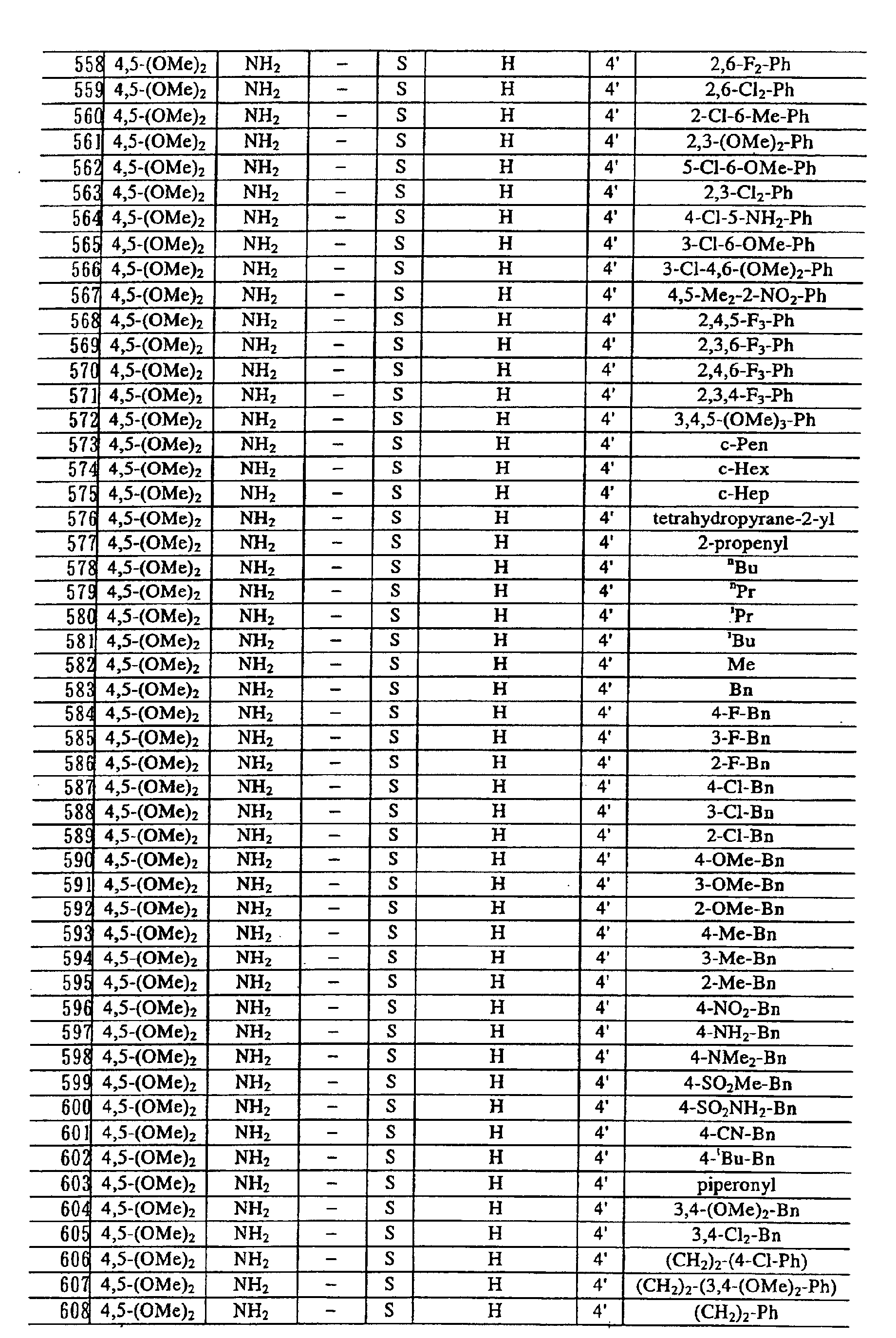

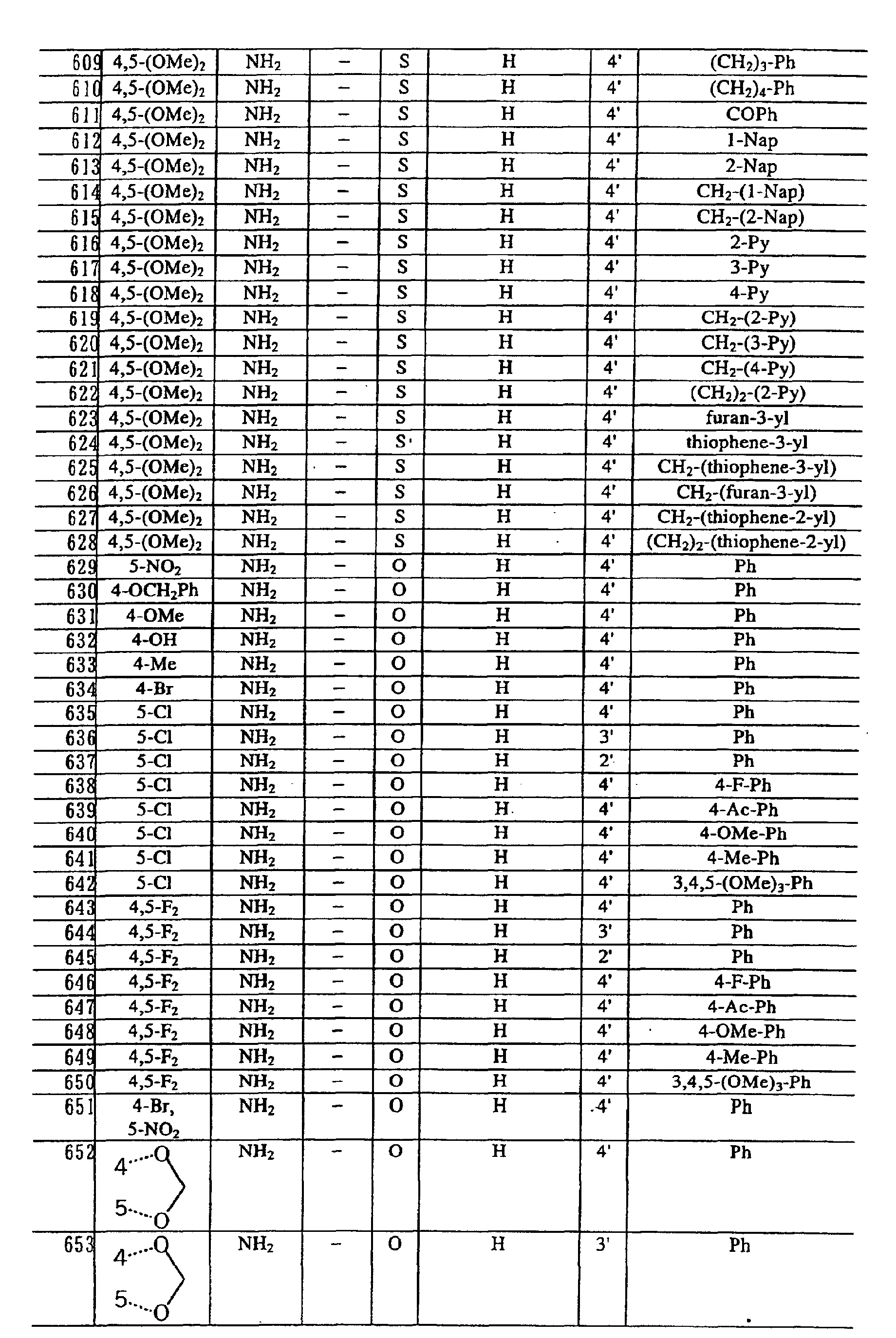

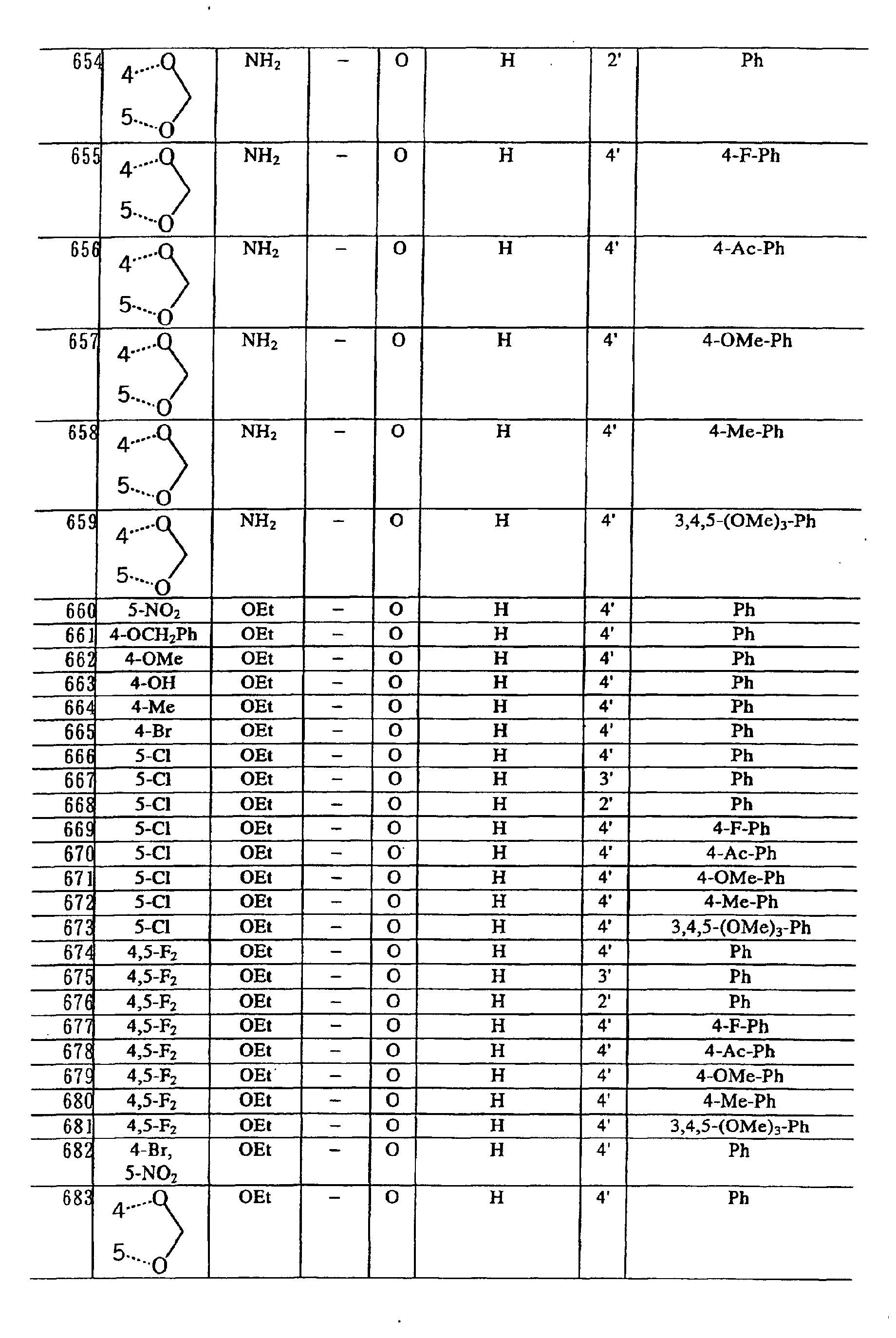

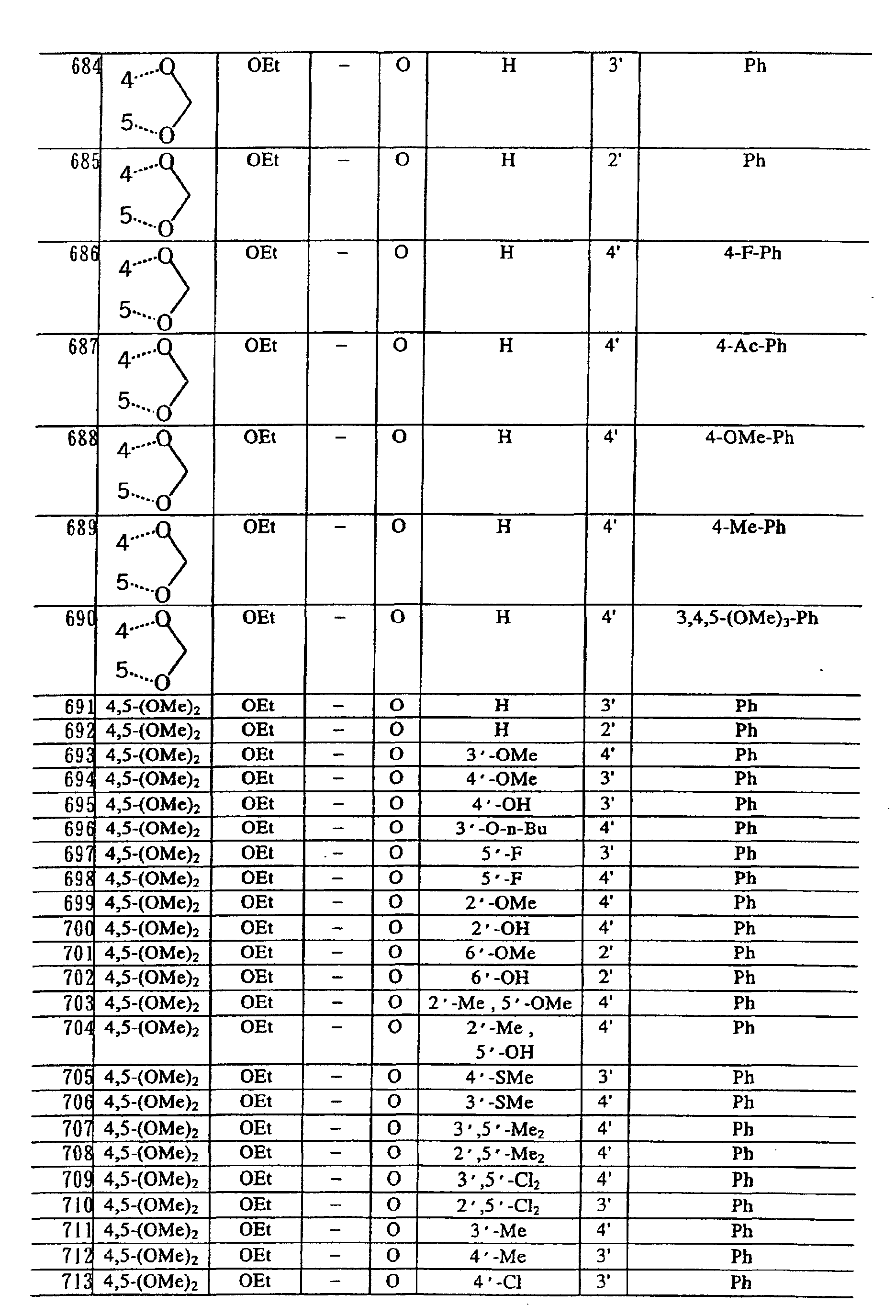

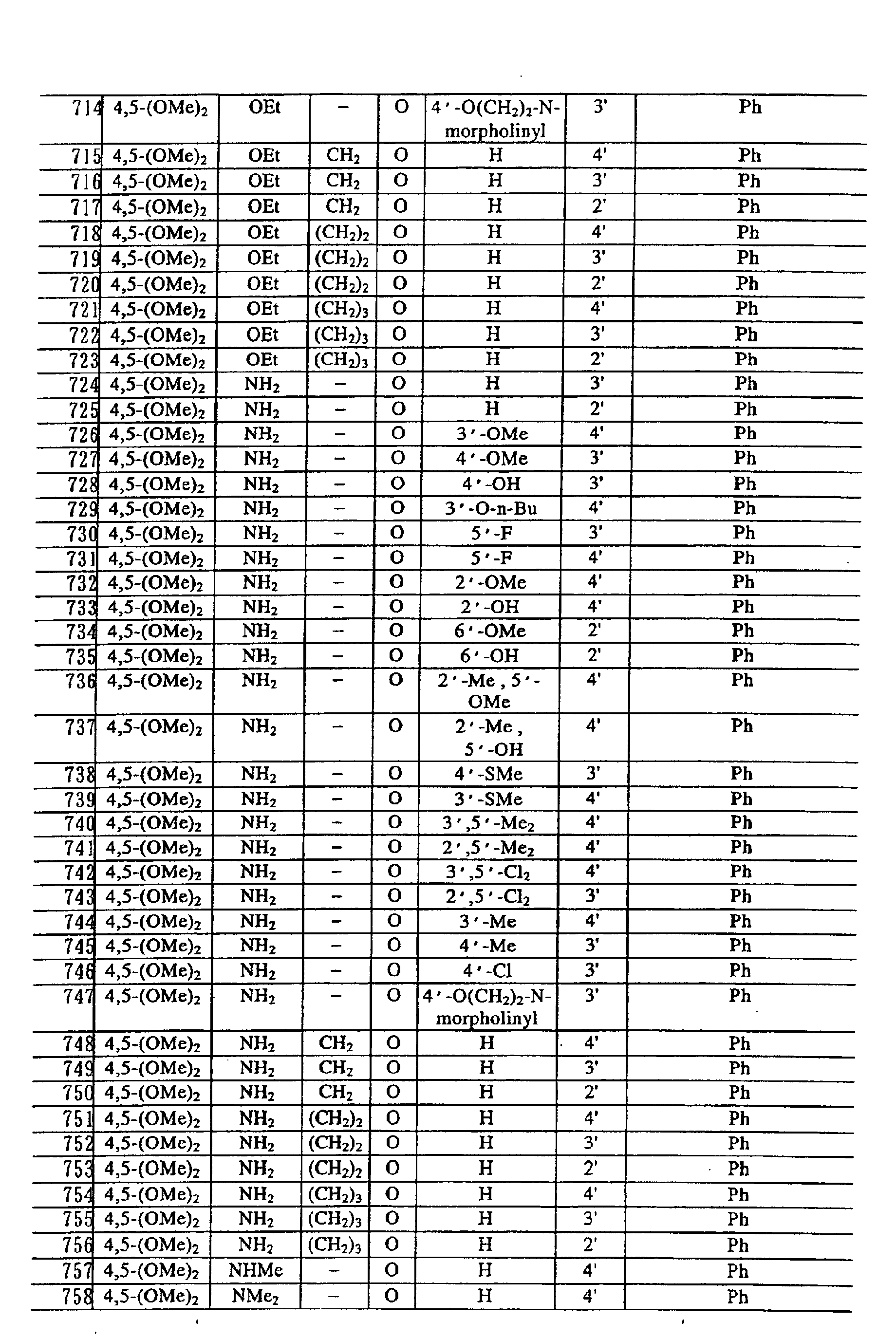

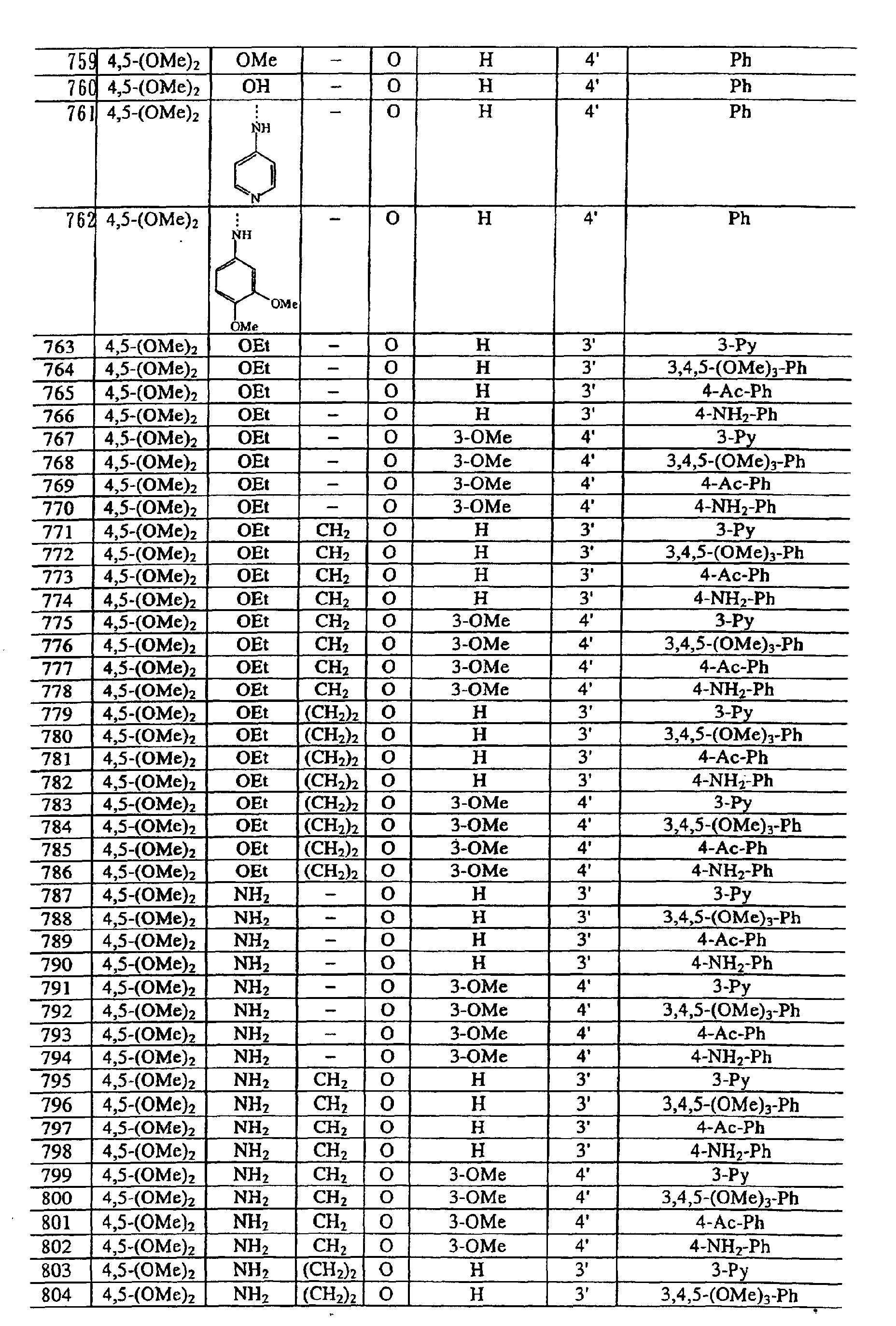

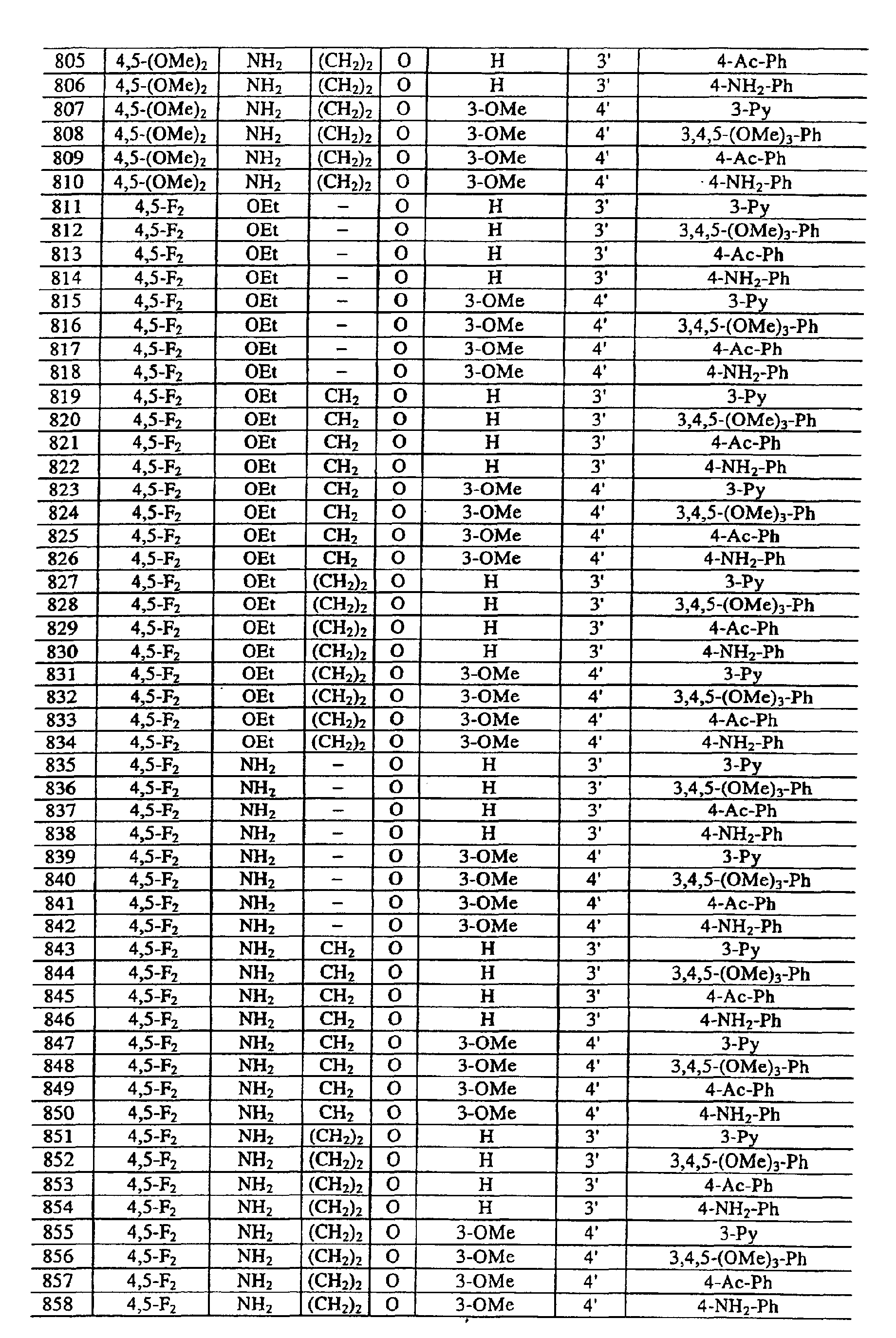

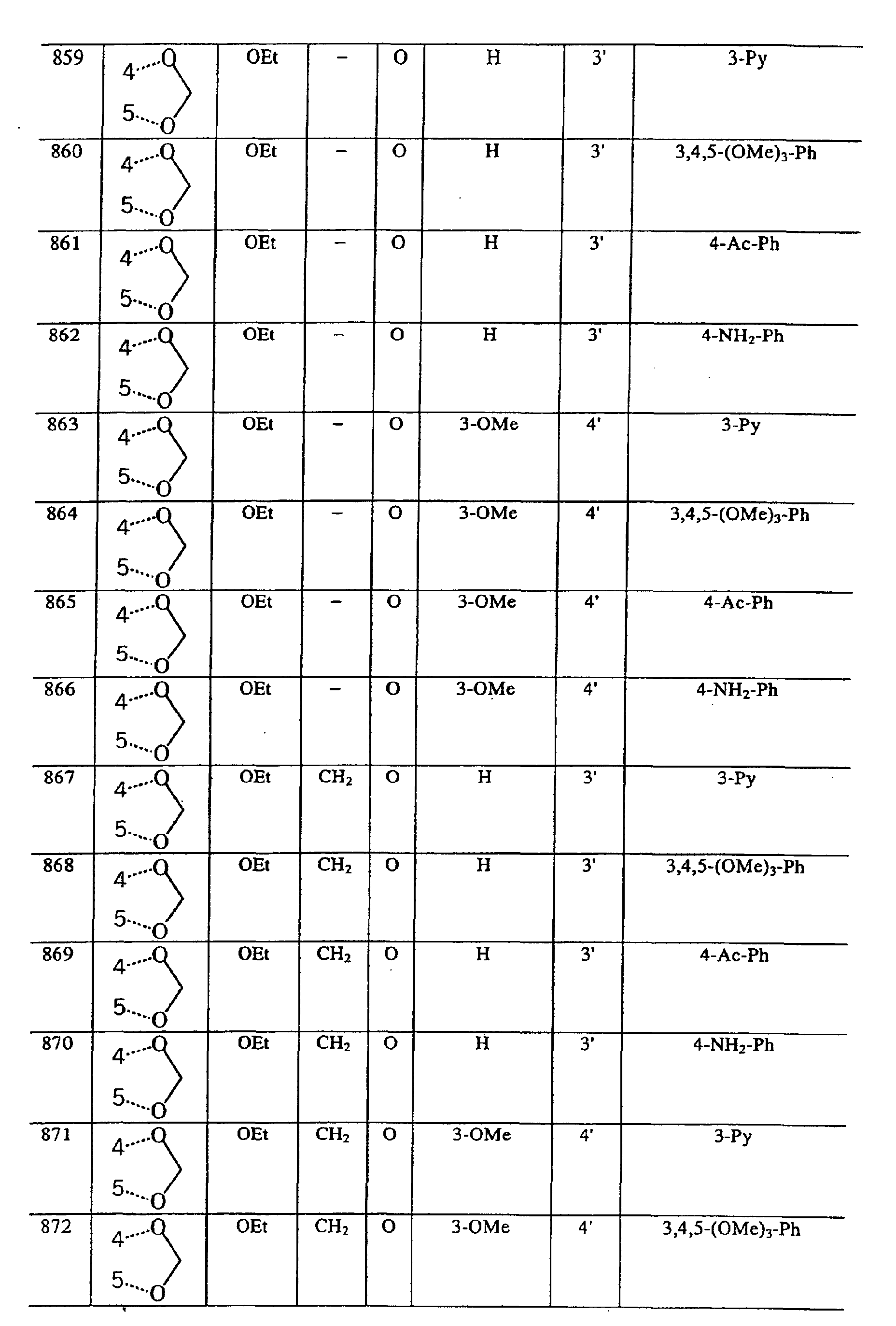

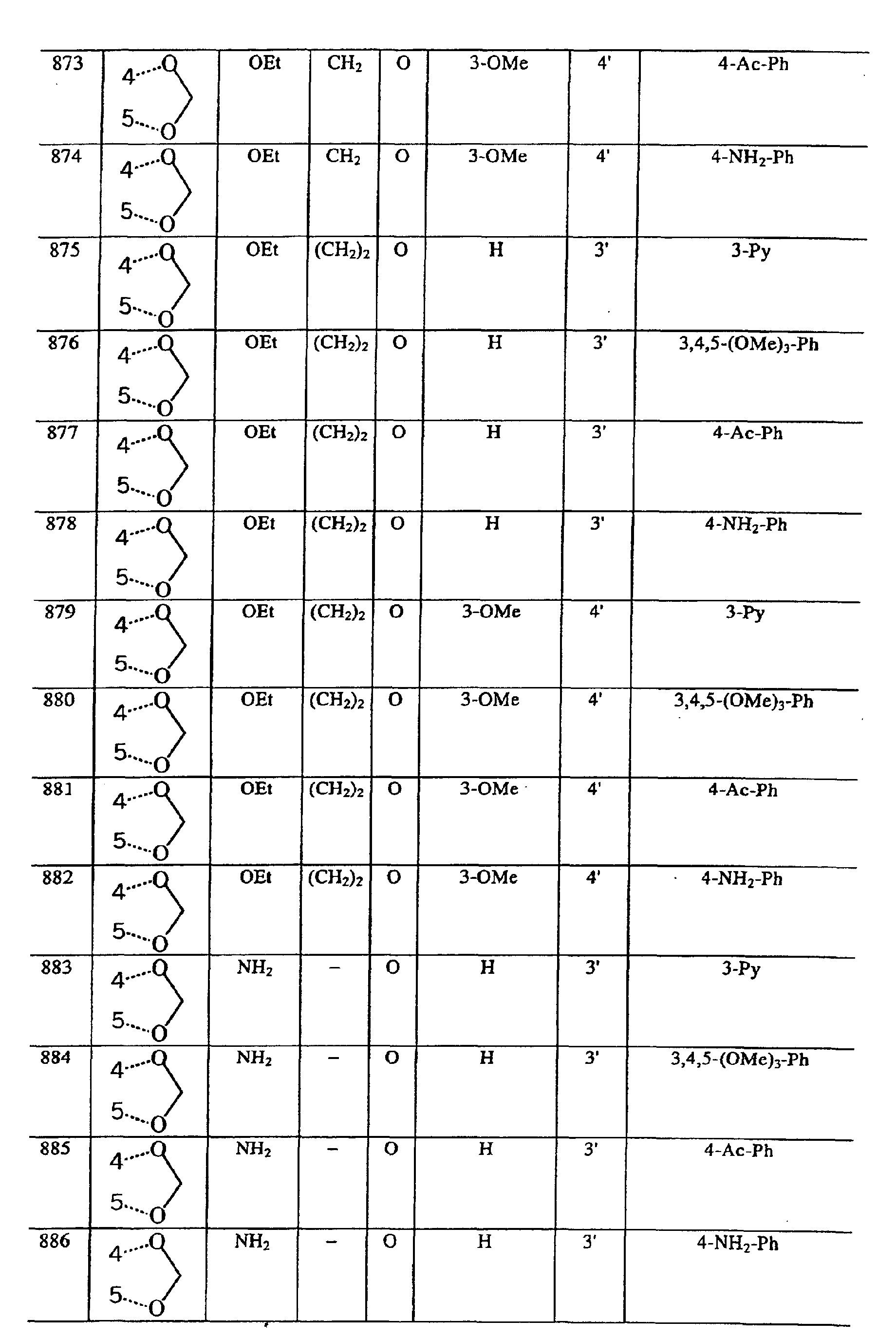

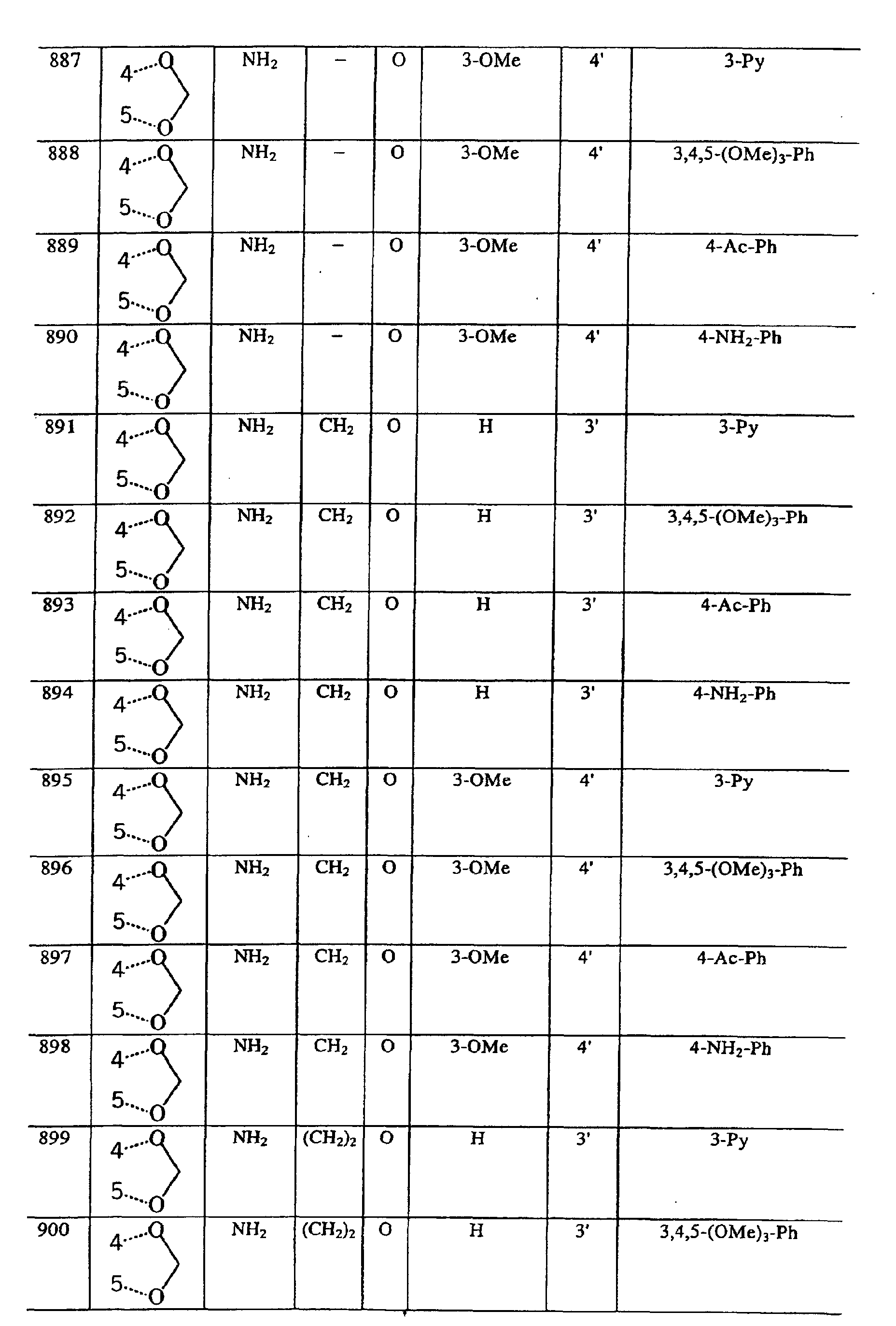

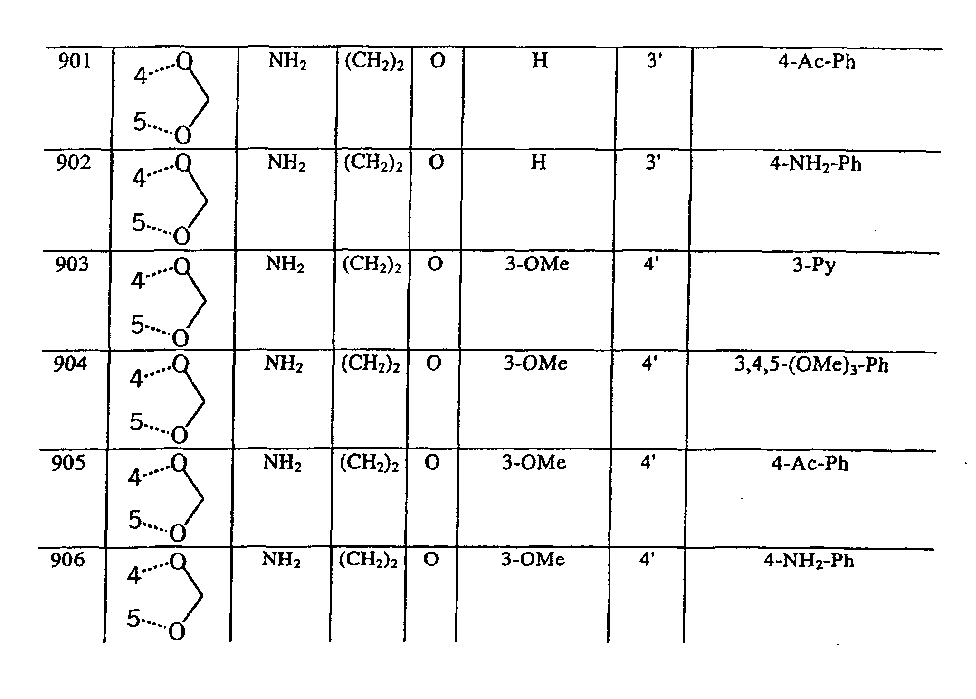

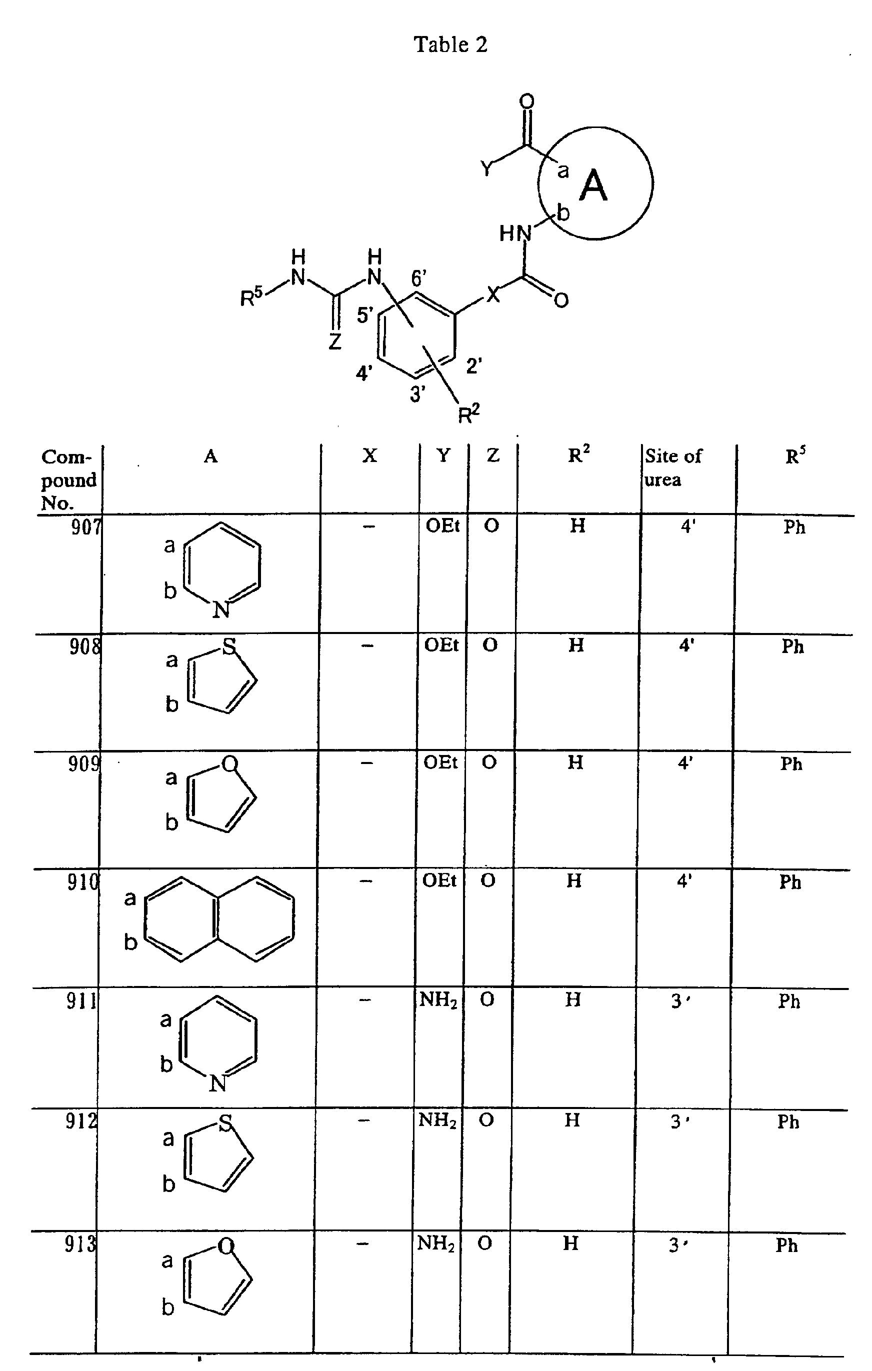

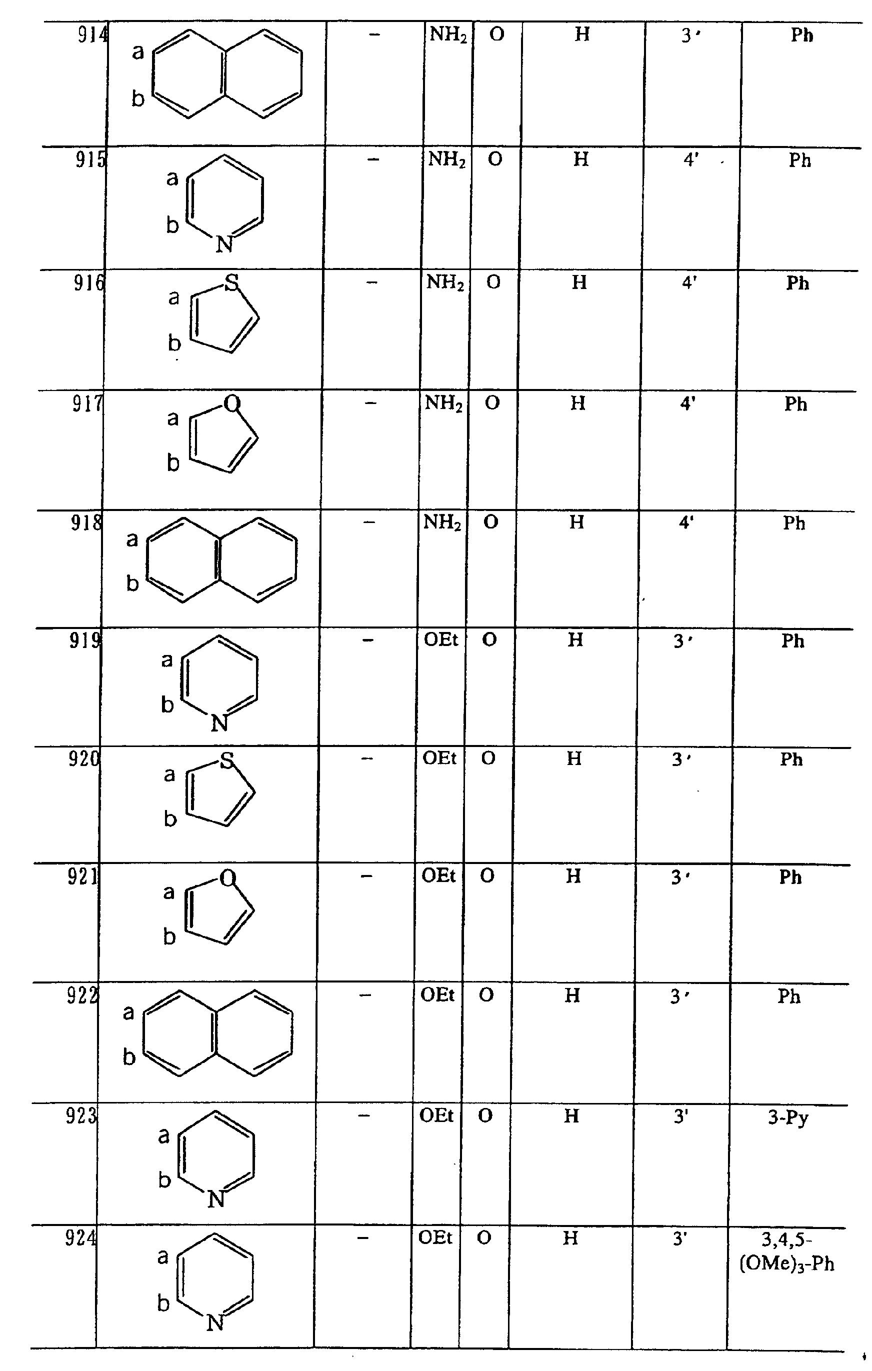

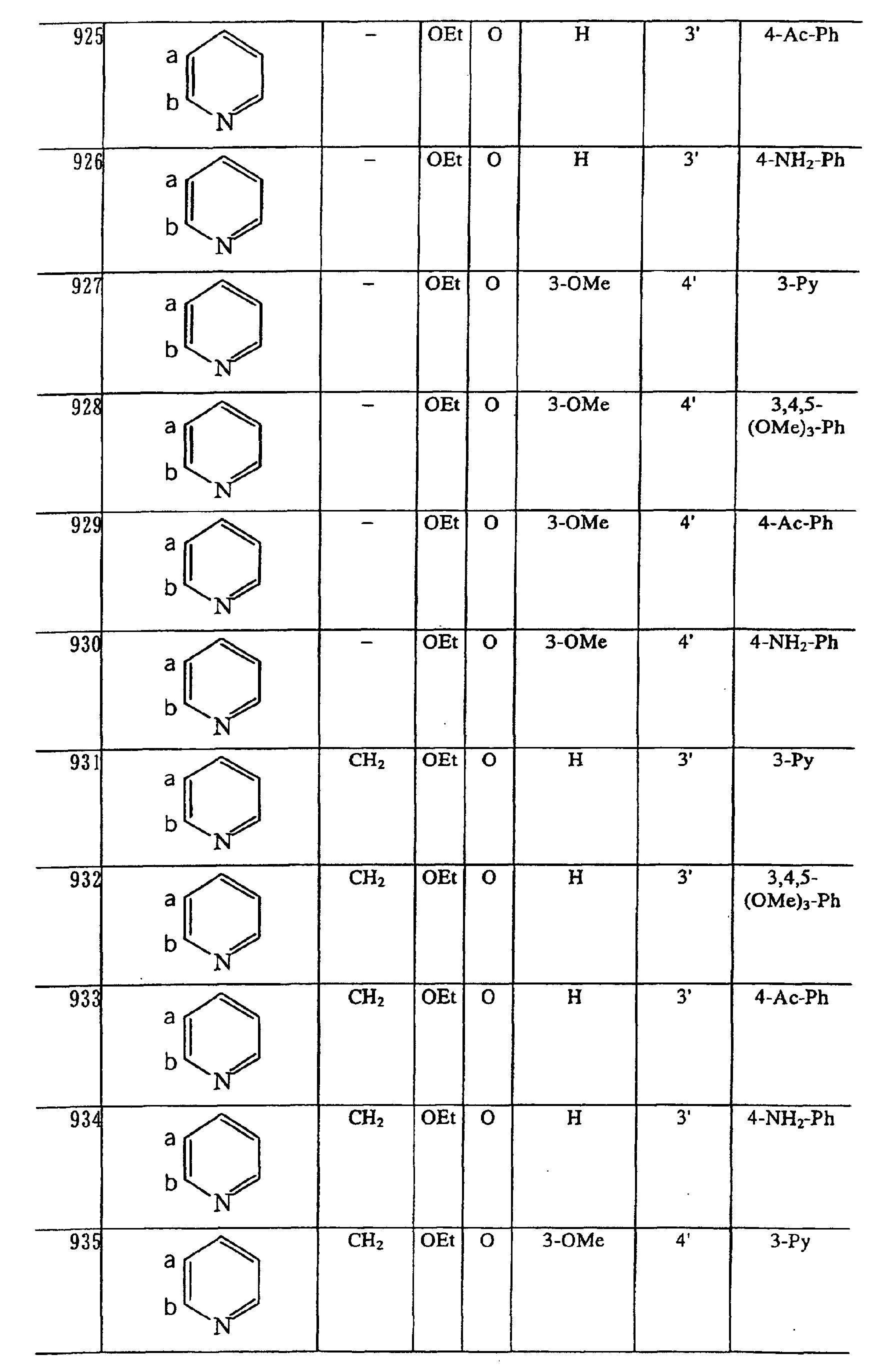

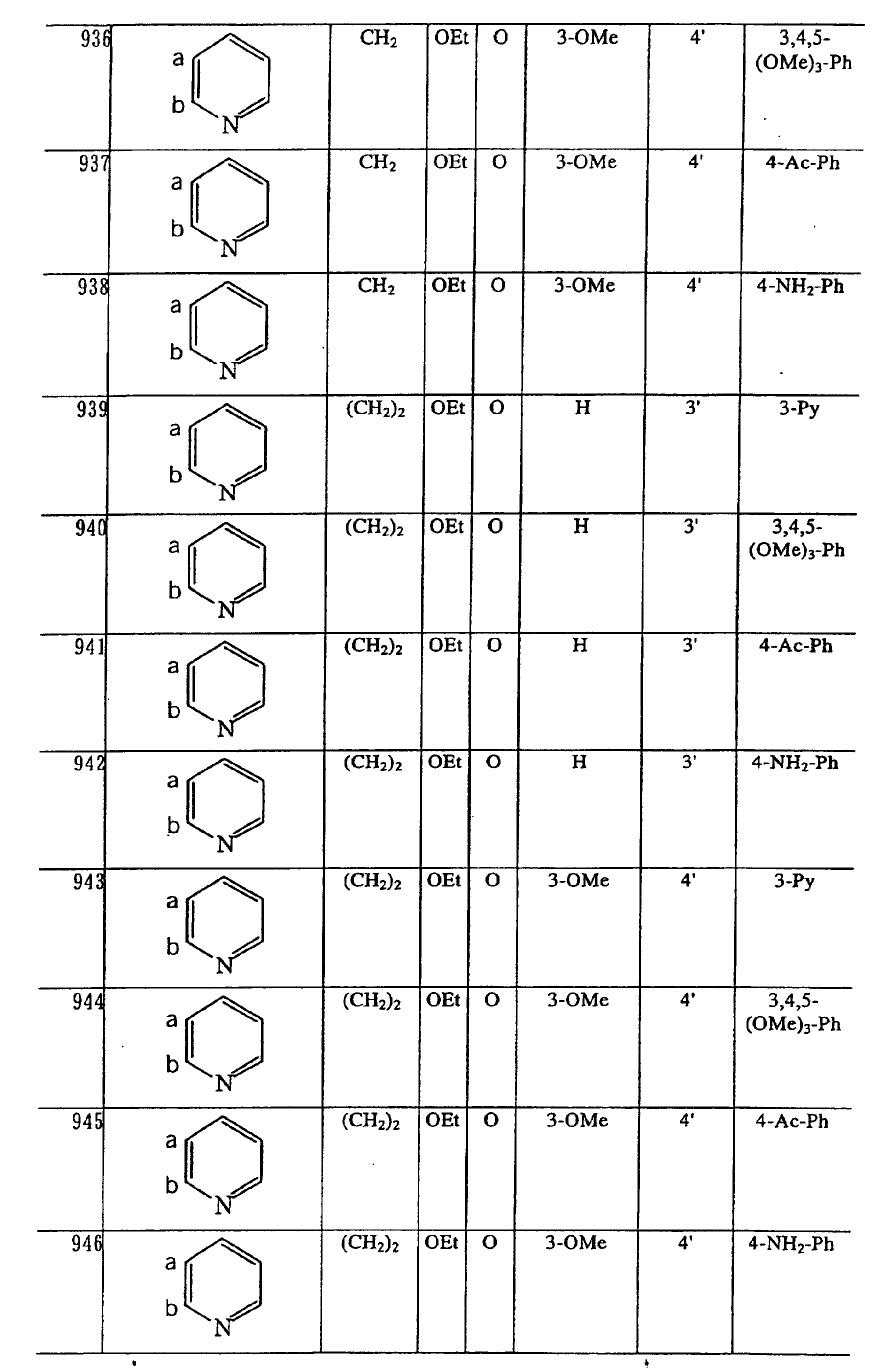

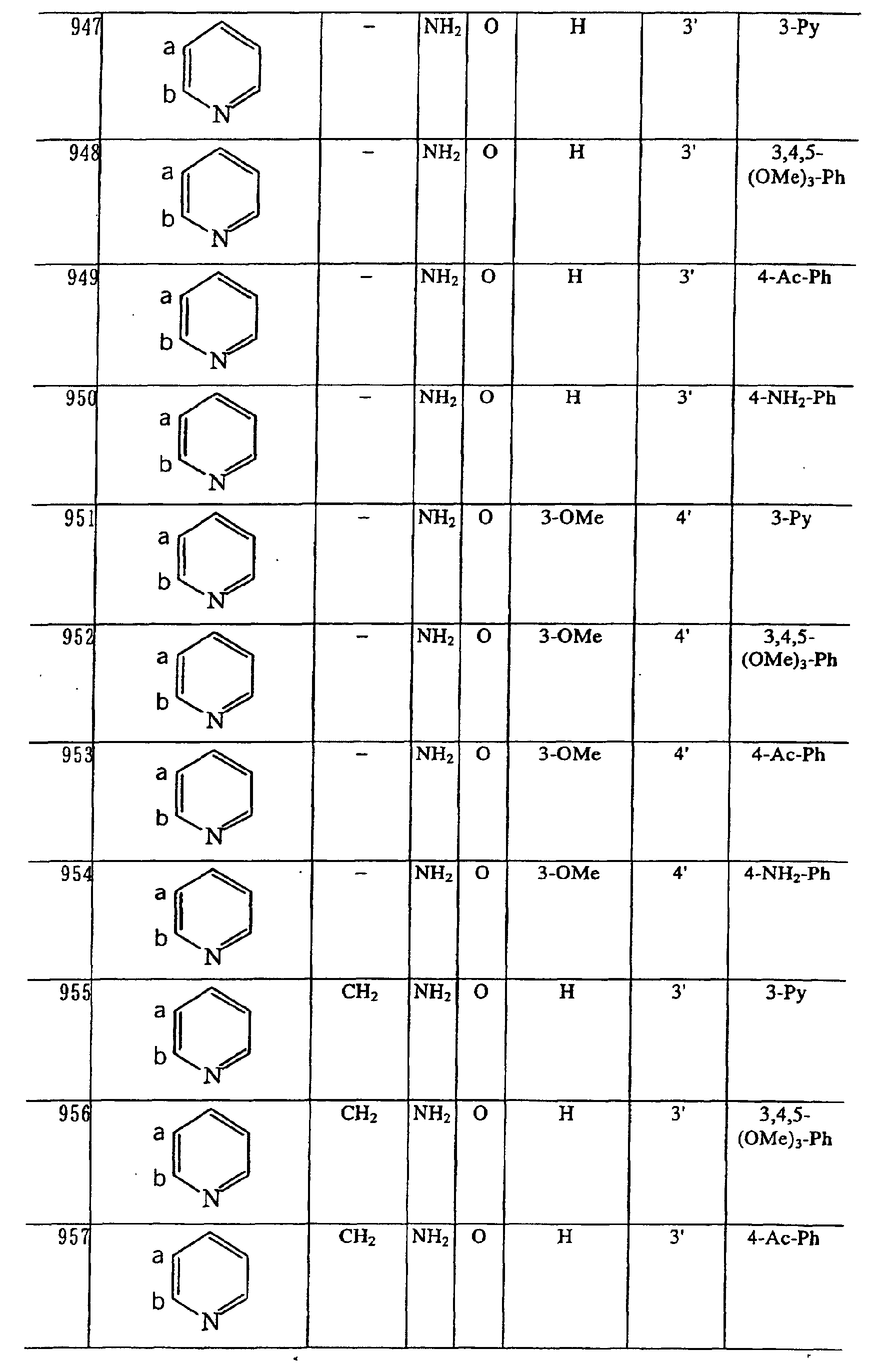

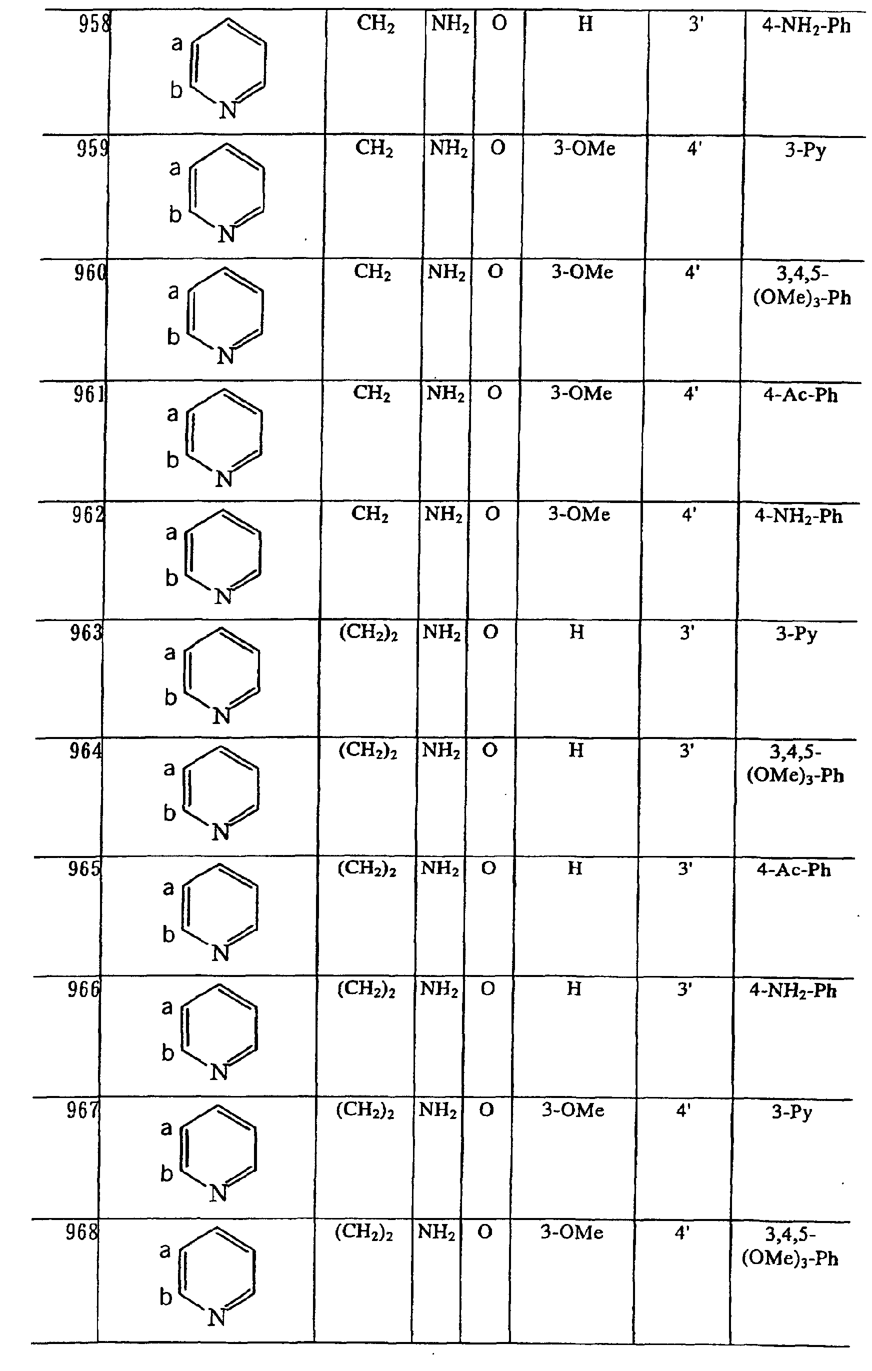

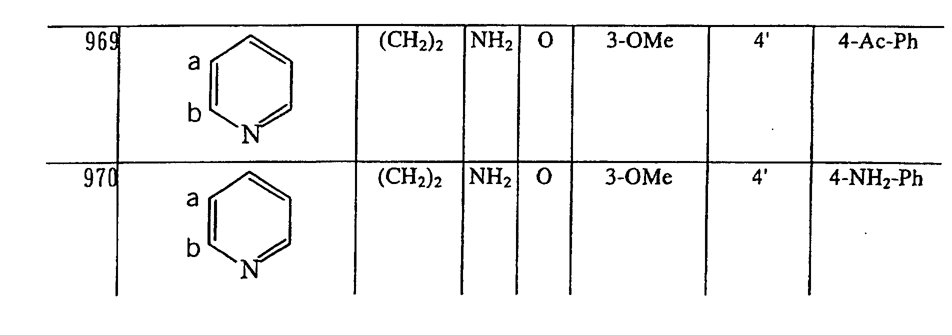

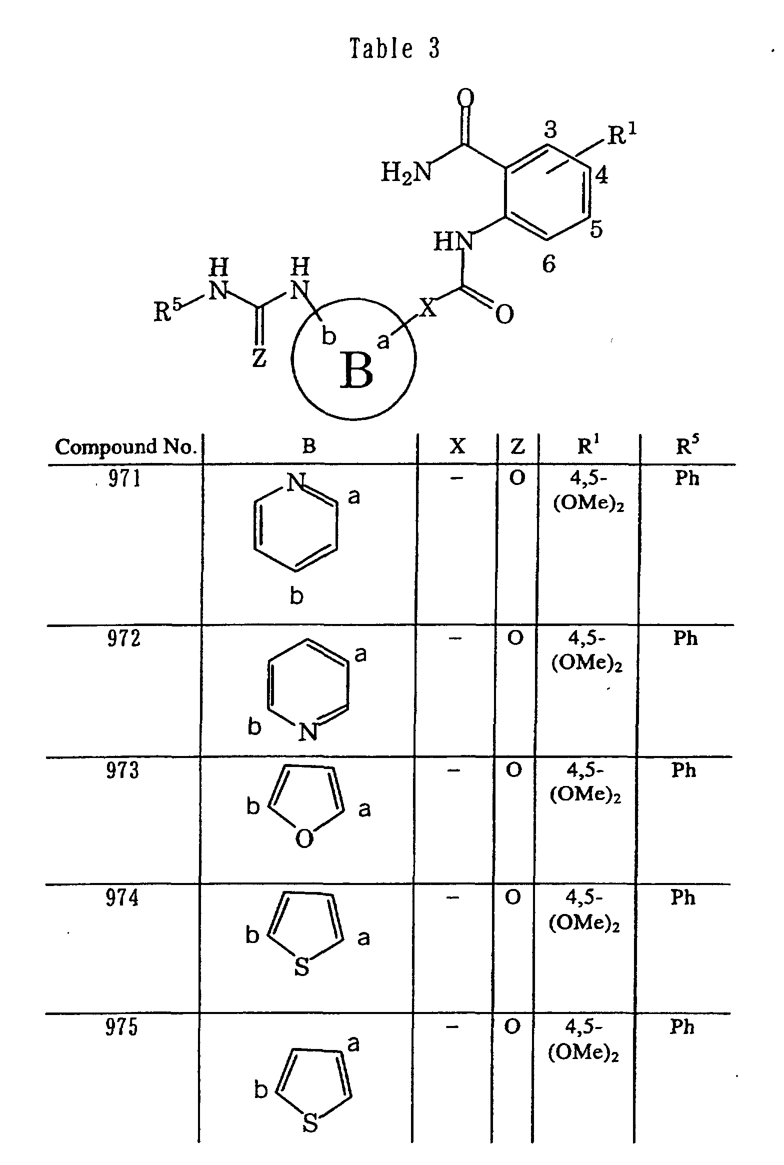

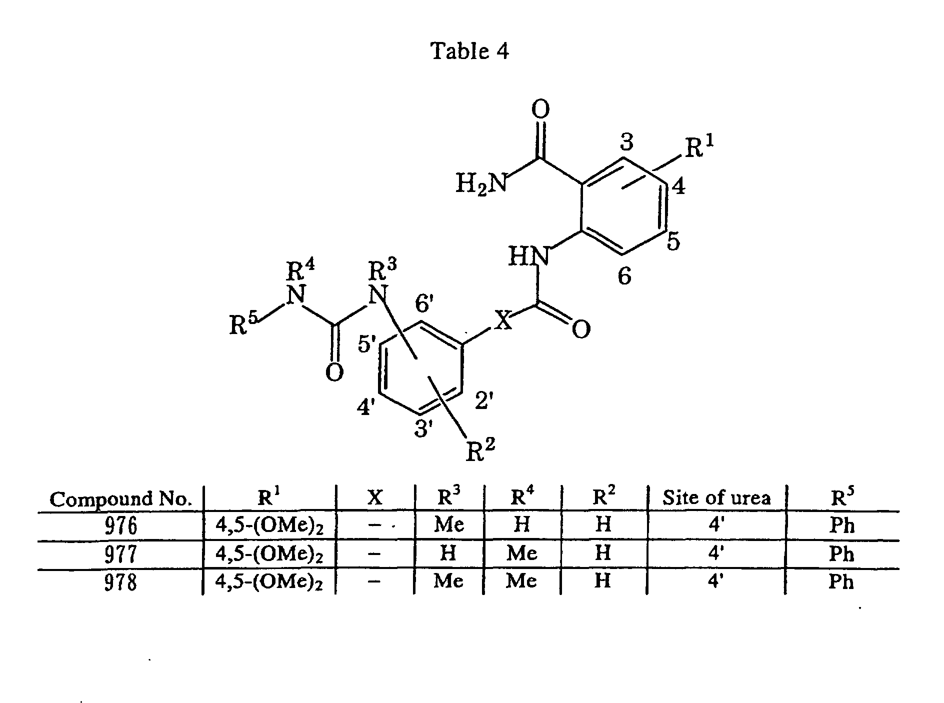

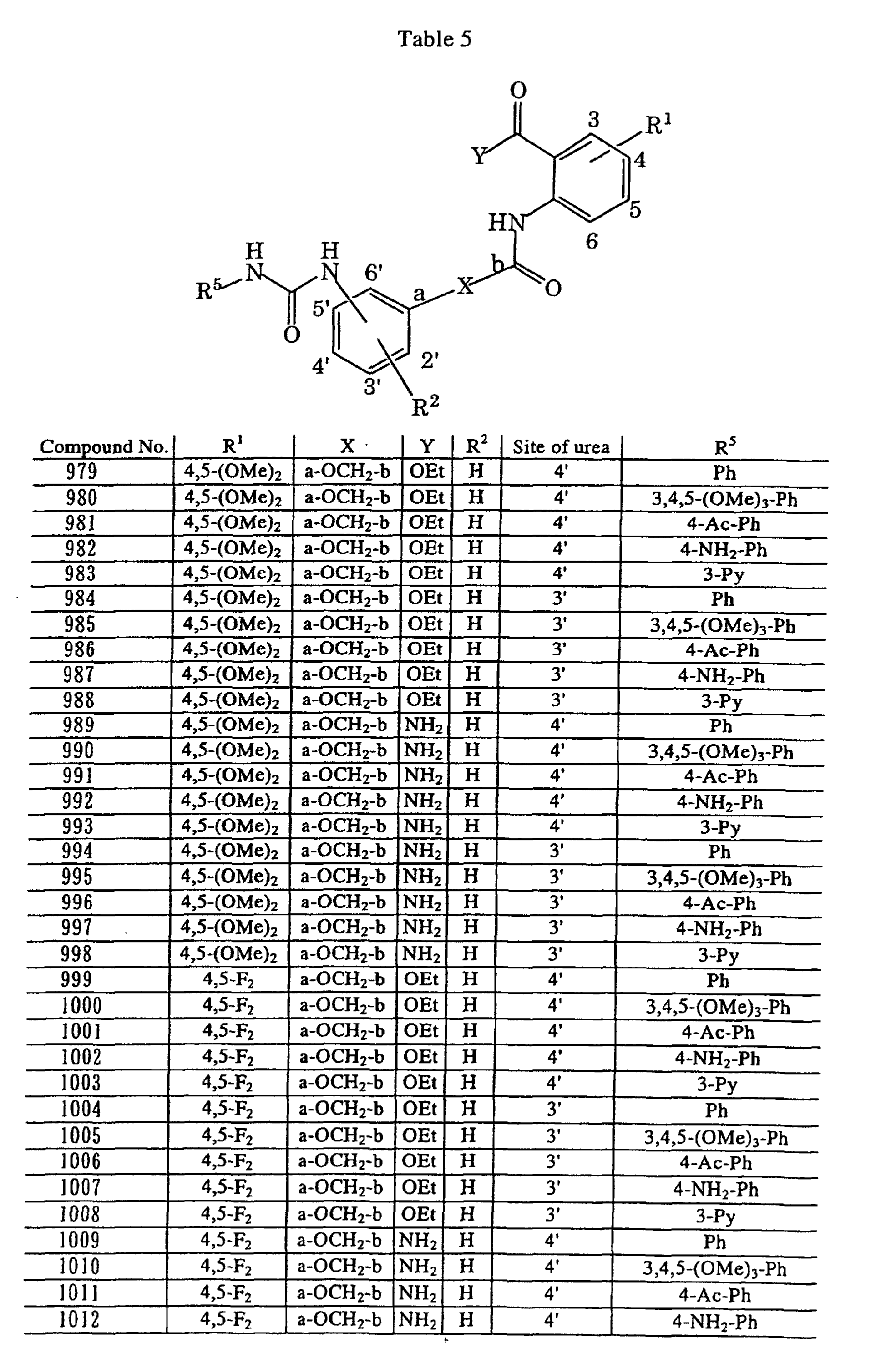

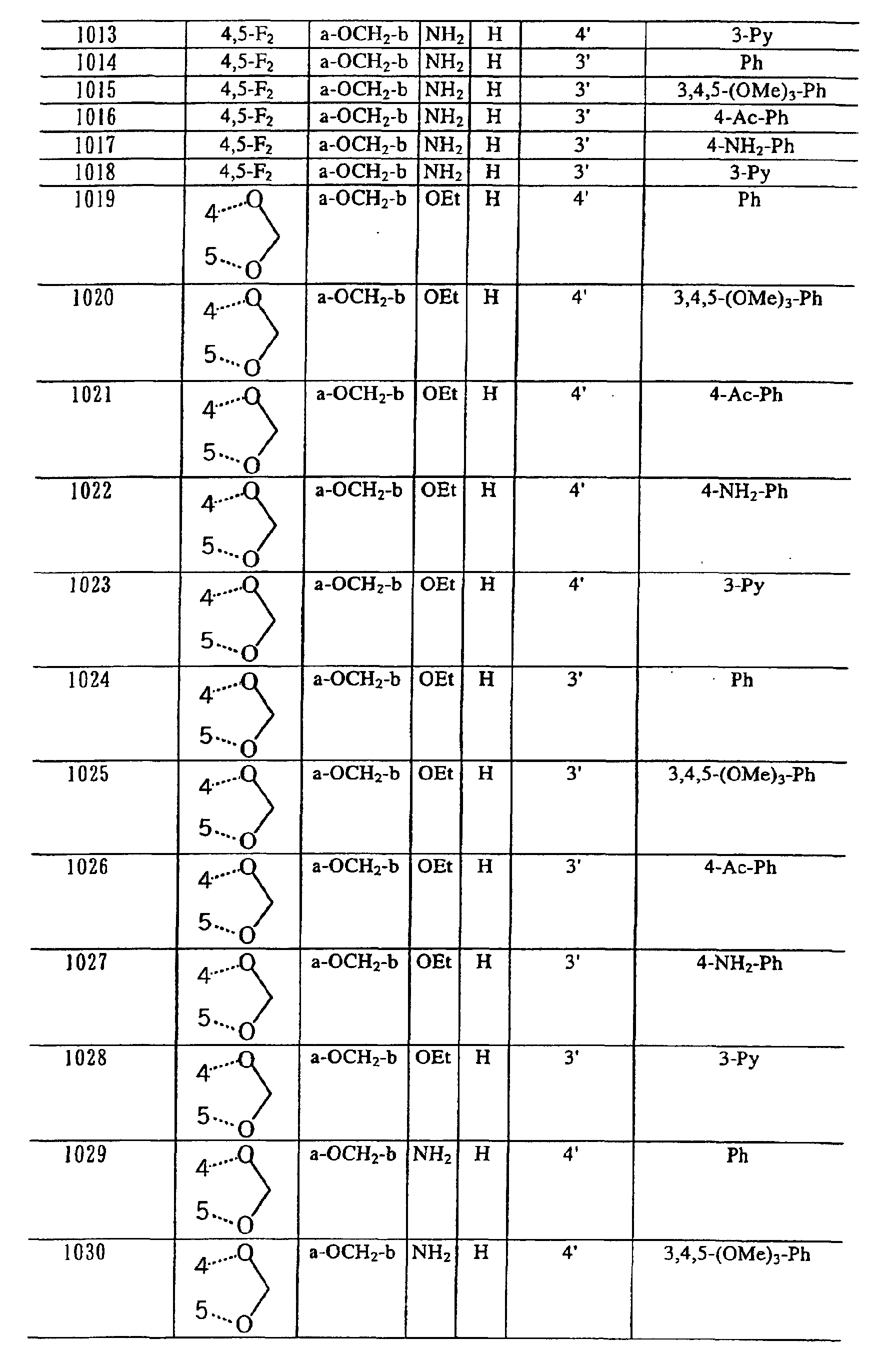

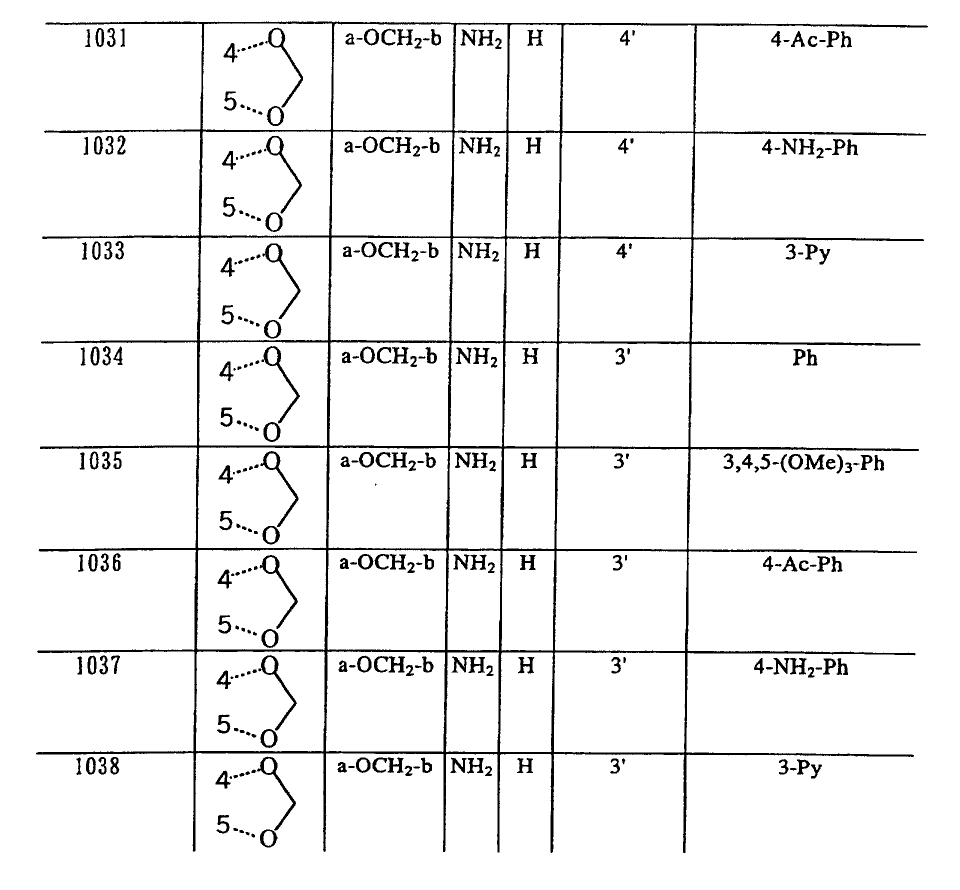

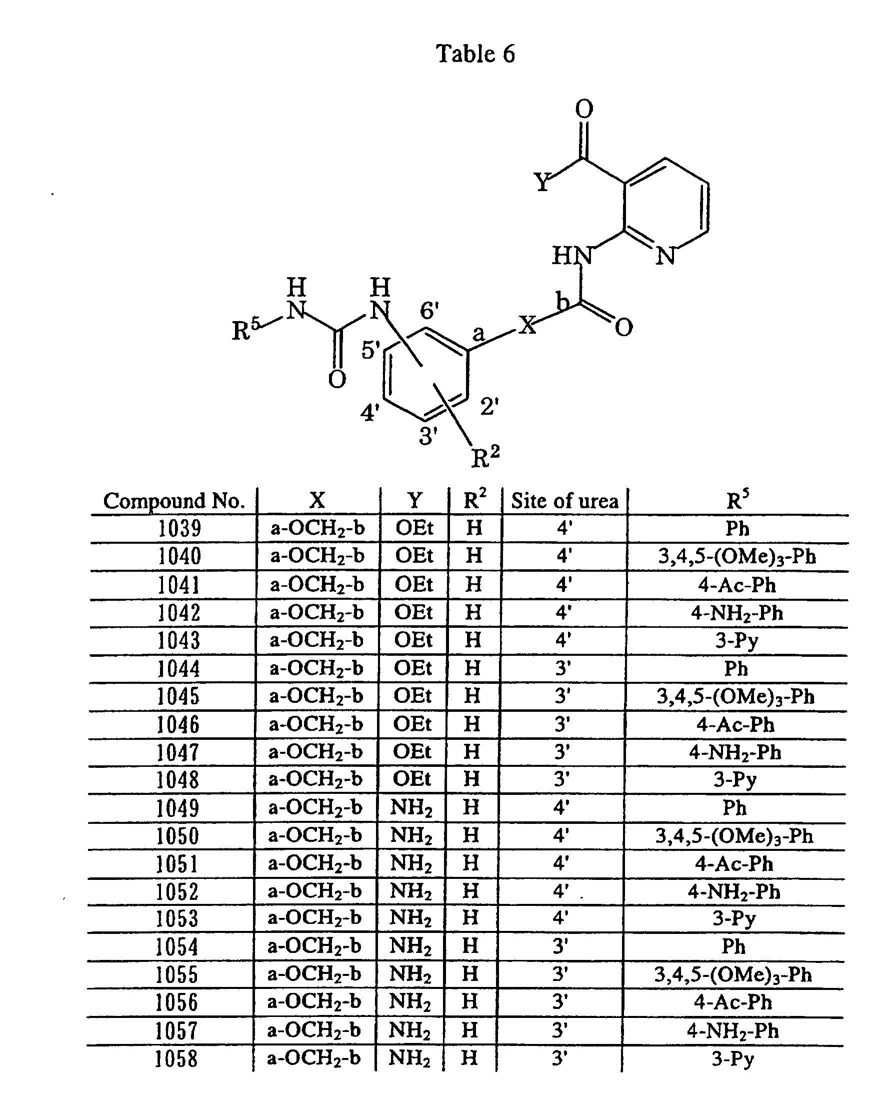

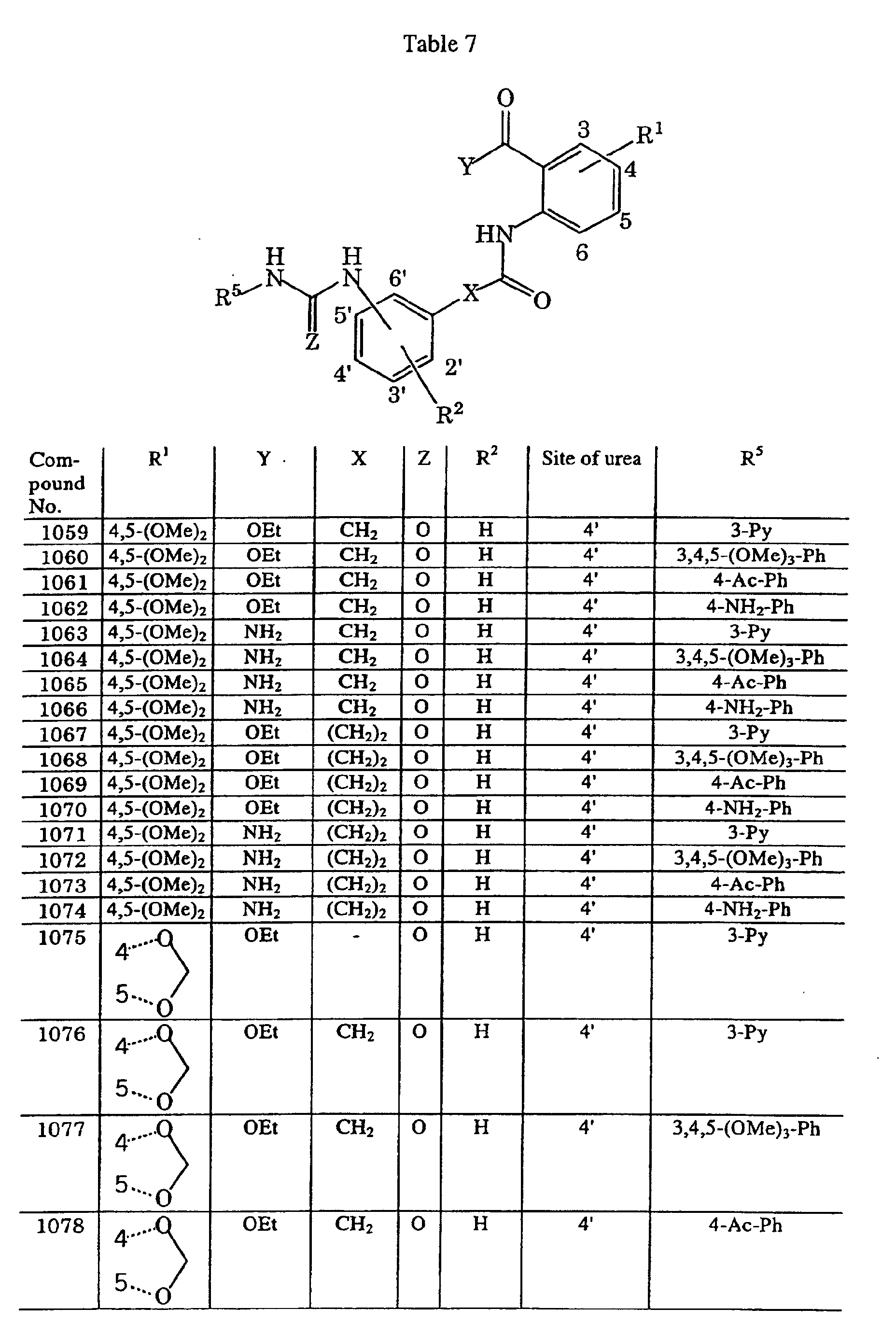

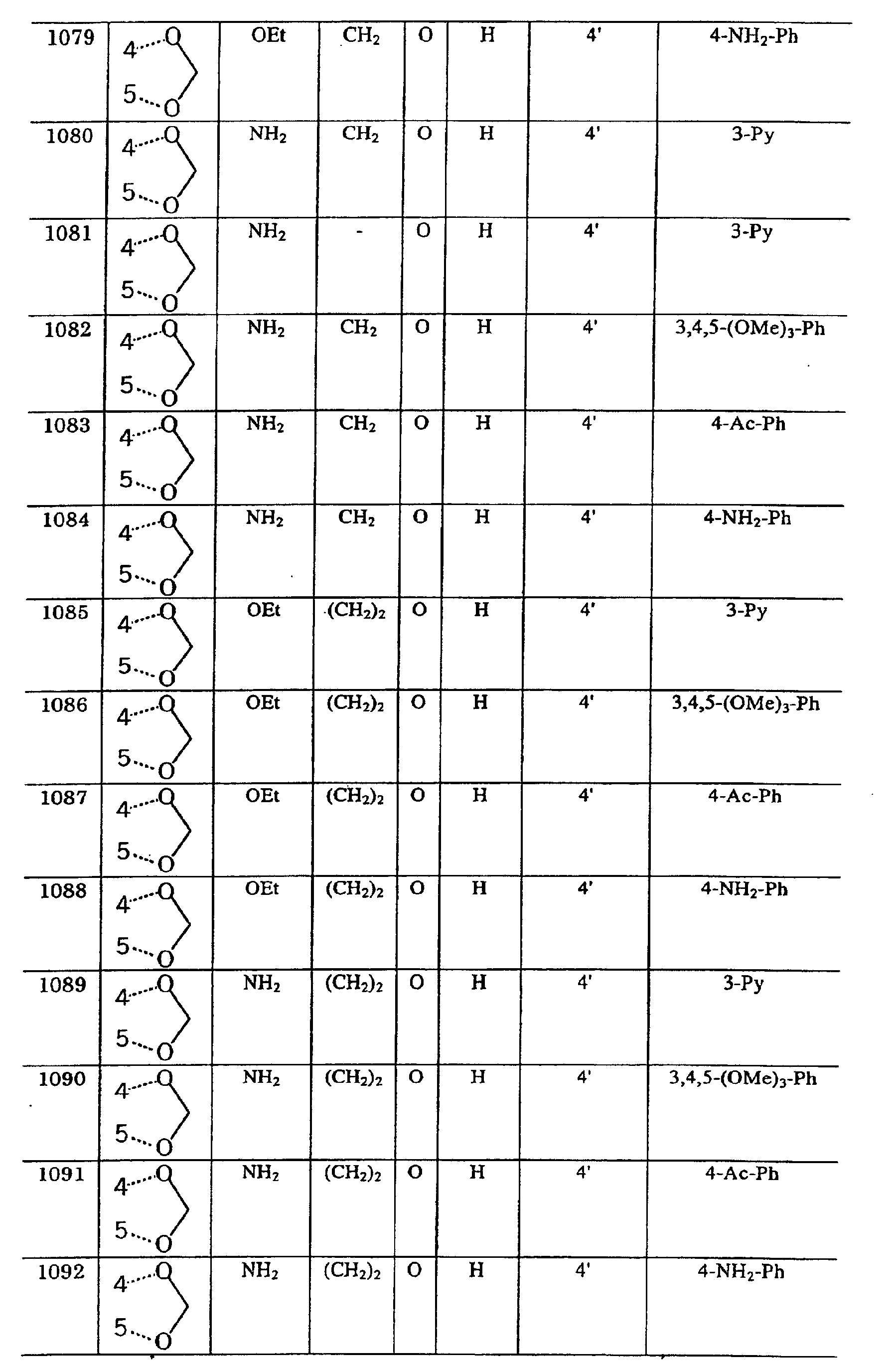

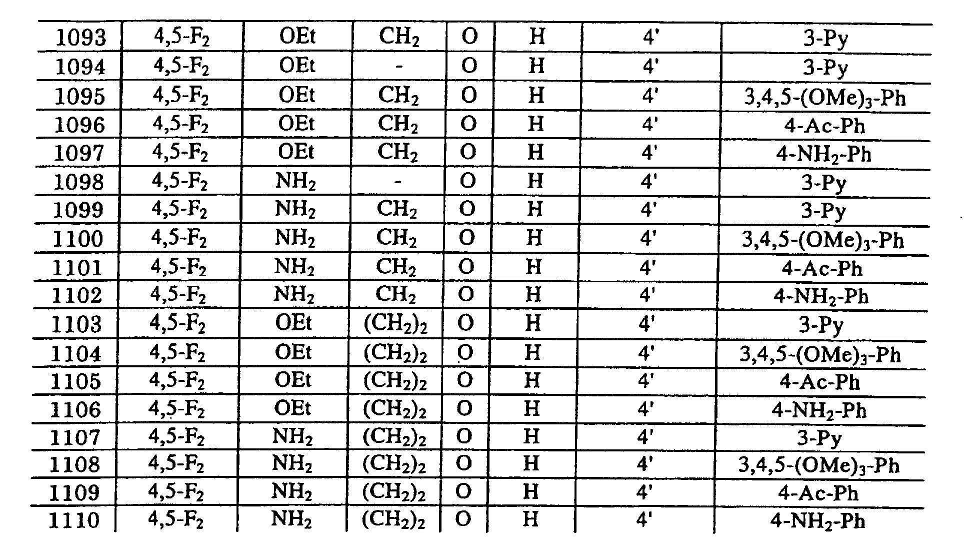

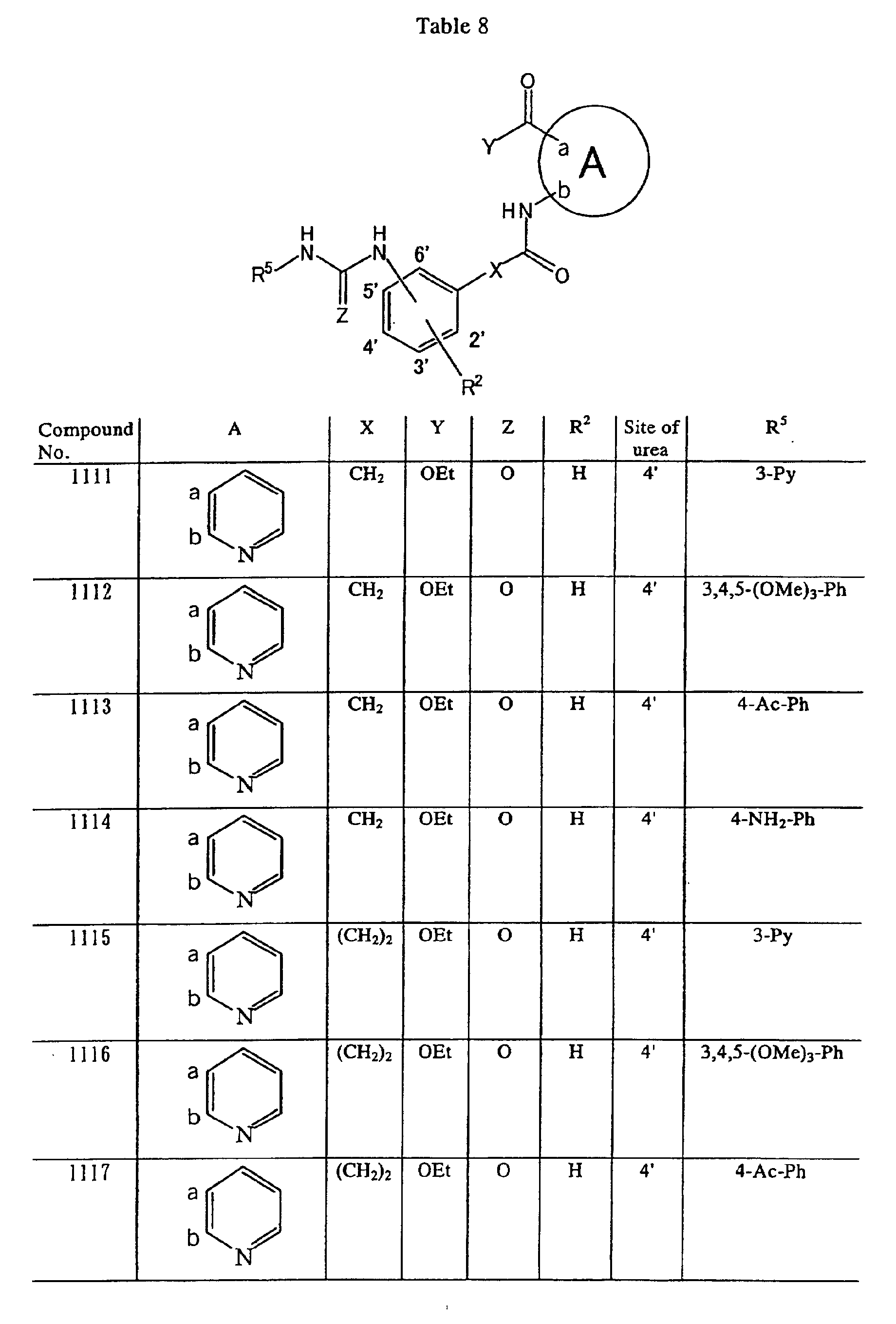

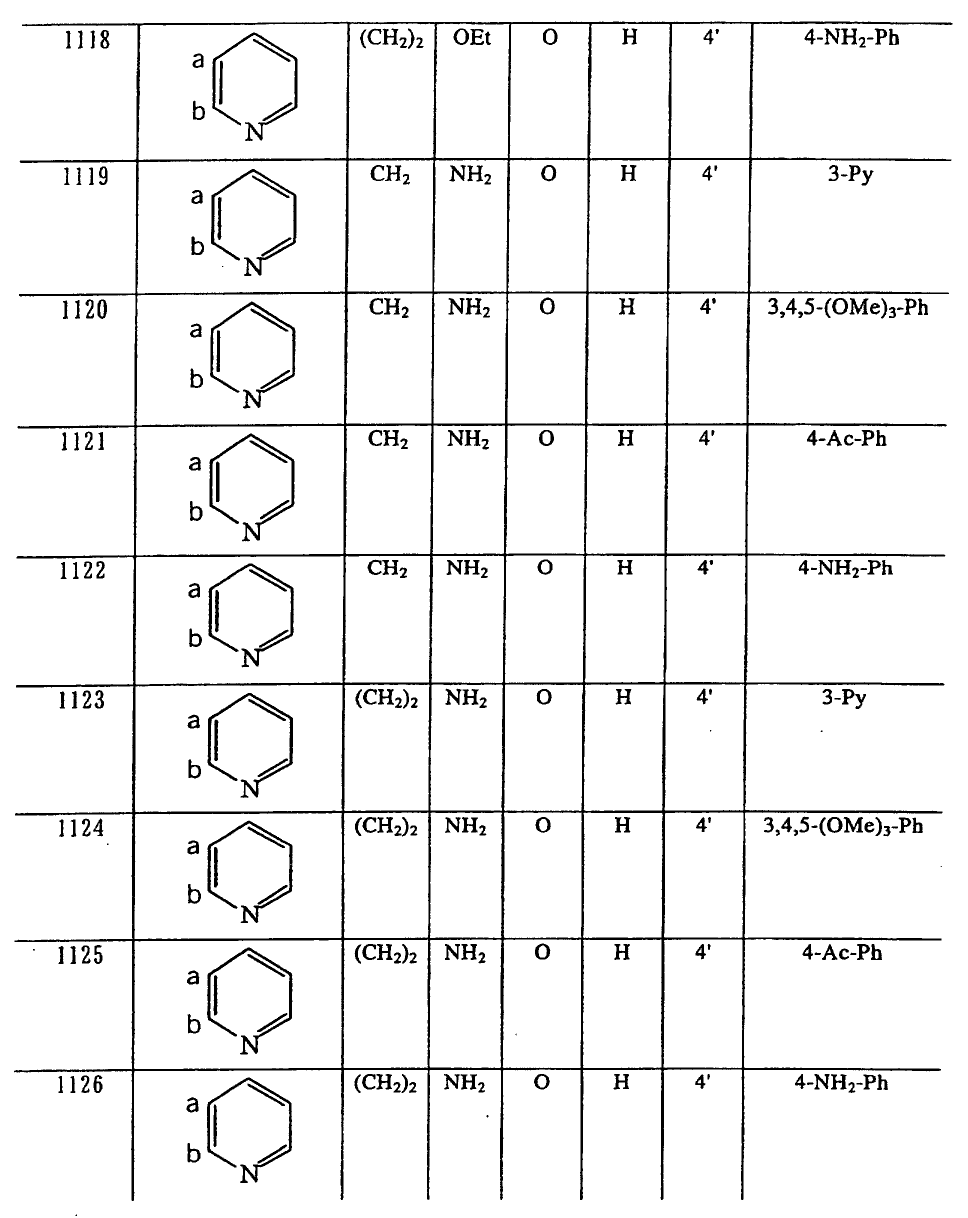

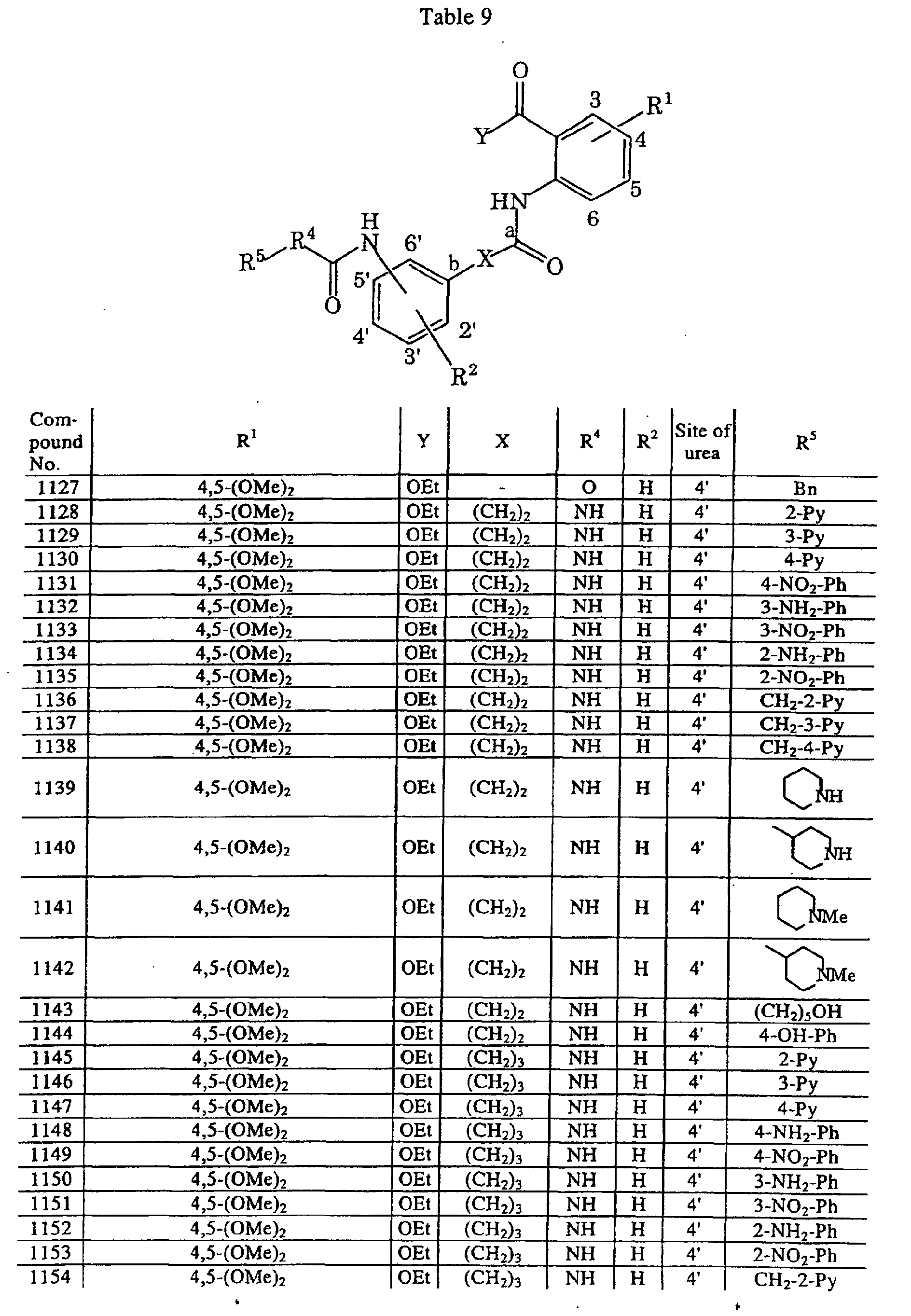

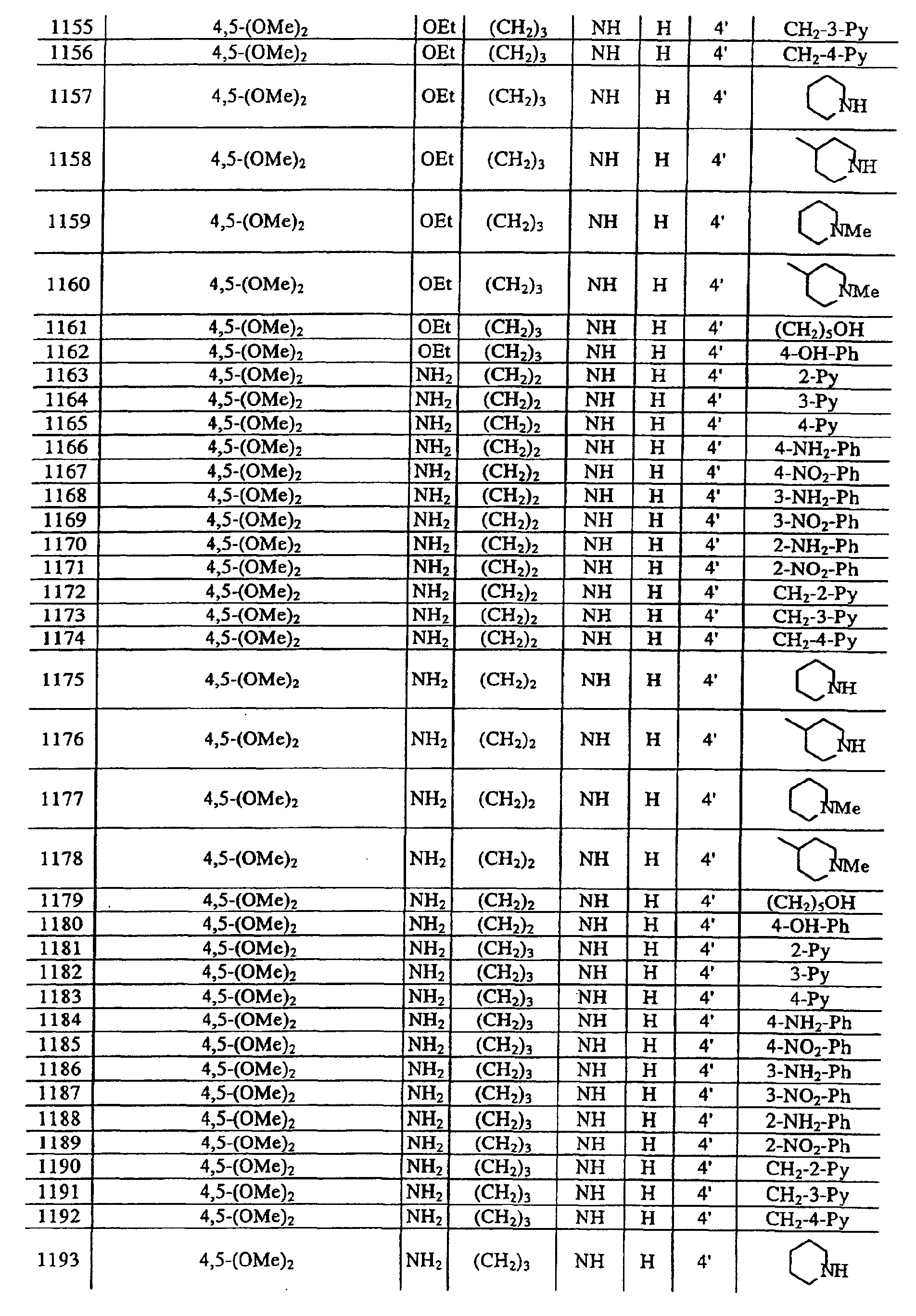

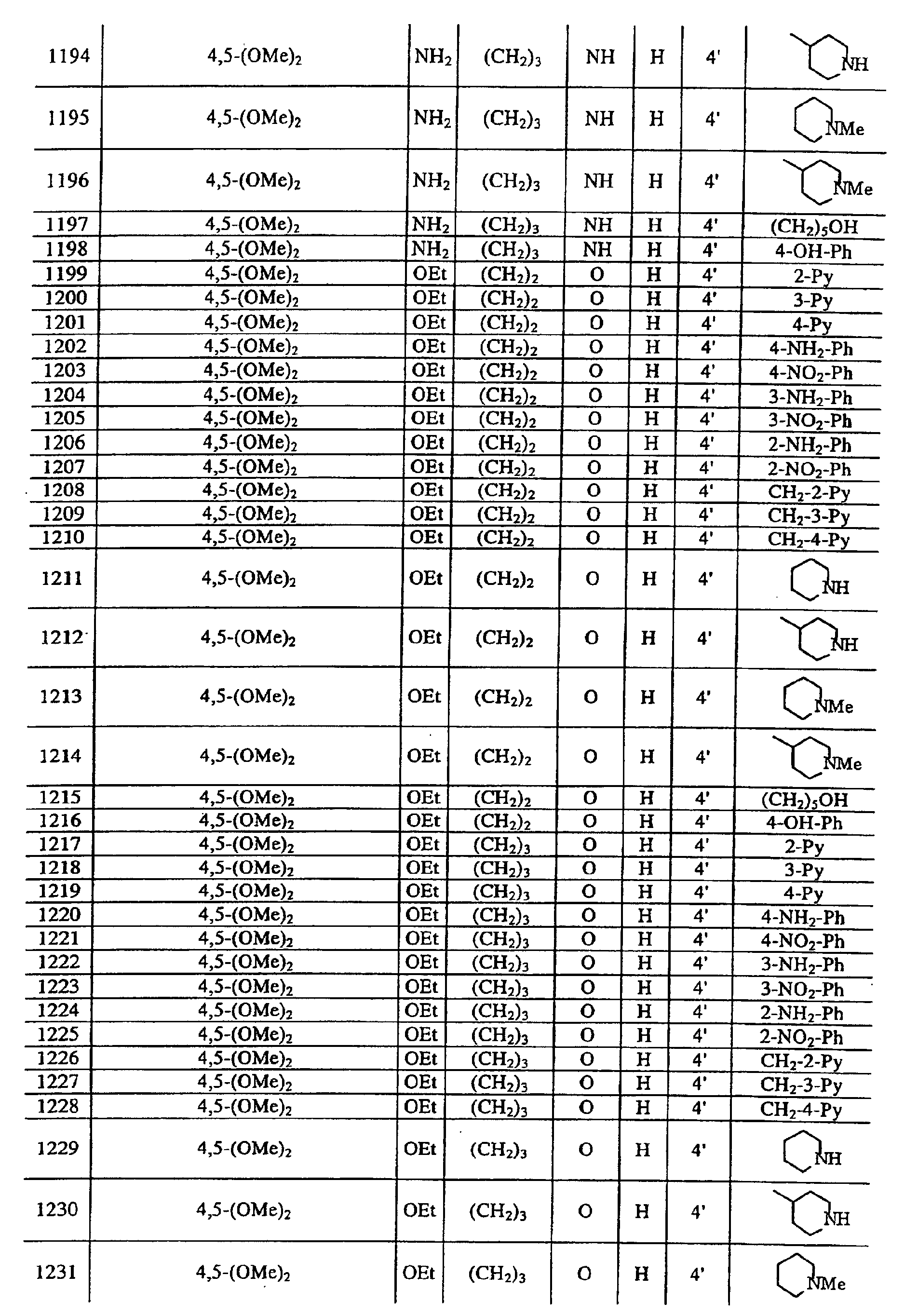

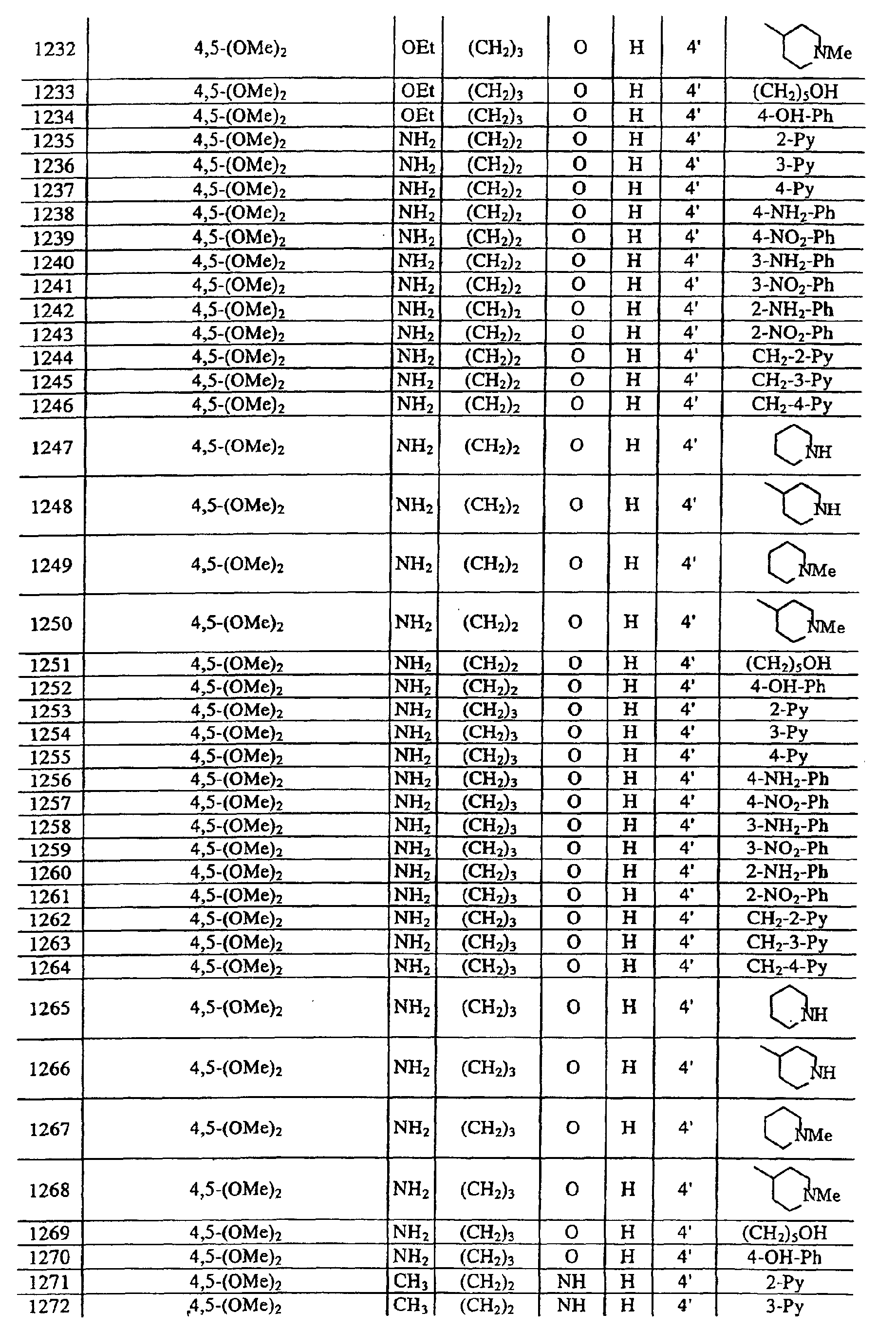

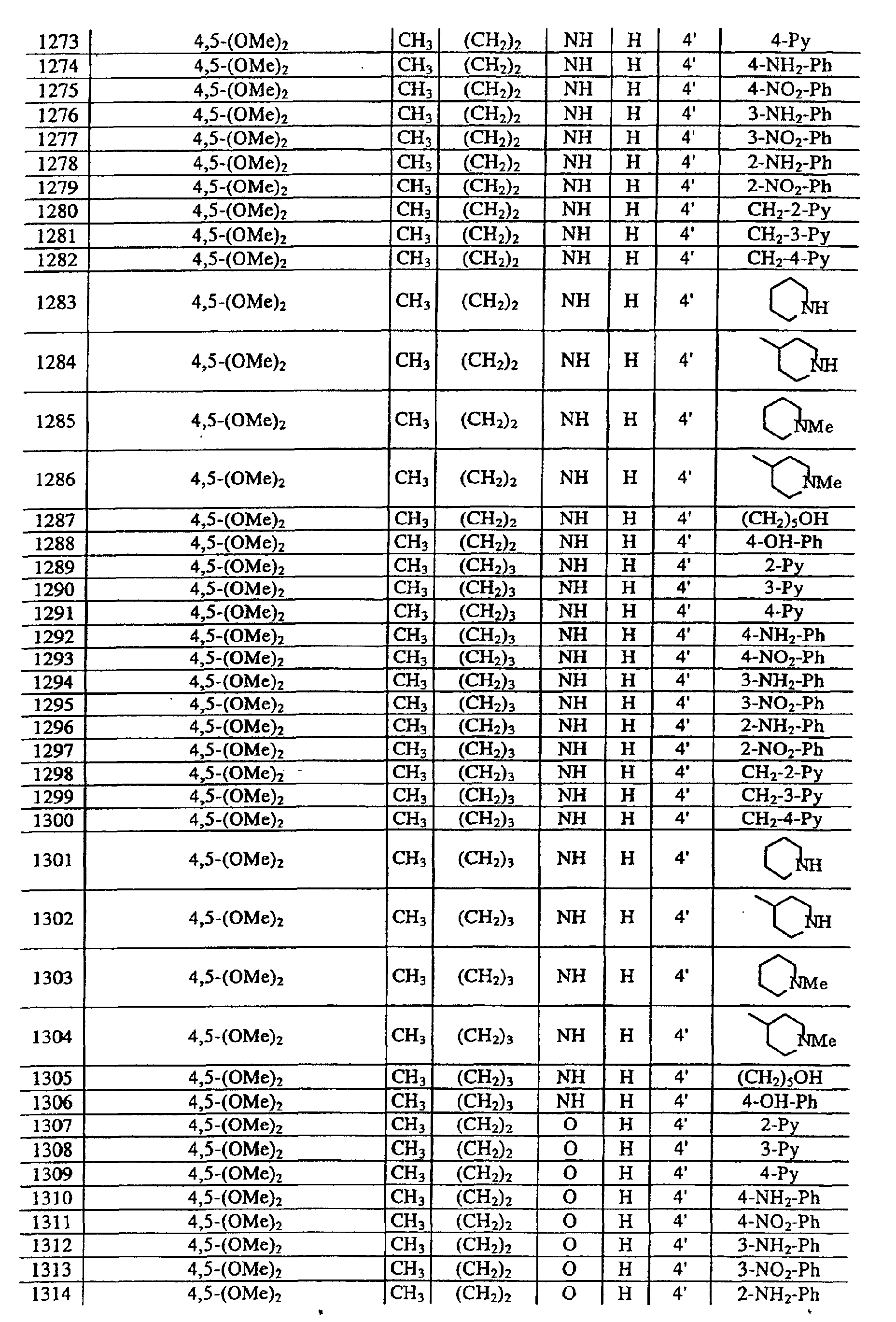

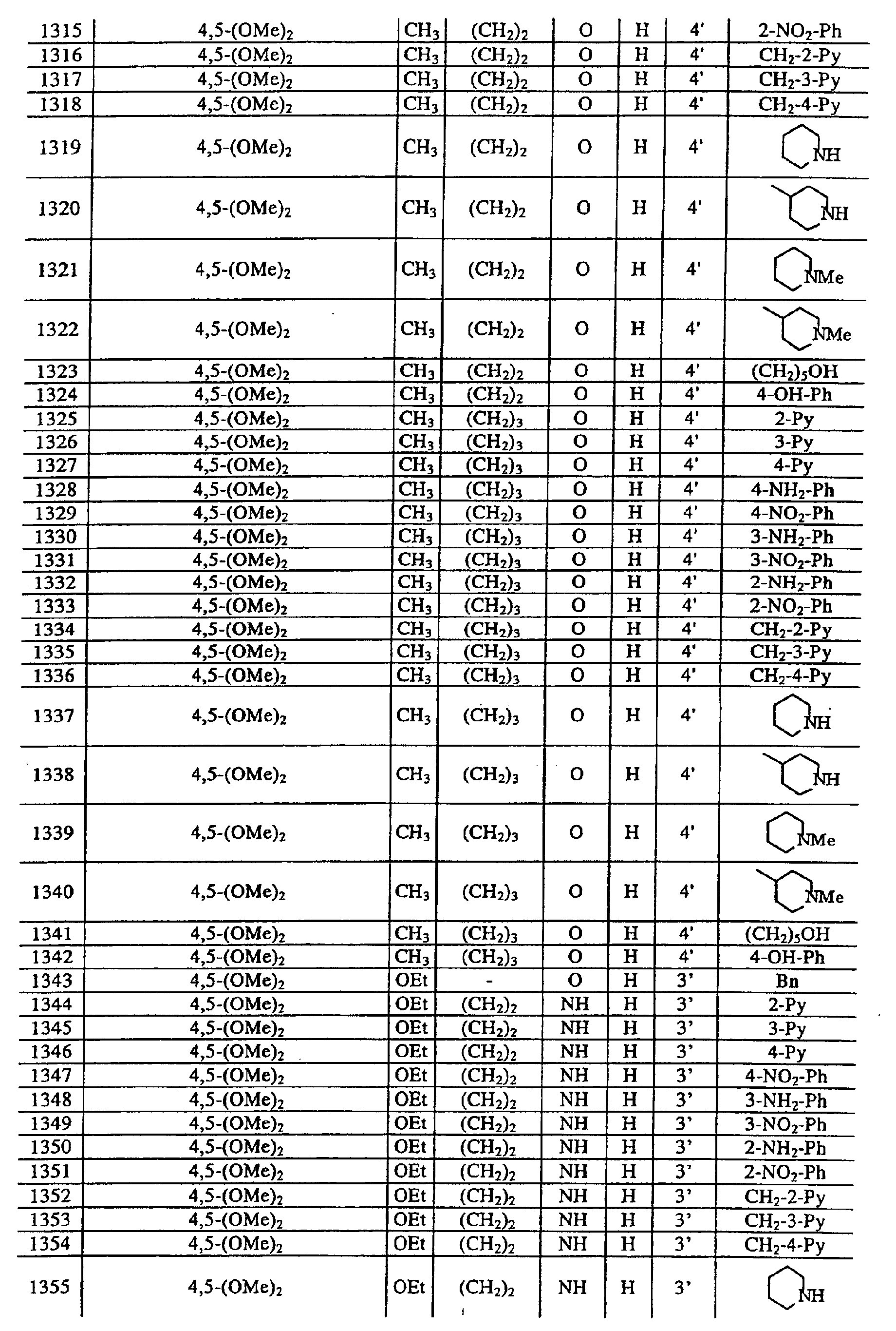

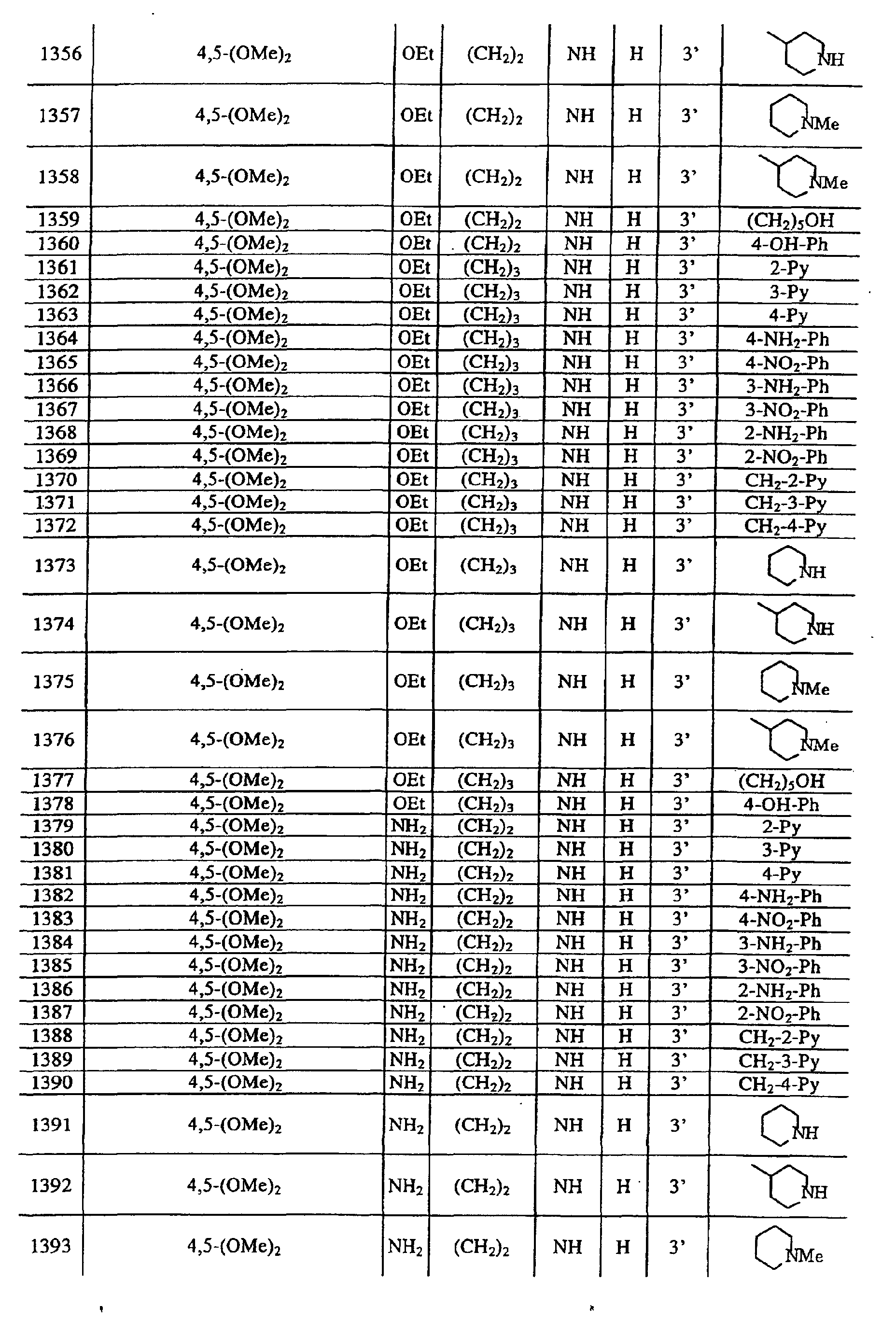

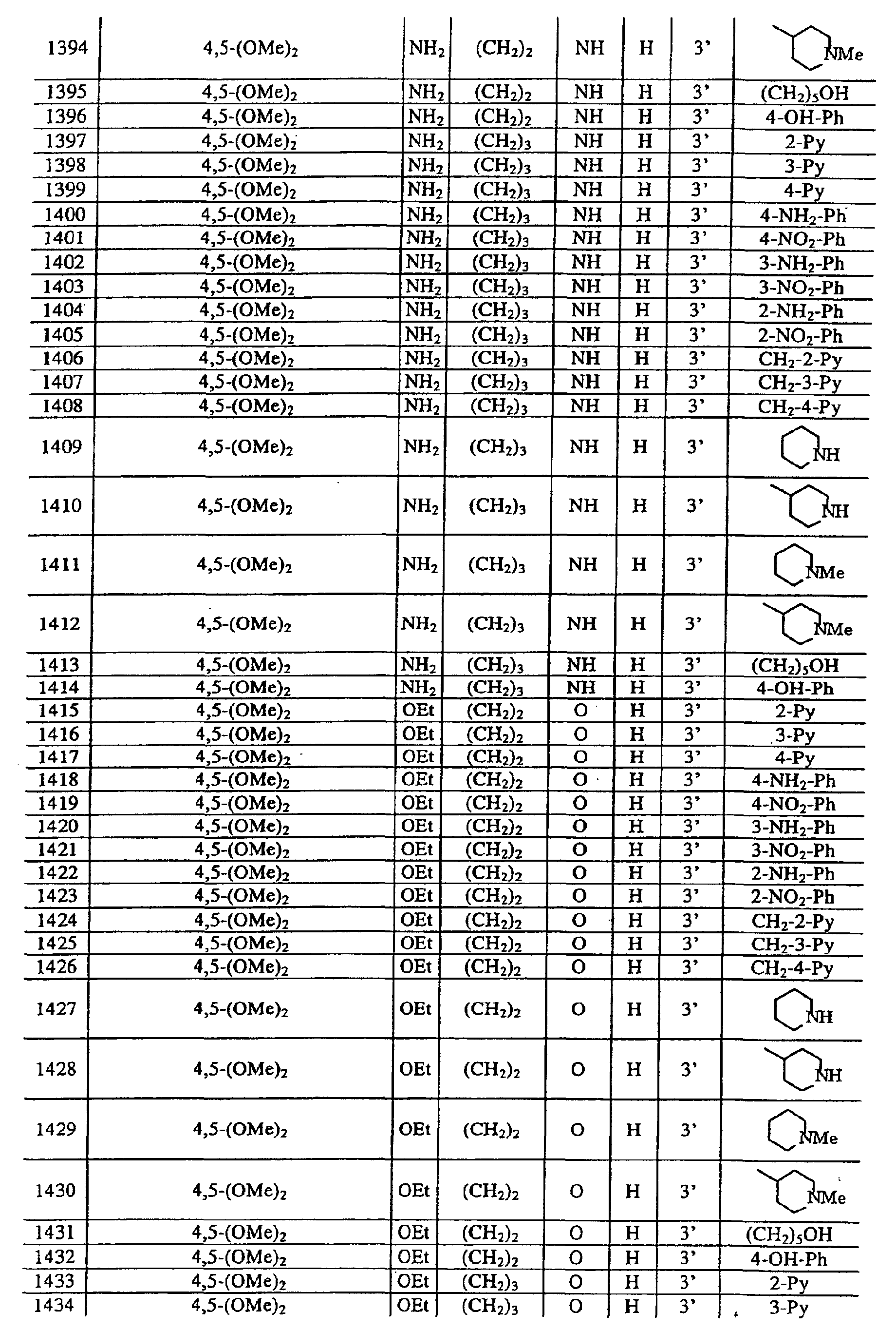

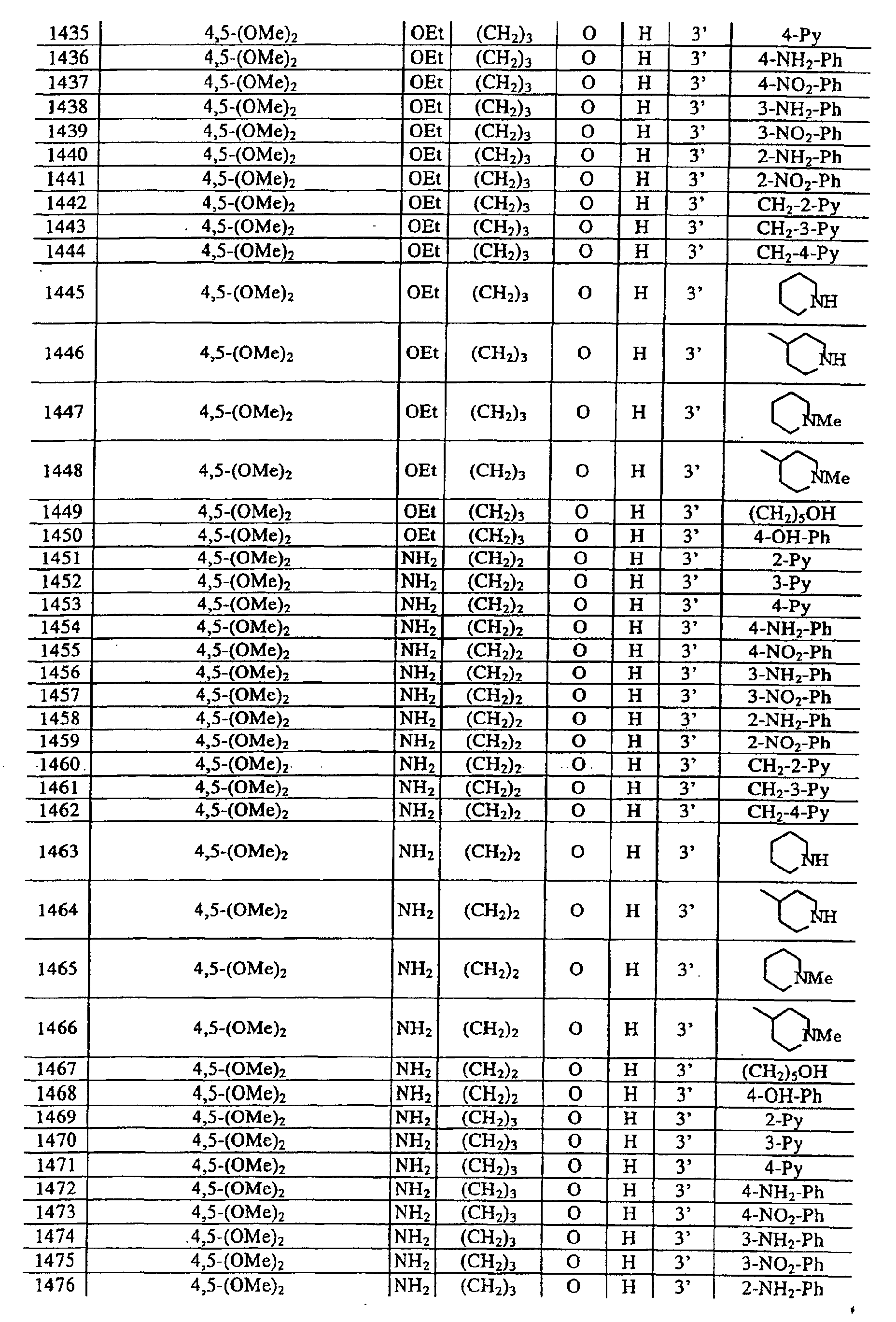

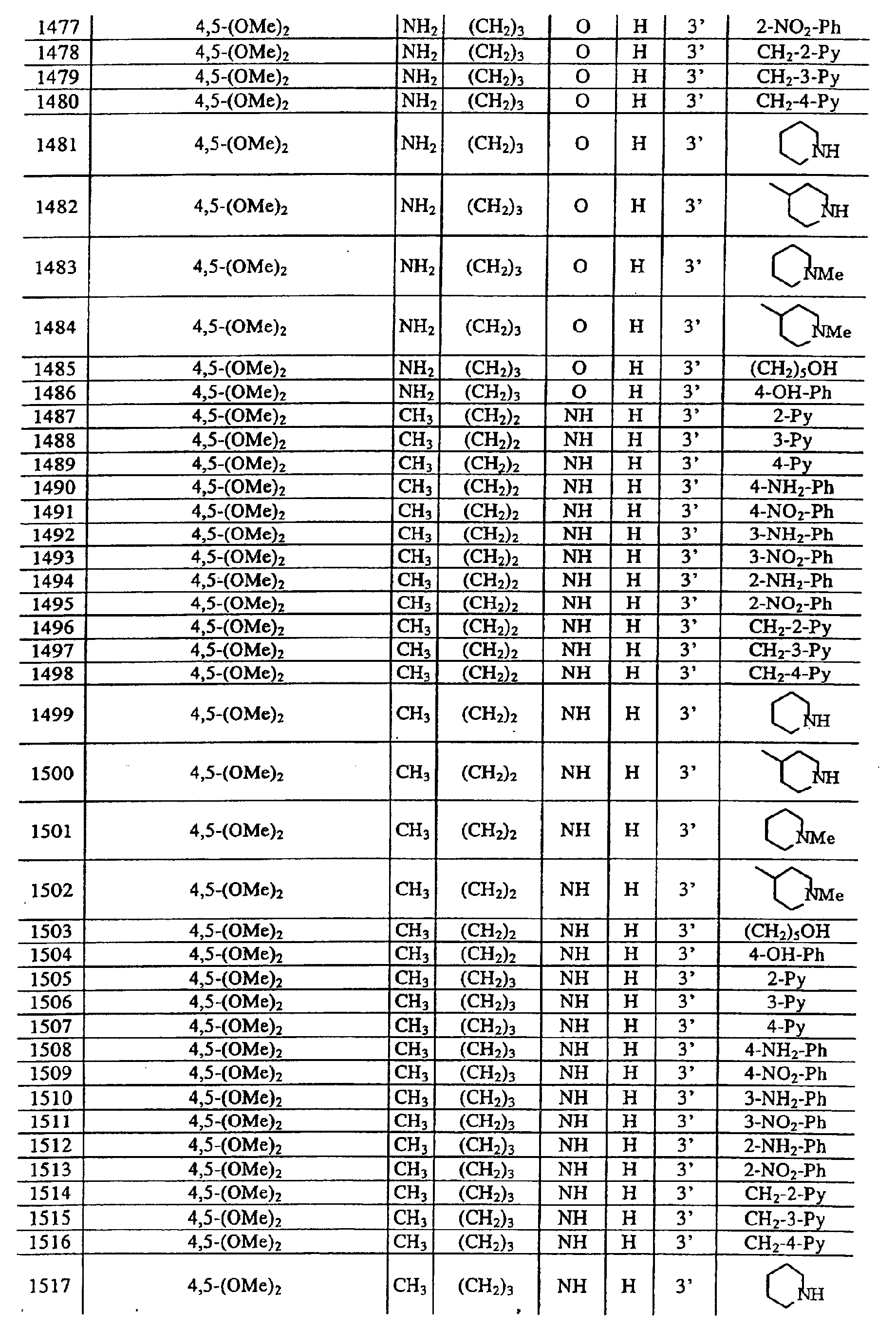

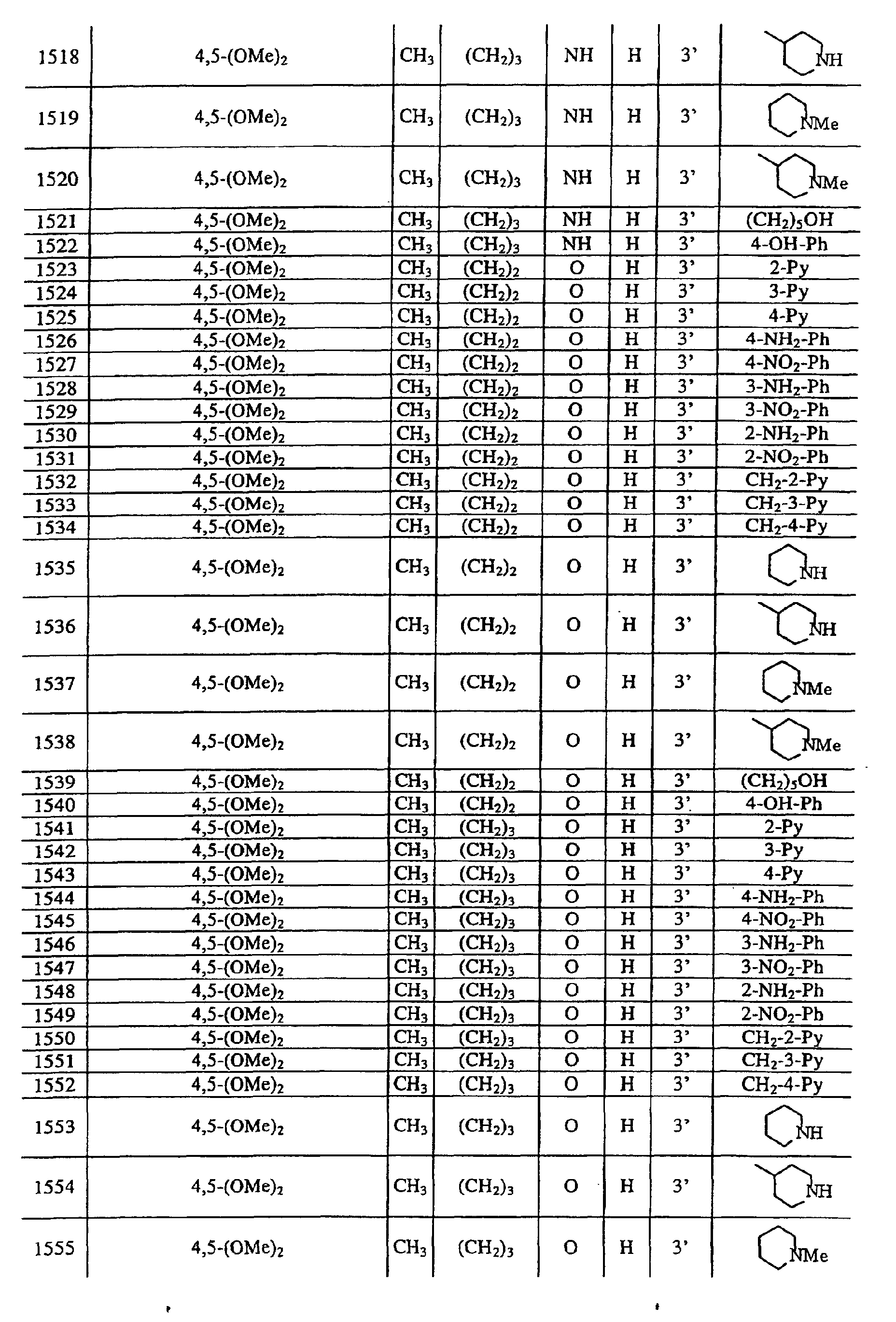

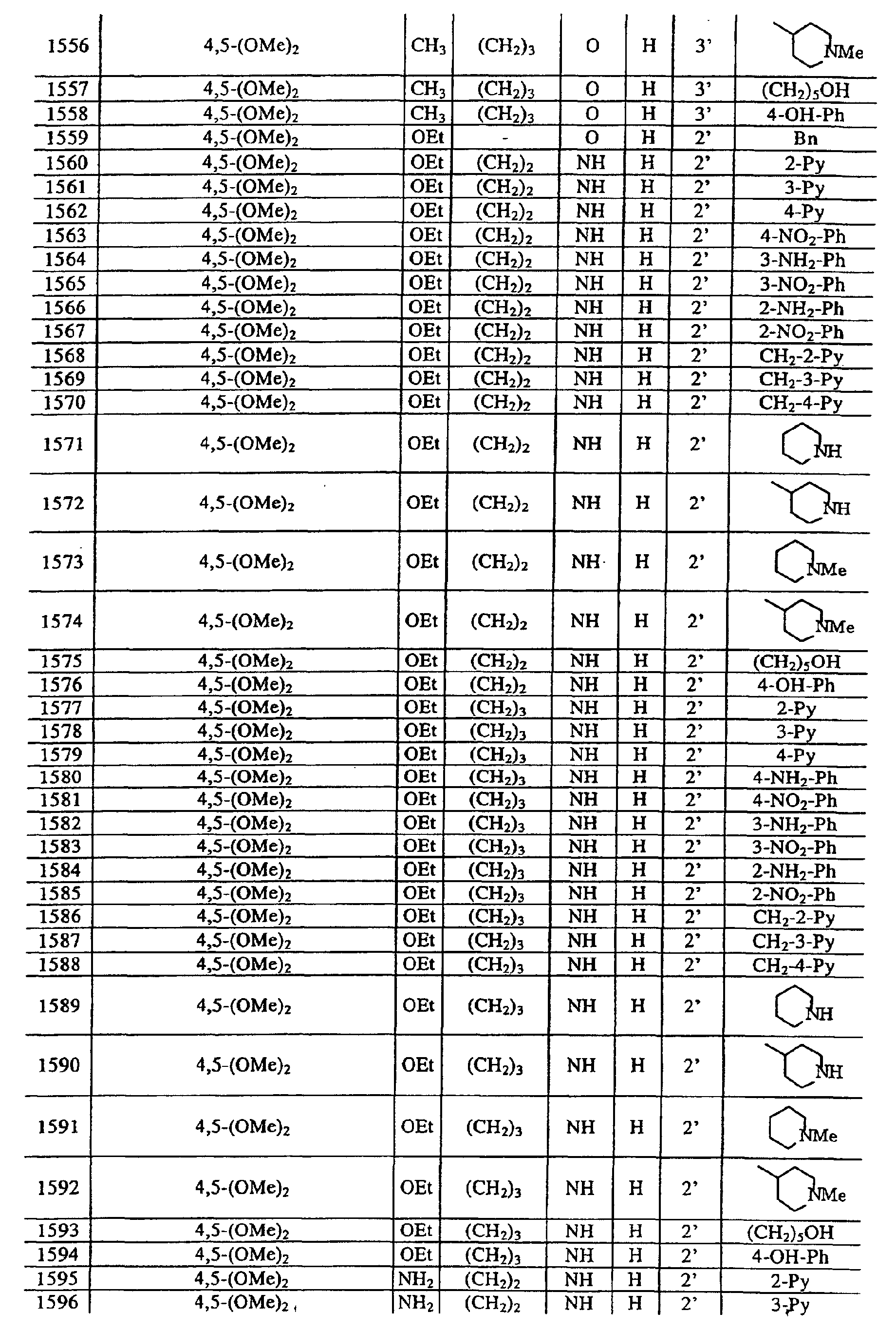

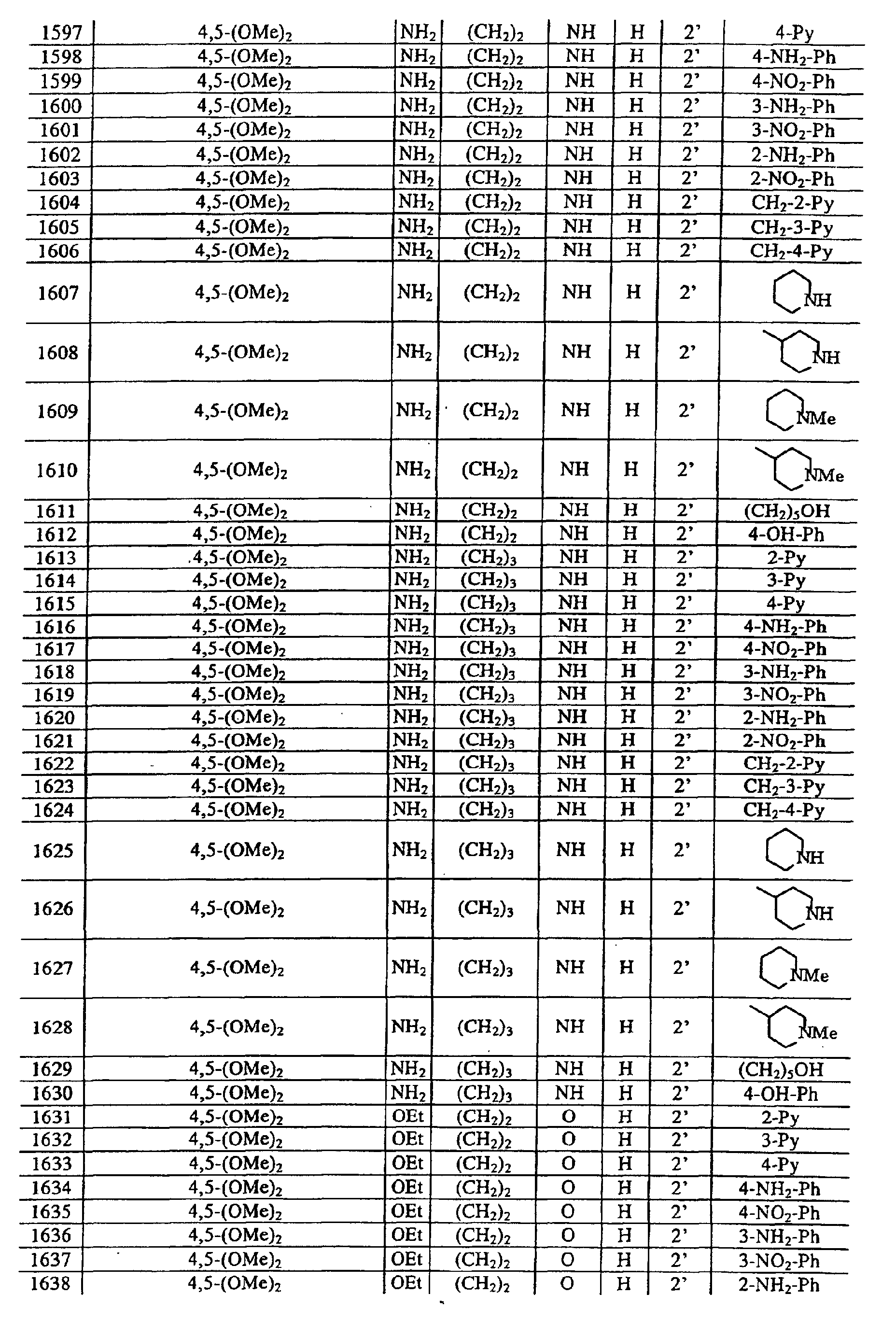

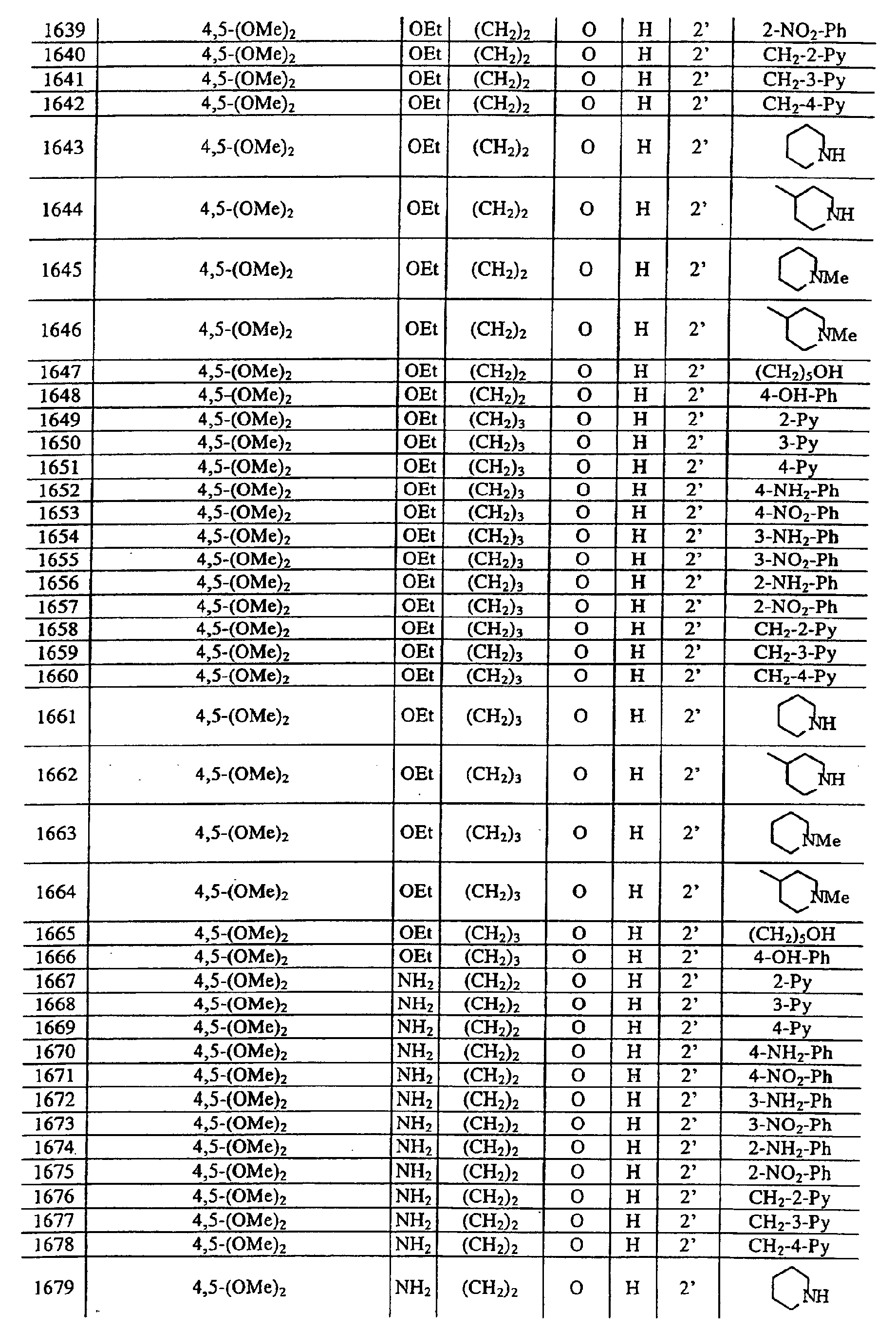

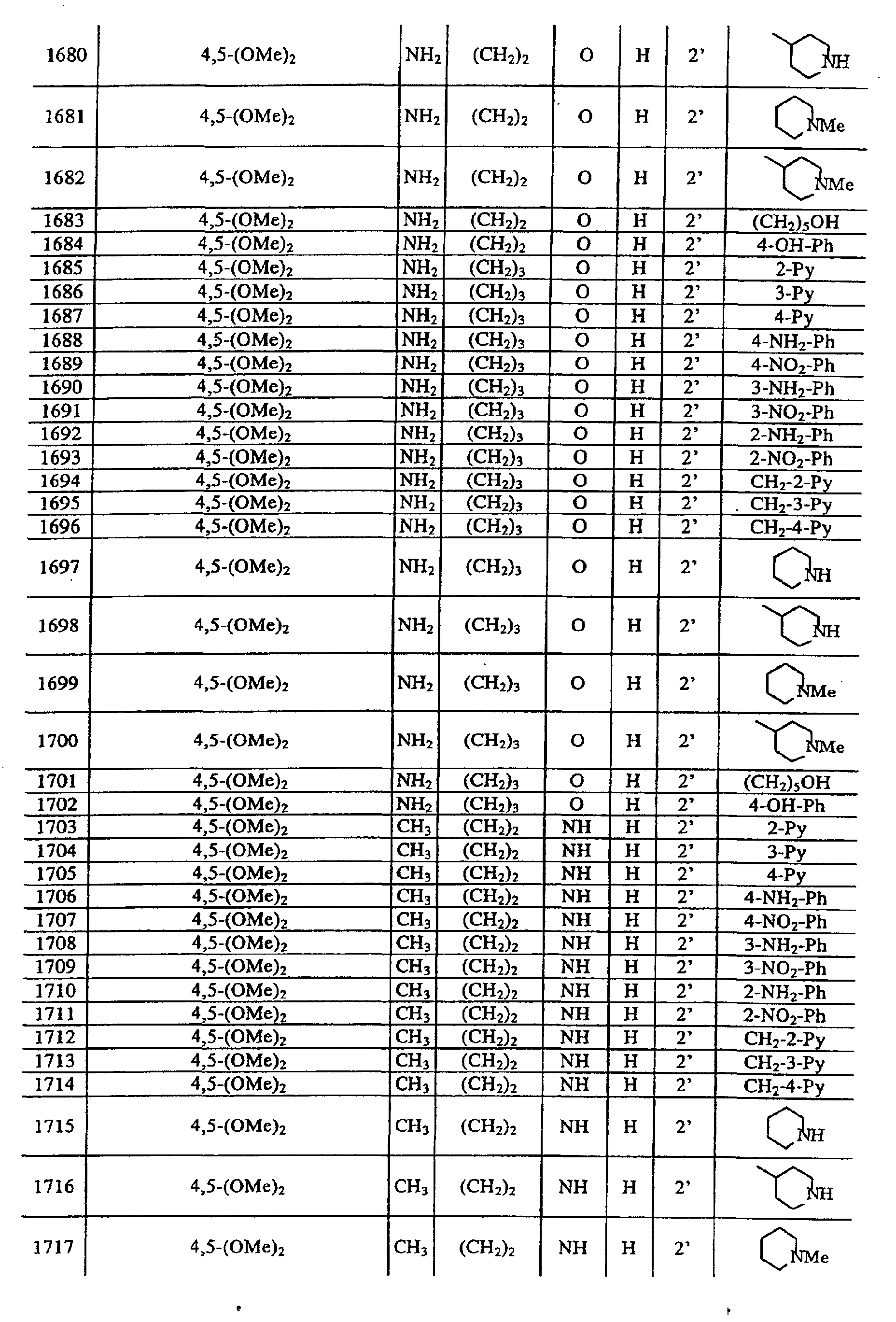

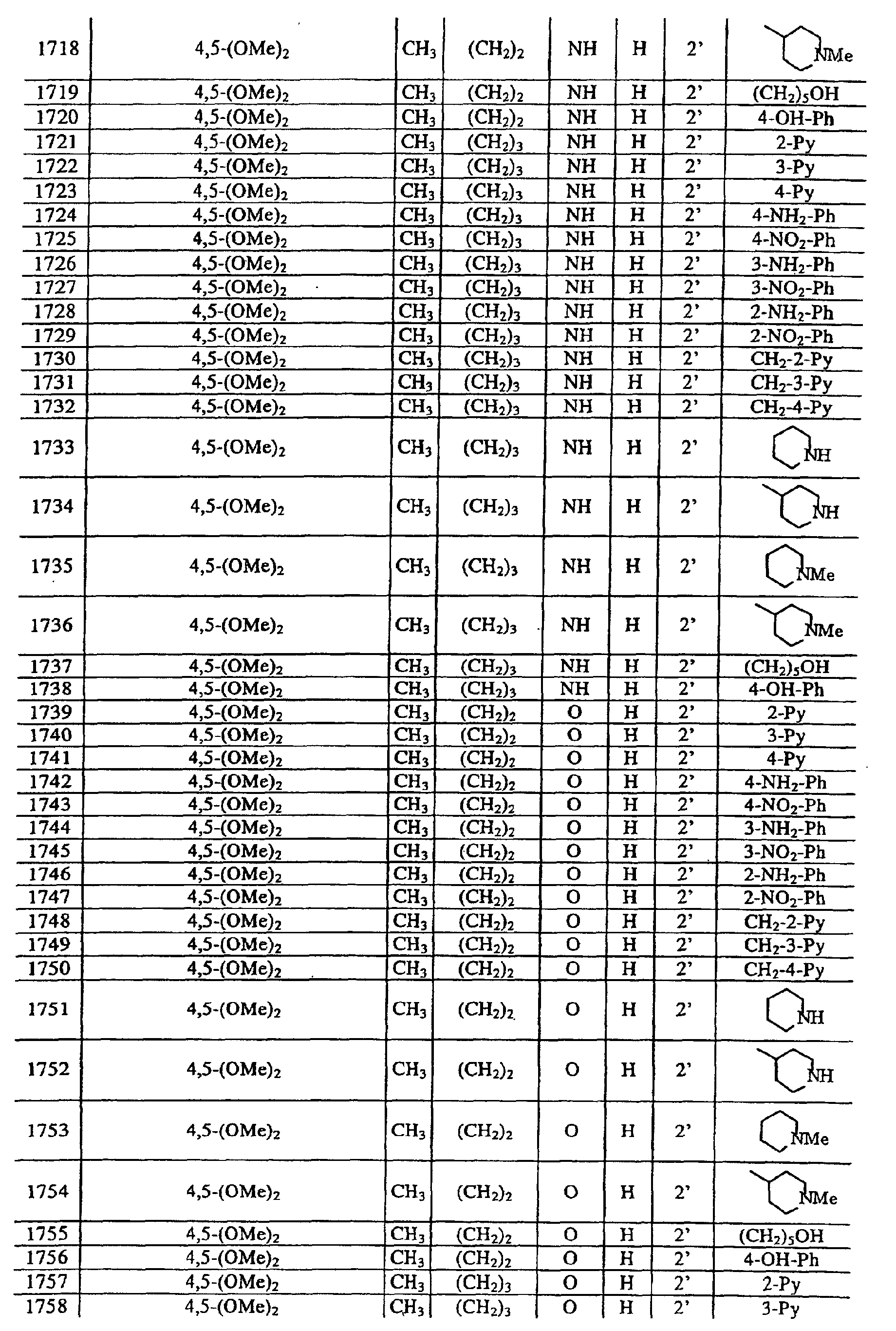

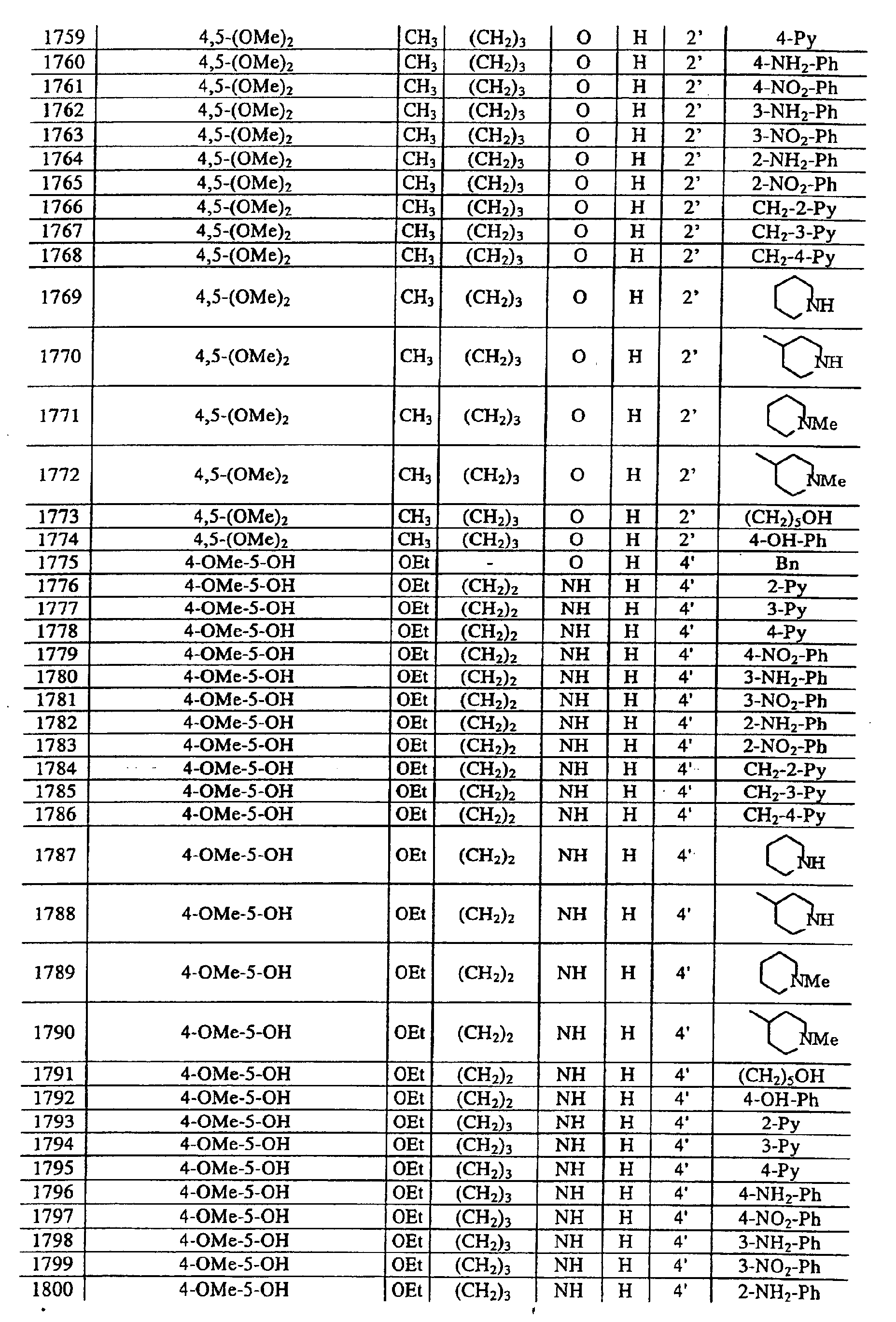

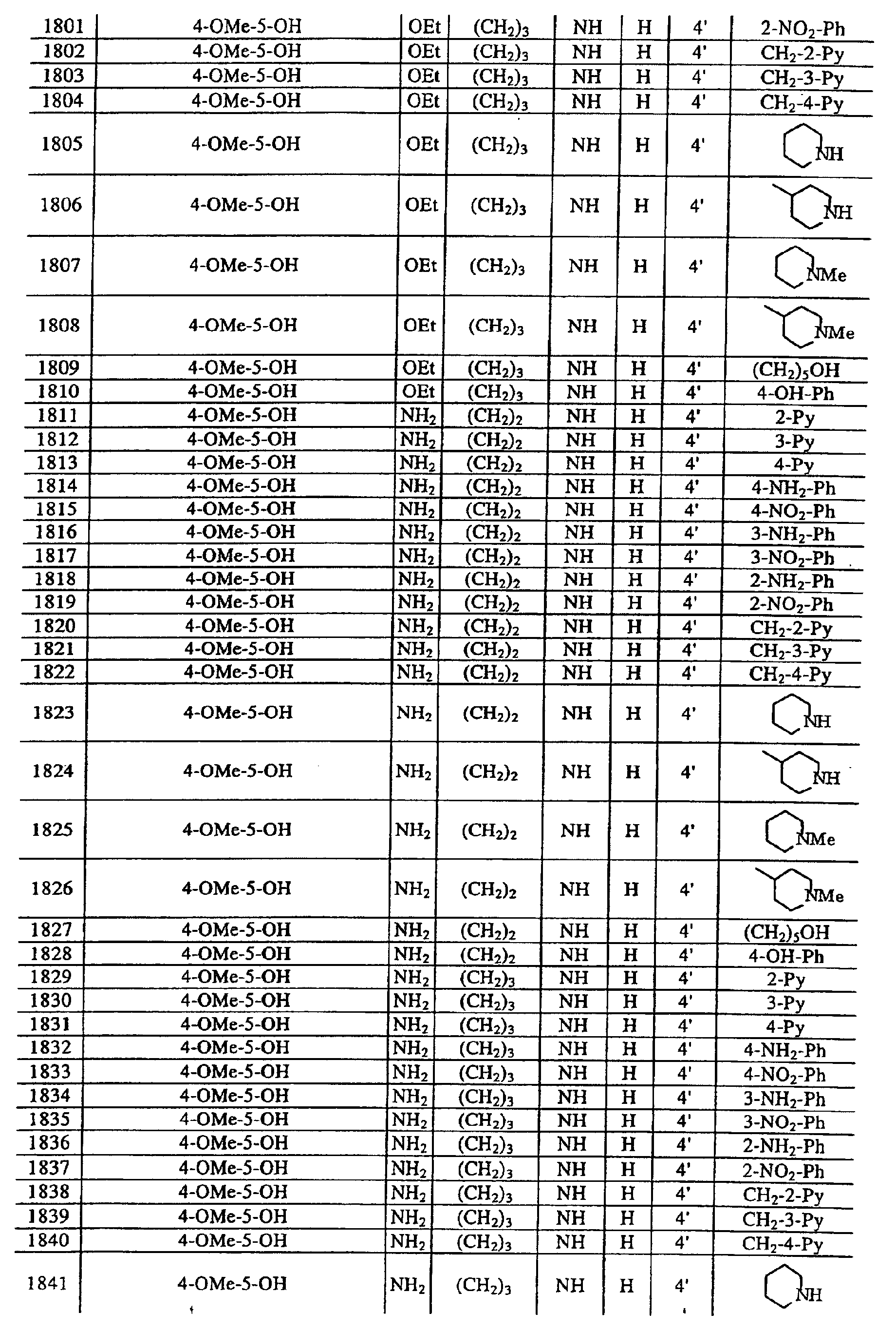

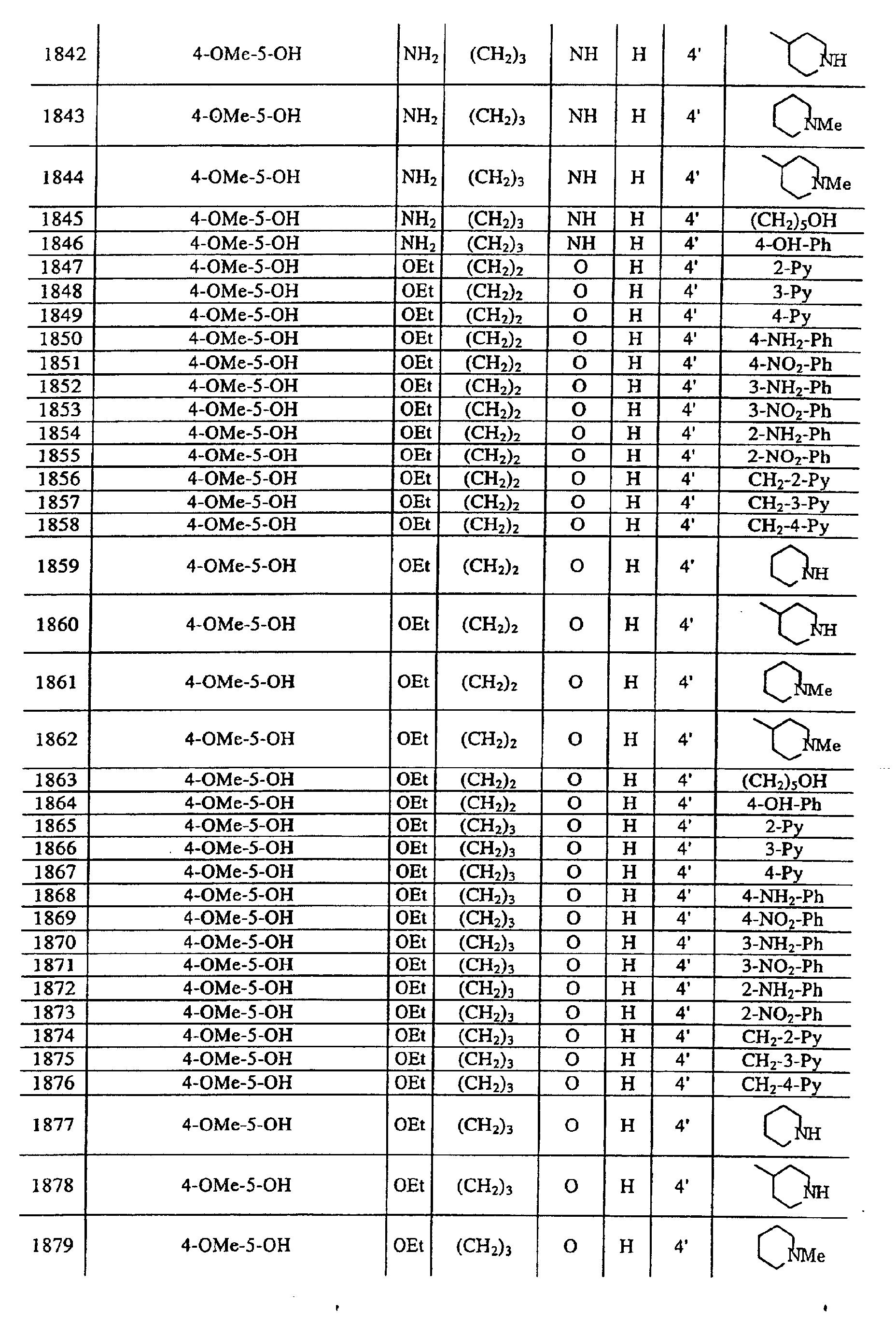

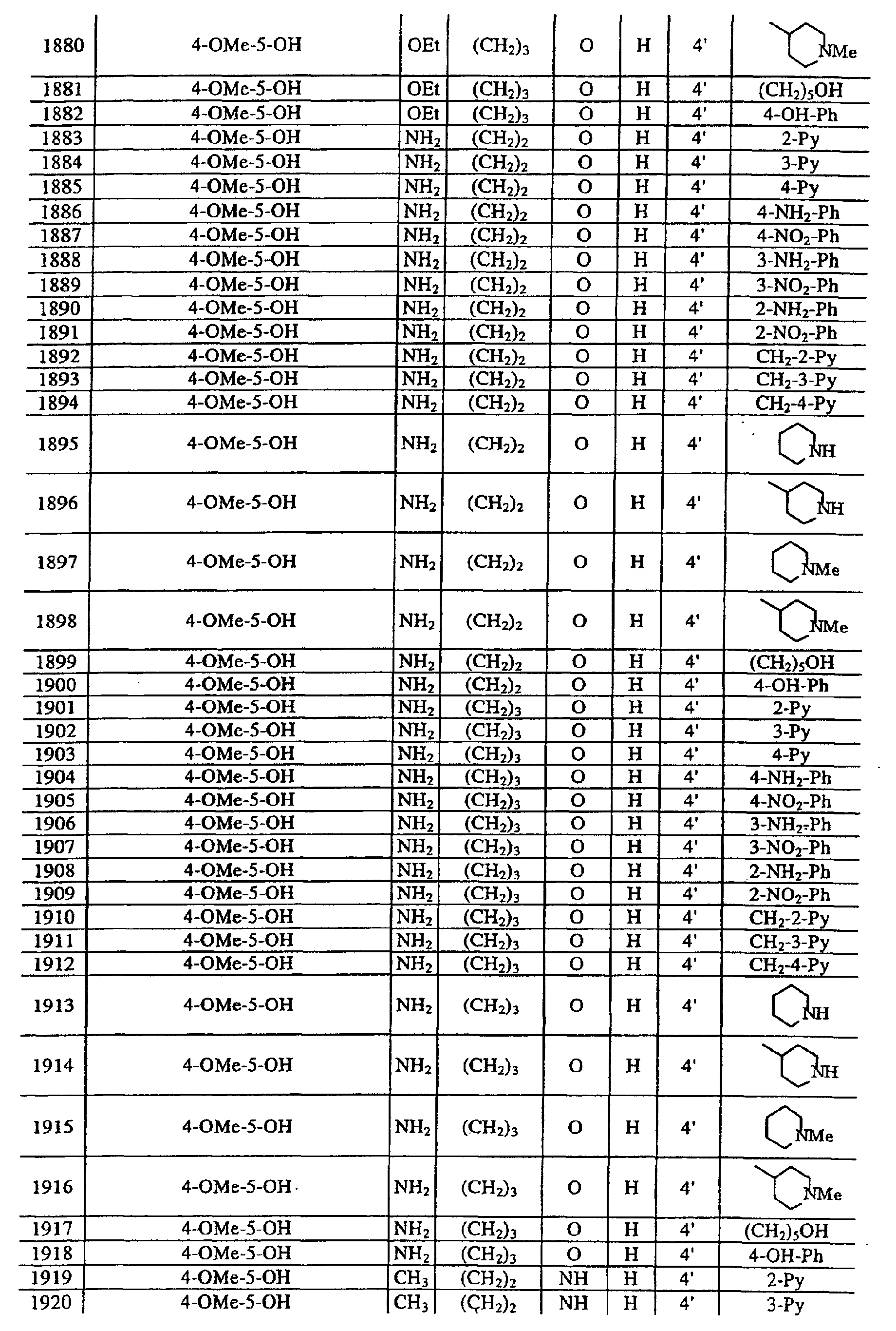

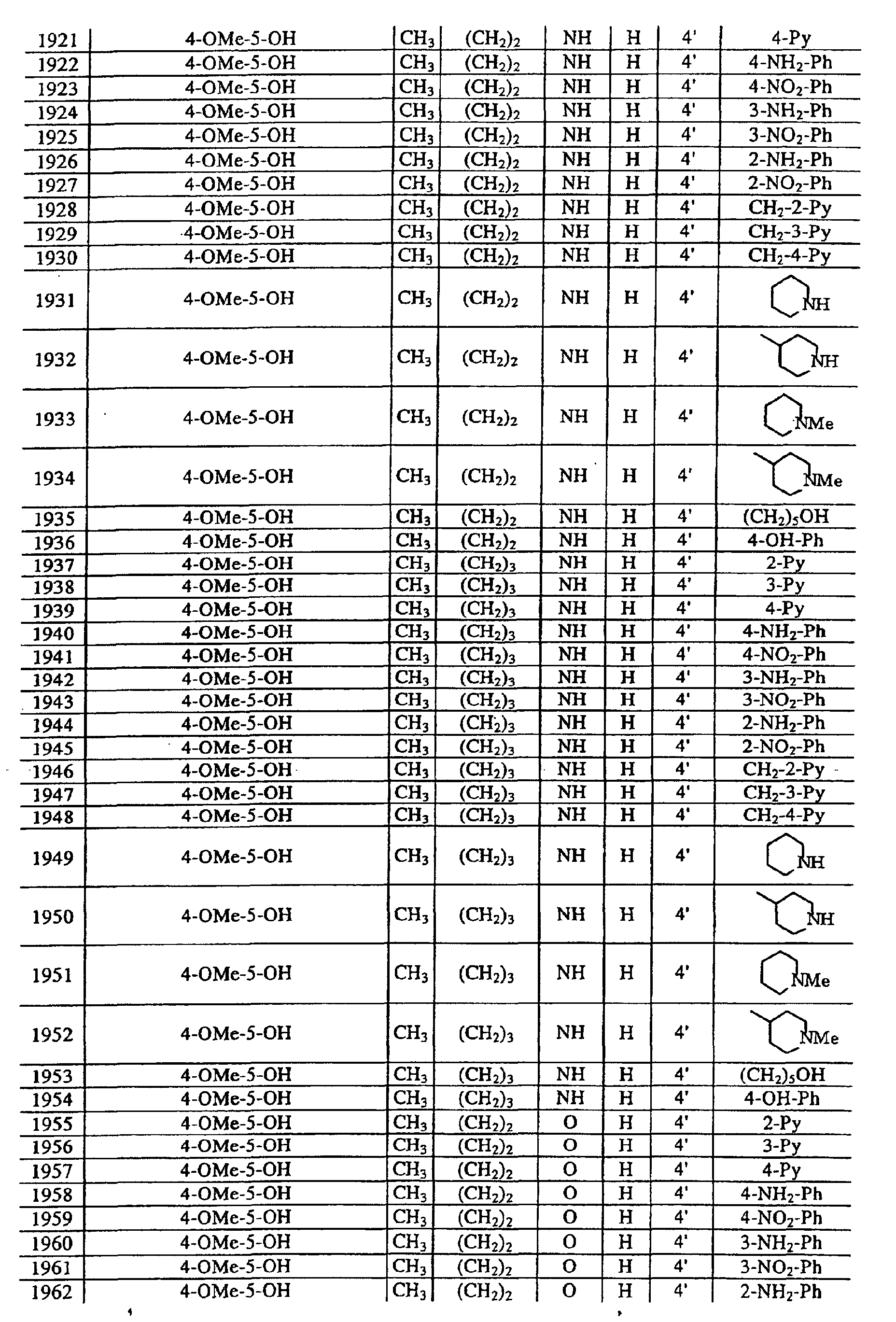

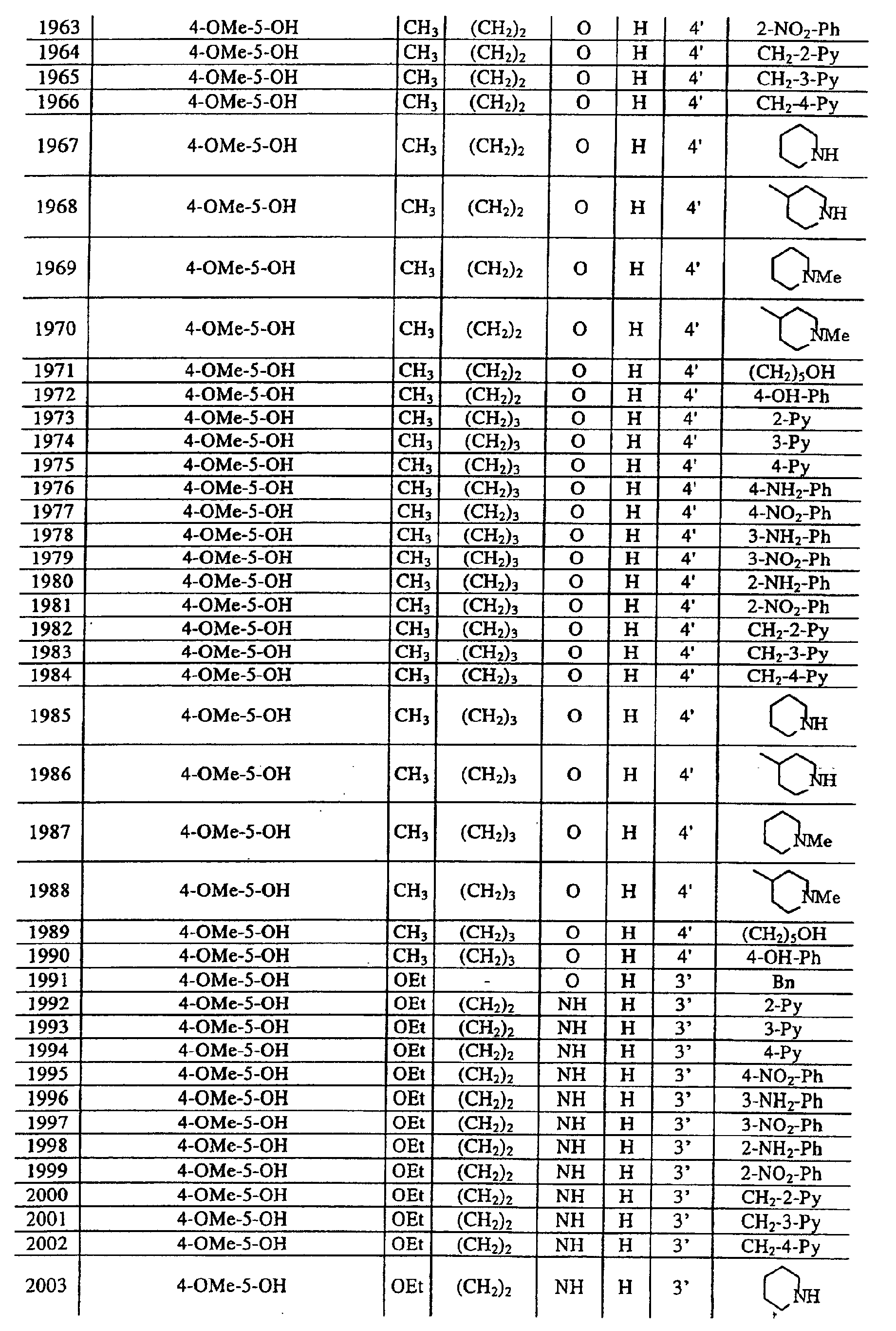

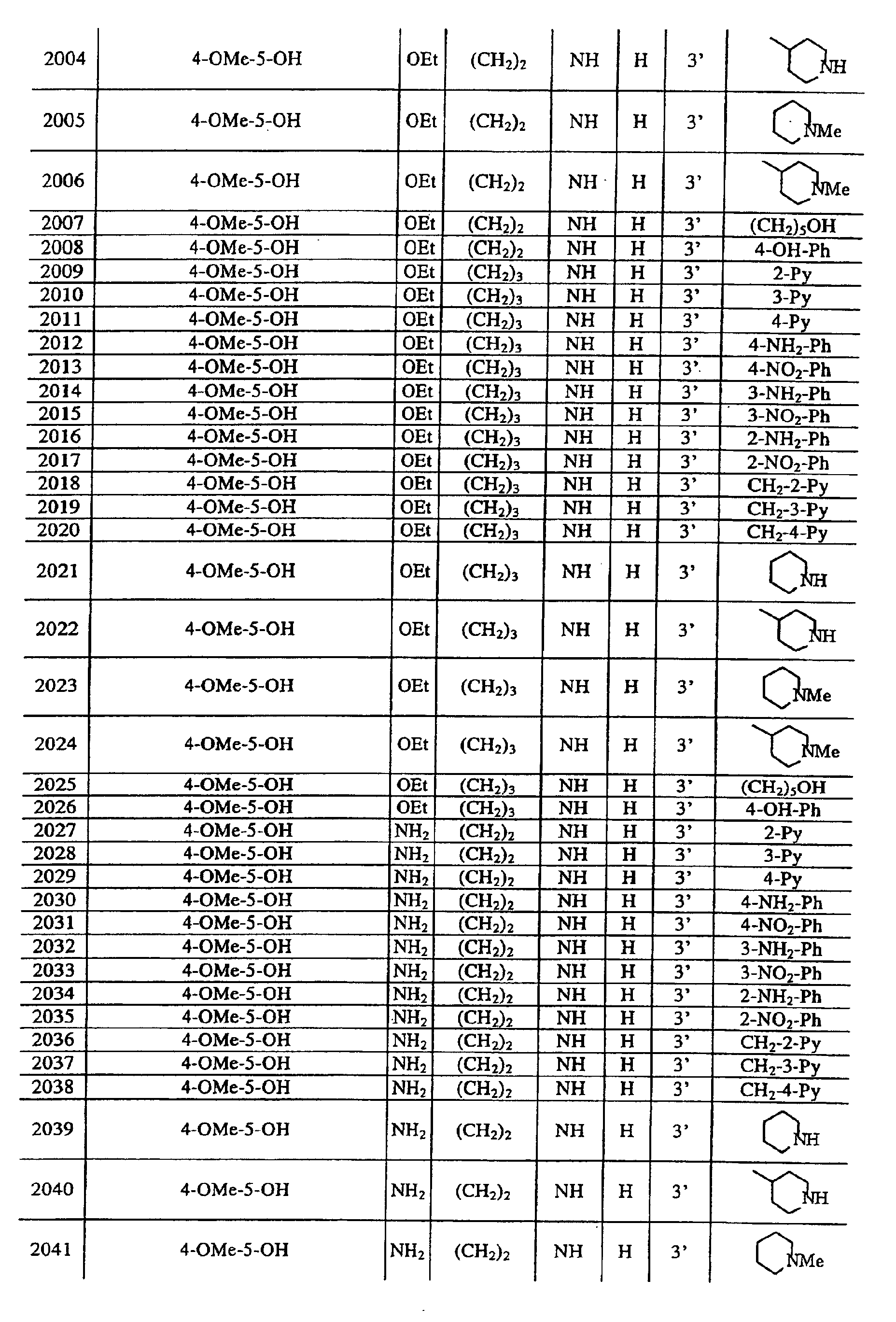

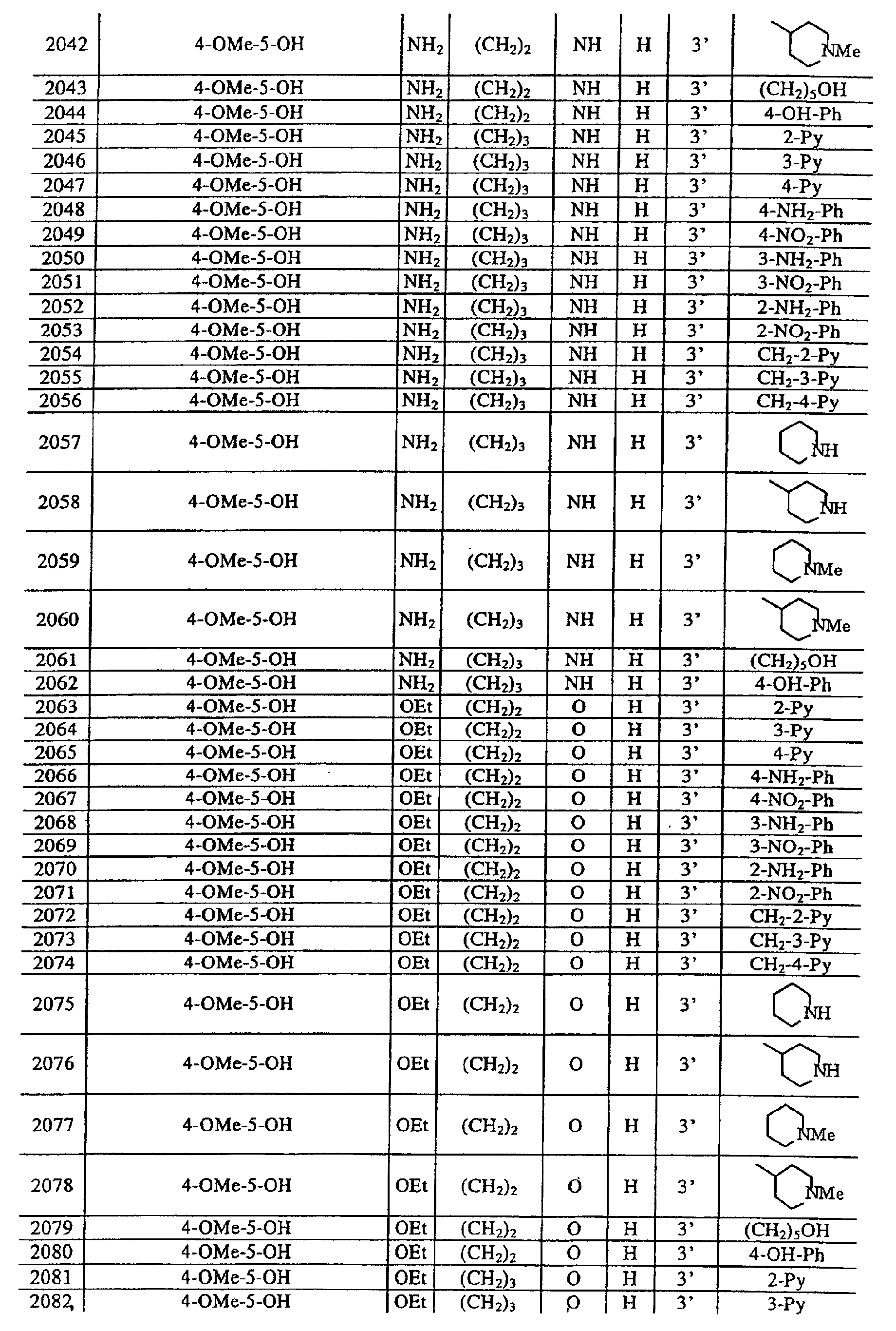

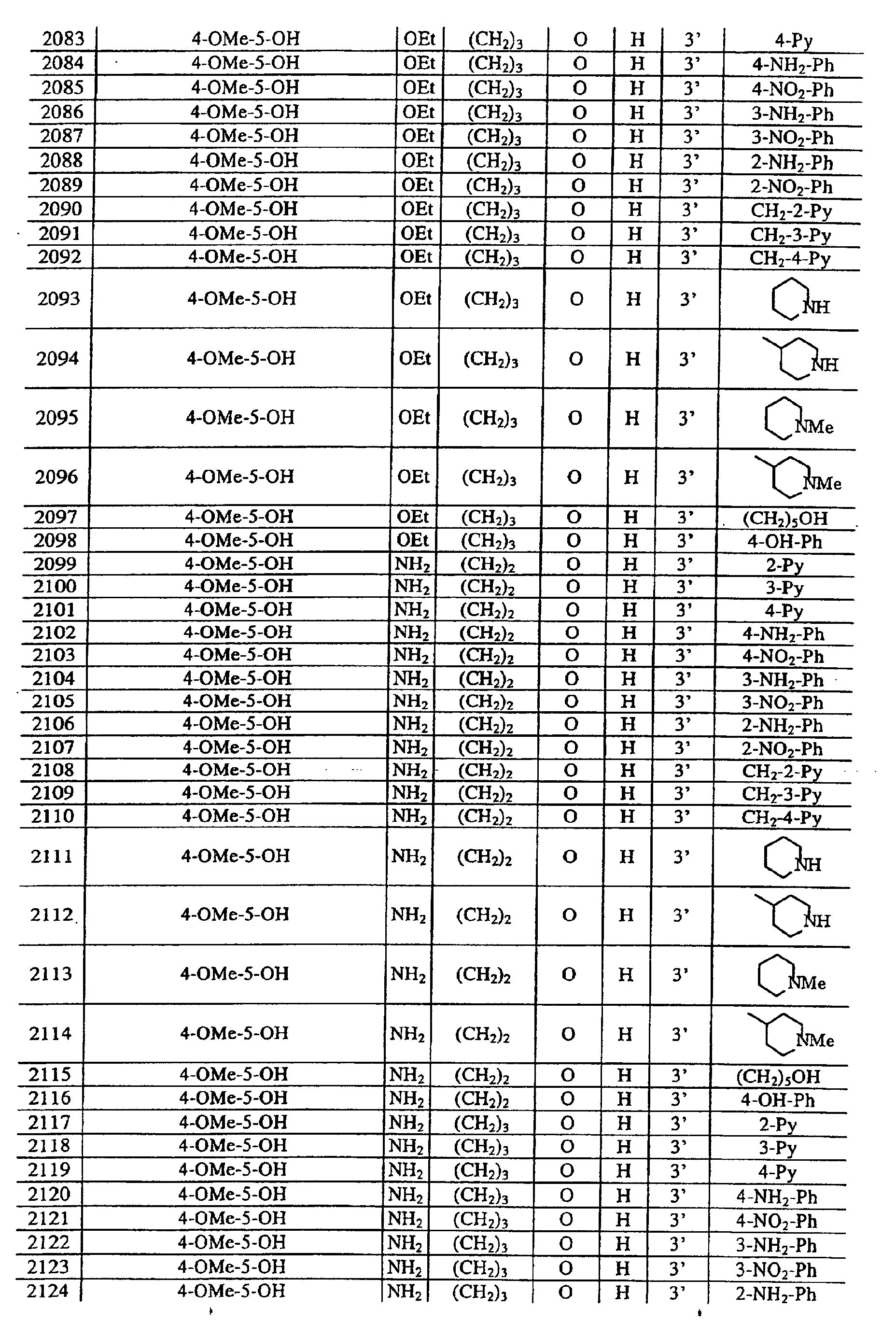

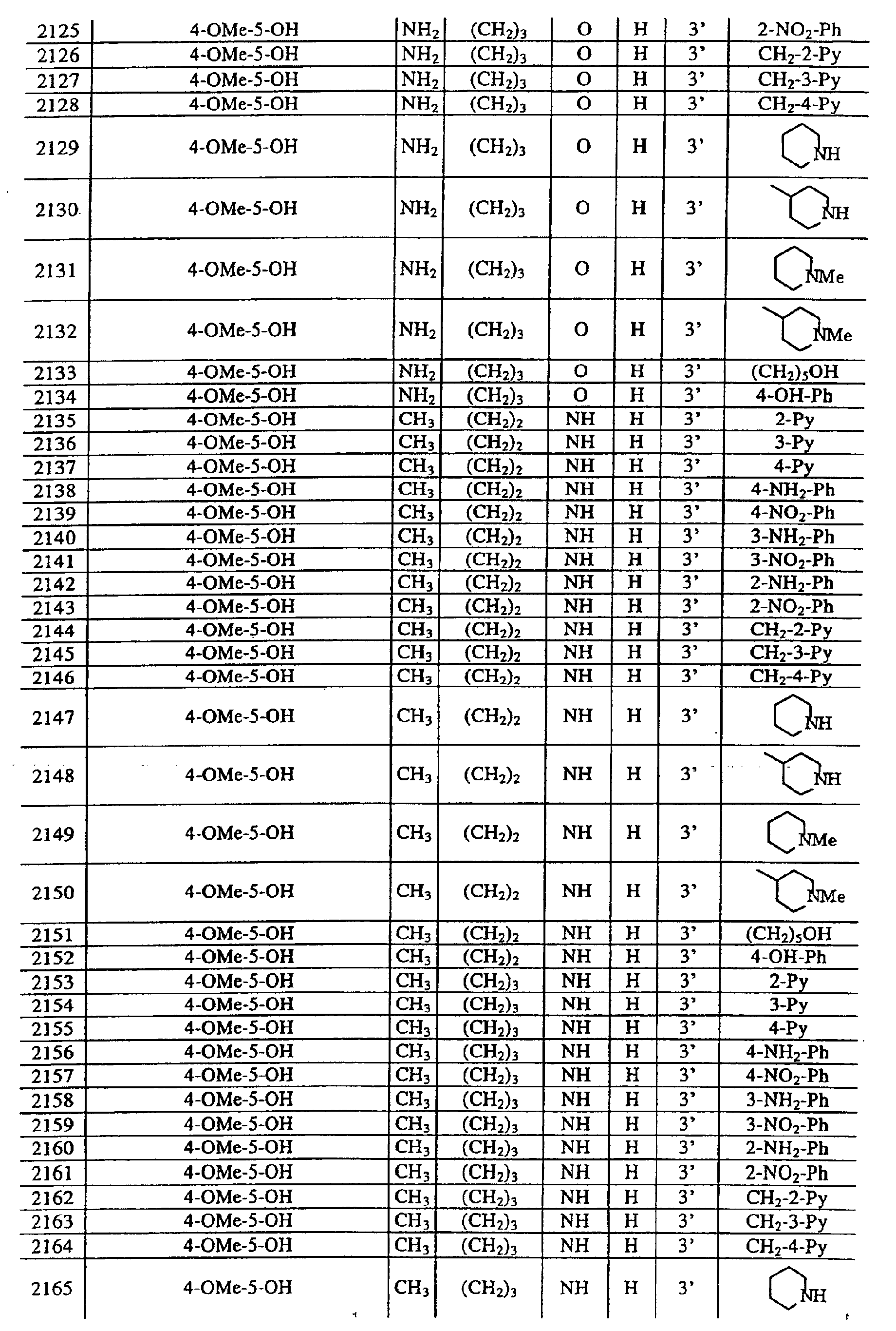

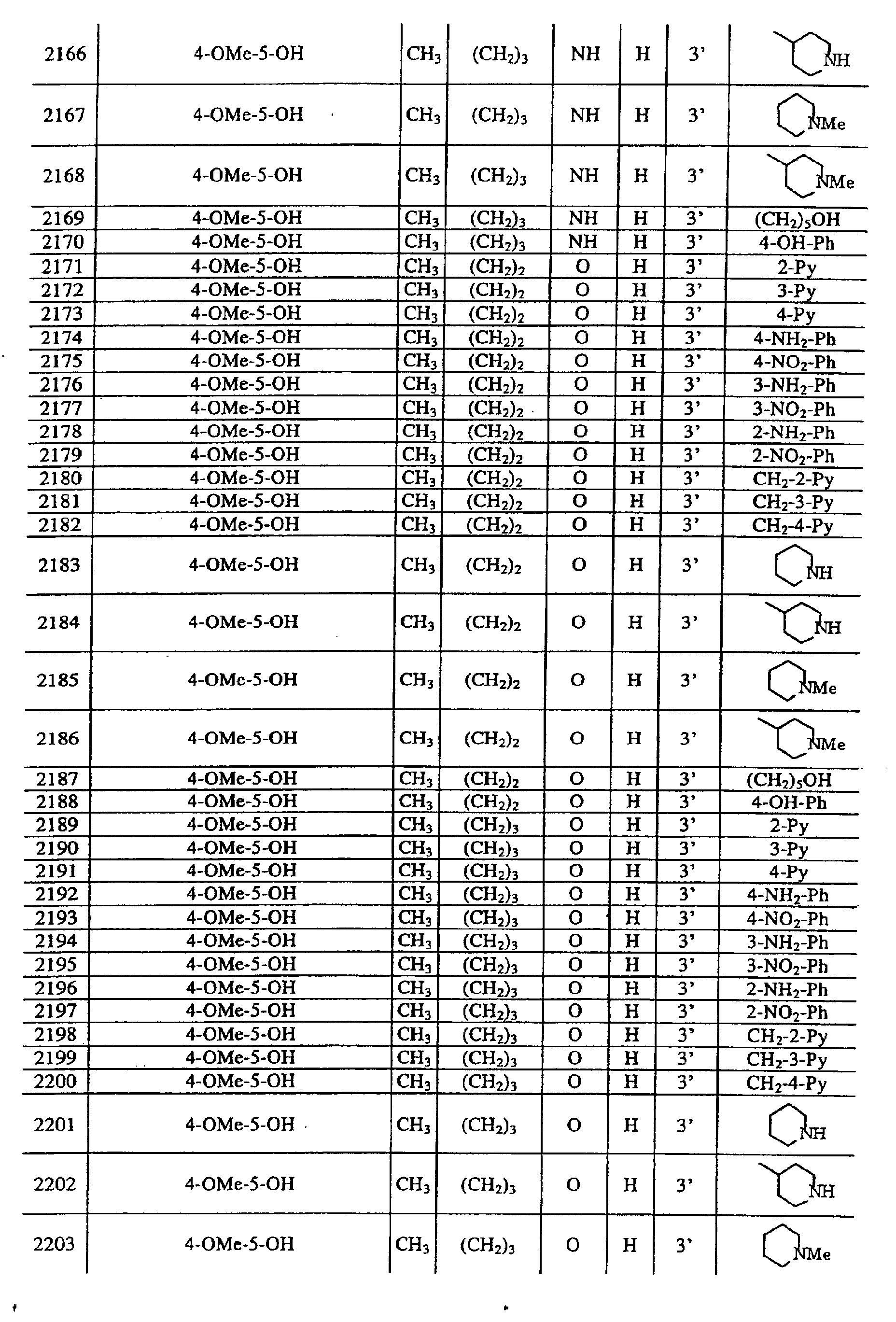

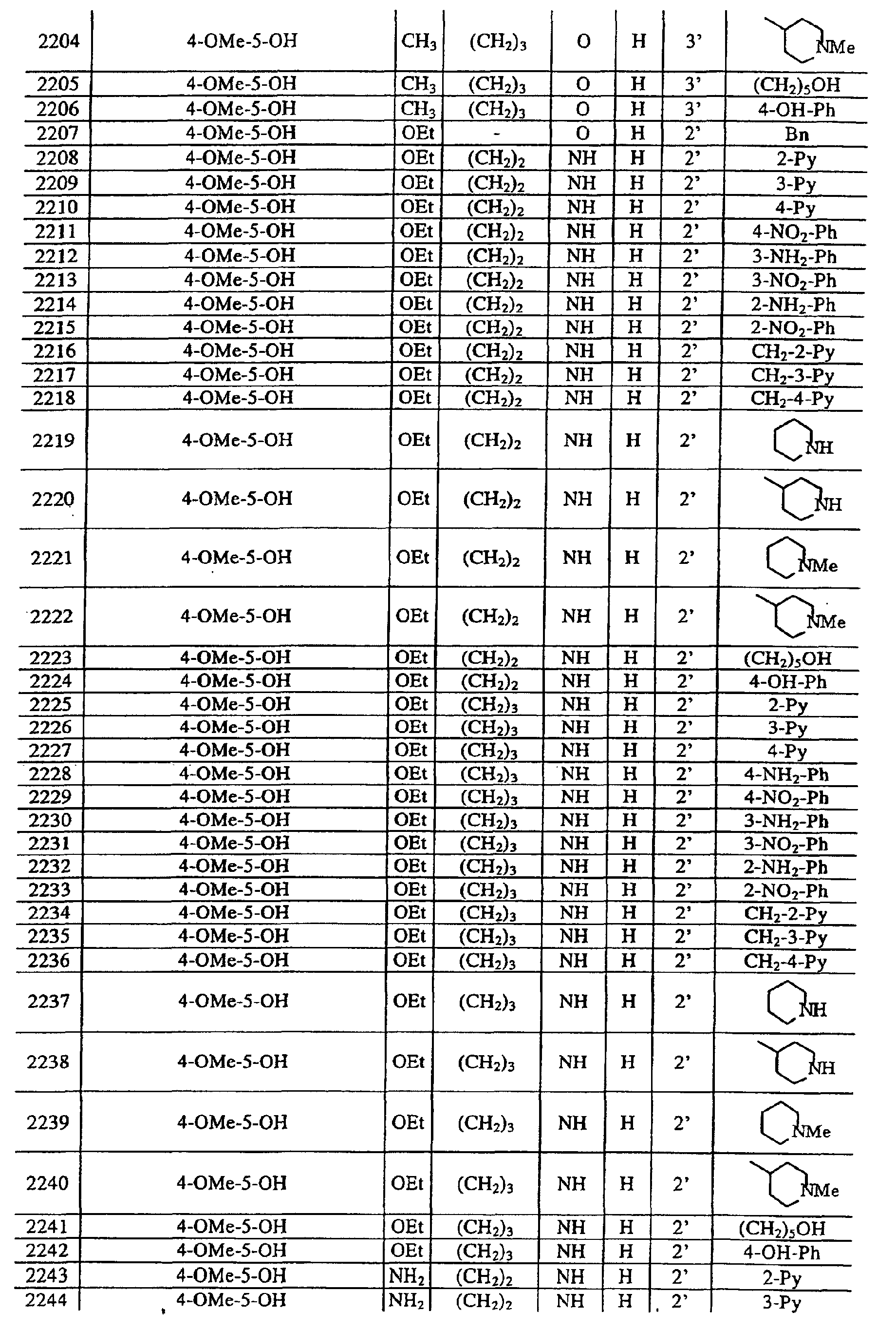

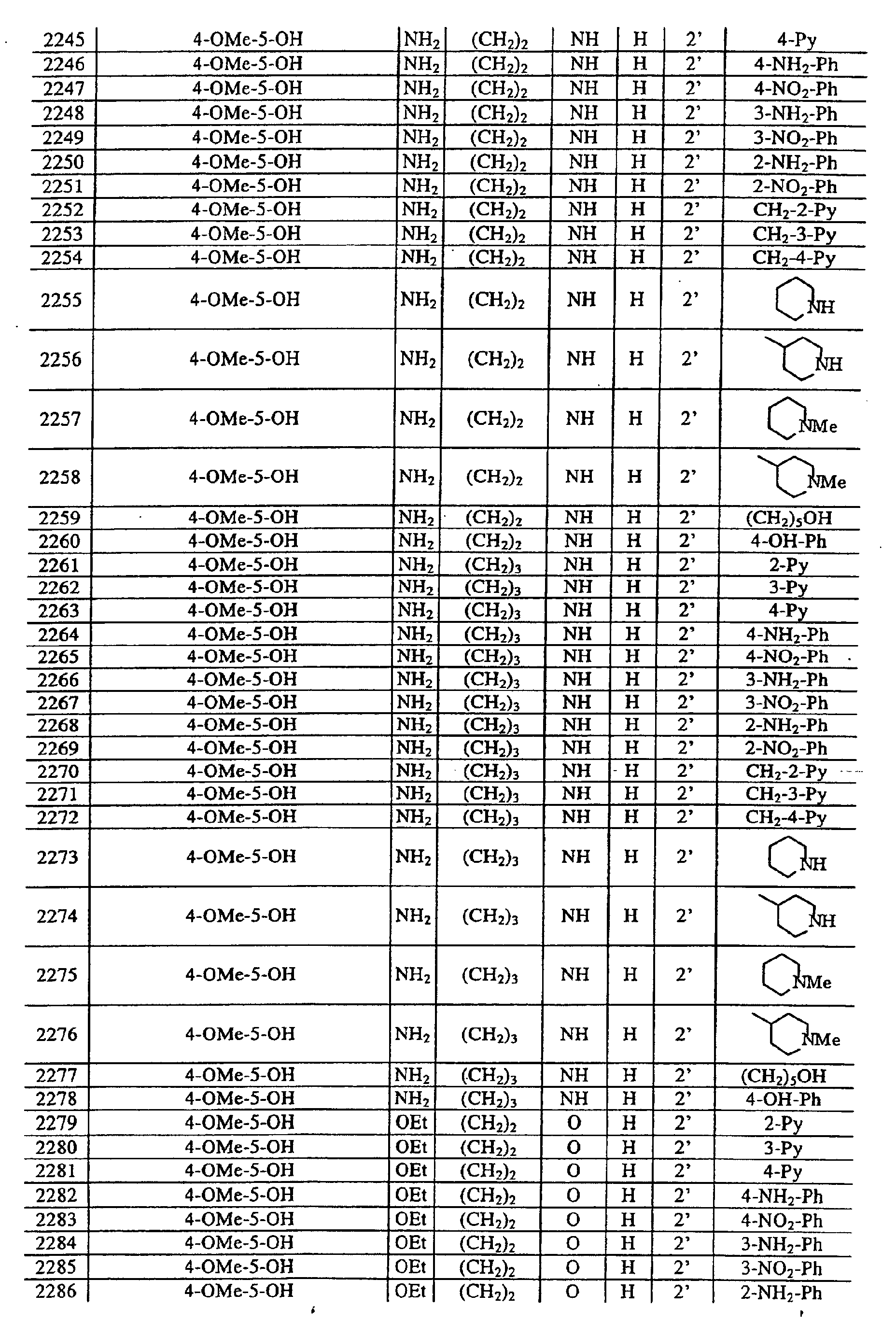

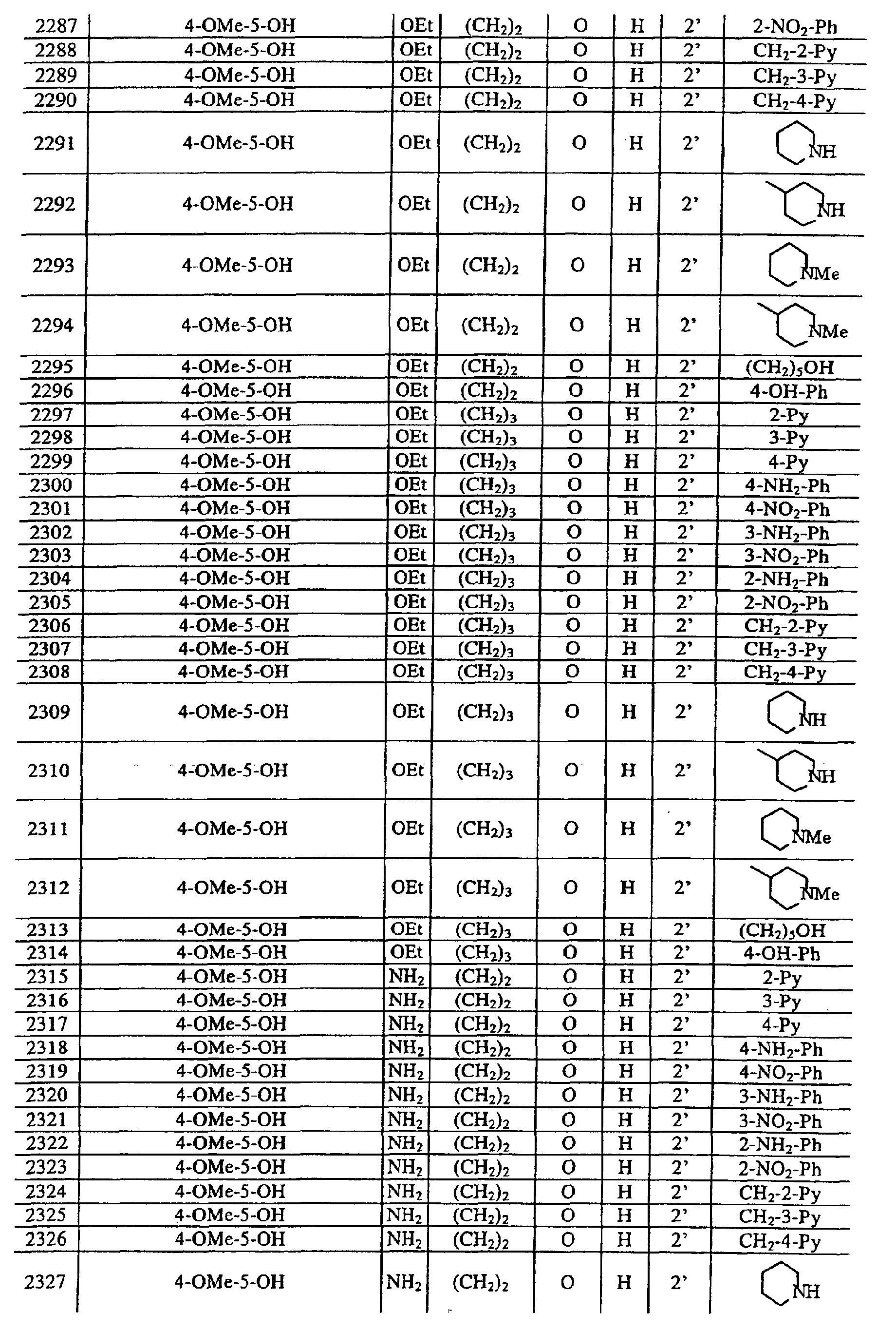

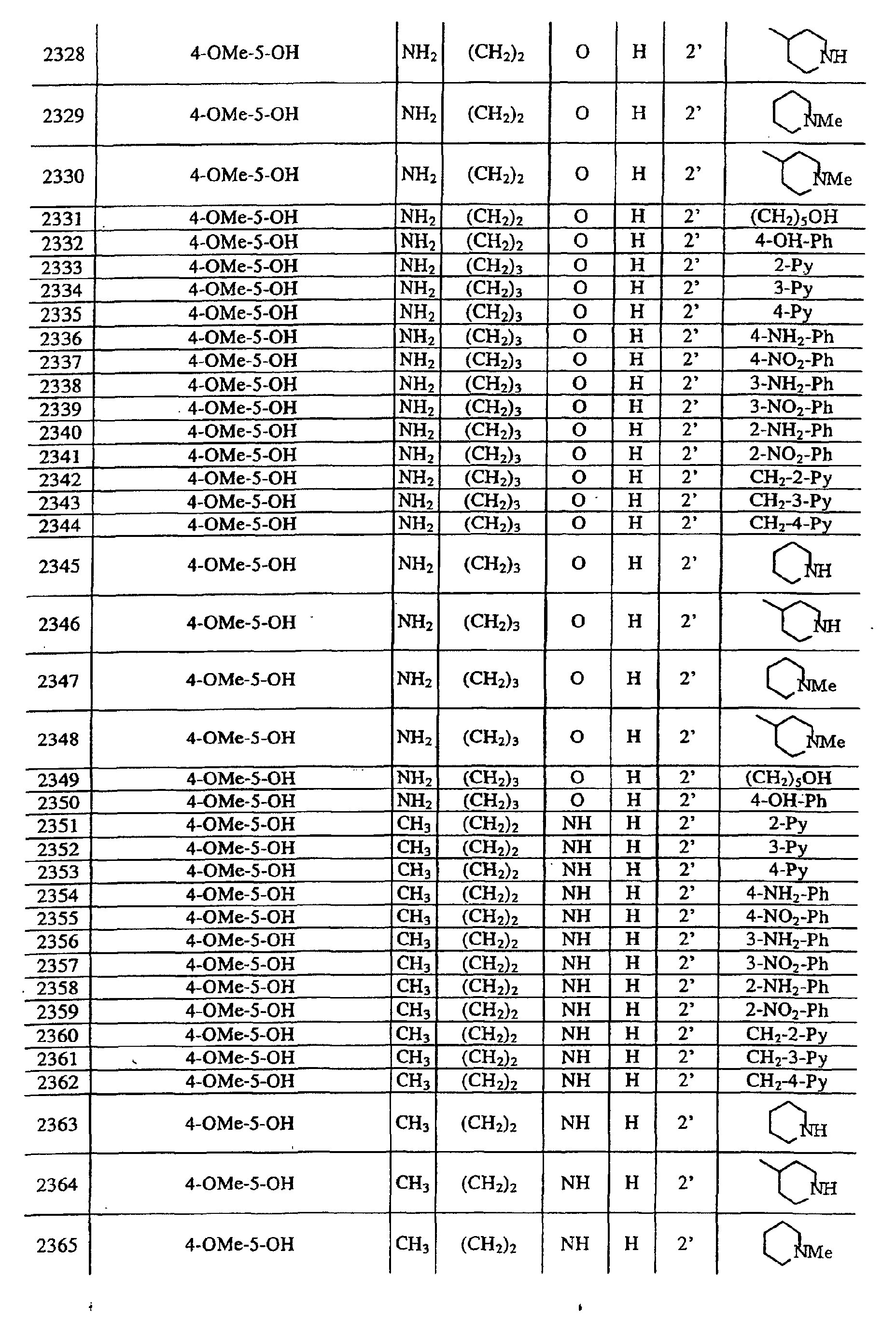

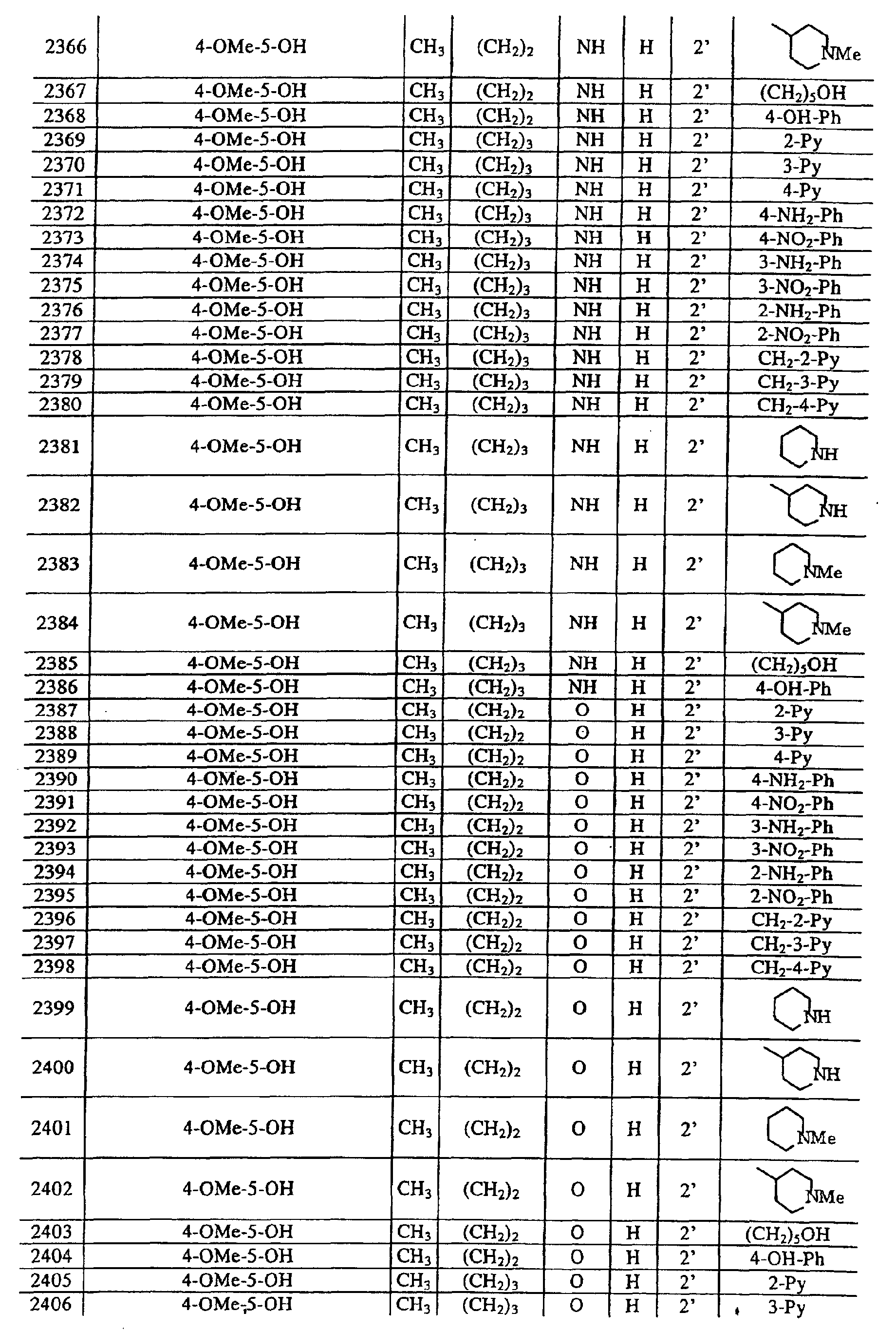

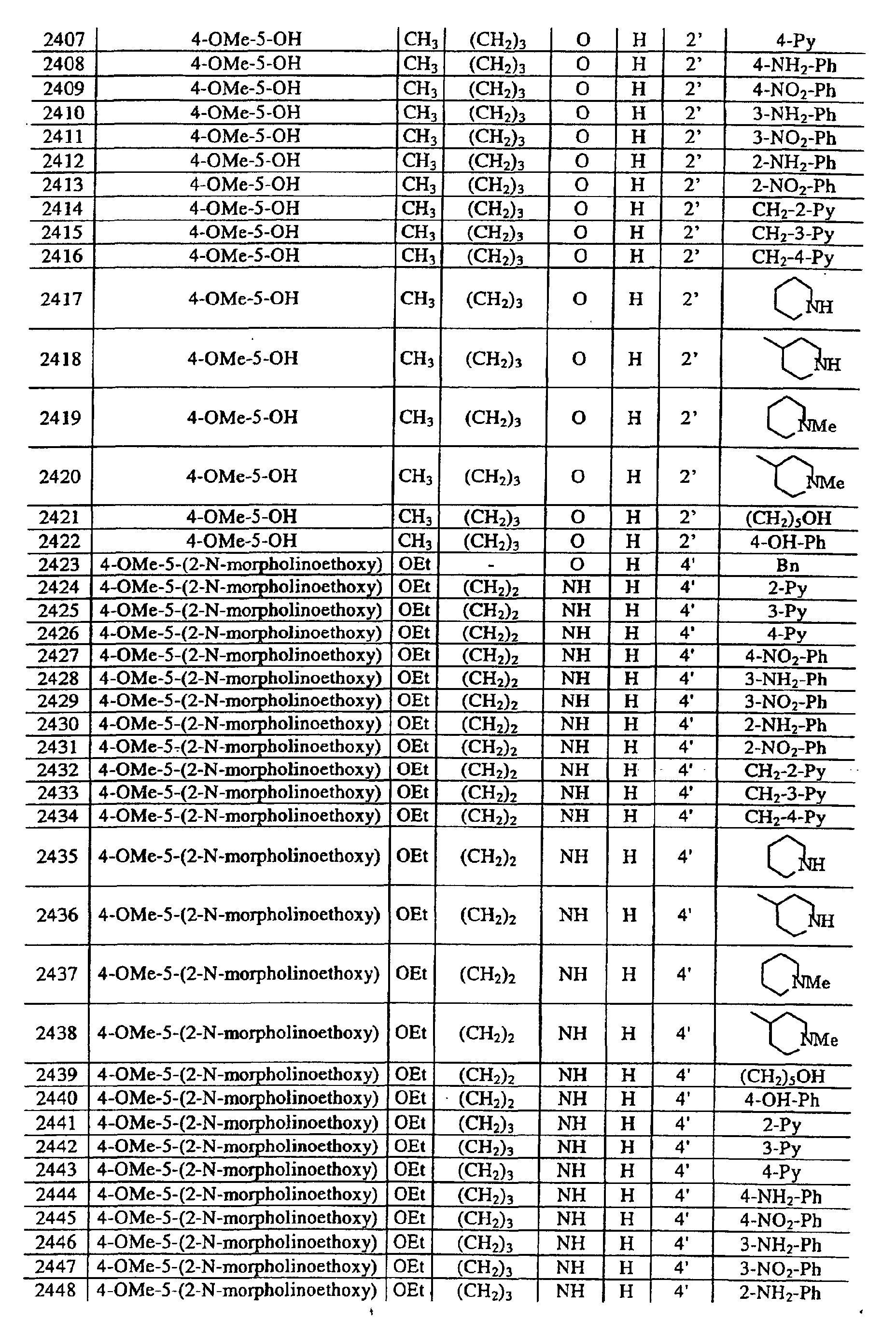

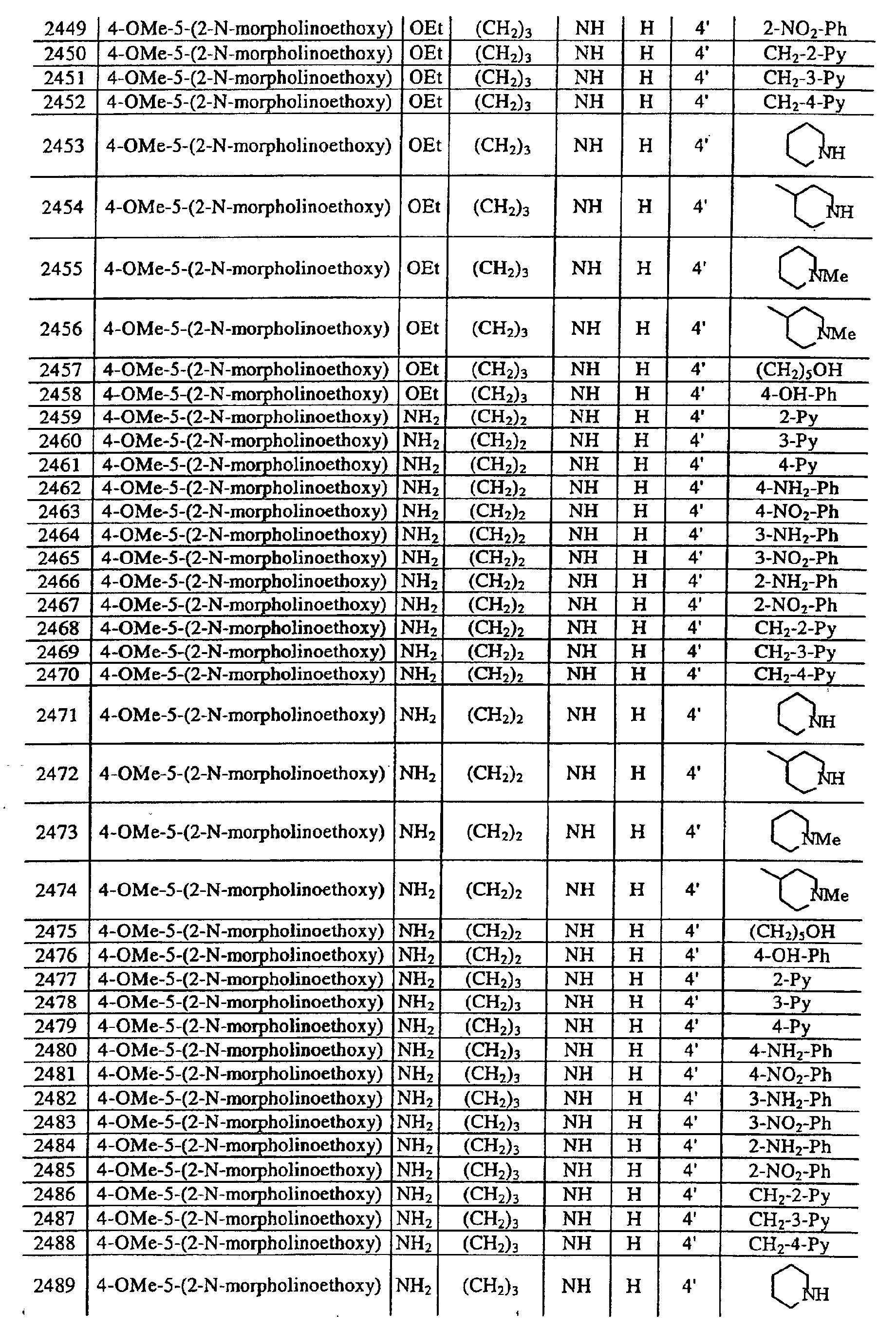

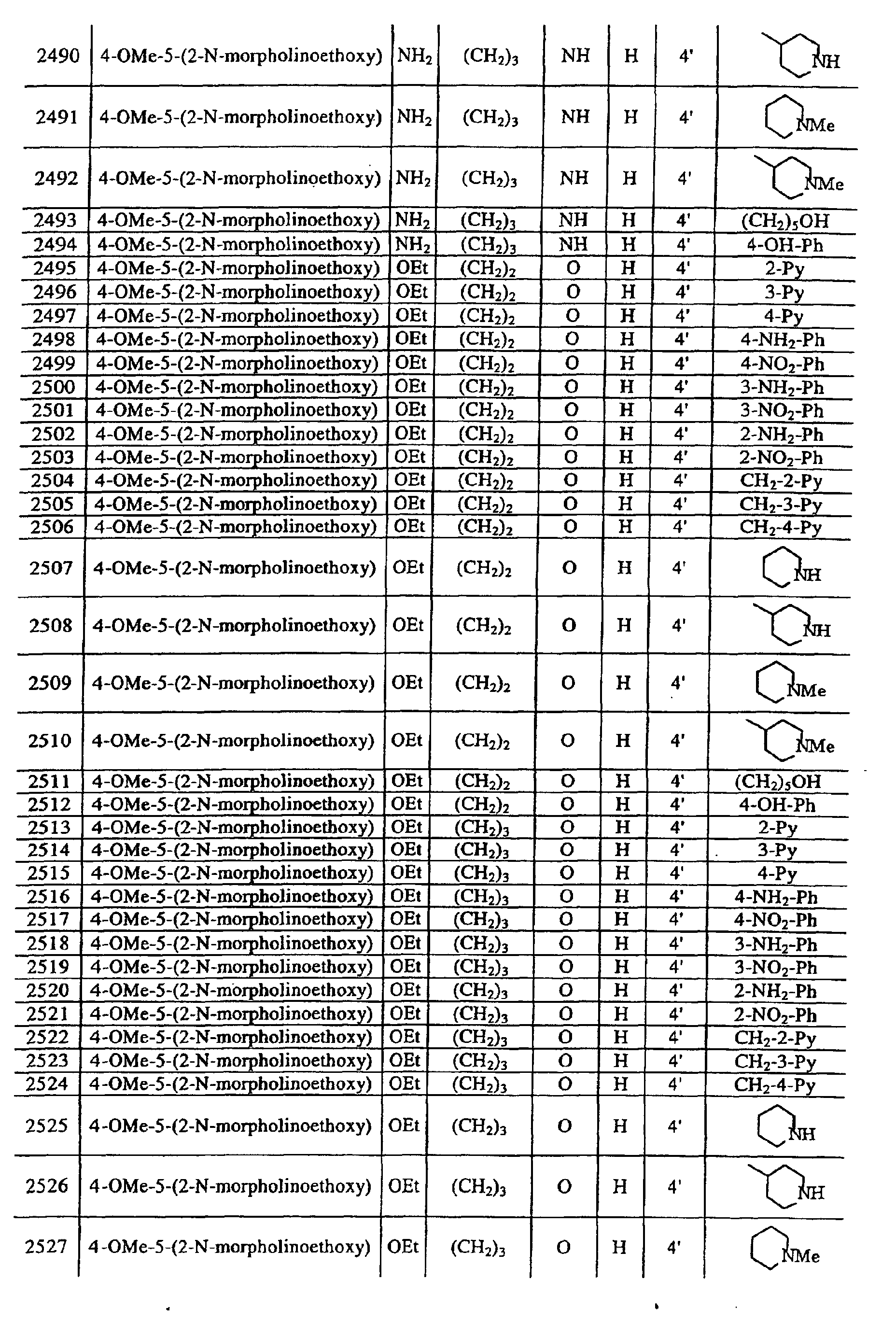

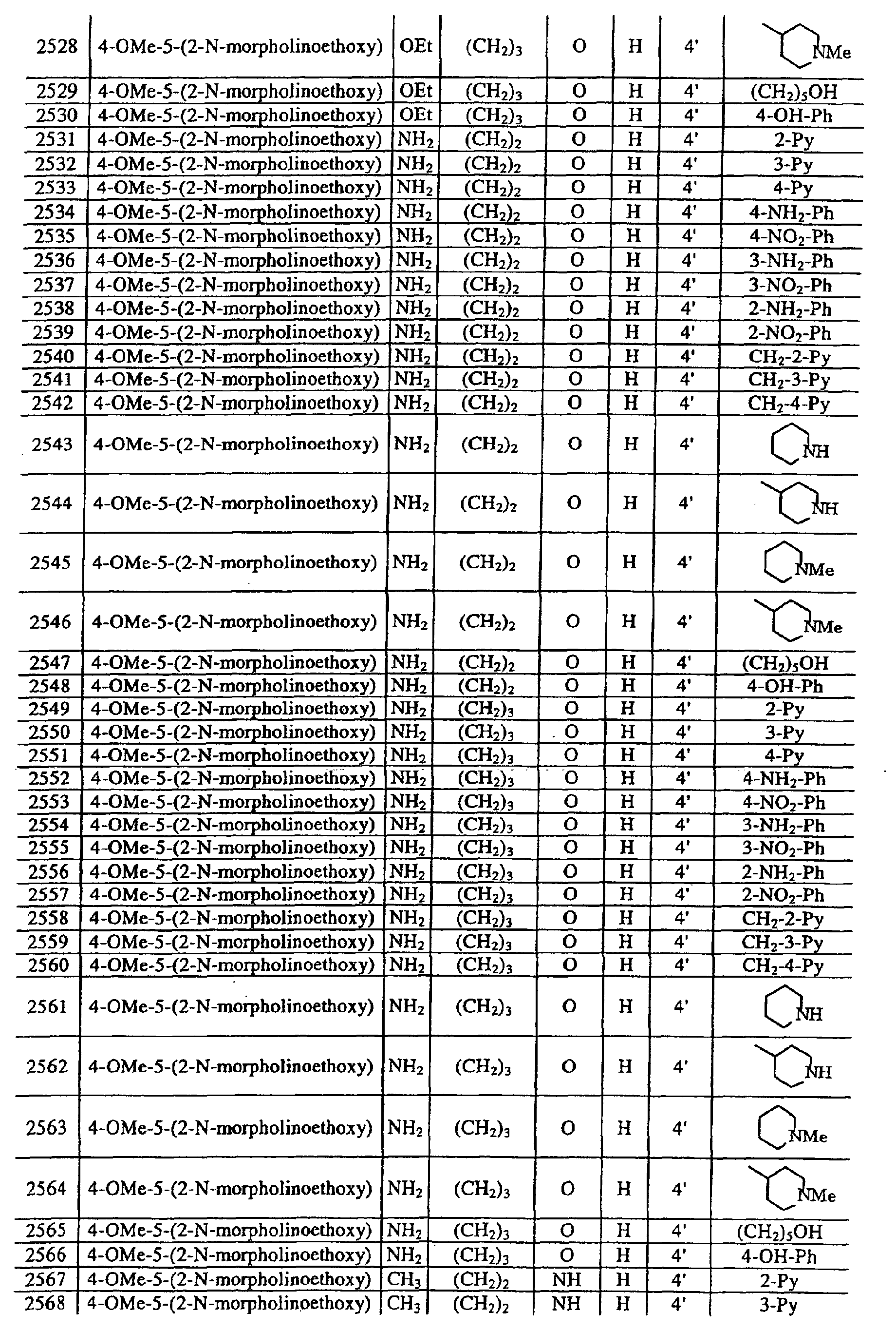

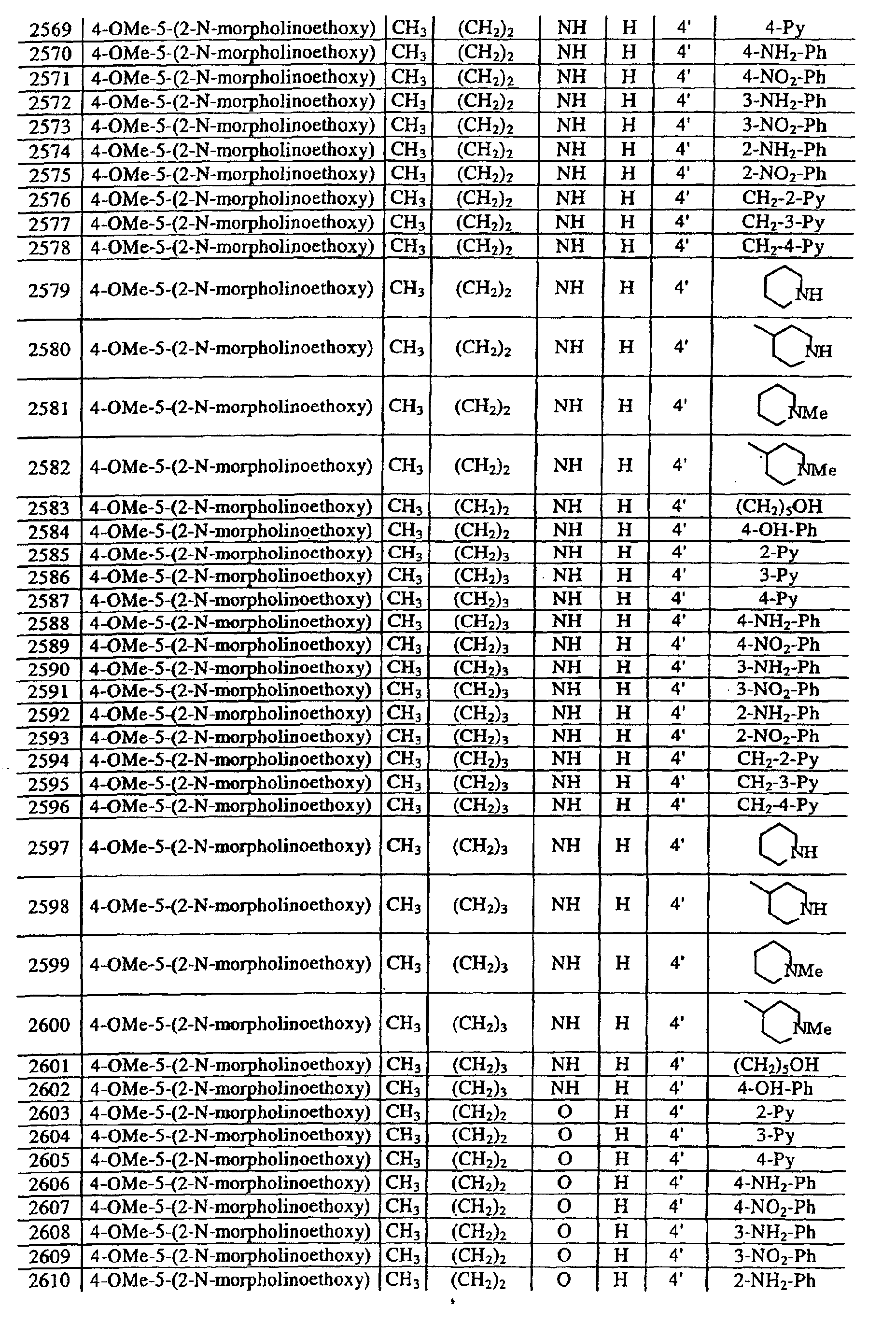

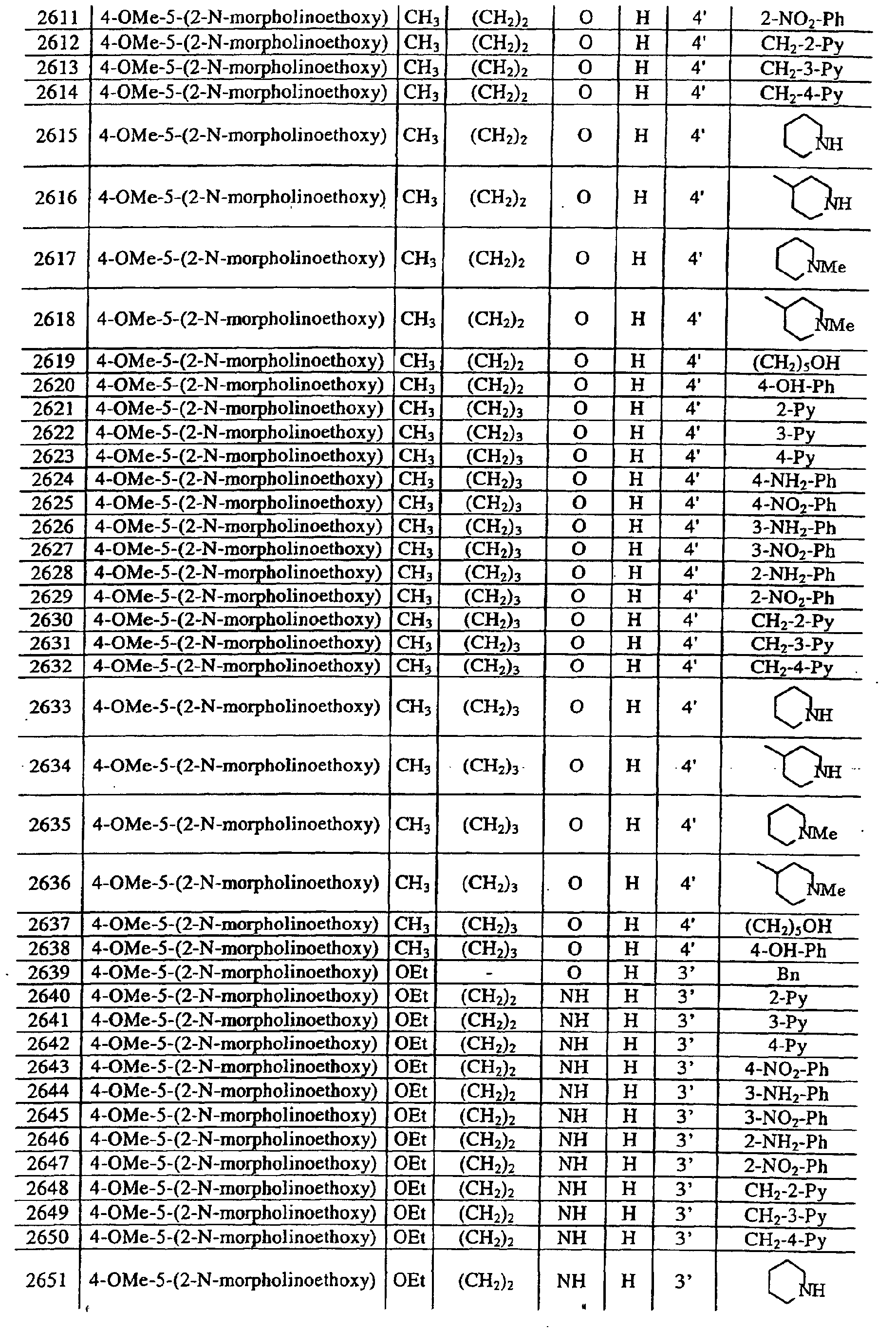

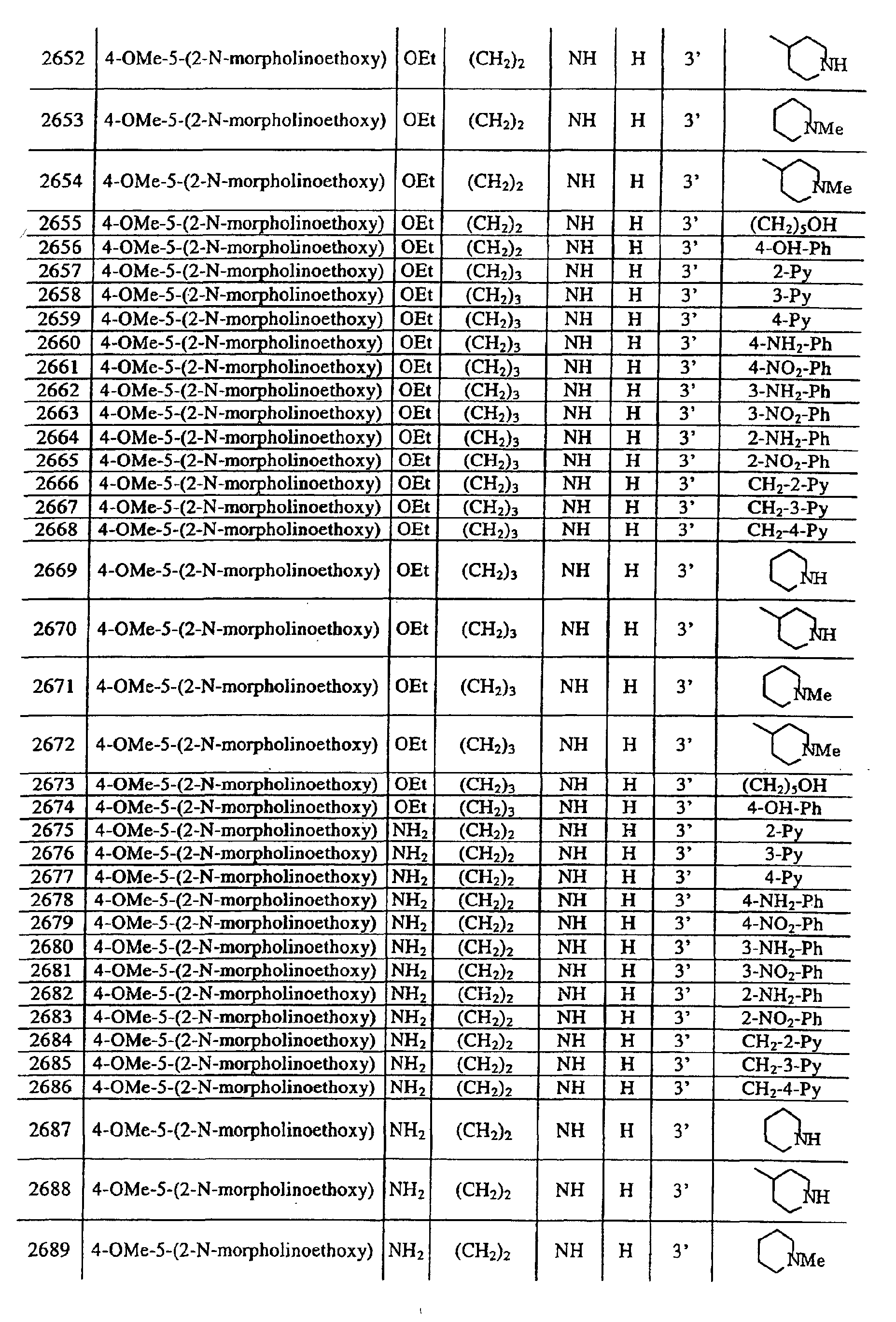

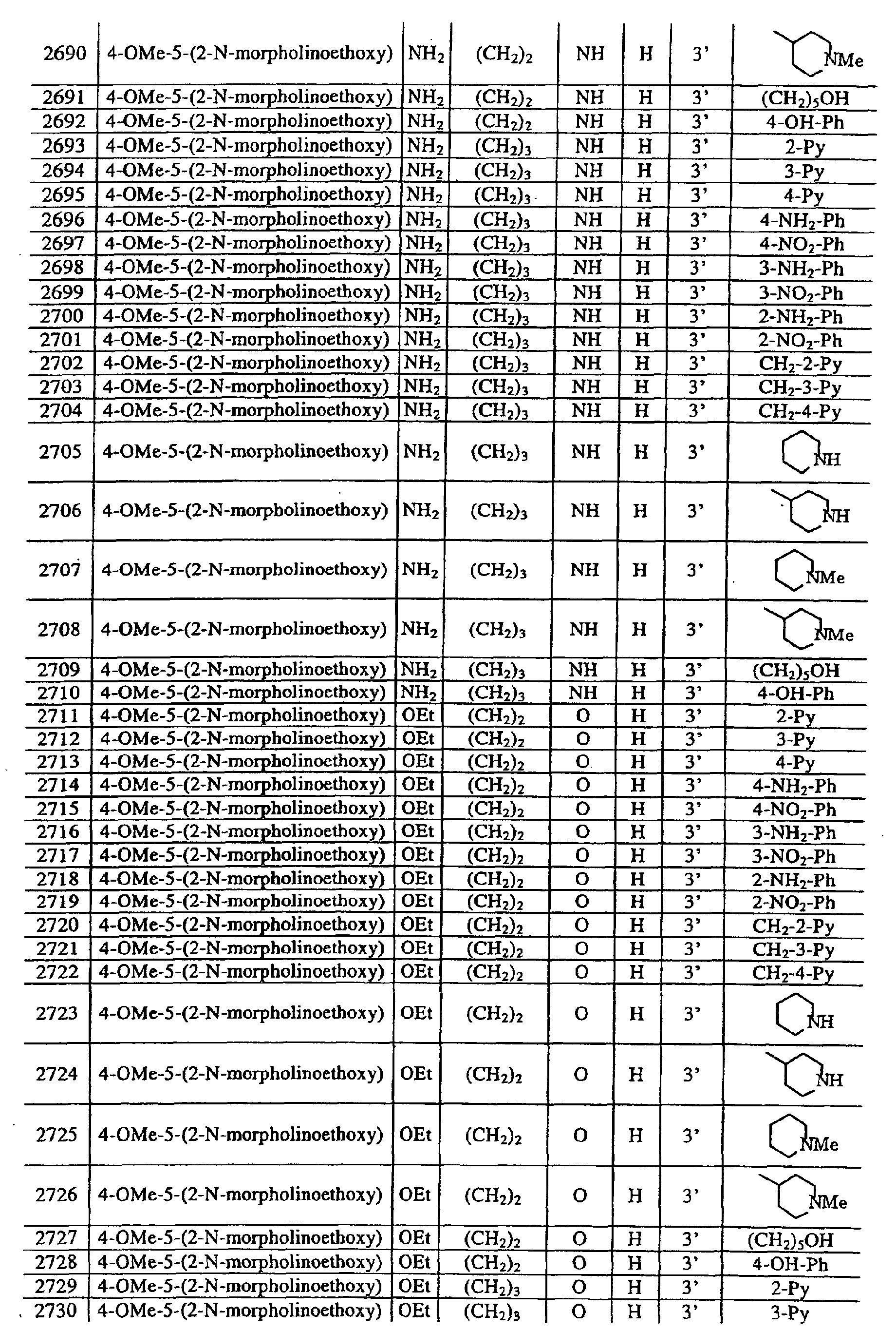

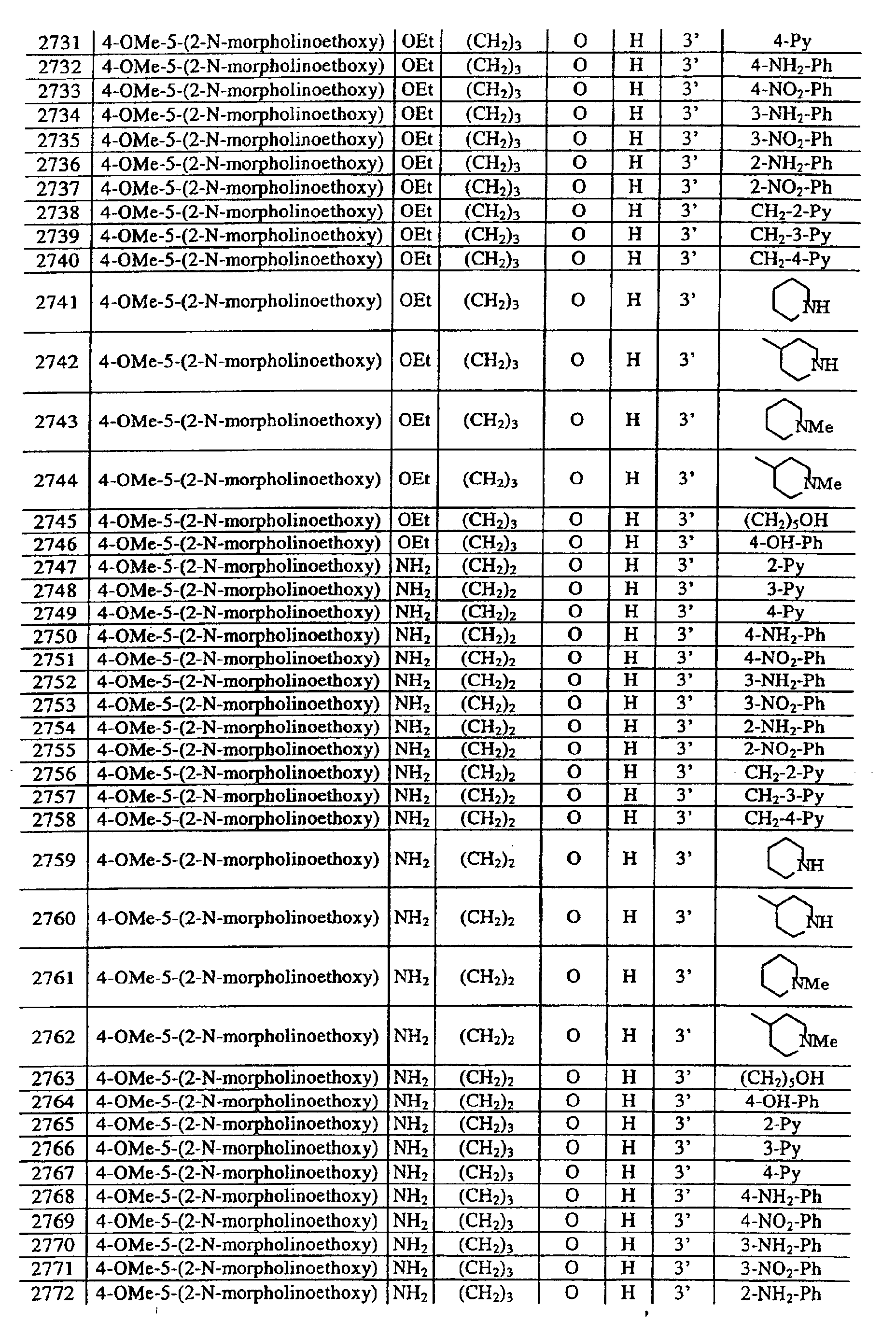

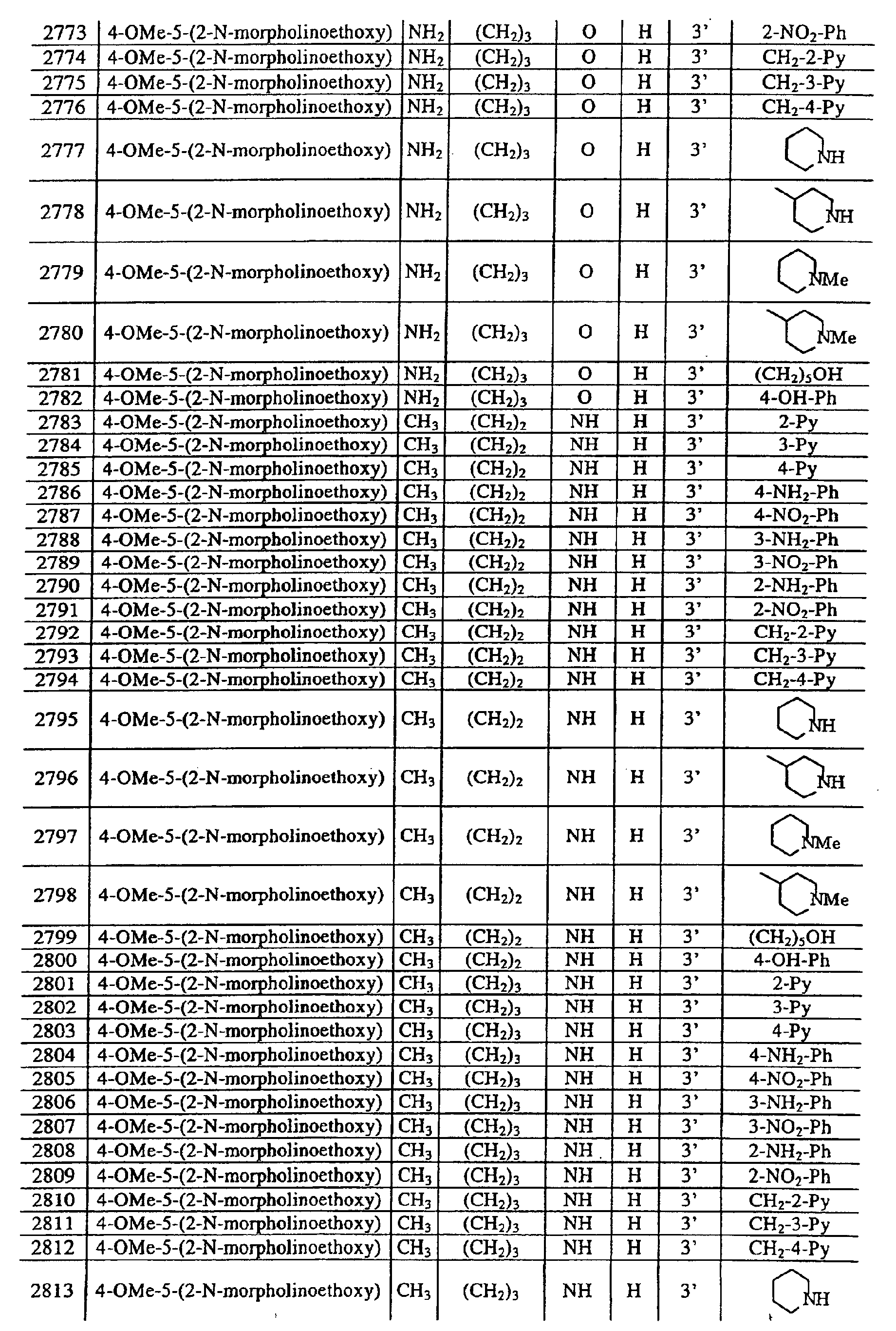

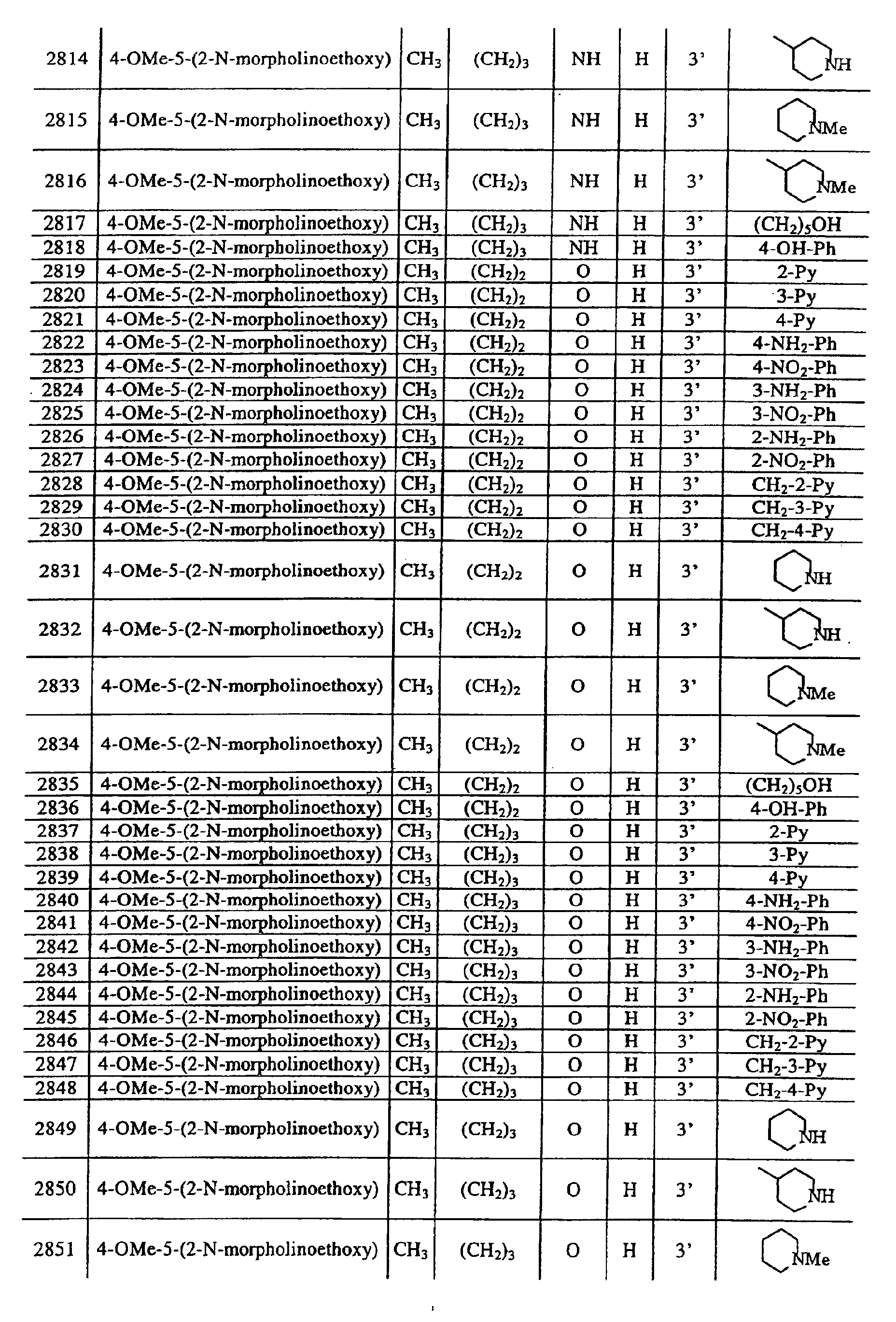

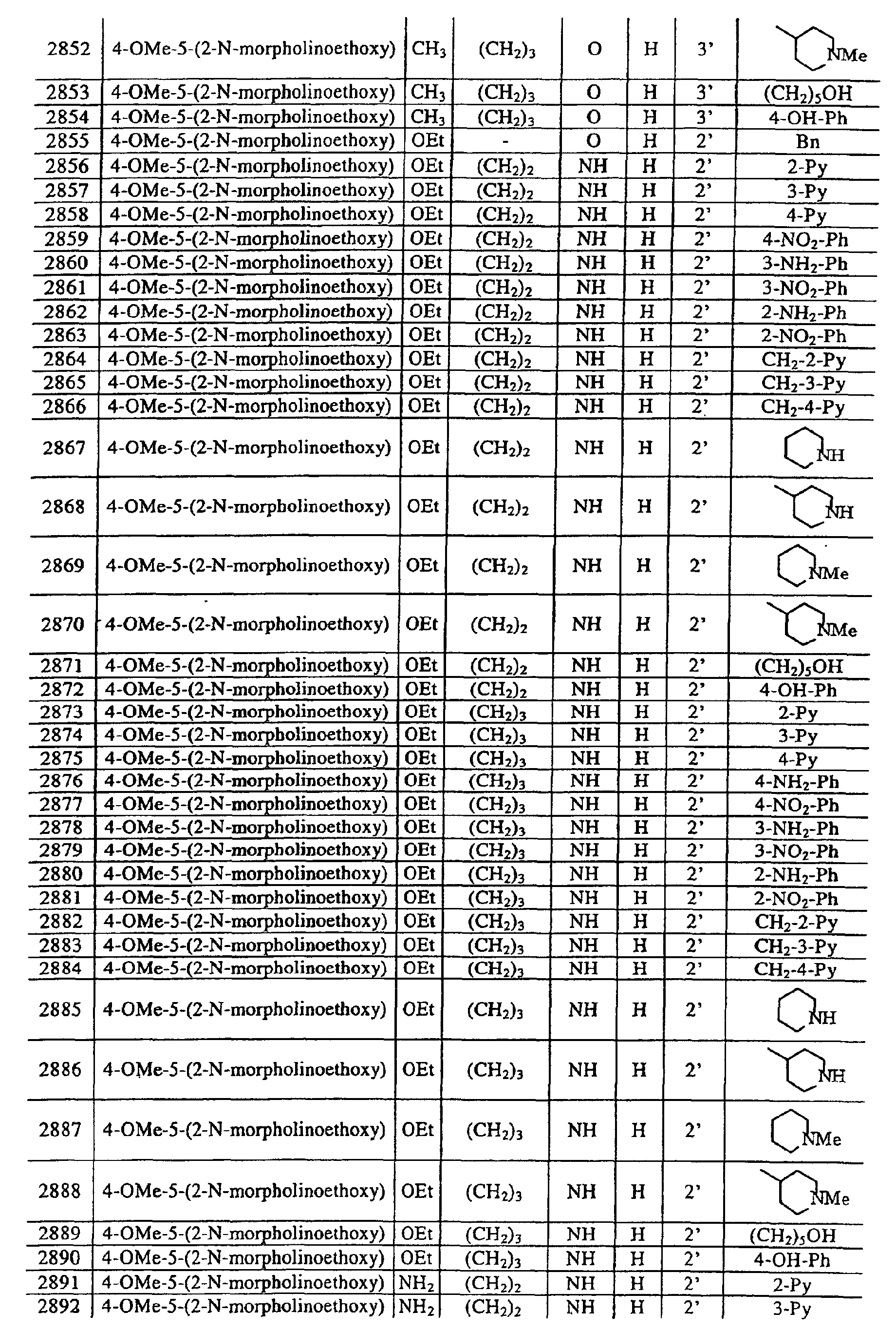

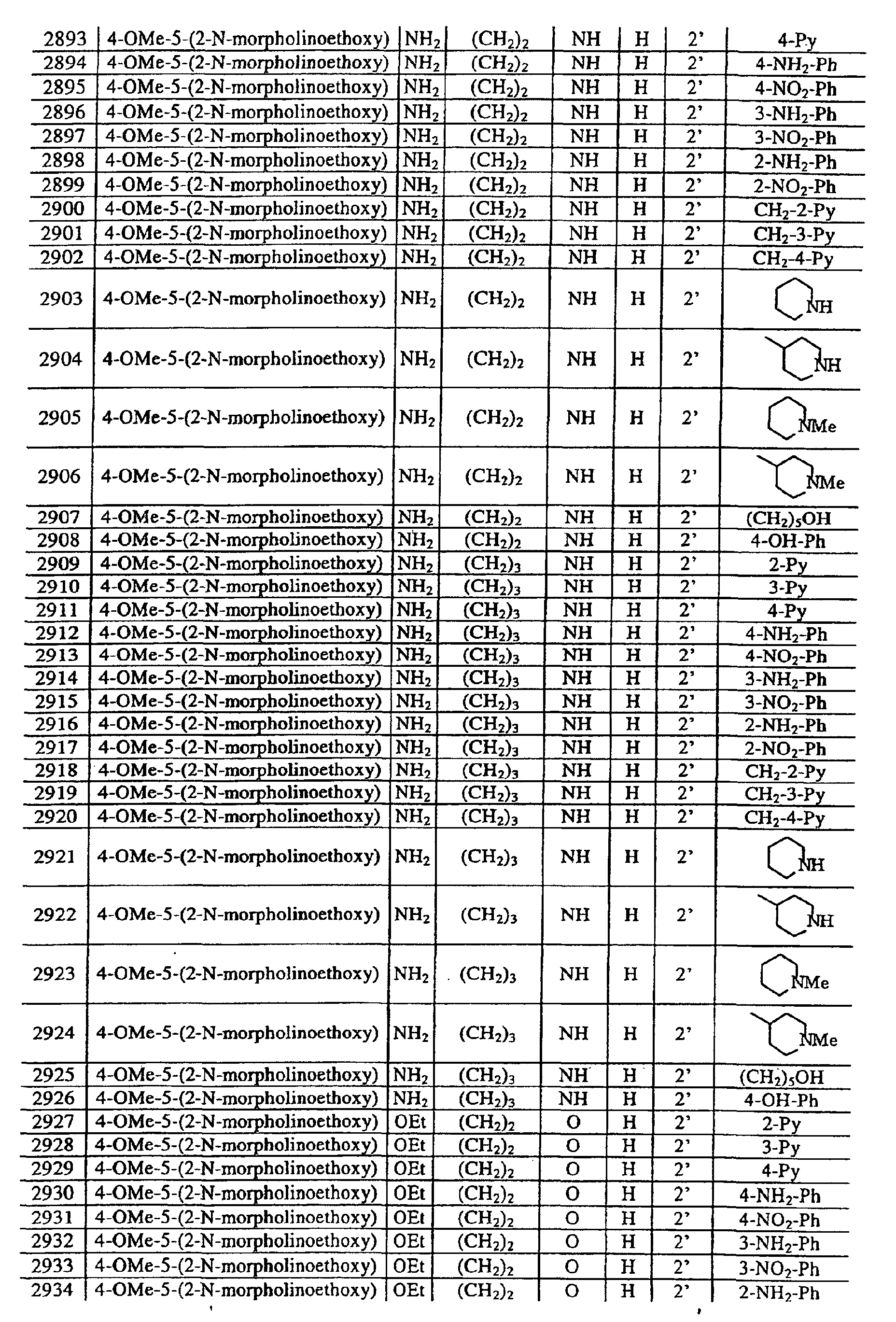

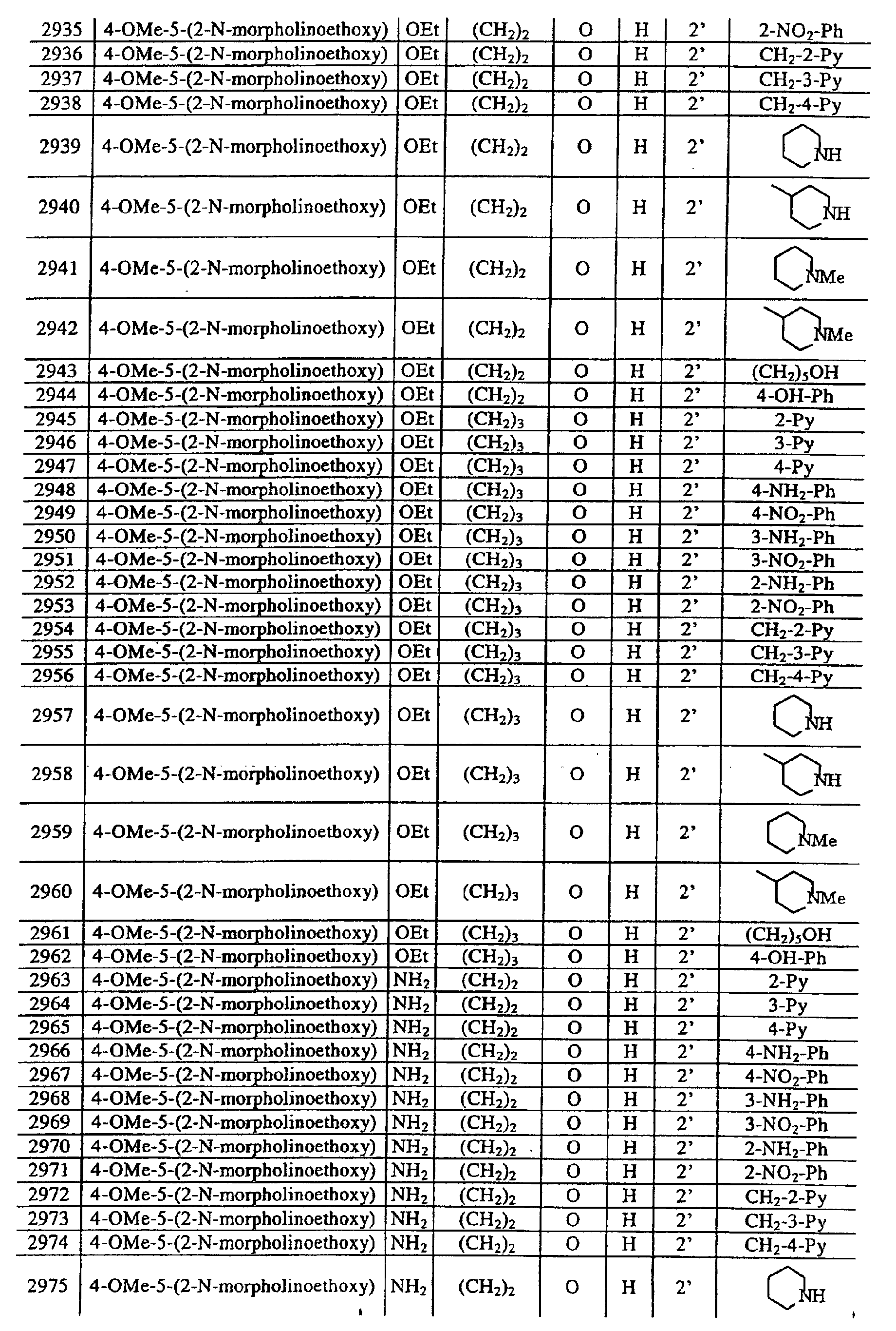

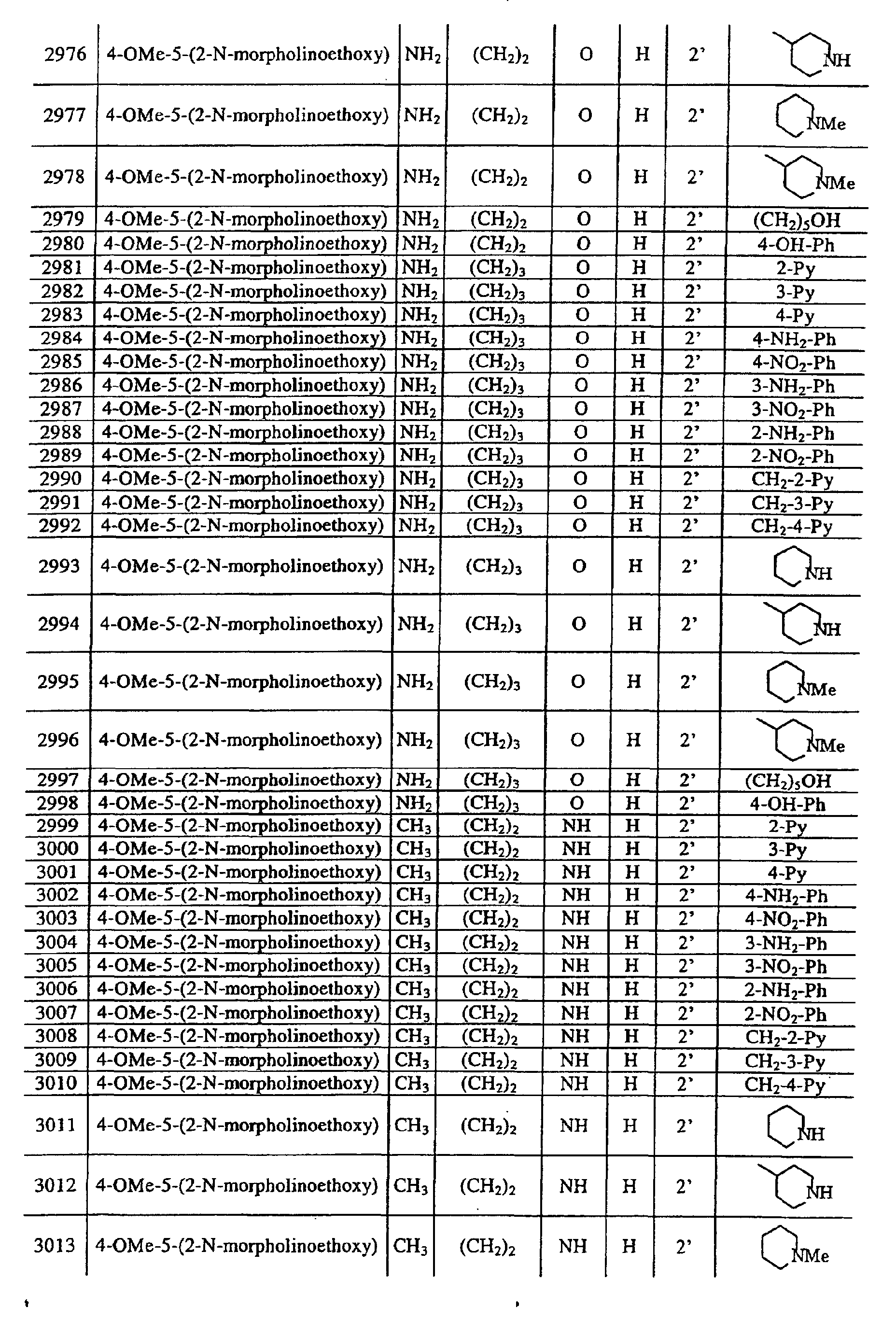

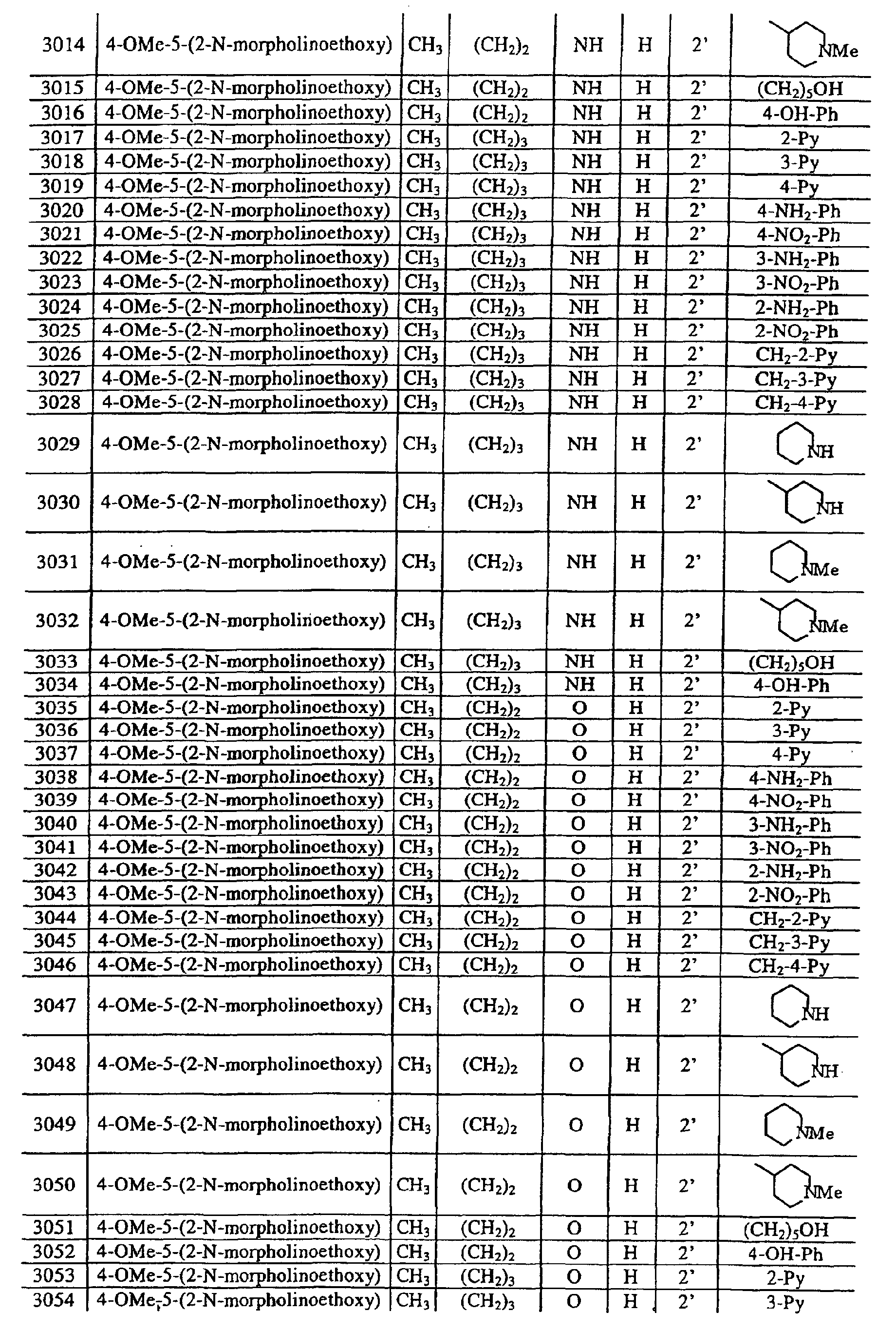

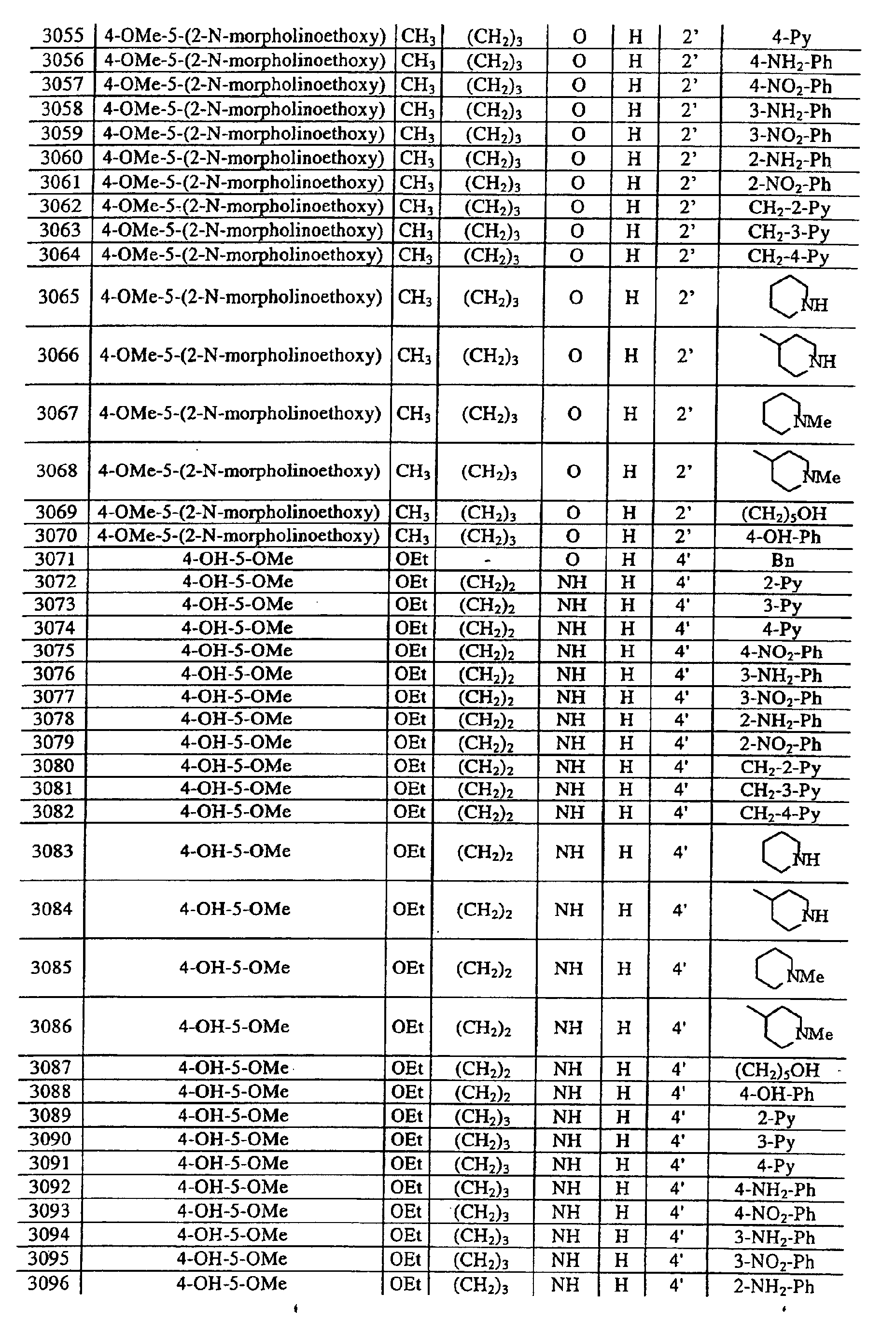

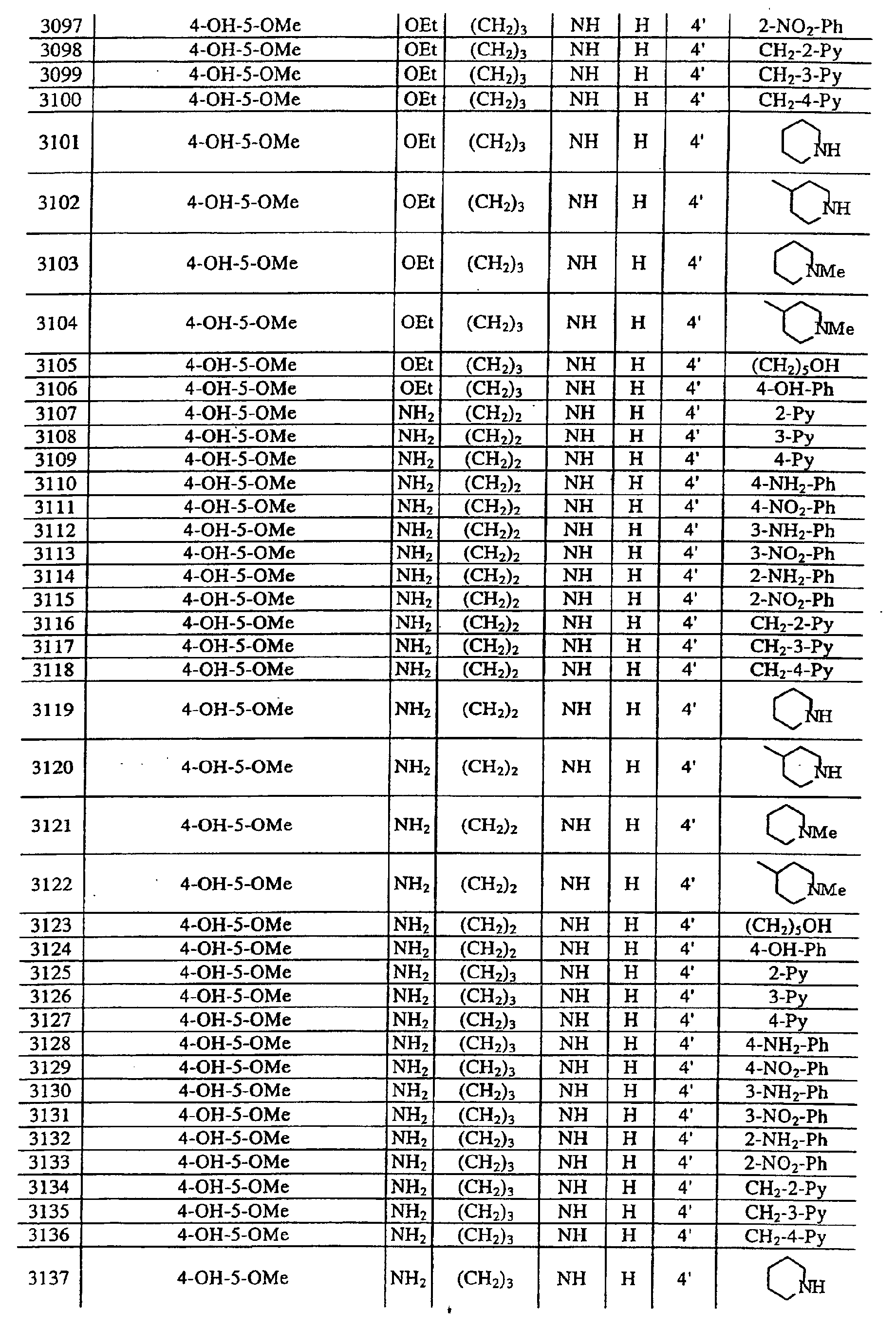

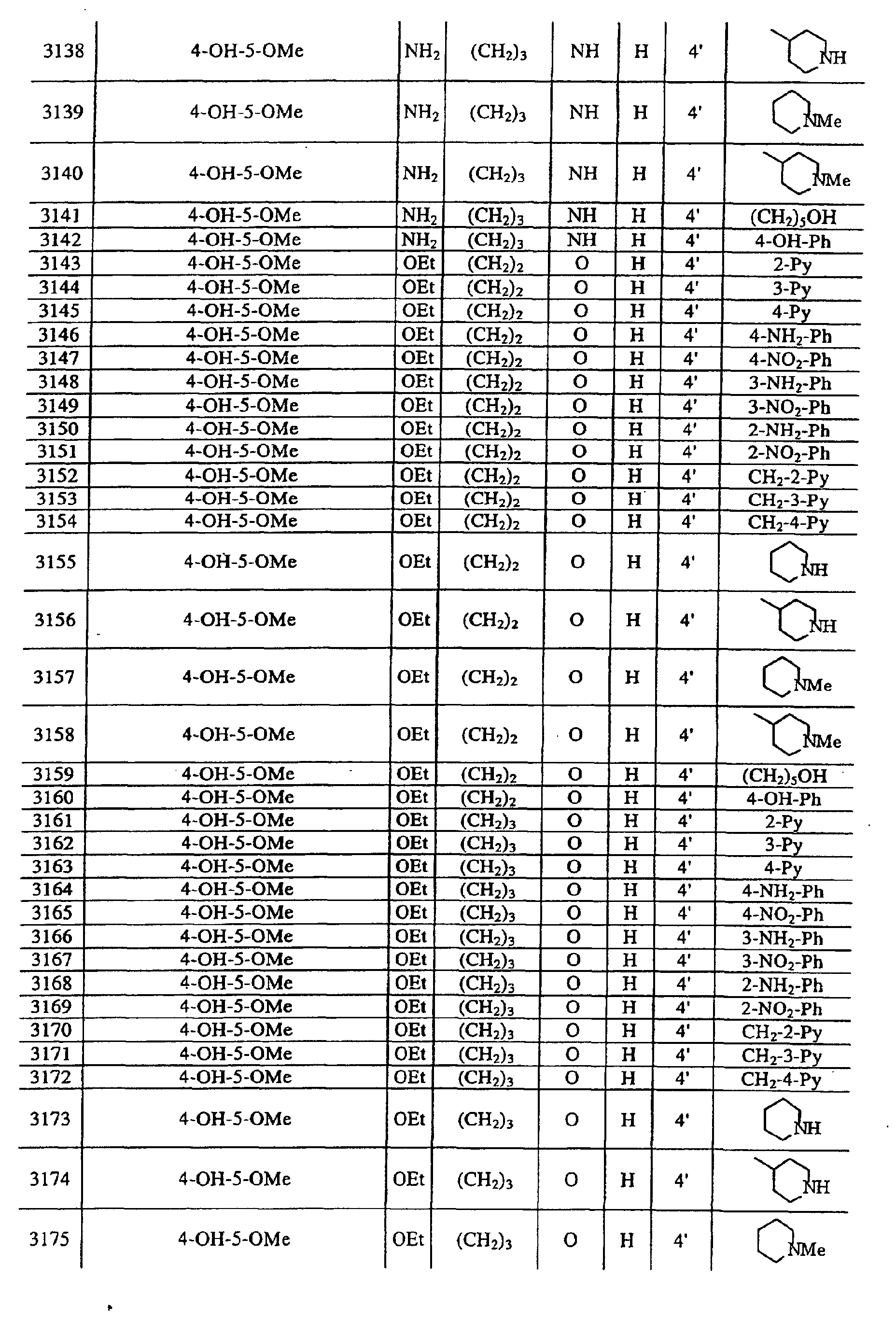

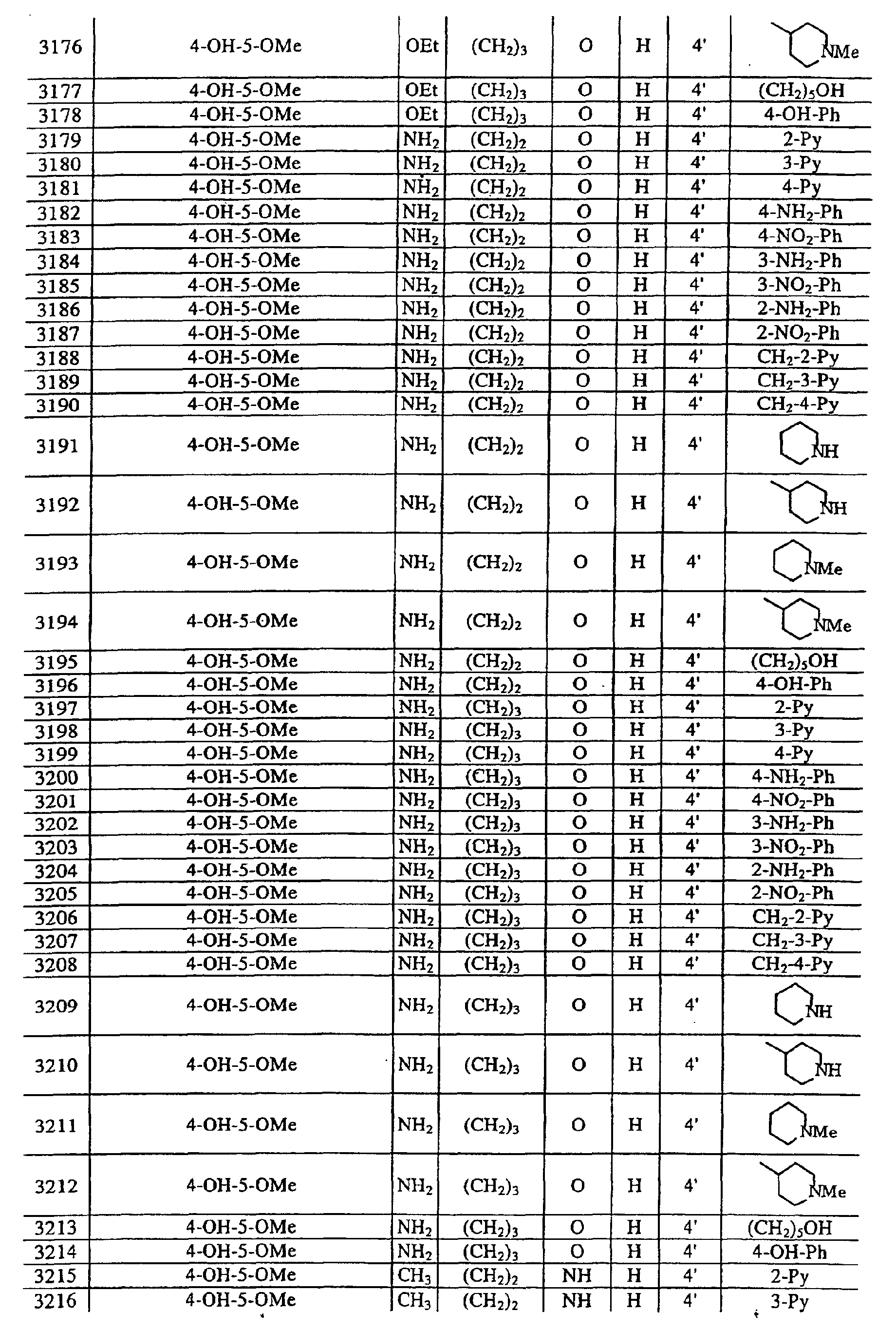

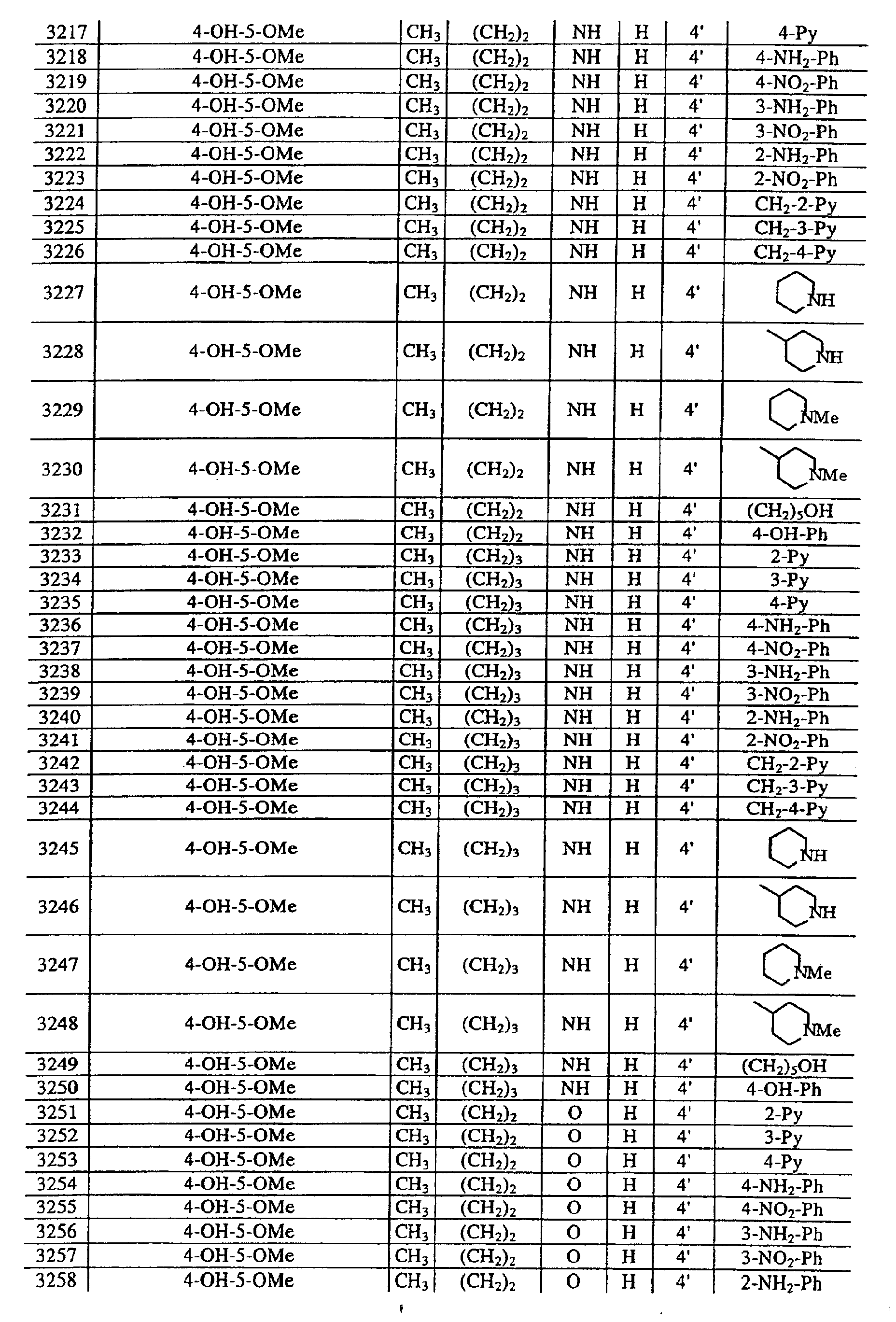

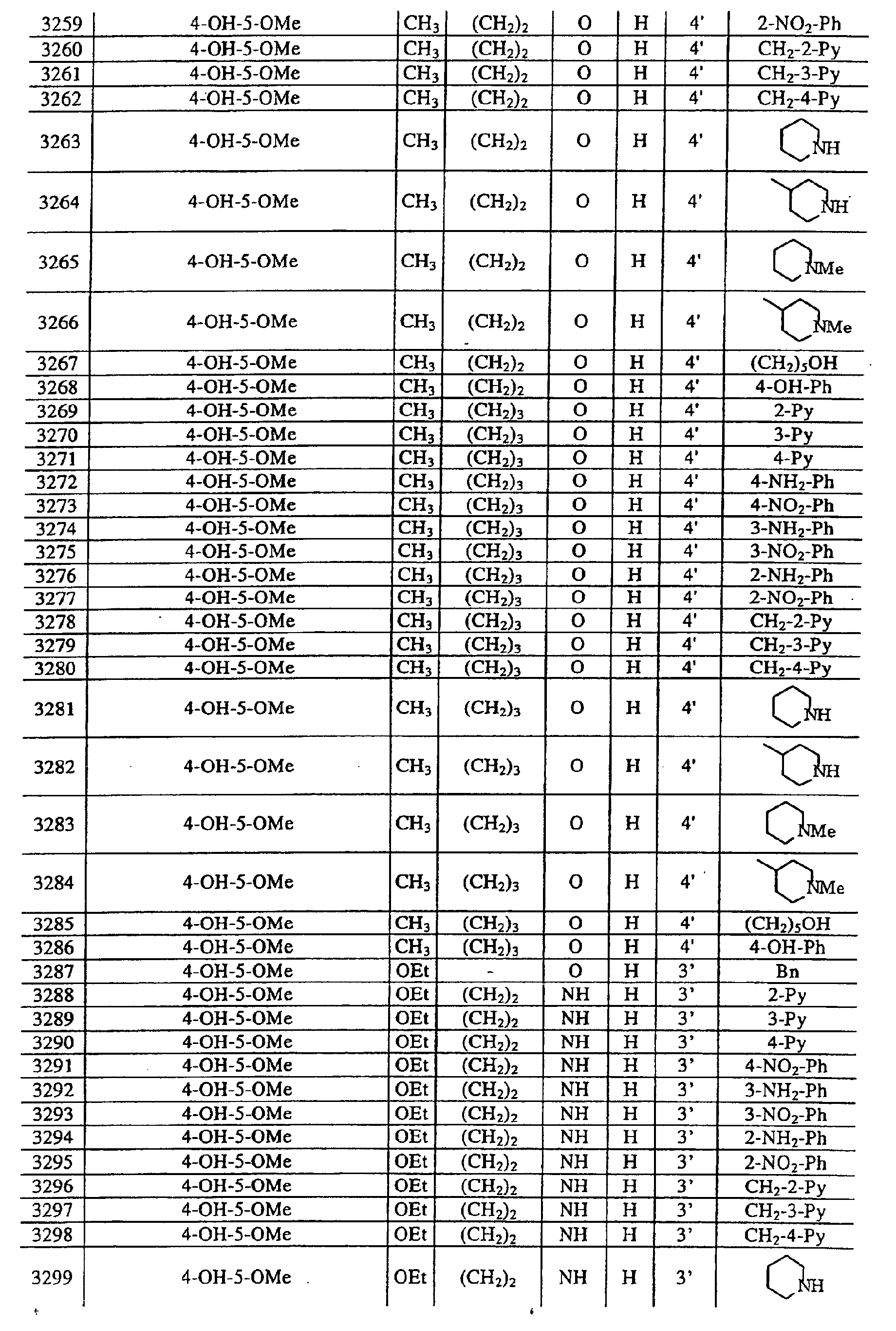

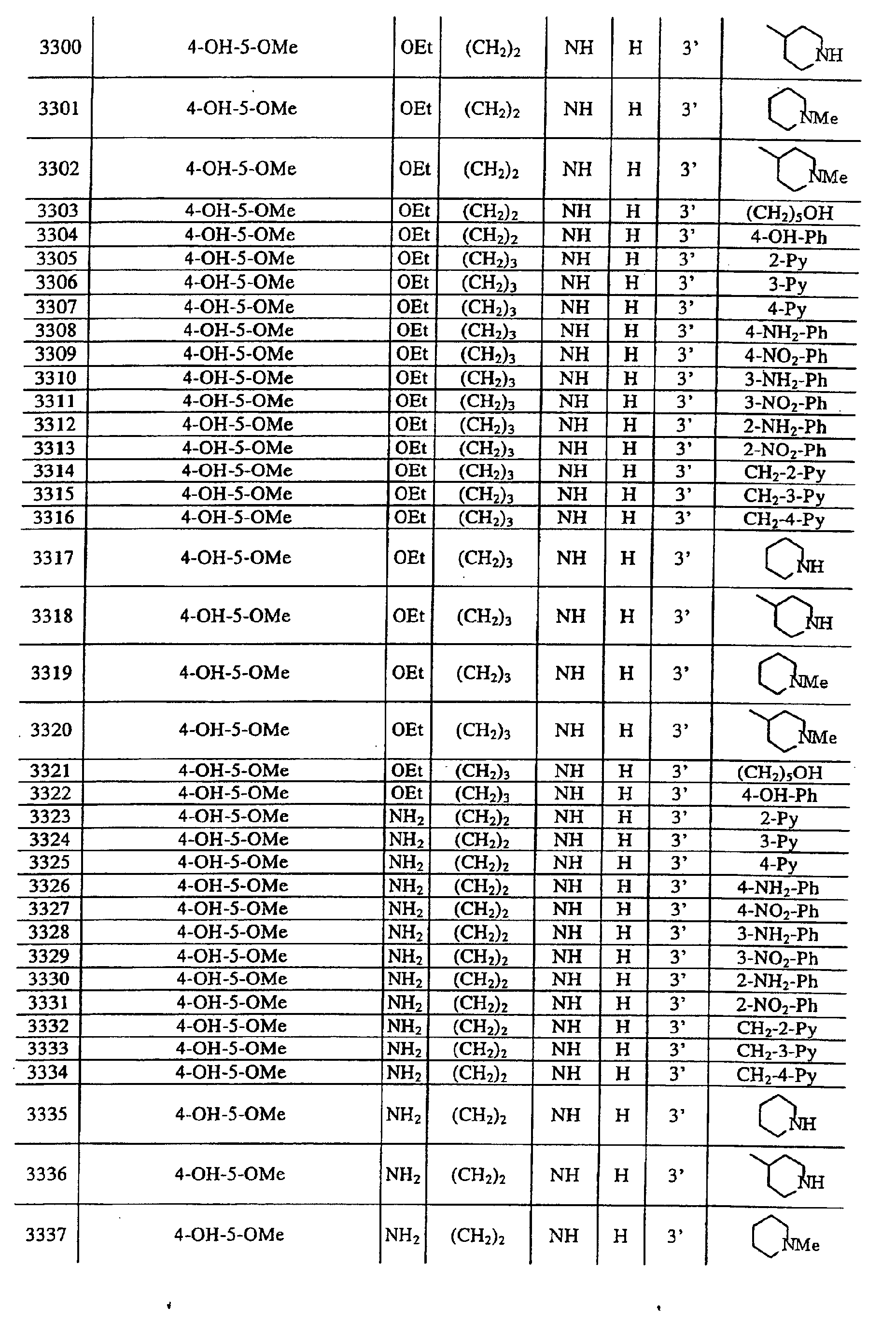

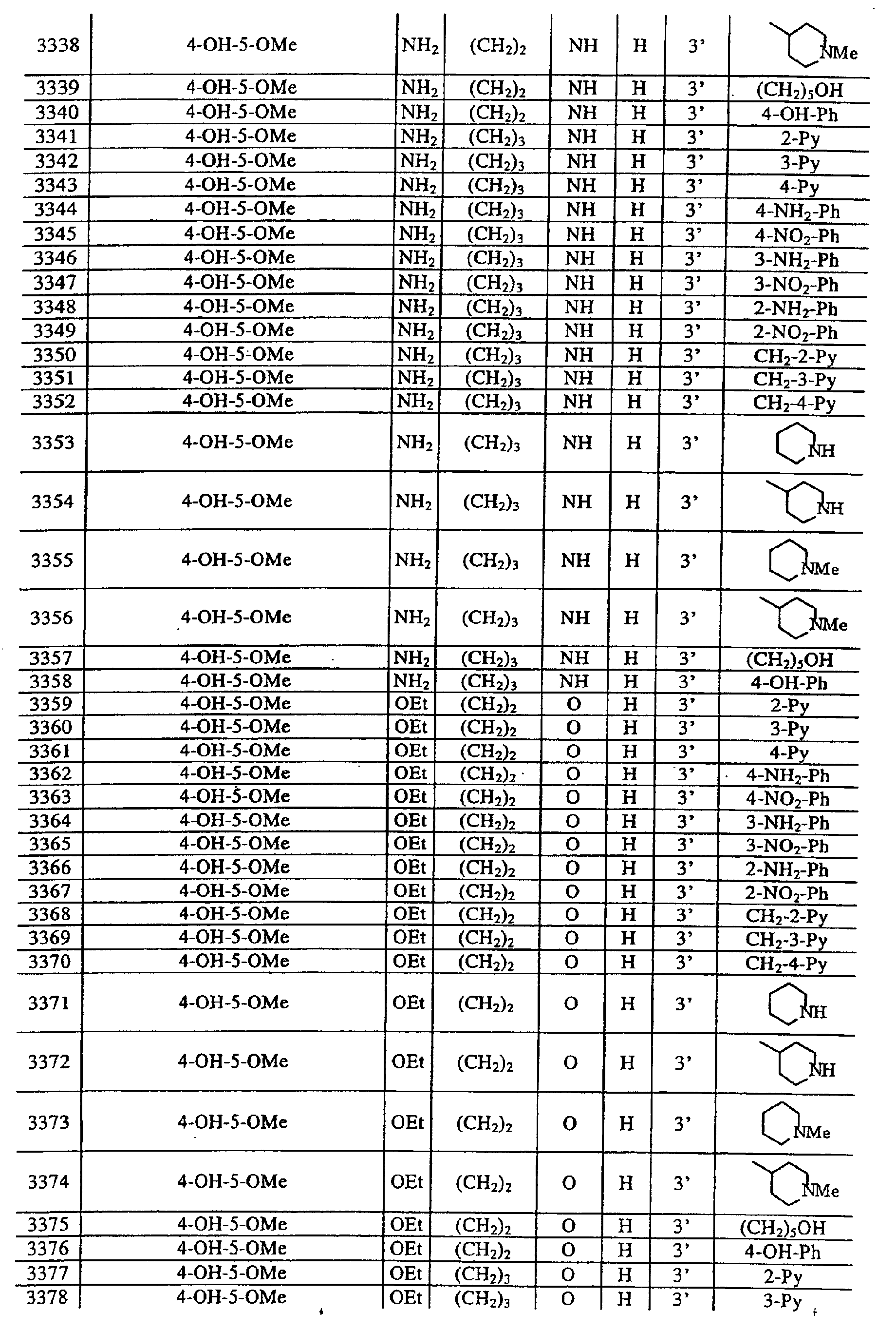

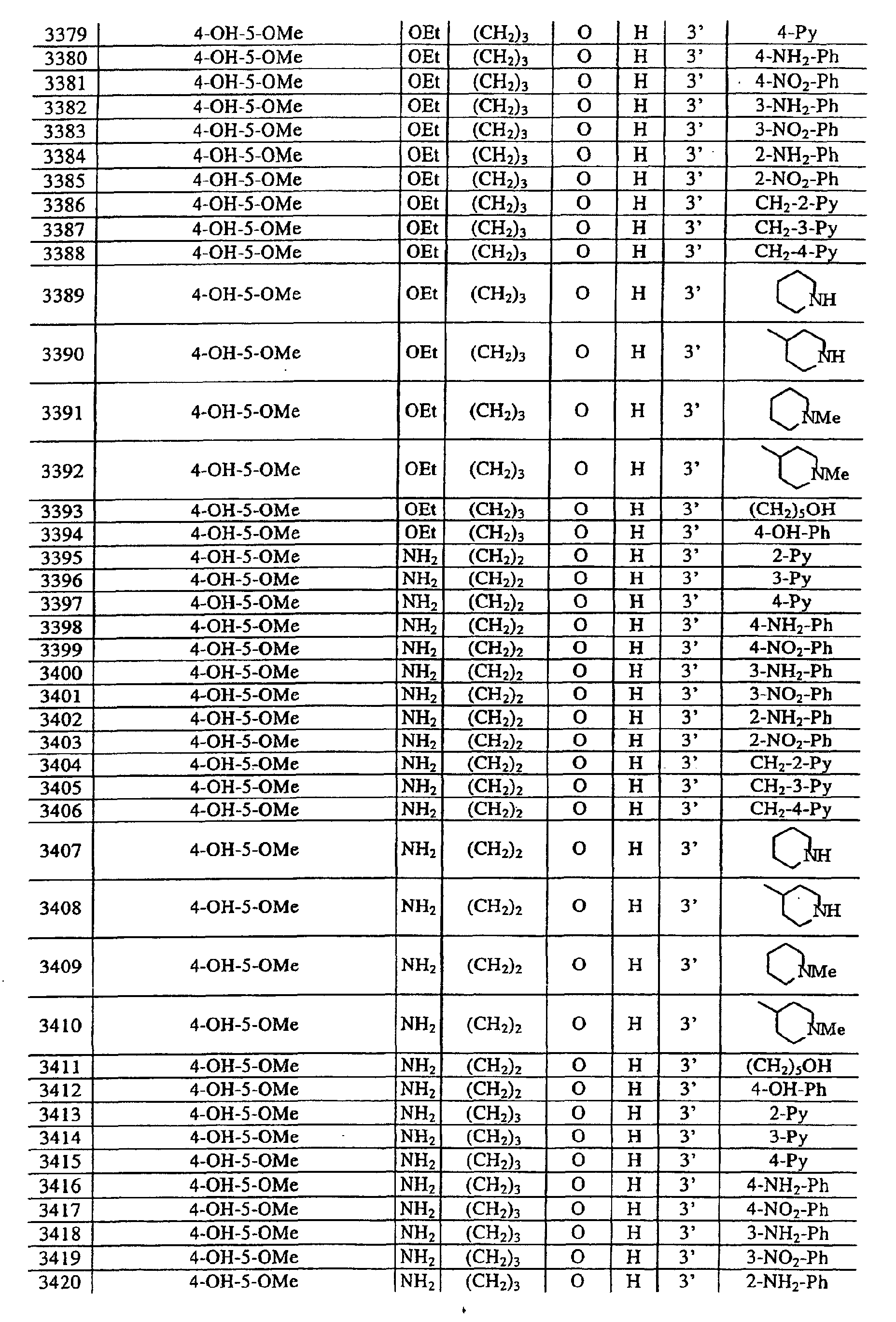

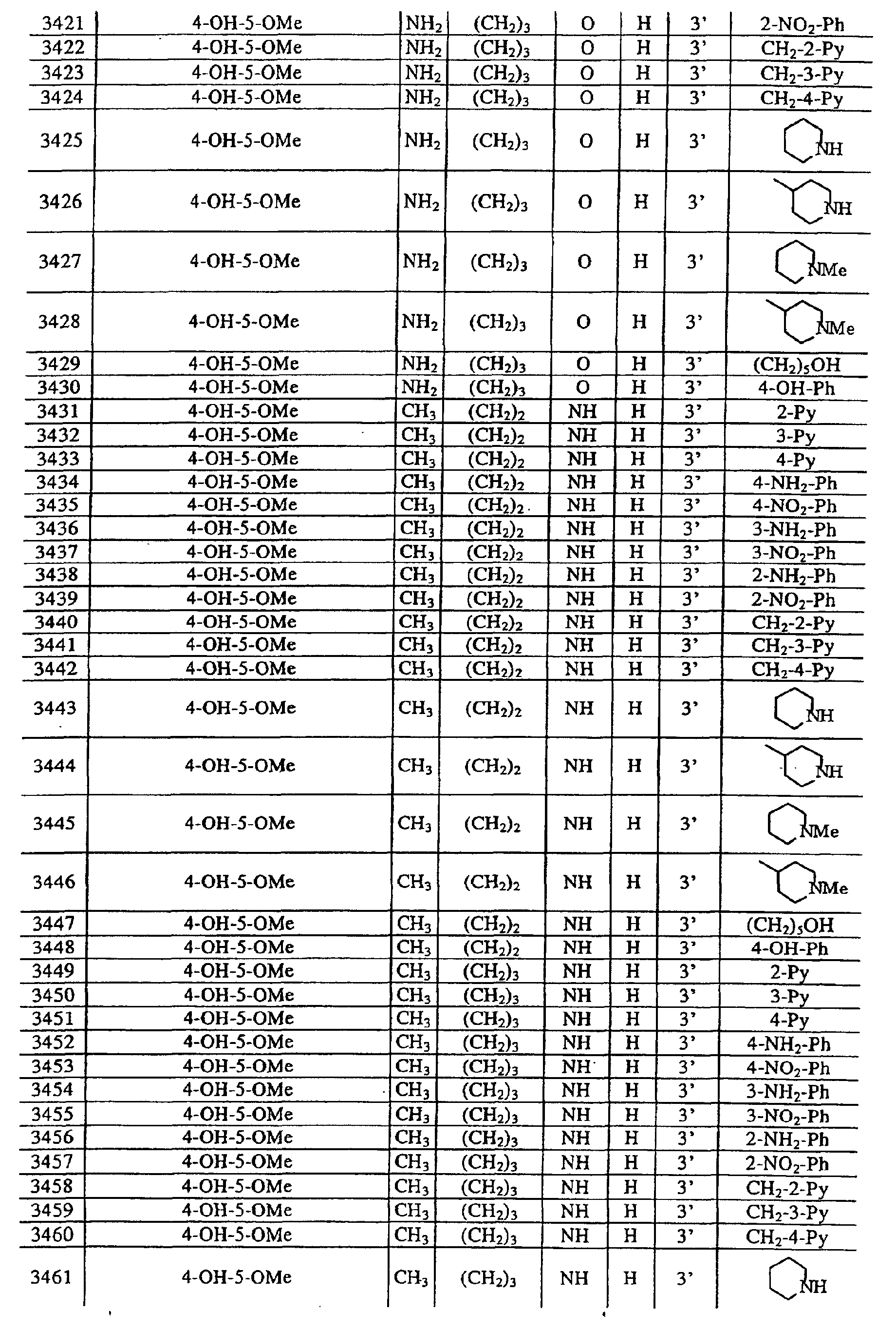

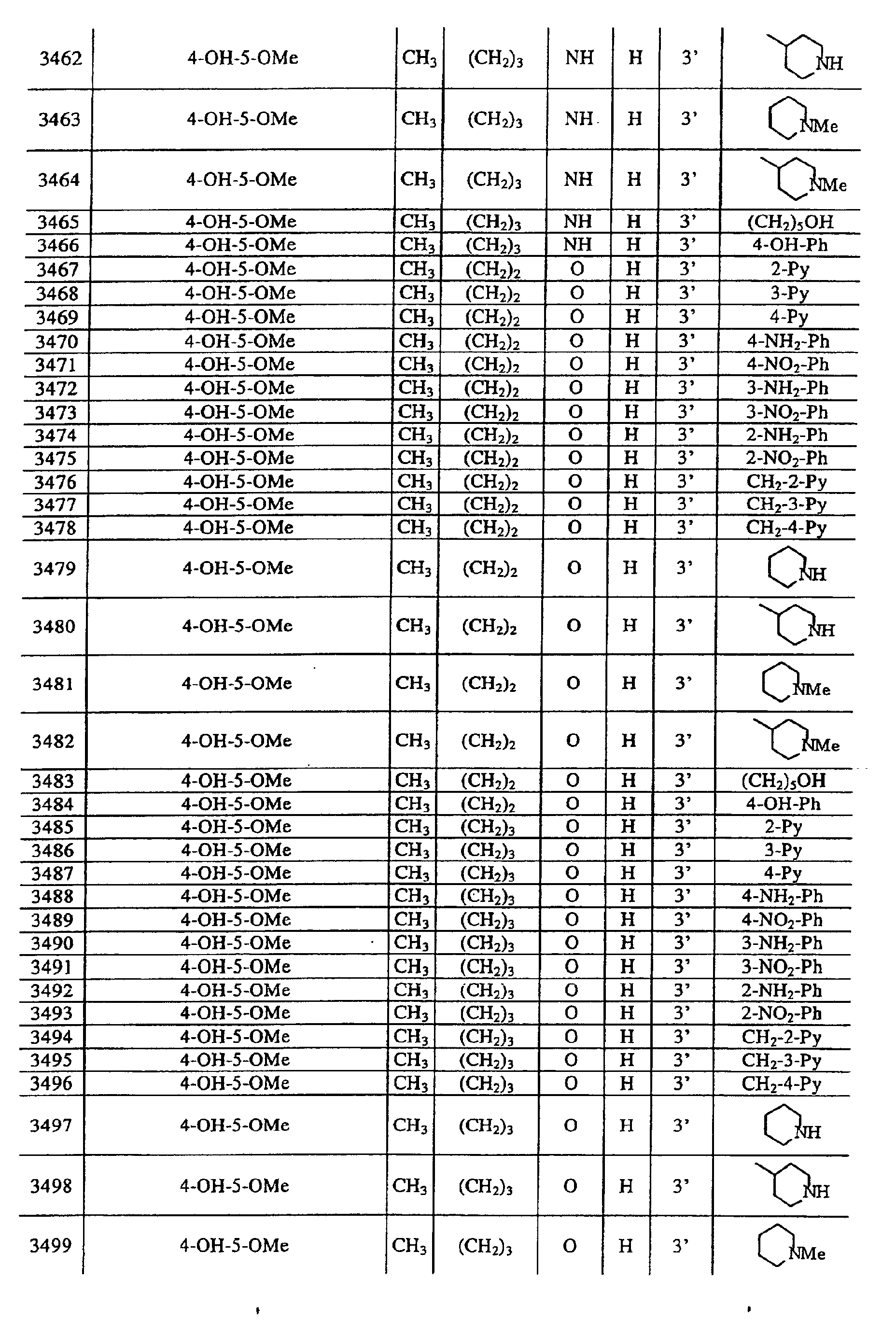

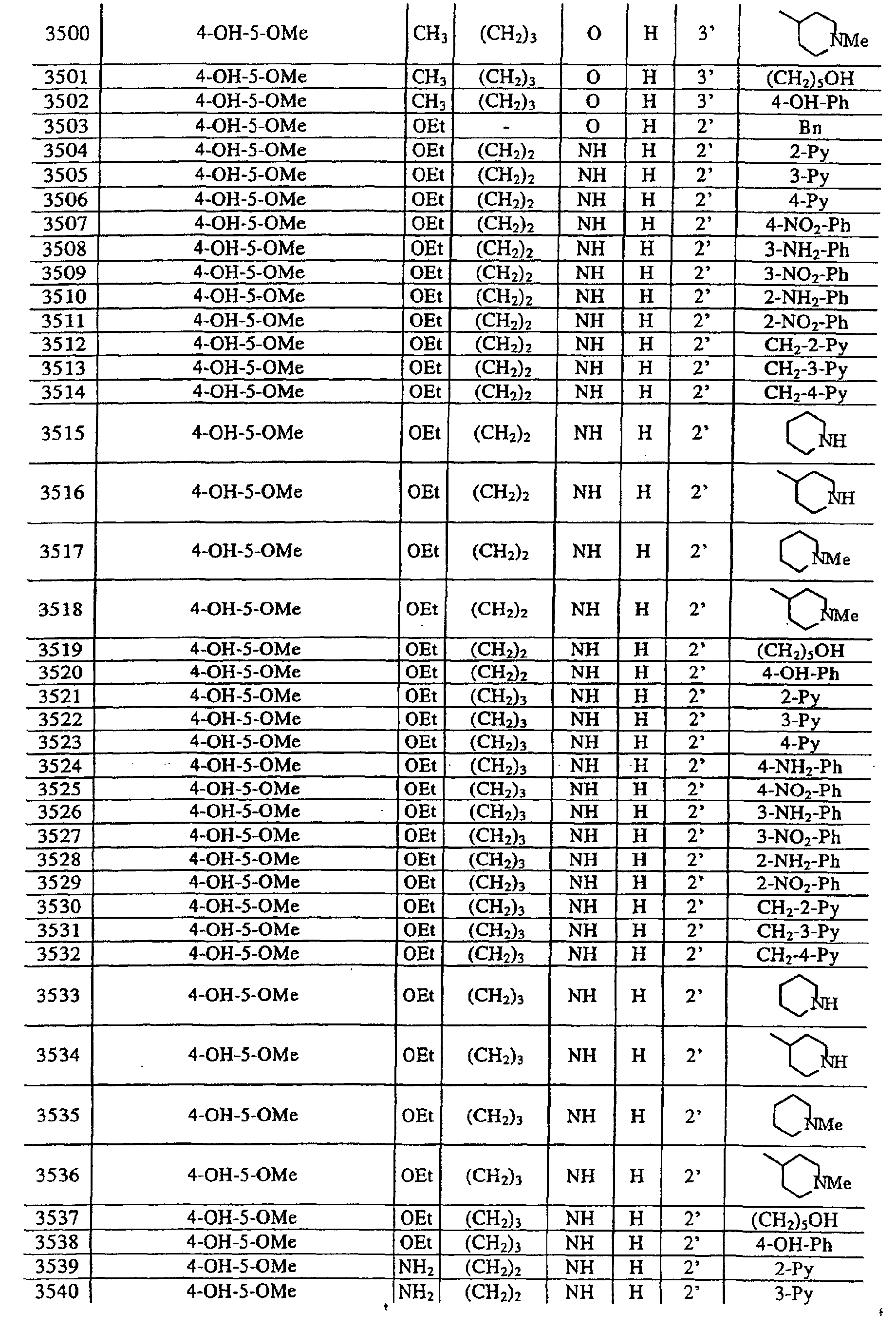

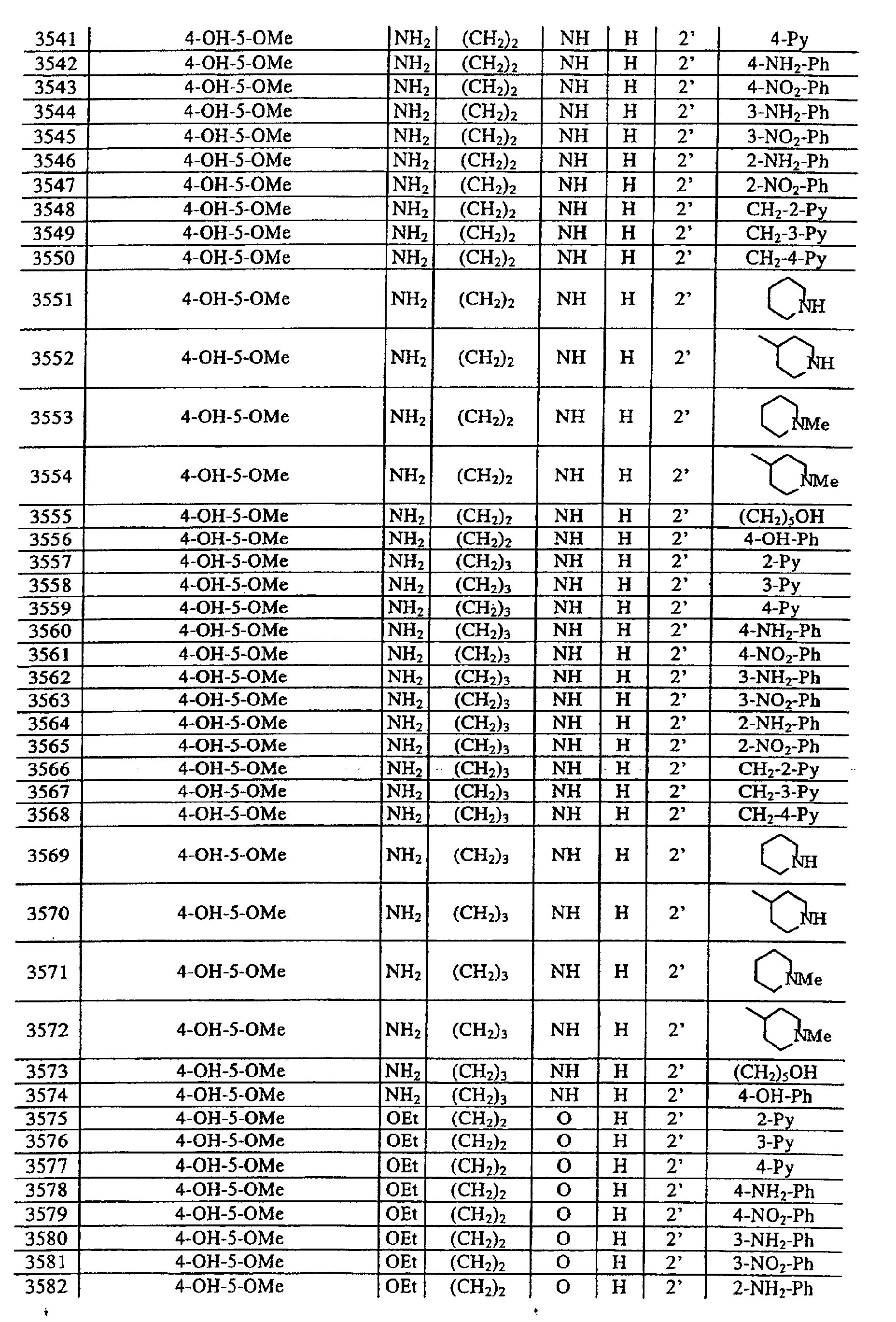

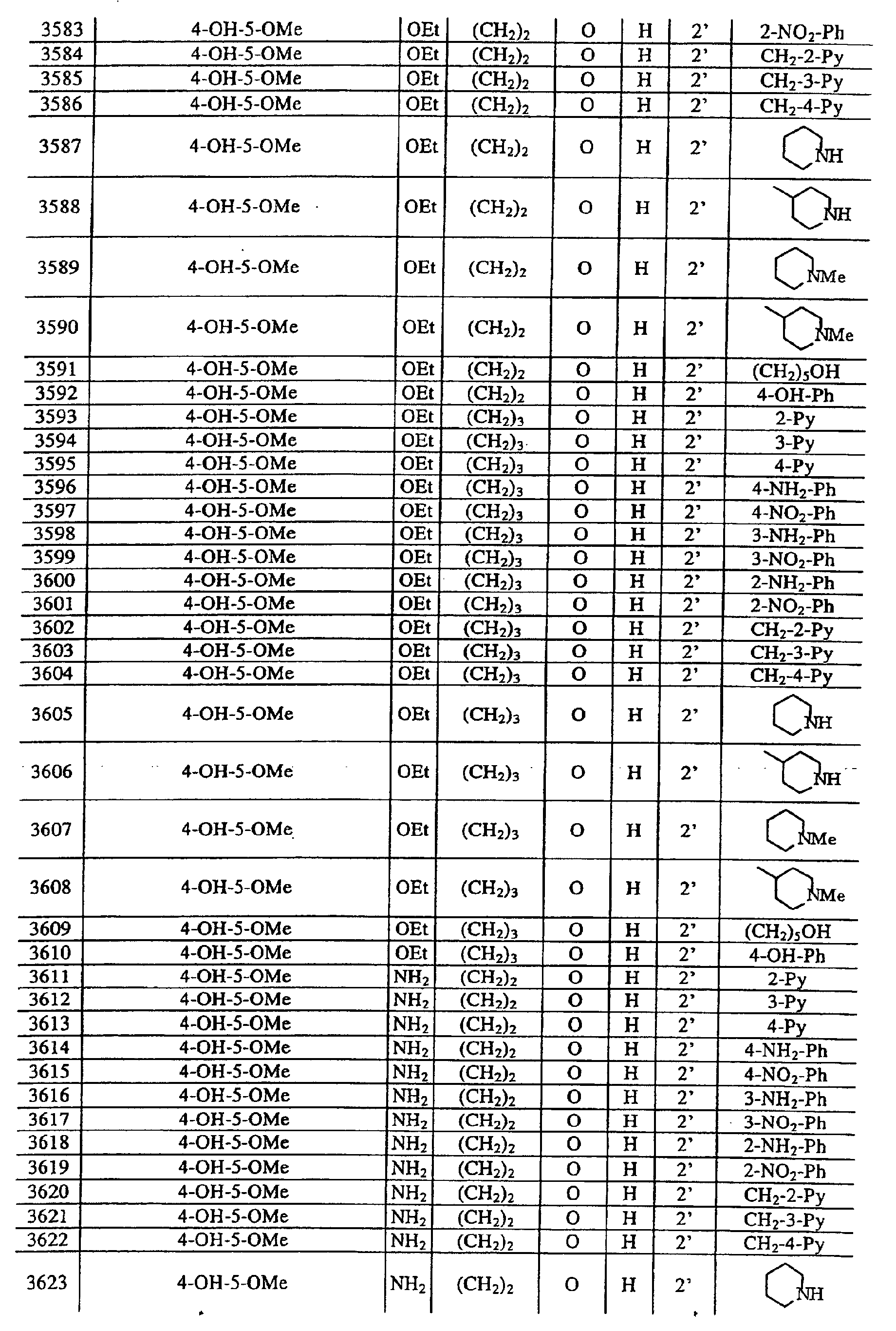

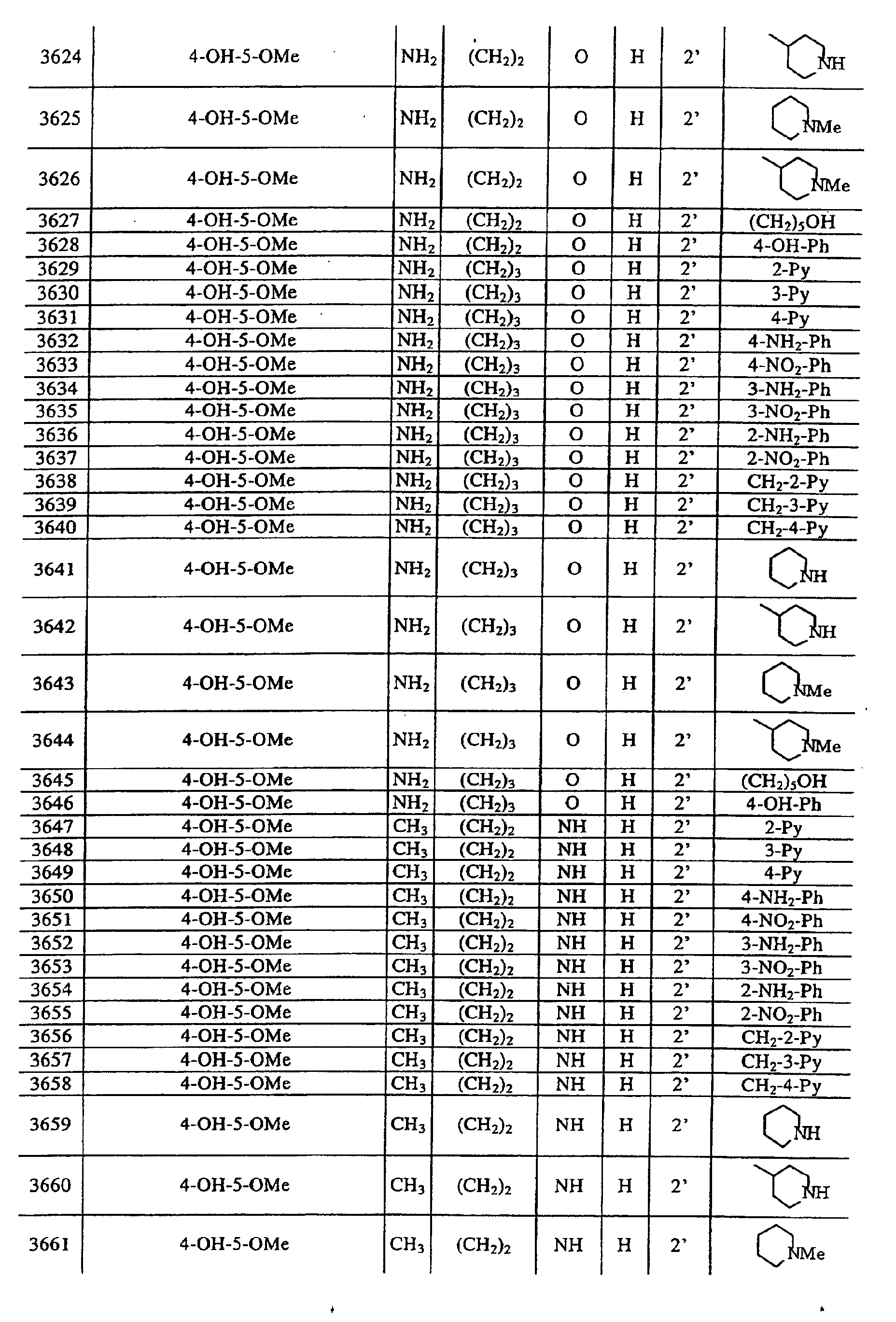

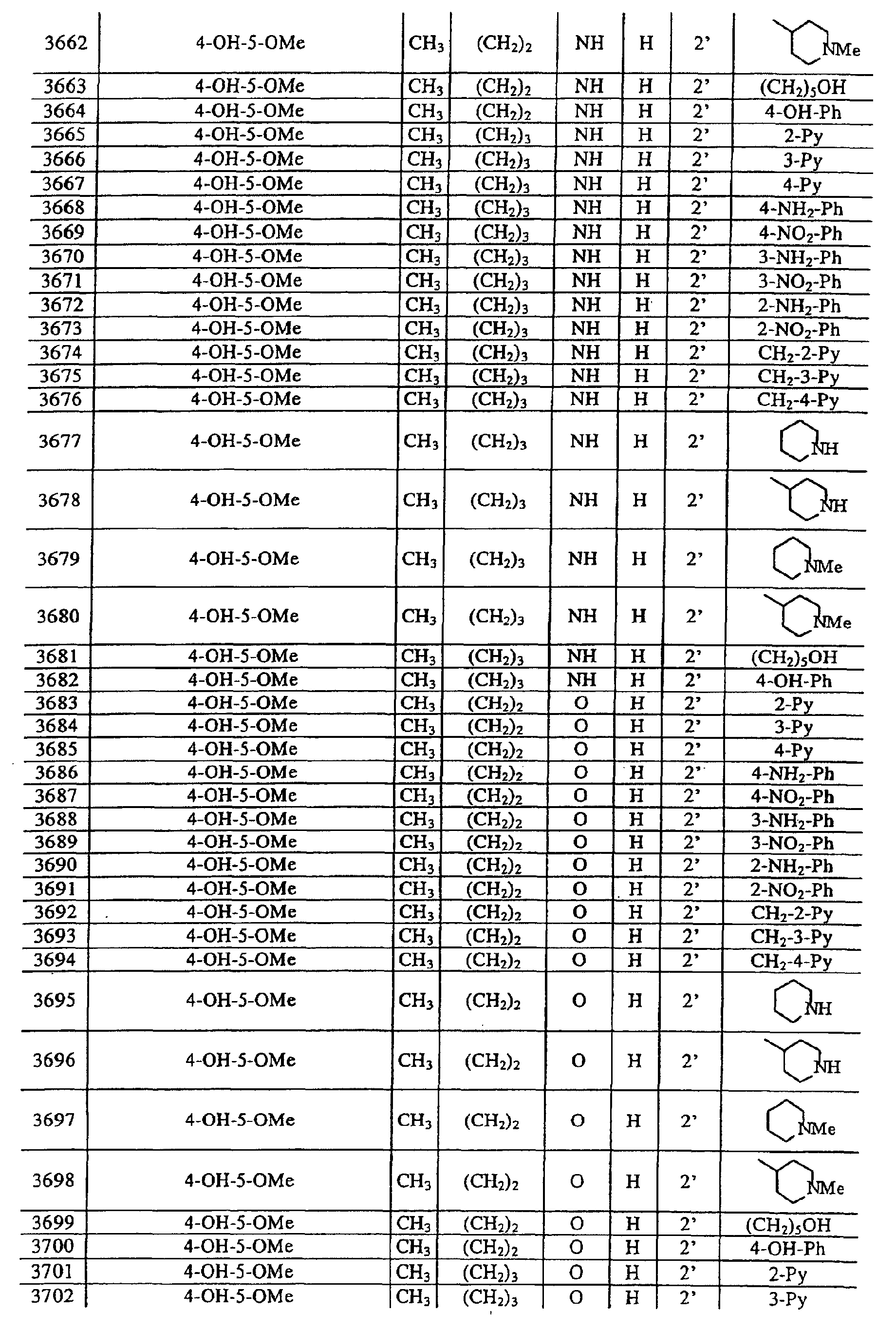

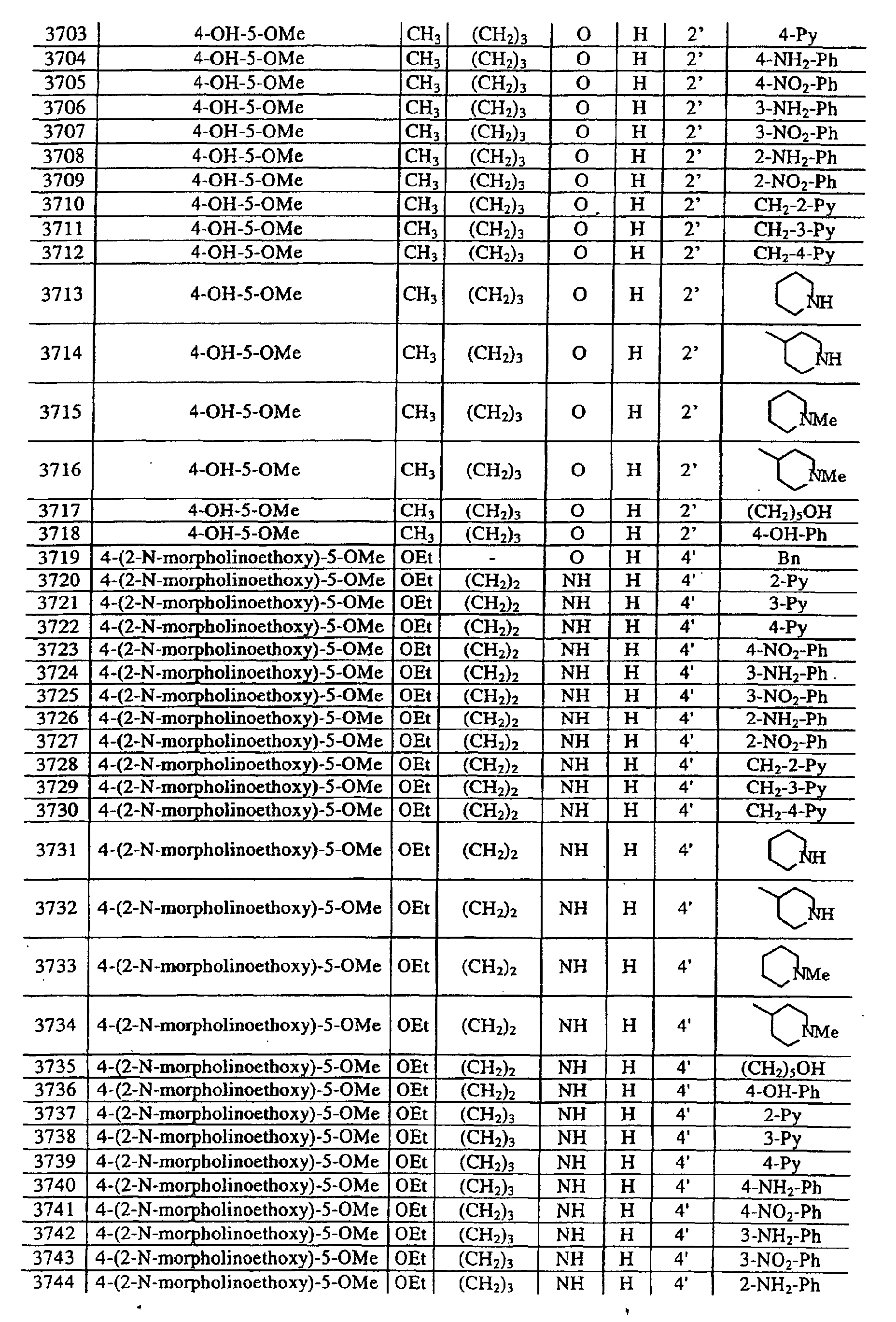

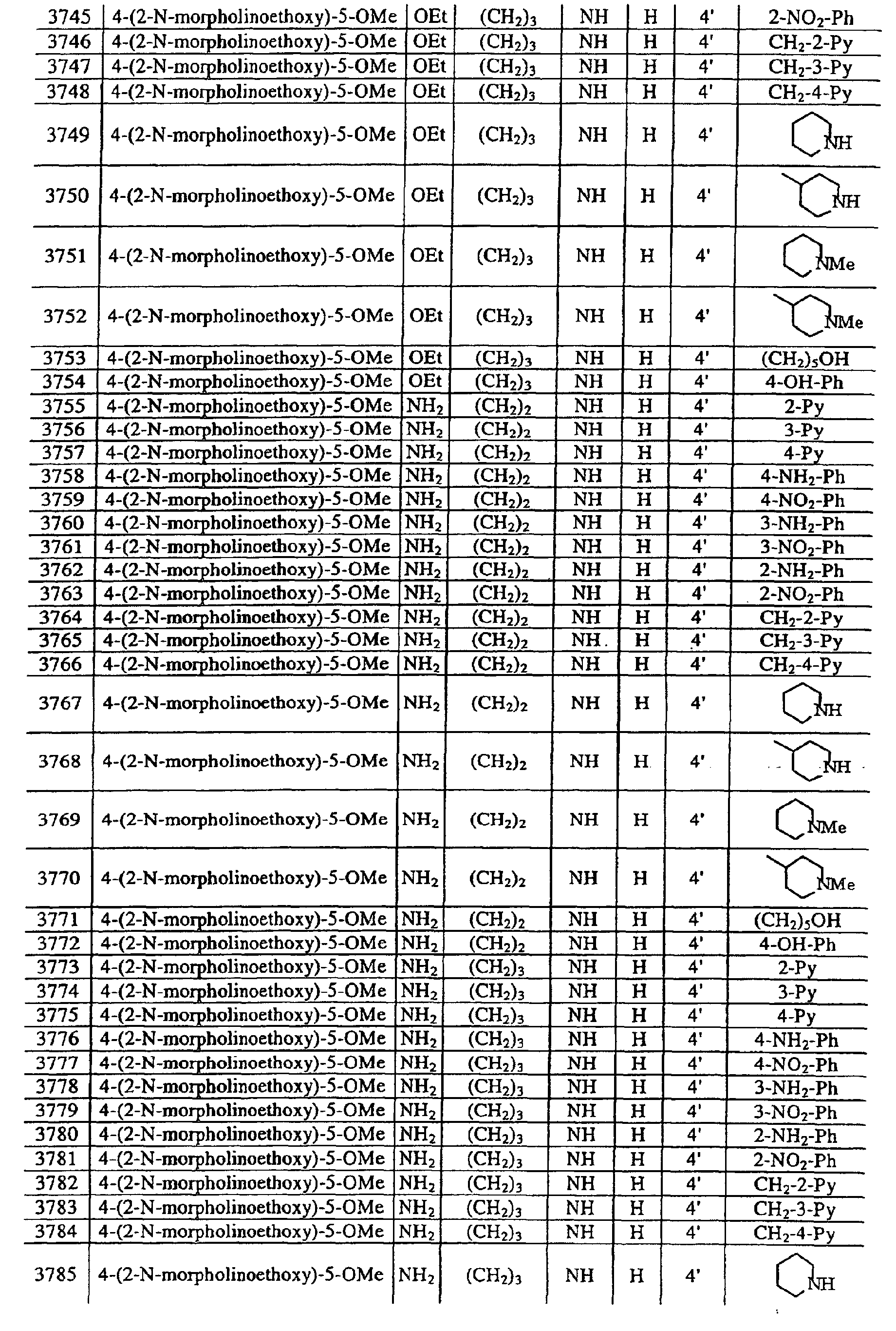

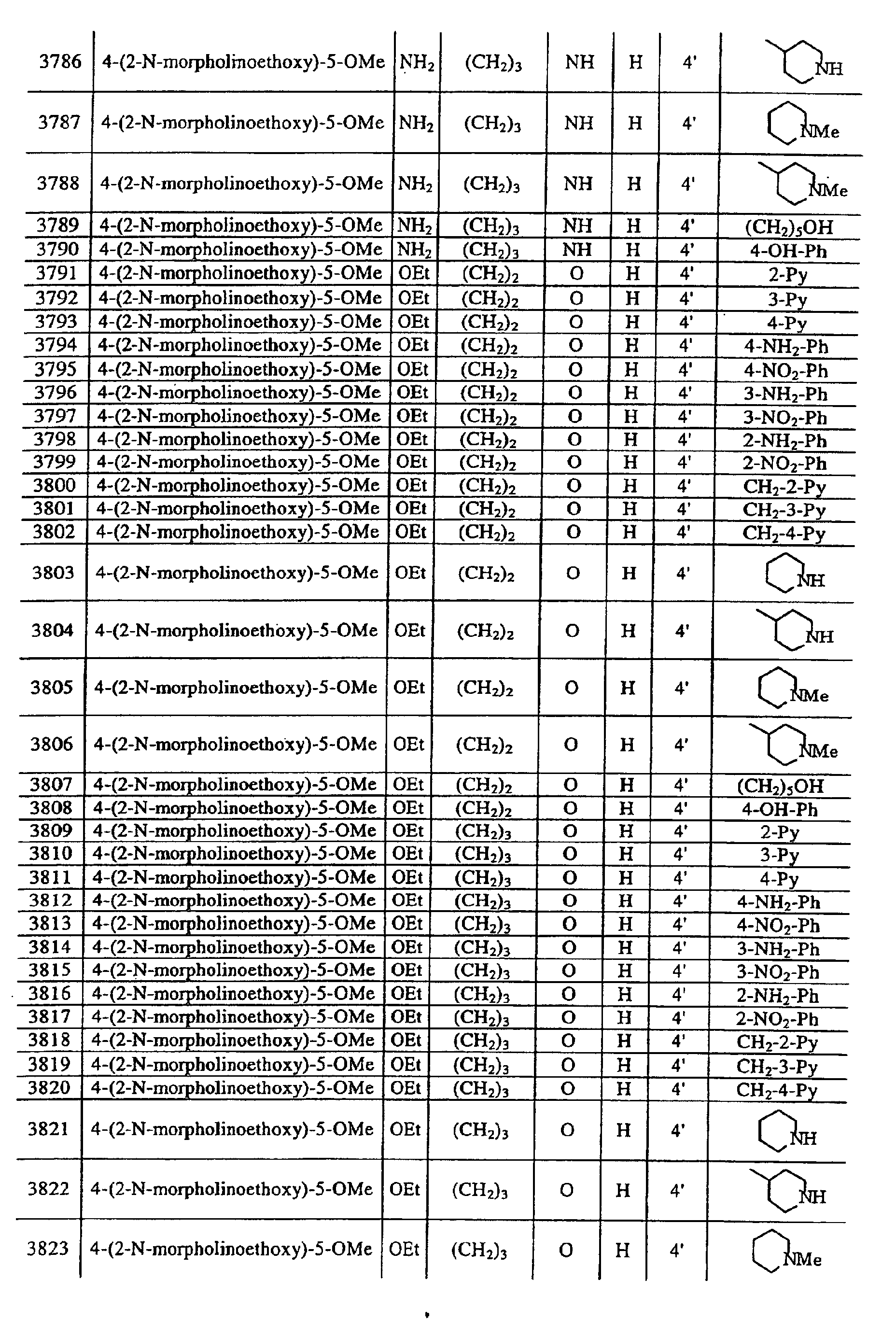

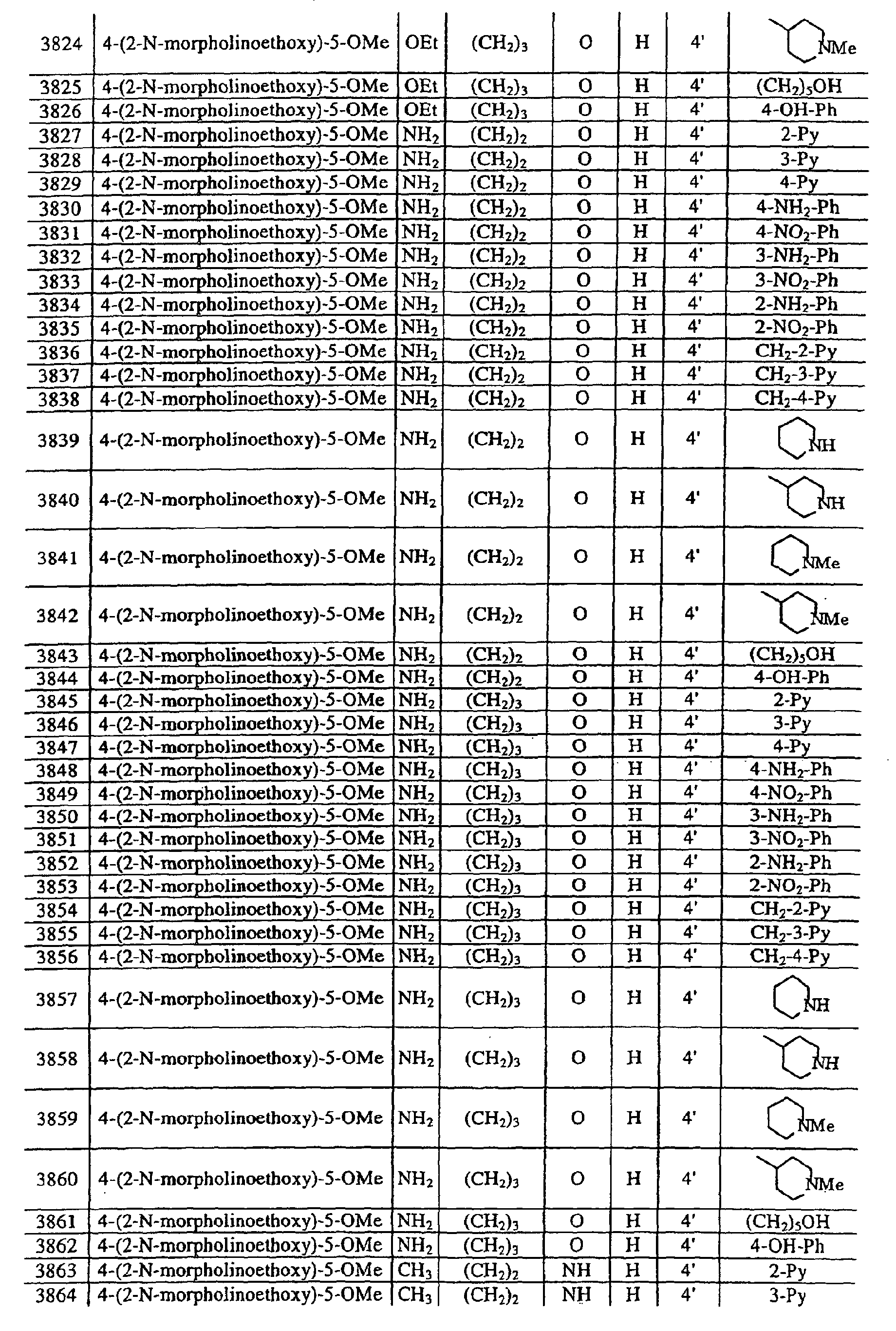

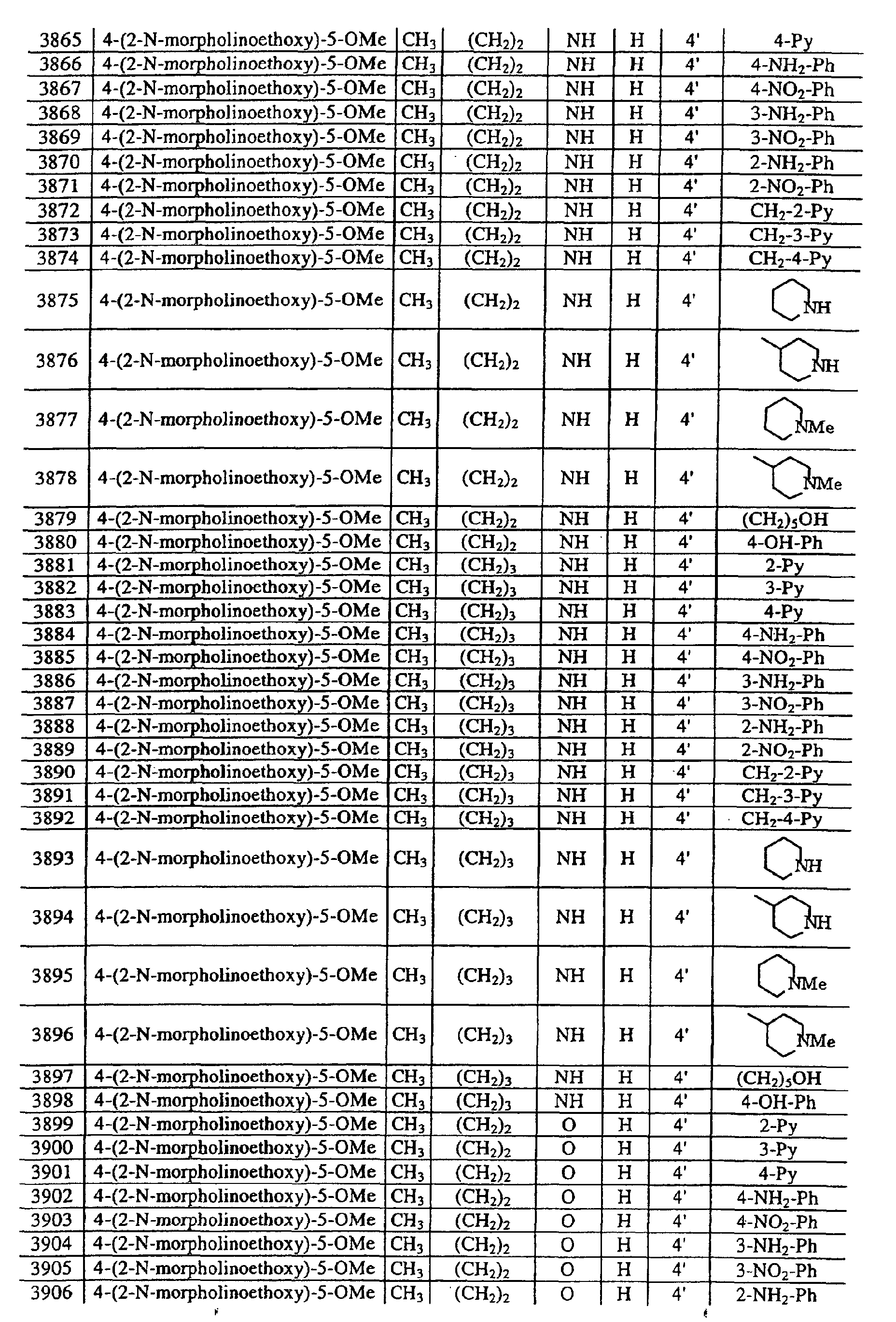

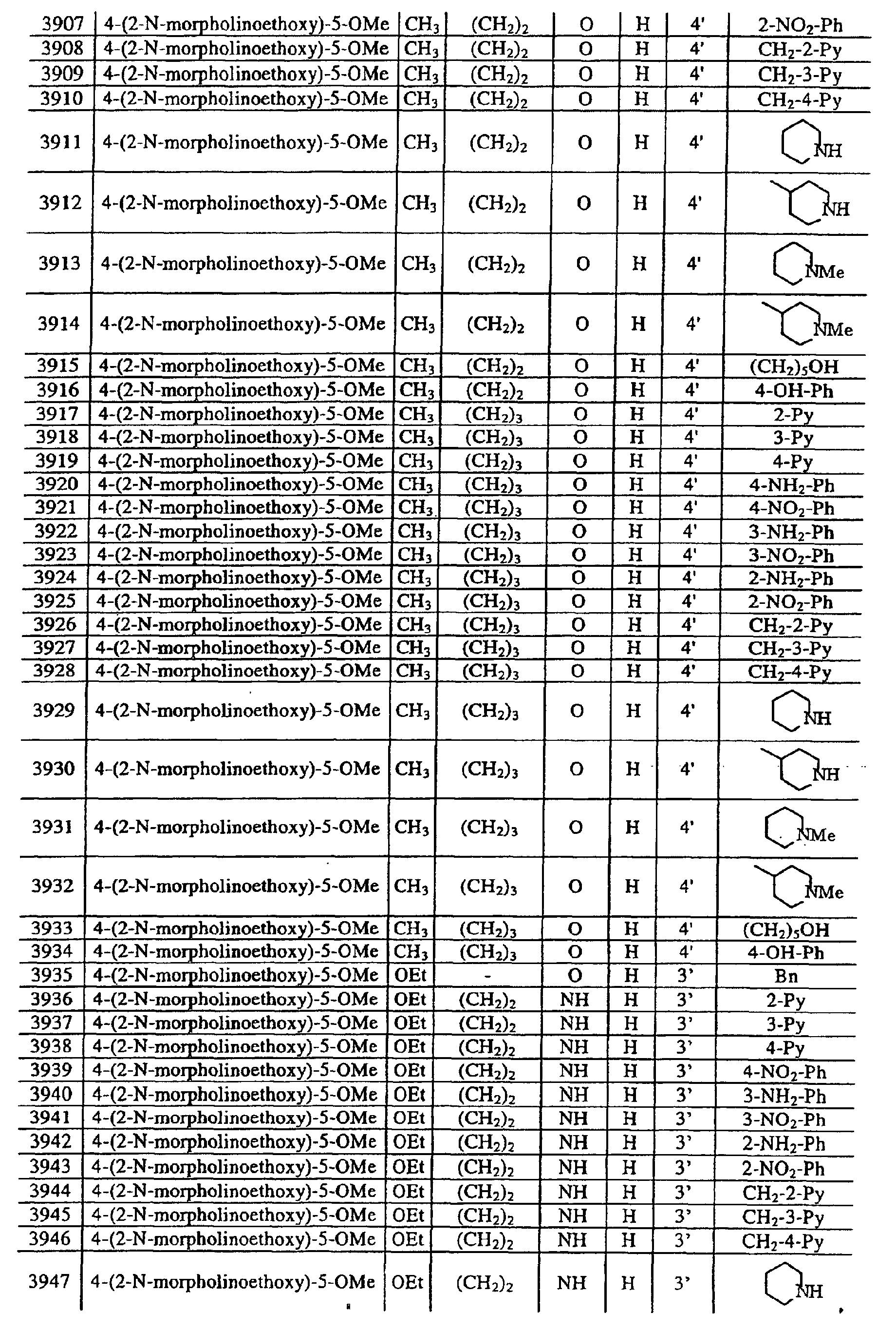

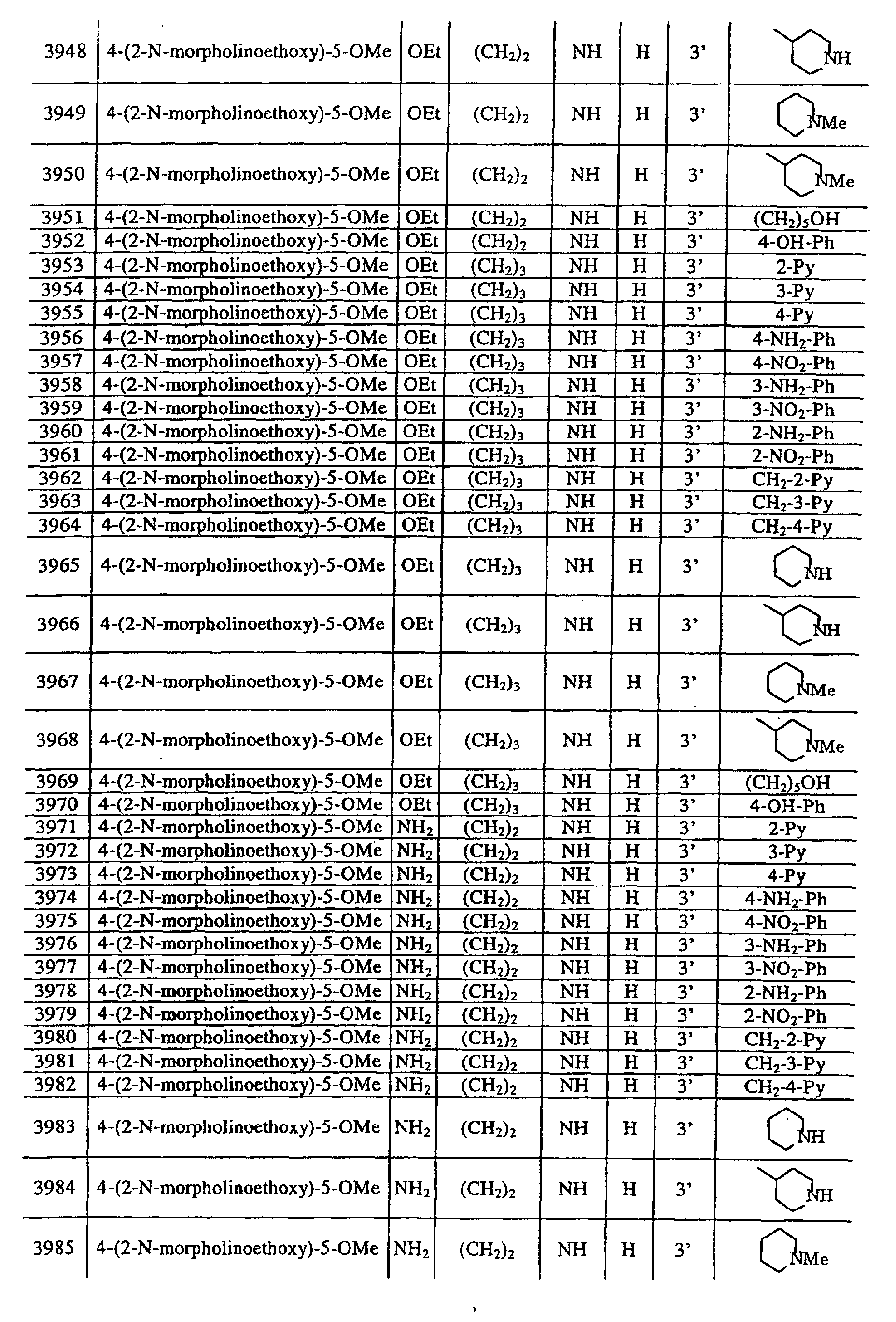

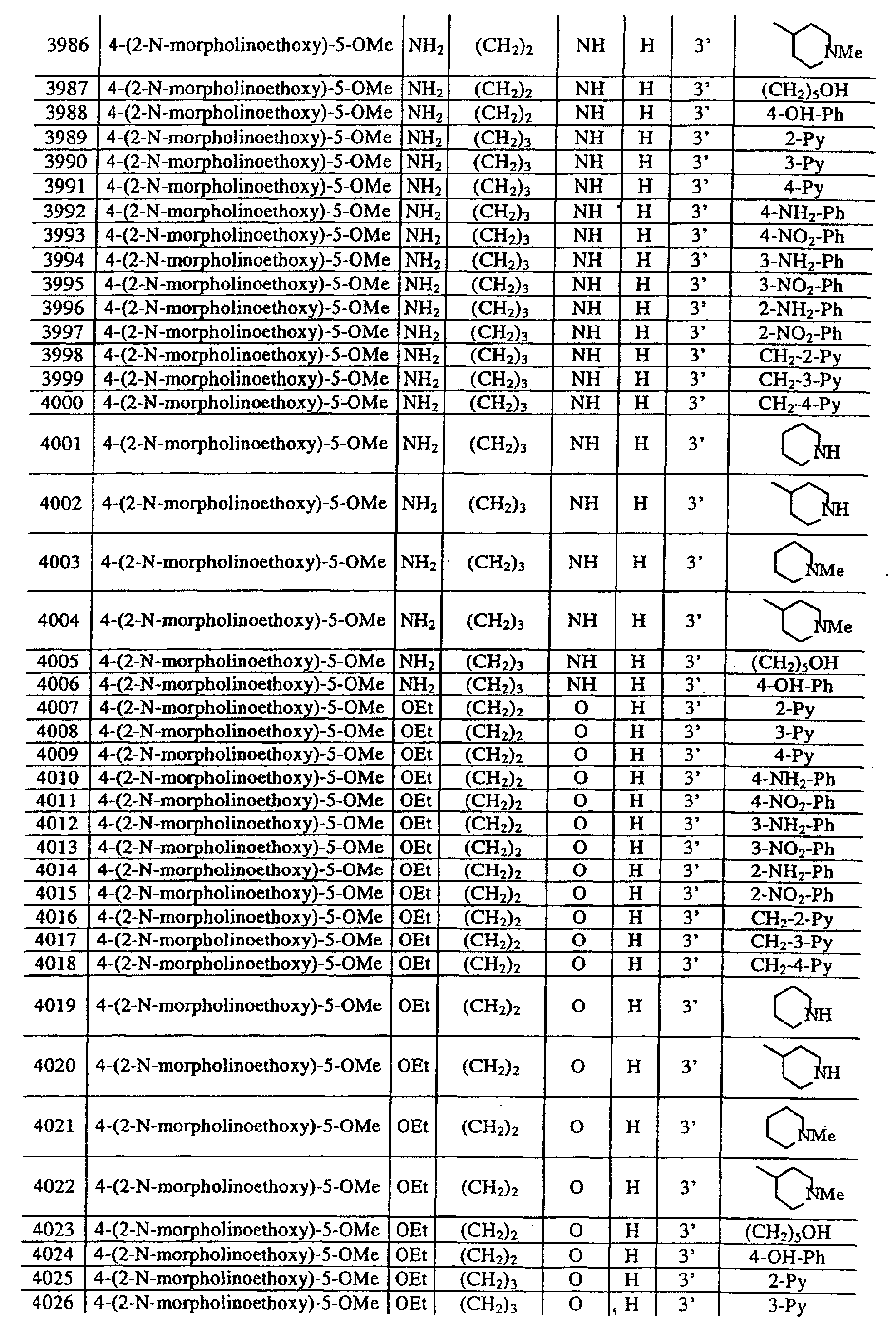

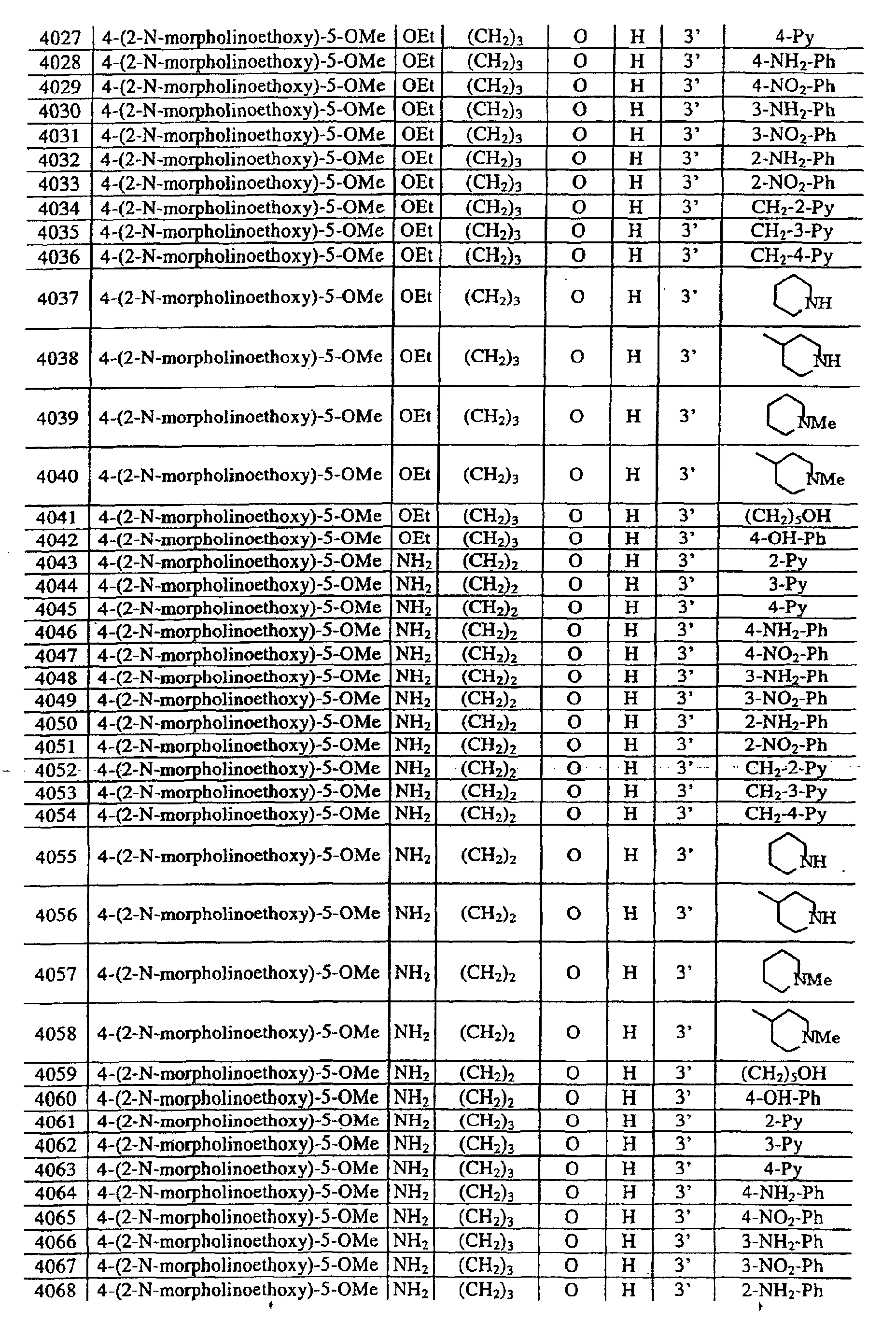

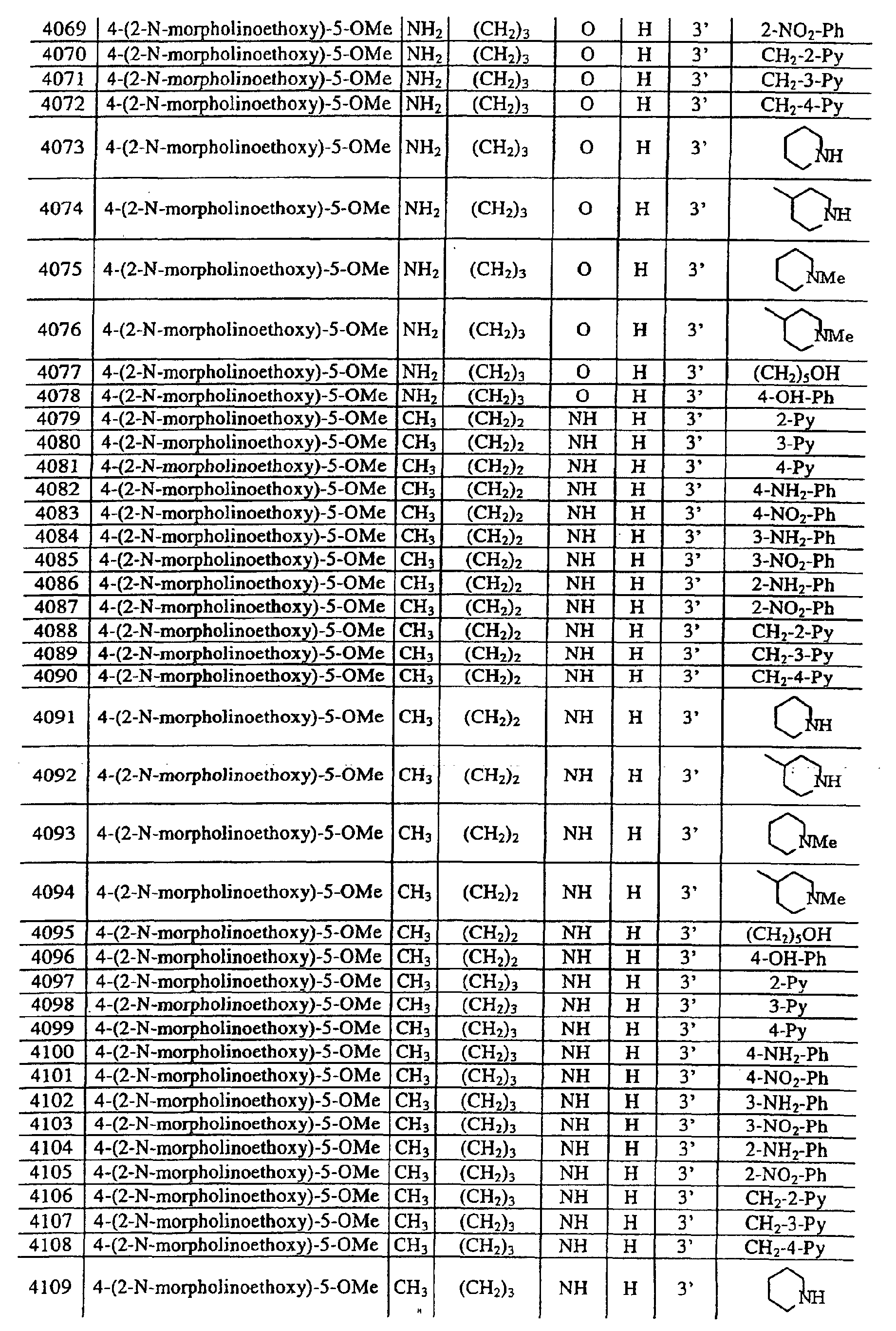

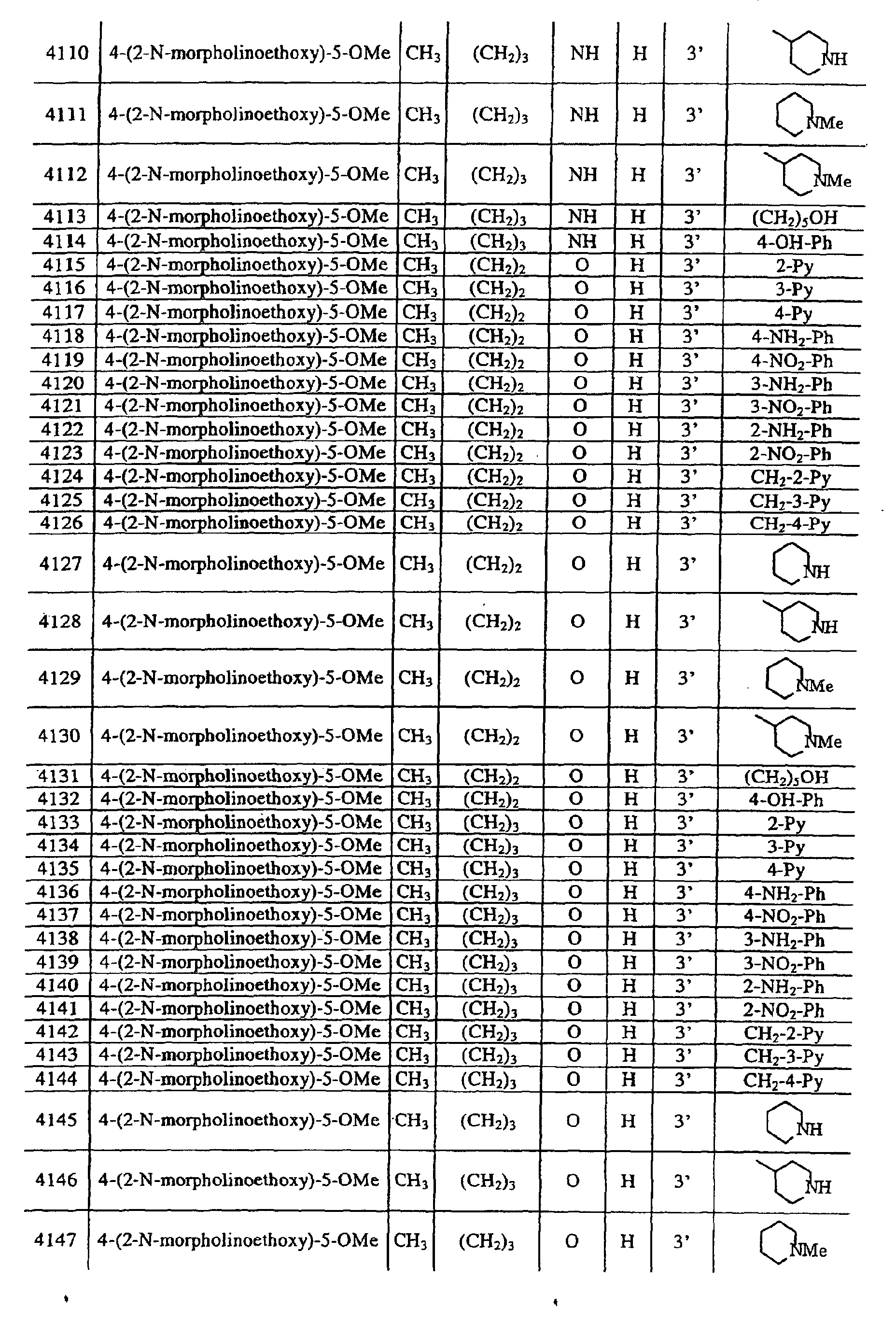

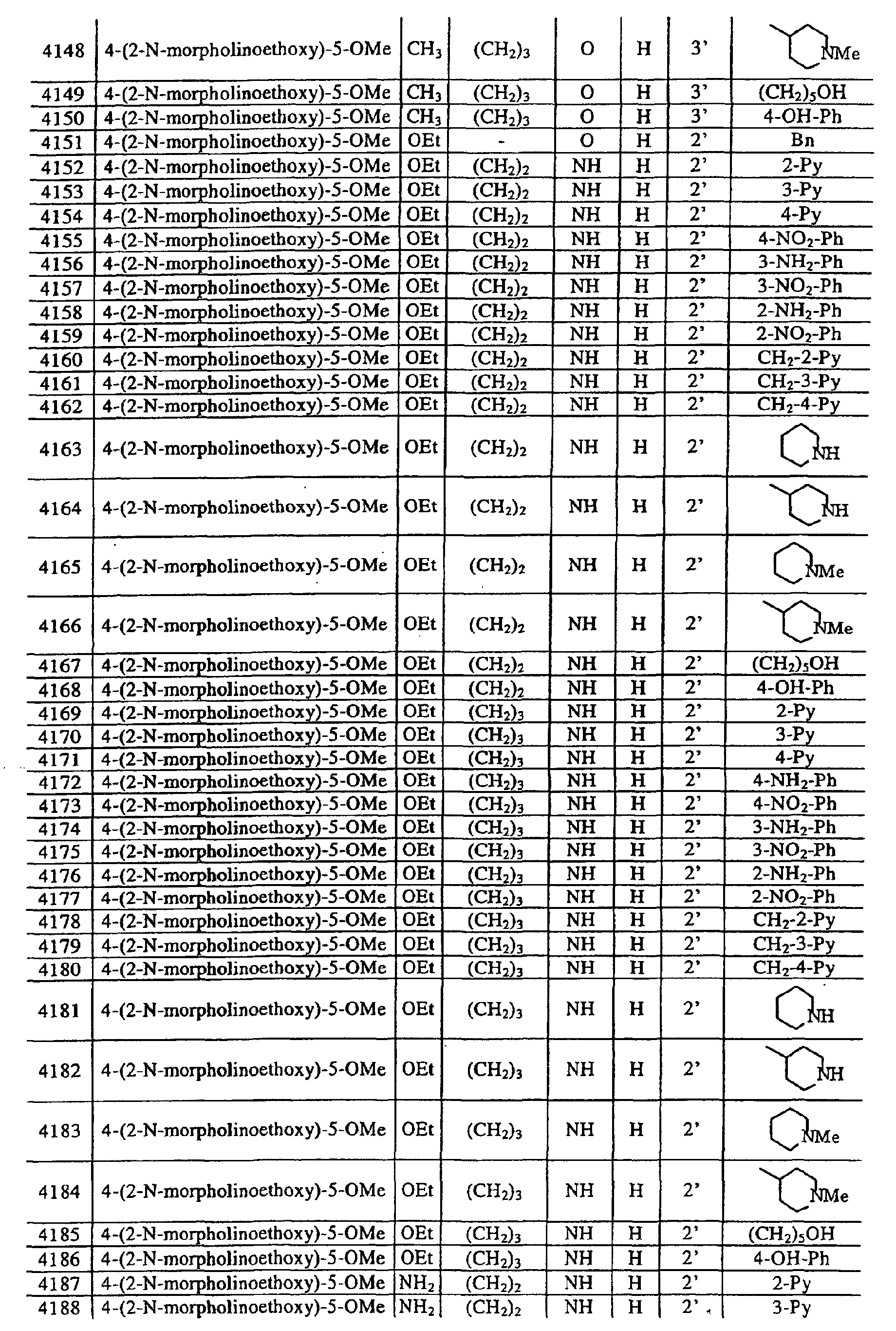

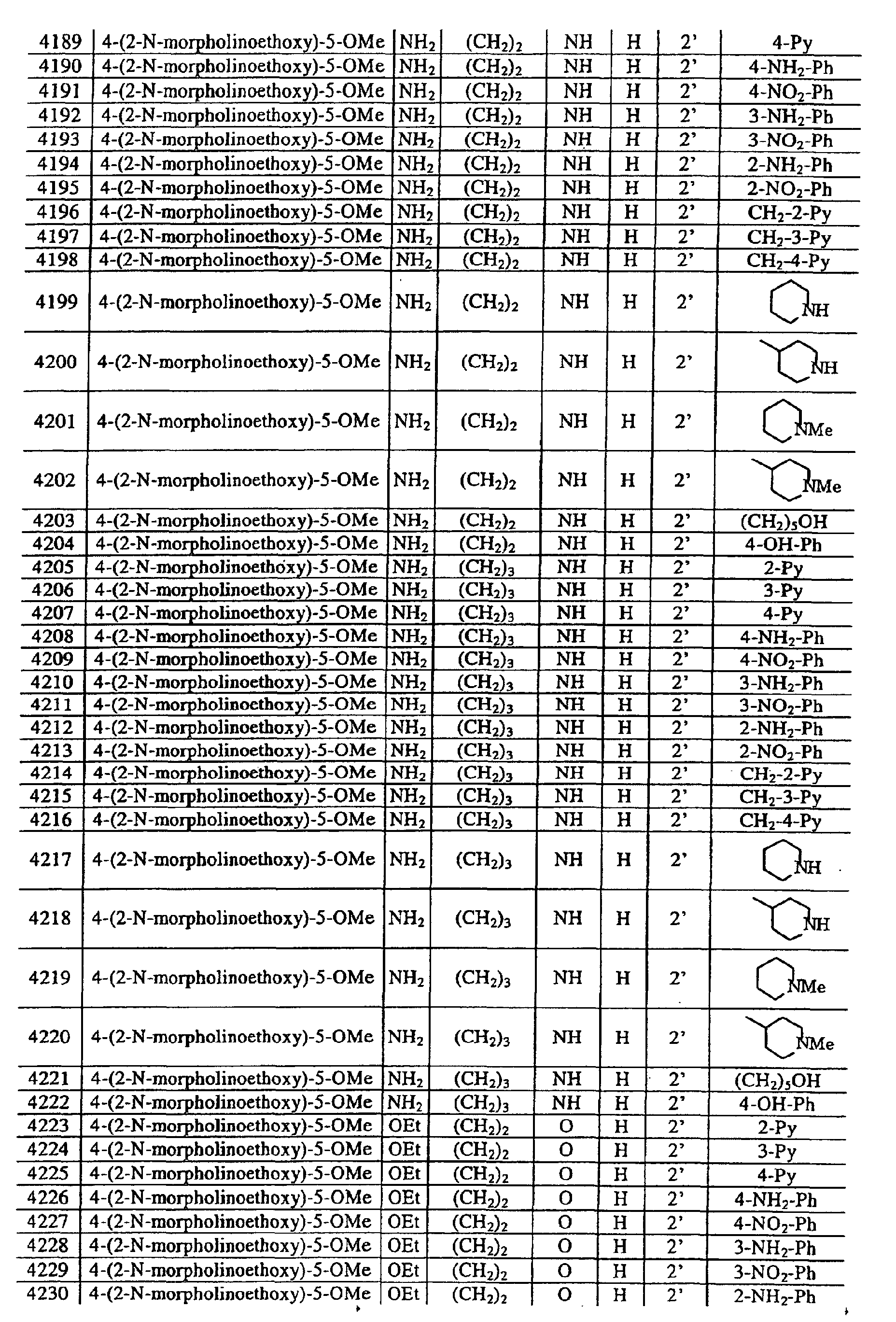

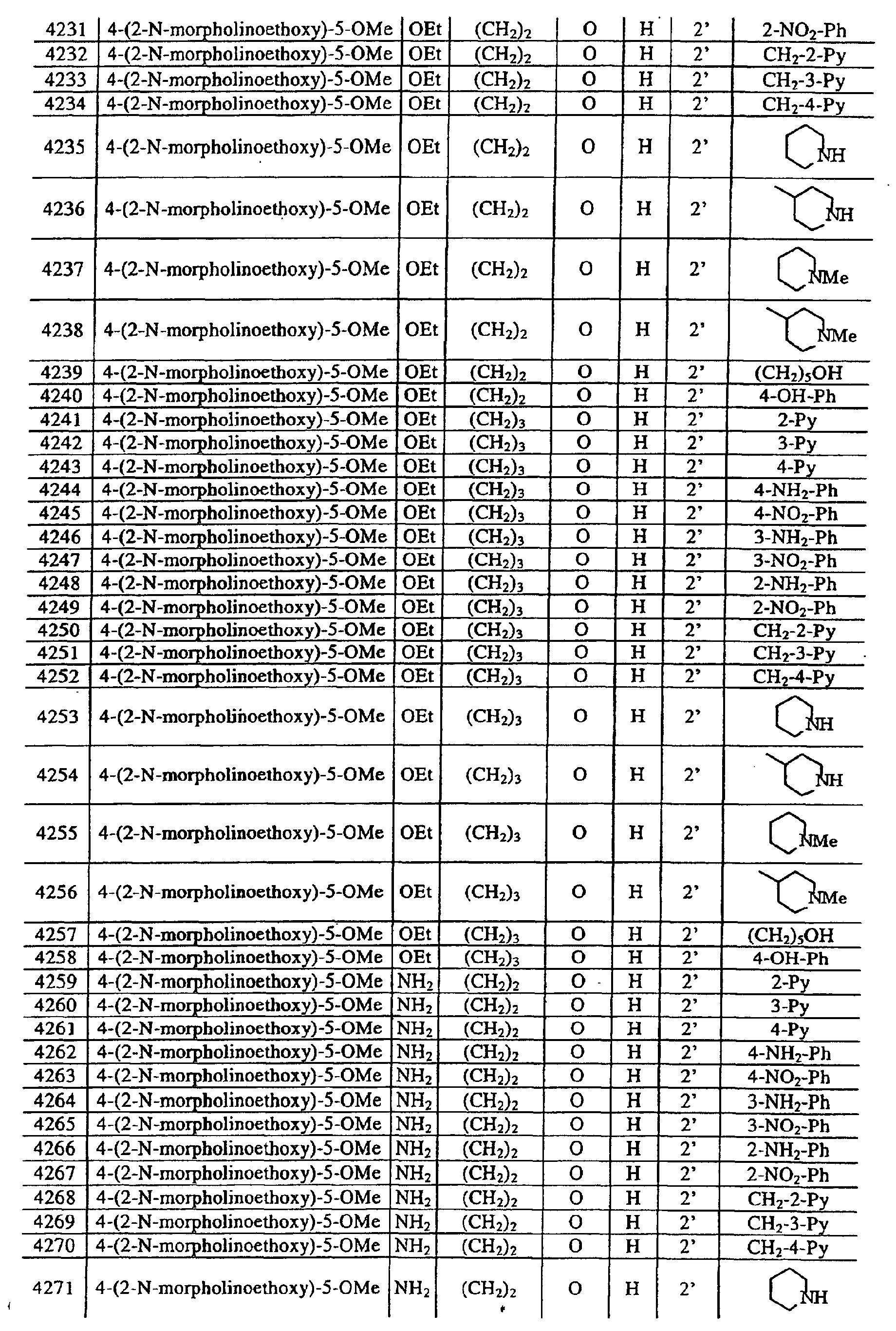

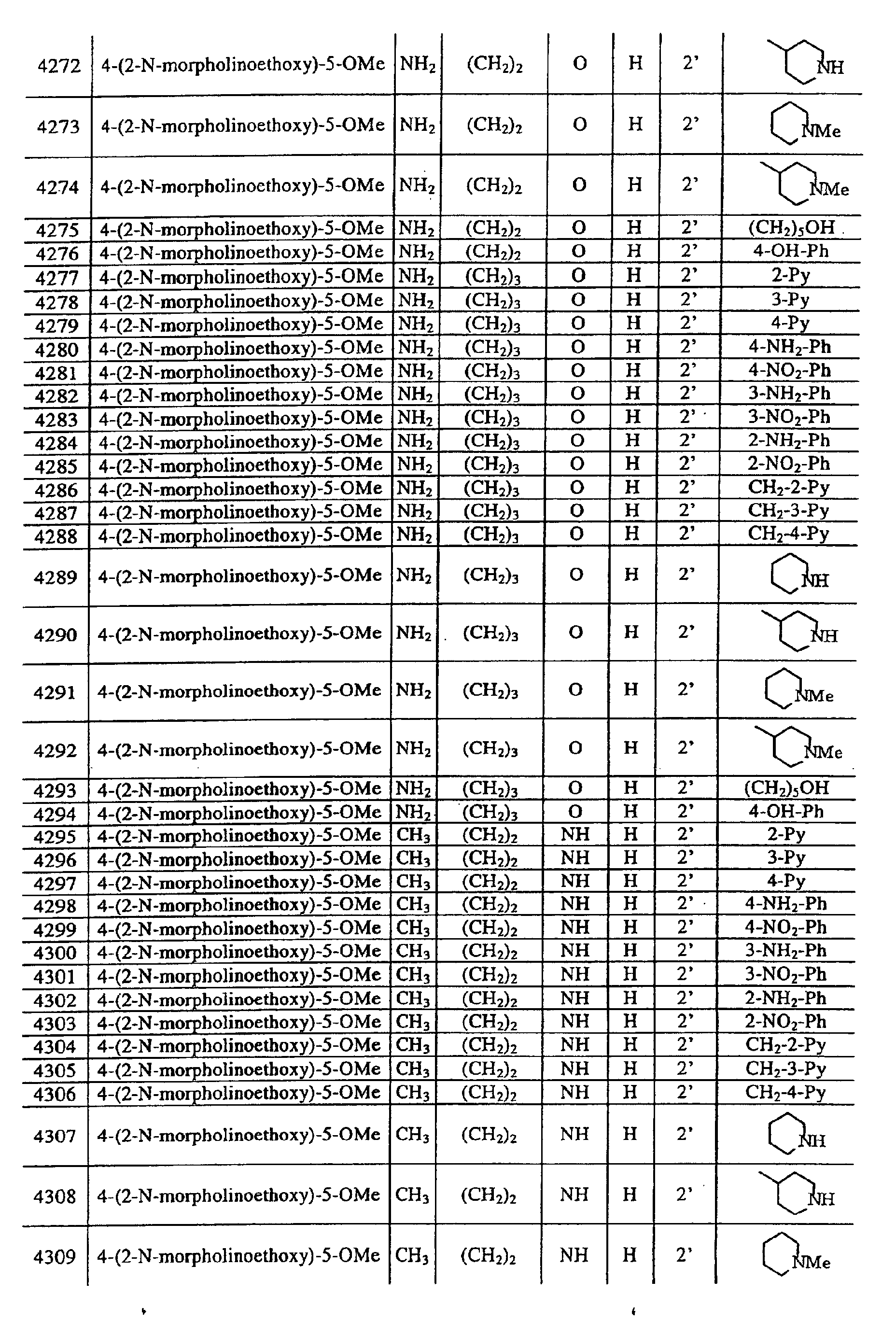

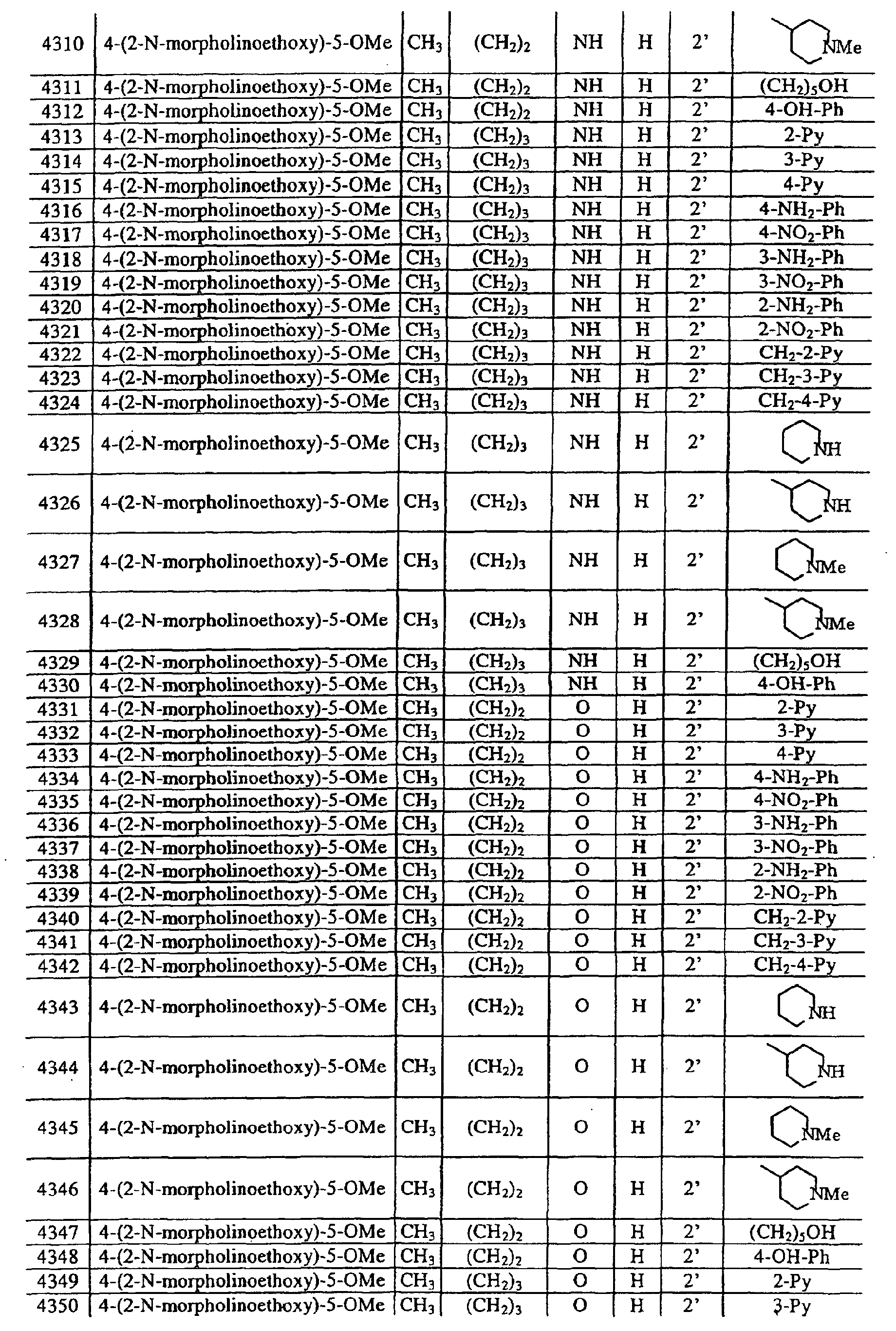

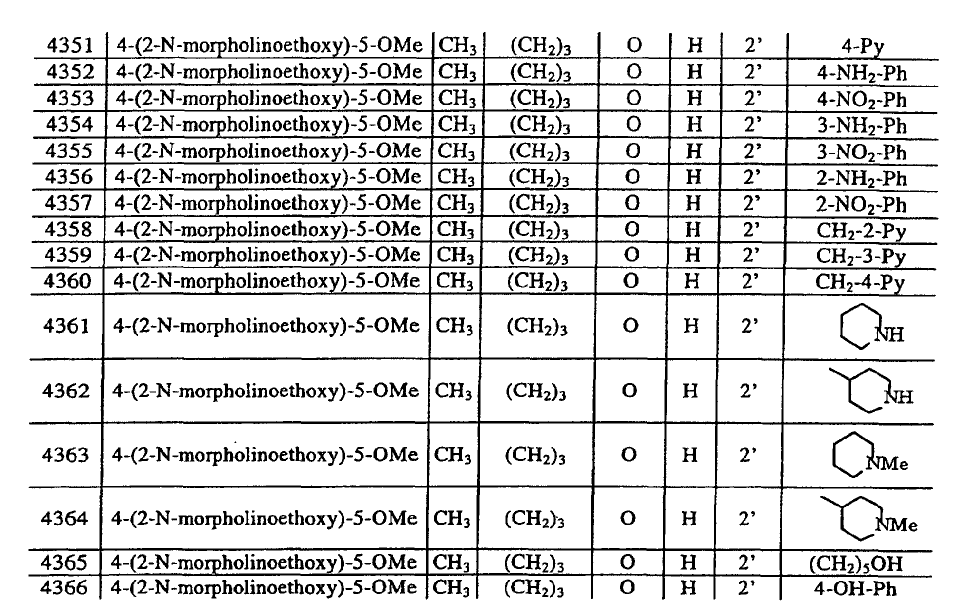

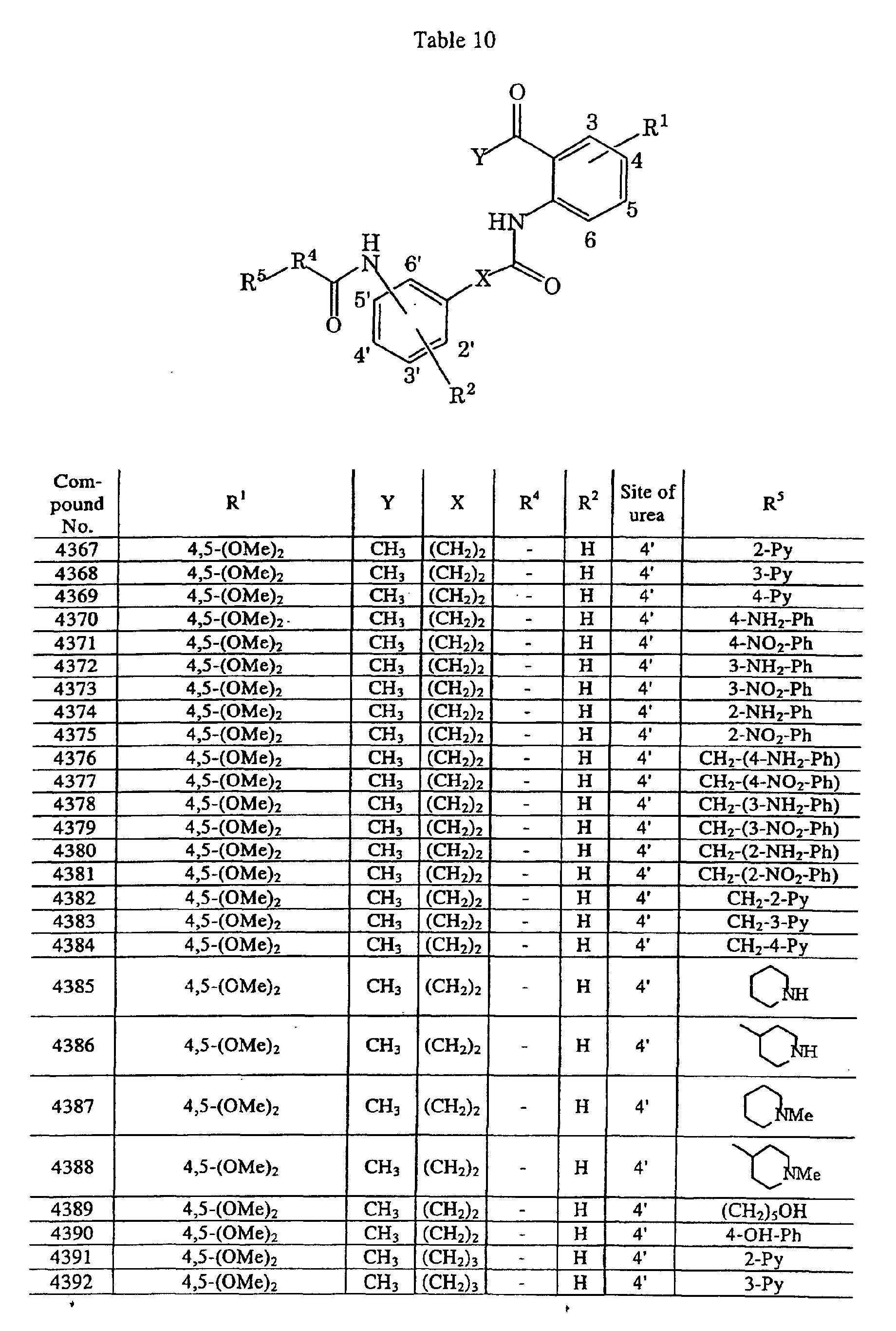

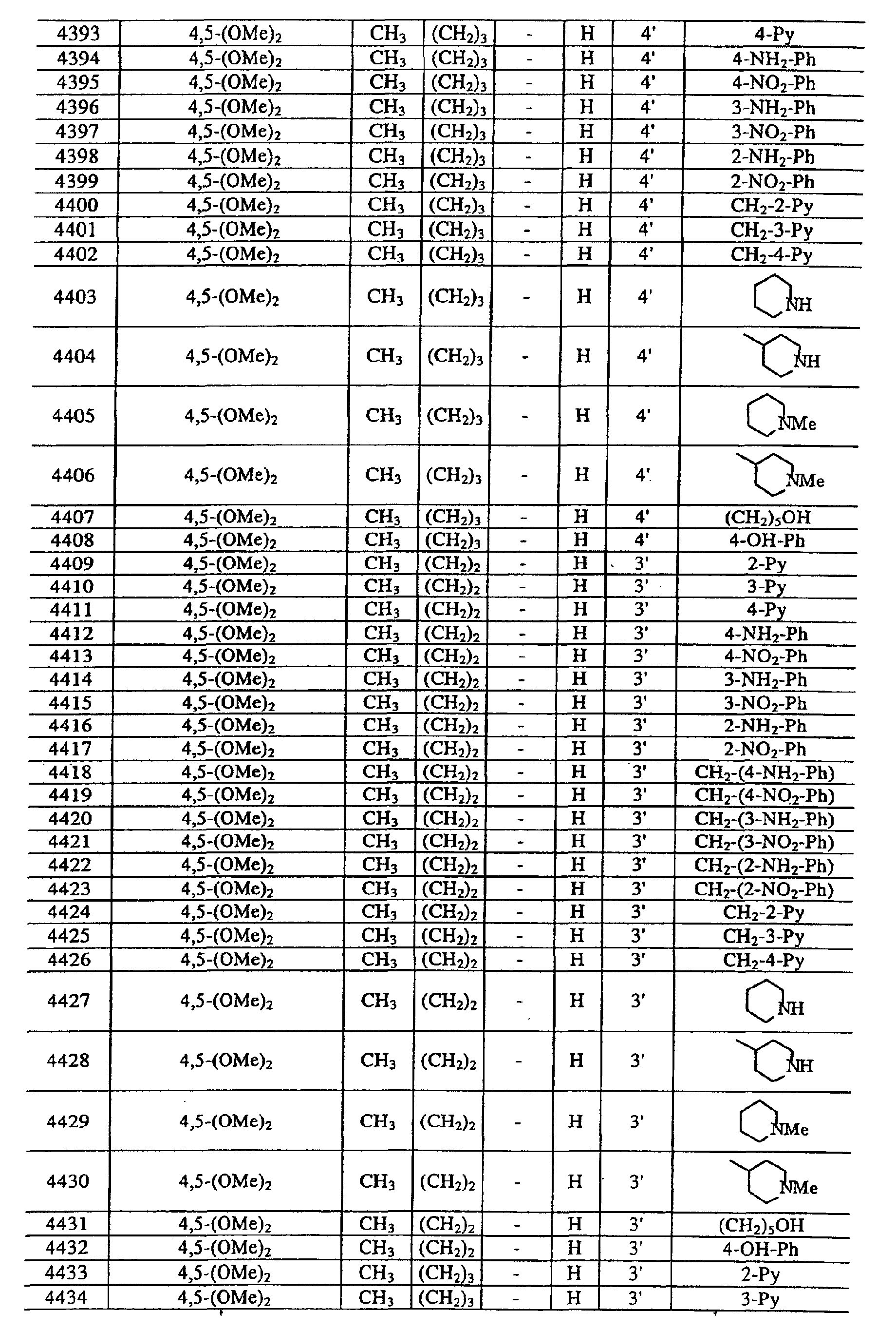

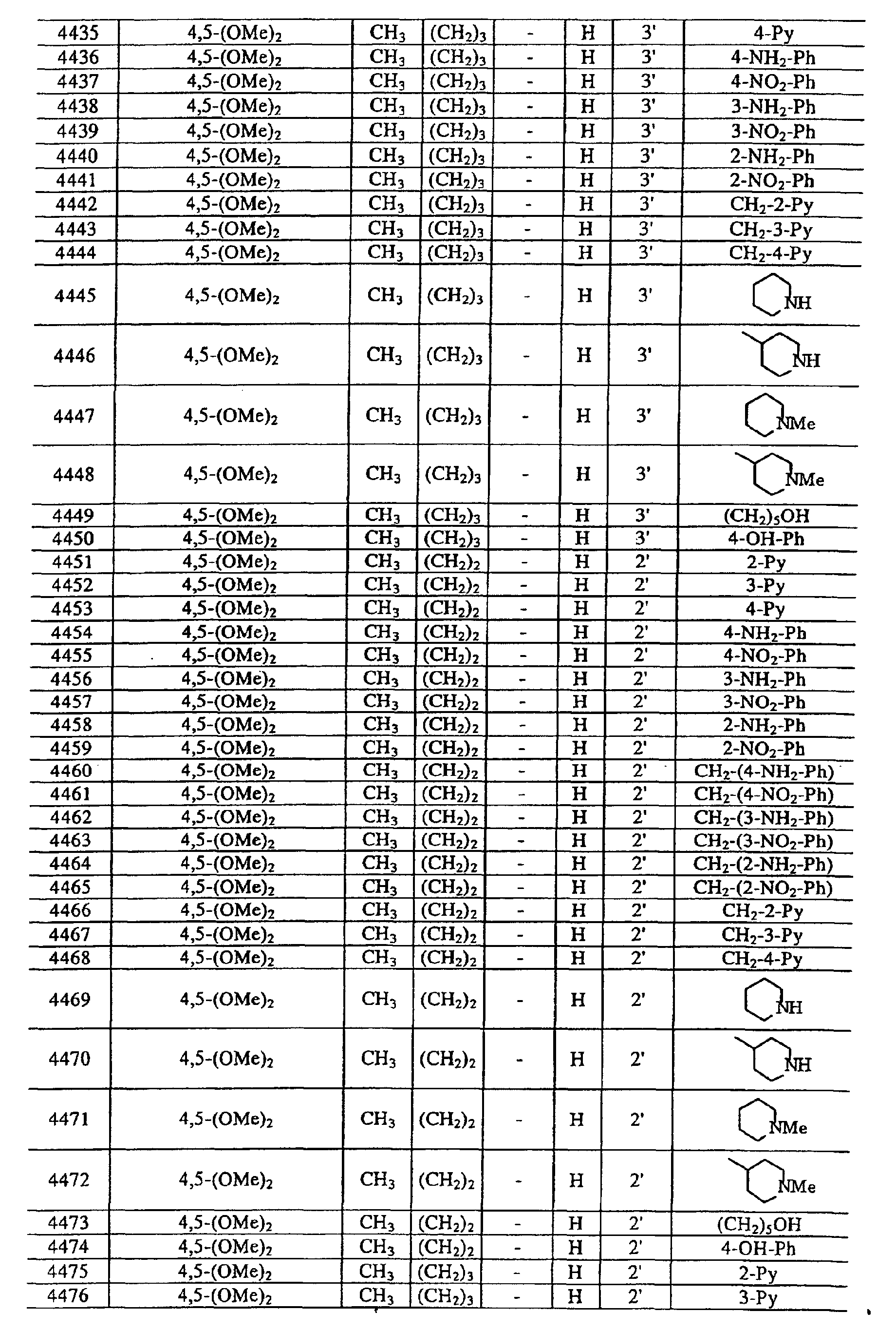

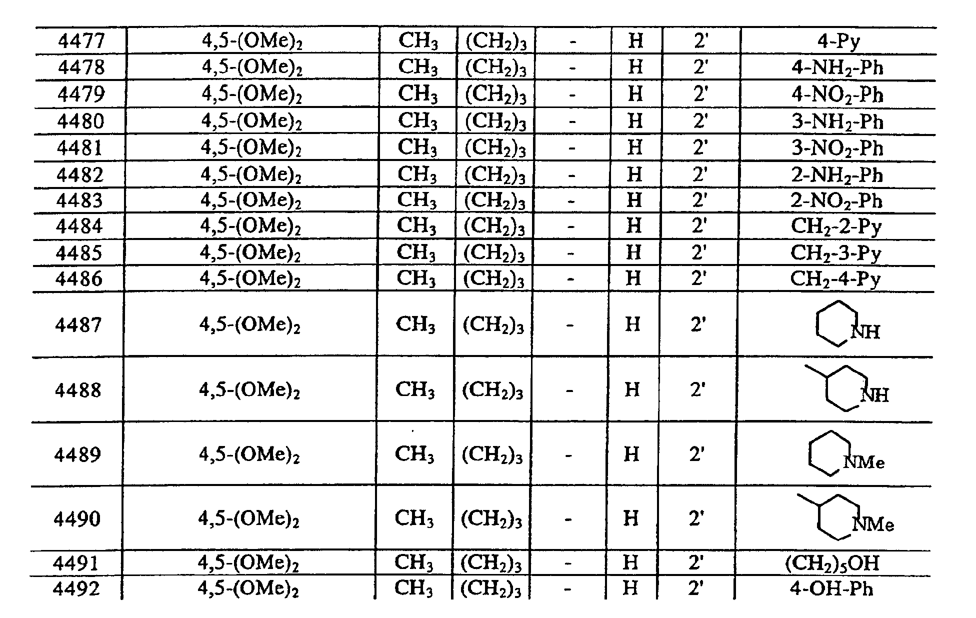

In Table I to Table 10, Py denotes a pyridyl group, Ph denotes a phenyl group, Me denotes a methyl group, Et denotes an ethyl group, "Pr denotes a n-propyl group, Ac denotes an acetyl group, ⁿBu denotes a n-butyl group, Bn denotes a benzyl group, c-Pen denotes a cyclopentyl group, c-Hex denotes a cyclohexyl group, c-Hep denotes a cycloheptyl group, ⁱPr denotes an isopropyl group, and Nap denotes a naphthyl group, respectively.

A pharmaceutical composition comprising, as an active ingredient, the diarylamide derivative of the present invention, that is, a medical composition, can be administered in various forms including injections such as intravenous injection, subcutaneous injection, and intramuscular injection and external preparations, in addition to internal preparations such as tablets, capsules, powders, and granules. For example, the diarylamide derivative of the present invention can be mixed with excipients such as lactose and starch, lubricants such as magnesium stearate and talc, and other conventional additives to prepare tablets. Distilled water, saline, alcohol and the like can be used to prepare injections, and buffers, isotonizing agents, preservatives, stabilizers and the like can be optionally added.

The dose of the diarylamide derivative of the present invention is properly determined in accordance with, for example, sex, age, and weight of a patient and a type and condition of disease. When internally administered, the dose can be in the range of approximately 0.1 to 100 mg/kg per day, preferably in the range of 1 to 10 mg/kg, in a single dose or several separate doses.

This specification includes part or all of the contents as disclosed in the specifications of Japanese Patent Applications Nos. 281271/1999 and 290789/1999, which are the base of the priority claim of the present application.

### BEST MODE FOR CARRYING OUT THE INVENTION

The present invention is further described by the following Reference Examples, Examples, and Preparation Example although these are not intended to limit the scope of the present invention.

### Example 1

### N-Phenyl-N'-[4-[(4,5-dimethoxy-2-ethoxycarbonylphenyl)aminocarbonyl]-phenyl]urea (compound number 1)

0.75 g of 4,5-dimethoxy-2-nitrobenzoic acid was dissolved in 100 ml of ethanol and 3 ml of concentrated sulfuric acid was then added thereto. The mixture was stirred for 18 hours under reflux and neutralized with 5% aqueous solution of sodium hydroxide. Thereafter, the precipitated solid was collected by suction filtration and washed with water, followed by drying. Thus, 0.53 g of white solid was obtained. Subsequently, 0.30 g of this solid and 60 mg of 5% Pd/C were added to 20 ml of ethanol, and the mixture was stirred under hydrogen atmosphere at room temperature for 14 hours. The reaction solution was filtered and the filtrate was concentrated. Thus, 0.27 g of ethyl 2-amino-4,5-dimethoxybenzoate was obtained as a white solid.

Subsequently, 0.26 g of this solid was dissolved in 20 ml of dichloromethane, 0.27 g of 4-nitrobenzoyl chloride and 0.5 ml of triethylamine were then added thereto. The mixture was stirred for 30 minutes at room temperature. The reaction solution was poured into saturated sodium bicarbonate water, extracted with dichloromethane, the organic layer was dried with anhydrous magnesium sulfate, followed by concentration. The residue was washed with methanol and then dried. Thus, 0.36 g of yellow solid was obtained.

Thereafter, 0.36 g of this solid and 50 mg of 5% Pd/C were added to 100 ml of methanol. The mixture was then stirred under hydrogen atmosphere at room temperature for 32 hours. The reaction solution was filtered and the filtrate was concentrated. Thus, 0.28 g of ethyl 2-(4-aminophenyl)carbonylamino-4,5-dimethoxybenzoate was obtained as a yellow solid.

Subsequently, 90 mg of this solid, 0.24 g of phenyl isocyanate, and 0.12 g of triethylamine were added to 20 ml of toluene and the mixture was then stirred under reflux for 18 hours. The reaction solution was poured into water and extracted with dichloromethane, and the organic layer was dried with anhydrous magnesium sulfate, followed by concentration. The residue was purified by column chromatography on silica gel (eluent, dichloromethane : ethyl acetate = 10:1 → dichloromethane : methanol = 30 : 1). Thus, 80 mg of the title compound was obtained as a white solid.

¹H-NMR (DMSO-d₆, 270 MHz) δ ppm: 1.35 (t, J = 7.2 Hz, 3H), 3.80 (s, 3H), 3.88 (s, 3H), 4.37 (q, J = 7.2 Hz, 3H), 6.99 (t, J = 7.3 Hz, 1H), 7.30 (m, 3H), 7.48 (d, J = 7.5 Hz, 2H), 7.48 (s, 1H), 7.67 (d, J = 7.3 Hz, 2H), 7.90 (d, J = 8.9 Hz, 2H), 8.45 (s, 1H), 9.05 (s, 1H), 9.31 (s, 1H), 11.75 (s, 1H)

### Example 2

### N-(4-Nitrophenyl)-N'-[4-[(4,5-dimethoxy-2-ethoxycarbonylphenyl)-aminocarbonyl]phenyl]urea (compound number 25)

The title compound was synthesized in the same manner as in Example 1.

¹H-NMR (DMSO-d₆, 270 MHz) δ ppm : 1.35 (t, J = 7.2 Hz, 3H), 3.80 (s, 3H), 3.88 (s, 3H), 4.37 (q, J = 7.2 Hz, 2H), 7.48 (s, 1H), 7.71 (m, 4H), 7.92 (d, J = 8.9 Hz, 2H), 8.22 (d, J = 9.2 Hz, 2H), 8.43 (s, 1H), 9.40 (s, 1H), 9.65 (s, 1H), 11.76 (s, 1H)

### Example 3

### N-(4-Aminophenyl)-N'-[4-[(4,5-dimethoxy-2-ethoxycarbonylphenyl)-aminocarbonyl]phenyl]urea (compound number 28)

The compound (90 mg) synthesized in Example 2 and 20 mg of 5% Pd/C were added to 10 ml of ethanol. The mixture was then stirred under hydrogen atmosphere at room temperature for 14 hours. The reaction solution was filtered and the filtrate was concentrated. The residue was purified by column chromatography on silica gel (eluent, dichloromethane : methanol = 50 : 1). Thus, 50 mg of the title compound was obtained as a pale pink solid.

¹H-NMR (DMSO-d₆, 270 MHz) δ ppm : 1.35 (t, J = 7.2 Hz, 3H), 3.80 (s, 3H), 3.87 (s, 3H), 4.37 (q, J = 7.2 Hz, 2H), 4.80 (s, 2H), 6.52 (d, J = 8.1 Hz, 2H), 7.10 (d, J = 8.9 Hz, 2H), 7.48 (s, 1H), 7.63 (d, J = 8.9 Hz, 2H), 7.87 (d, J = 8.9 Hz, 2H), 8.22 (d, J = 9.2 Hz, 2H), 8.42 (s, 1H), 8.45 (s, 1H), 9.03 (s, 1H), 11.74 (s, 1H)

### Example 4

### N-(4-Fluorophenyl)-N'-[4-[(4,5-dimethoxy-2-ethoxycarbonylphenyl)-aminocarbonyl]phenyl]urea (compound number 19)

60 mg of Ethyl 2-(4-aminophenyl)carbonylamino-4,5-dimethoxybenzoate, 0.11 g of 4-fluorophenyl isocyanate, and 70 mg of 4-dimethylaminopyridine were added to 20 ml of tetrahydrofuran. The mixture was then stirred at 70°C for 5 hours. The reaction solution was poured into water and extracted with dichloromethane, and the organic layer was dried with anhydrous magnesium sulfate, followed by concentration. The residue was purified by column chromatography on silica gel (eluent, dichloromethane : ethyl acetate =10 : 1 → dichloromethane : methanol = 30 : 1). Thus, 60 mg of the title compound was obtained as a white solid.

¹H-NMR (DMSO-d₆, 270 MHz) δ ppm : 1.35 (t, J=7.2Hz, 3H), 3.80 (s, 3H), 3.88 (s, 3H), 4.37 (q, J = 7.2 Hz, 2H), 7.14 (t, J = 6.2 Hz, 2H), 7.48 (s, 1H), 7.49 (dd, J = 3.8, 8.6 Hz, 2H), 7.67 (d, J = 8.6 Hz, 2H), 7.89 (d, J = 8.9 Hz, 2H), 8.44 (s, 1H), 9.12 (s, 1H), 9.34 (s, 1H), 11.75 (s, 1H)

### Example 5

### N-(4-Ethoxycarbonylphenyl)-N'-[4-[(4,5-dimethoxy-2-ethoxycarbonylphenyl)aminocarbonyl]phenyl]urea (compound number 14)

The title compound was synthesized in the same manner as in Example 4.

¹H-NMR (DMSO-d₆, 270 MHz) δ ppm : 1.32 (m, 6H), 3.80 (s, 3H), 3.88 (s, 3H), 4.33 (m, 4H), 7.48 (s, 1H), 7.62 (d, J = 8.4 Hz, 2H), 7.68 (d, J = 8.6 Hz, 2H), 7.91 (m, 4H), 8.44 (s, 1H), 9.29 (s, 1H), 9.34 (s, 1H), 11.76 (s, 1H)

### Example 6

### N-(4-Acetylphenyl)-N'-[4-[(4,5-dimethoxy-2-ethoxycarbonylphenyl)-aminocarbonyl]phenyl]urea (compound number 12)

The title compound was synthesized in the same manner as in Example 4.

¹H-NMR (DMSO-d₆, 270 MHz) δ ppm : 1.35 (t, J = 7.2 Hz, 3H), 3.80 (s, 3H), 3.88 (s, 3H), 4.37 (q, J = 7.2 Hz, 2H), 7.48 (s, 1H), 7.62 (d, J = 8.9 Hz, 2H), 7.68 (d, J = 8.9 Hz, 2H), 7.93 (m, 4H), 8.44 (s, 1H), 9.34 (s, 1H), 9.38 (s, 1H), 11.76 (s, 1H)

### Example 7

### N-(4-Methoxyphenyl)-N'-[4-[(4,5-dimethoxy-2-ethoxycarbonylphenyl)-aminocarbonyl]phenyl]urea (compound number 35)

The title compound was synthesized in the same manner as in Example 4.

¹H-NMR (DMSO-d₆, 270 MHz) δ ppm : 1.35 (t, J = 7.2 Hz, 3H), 3.73 (s, 3H), 3.80 (s, 3H), 3.88 (s, 3H), 4.37 (q, J = 7.2 Hz, 2H), 6.89 (d, J = 9.2 Hz, 2H), 7.38 (d, J = 8.6 Hz, 2H), 7.48 (s, 1H), 7.65 (d, J = 8.9 Hz, 2H), 7.89 (d, J = 8.9 Hz, 2H), 8.45 (s, 1H), 8.73 (s, 1H), 9.11 (s, 1H), 11.75 (s, 1H)

### Example 8

### N-(2-Methoxyphenyl)-N'-[4-[(4,5-dimethoxy-2-ethoxycarbonylphenyl)-aminocarbonyl]phenyl]urea (compound number 37)

The title compound was synthesized in the same manner as in Example 4.

¹H-NMR (DMSO-d₆, 270 MHz) δ ppm : 1.35 (t, J = 7.2 Hz, 3H), 3.80 (s, 3H), 3.88 (s, 3H), 3.89 (s, 3H), 4.37 (q, J = 7.2 Hz, 2H), 7.00 (m, 3H), 7.48 (s, 1H), 7.66 (d, J = 8.4 Hz, 2H), 7.90 (d, J = 8.9 Hz, 2H), 8.13 (dd, J = 1.6, 7.3 Hz, 1H), 8.41 (s, 1H), 8.45 (s, 1H), 9.75 (s, 1H), 11.76 (s, 1H)

### Example 9

### N-(3-Methoxyphenyl)-N'-[4-[(4,5-dimethoxy-2-ethoxycarbonylphenyl)-aminocarbonyl]phenyl]urea (compound number 36)

The title compound was synthesized in the same manner as in Example 4.

¹H-NMR (DMSO-d₆, 270 MHz) δ ppm : 1.35 (t, J = 7.2 Hz, 3H), 3.74 (s, 3H), 3.80 (s, 3H), 3.88 (s, 3H), 4.37 (q, J = 7.2 Hz, 2H), 6.58 (dd, J = 2.4, 8.1 Hz, 1H), 6.96 (d, J = 9.5 Hz, 1H), 7.20 (m, 2H), 7.48 (s, 1H), 7.66 (d, J = 8.6Hz, 2H), 7.90 (d, J = 8.9 Hz, 2H), 8.44 (s, 1H), 8.97 (s, 1H), 9.21 (s, 1H), 11.75 (s, 1H)

### Example 10

### N-(3,4,5-Trimethoxyphenyl)-N'-[4-[(4,5-dimethoxy-2-ethoxycarbonylphenyl)aminocarbonyl]phenyl]urea (compound number 101)

The title compound was synthesized in the same manner as in Example 4.

¹H-NMR (DMSO-d₆, 270 MHz) δ ppm : 1.35 (t, J = 7.2 Hz, 3H), 3.61 (s, 3H), 3.76 (s, 6H), 3.80 (s, 3H), 3.88 (s, 3H), 4.37 (q, J = 7.2 Hz, 2H), 6.83 (s, 2H), 7.48 (s, 1H), 7.67 (d, J = 8.4 Hz, 2H), 7.90 (d, J = 8.9 Hz, 2H), 8.44 (s, 1H), 8.93 (s, 1H), 9.19 (s, 1H), 11.74 (s, 1H)

### Example 11

### N-(3-Pyridyl)-N'-[4-[(4,5-dimethoxy-2-ethoxycarbonylphenyl)-aminocarbonyl]phenyl]urea (compound number 972)

The title compound was synthesized in the same manner as in Example 4.

¹H-NMR (DMSO-d₆, 270 MHz) δ ppm : 1.35 (t, J = 7.2 Hz, 3H), 3.80 (s, 3H), 3.88 (s, 3H), 4.37 (q, J = 7.2 Hz, 2H), 7.34 (m, 1H), 7.48 (s, 1H), 7.69 (d, J = 8.6 Hz, 2H), 7.90 (d, J = 8.9 Hz, 2H), 7.97 (d, J = 8.9 Hz, 1H), 8.20 (d, J = 4.3 Hz, 1H), 8.44 (s, 1H), 8.66 (s, 1H), 9.50 (s, 1H), 9.70 (s, 1H), 11.75 (s, 1H)

### Example 12

### N-Benzyl-N'-[4-[(4,5-dimethoxy-2-ethoxycarbonylphenyl)aminocarbonyl]-phenyl]urea (compound number 112)

The title compound was synthesized in the same manner as in Example 4.

¹H-NMR (DMSO-d₆, 270 MHz) δ ppm : 1.34 (t, J = 7.2 Hz, 3H), 3.80 (s, 3H), 3.87 (s, 3H), 4.37 (m, 4H), 6.99 (t, J = 6.5 Hz, 1H), 7.28 (m, 5H), 7.47 (s, 1H), 7.61 (d, J = 8.6 Hz, 2H), 7.84 (d, J = 8.9 Hz, 1H), 8.44 (s, 1H), 9.18 (s, 1H), 11.72 (s, 1H)

### Example 13

### N-Cyclohexyl-N'-[4-[(4,5-dimethoxy-2-ethoxycarbonylphenyl)-aminocarbonyl]phenyl]urea (compound number 103)

The title compound was synthesized in the same manner as in Example 4.

¹H-NMR (DMSO-d₆, 270 MHz) δ ppm : 1.20 (m, 6H), 1.34 (t, J = 7.2Hz, 3H), 1.65 (m, 4H), 3.48 (m, 1H), 3.79 (s, 3H), 3.87 (s, 3H), 4.37 (m, 4H), 6.42 (d, J = 7.8 Hz, 1H), 7.47 (s, 1H), 7.57 (d, J = 8.9 Hz, 2H), 7.83 (d, J = 8.9 Hz, 1H), 8.45 (s, 1H), 8.88 (s, 1H), 11.72 (s, 1H)

### Example 14

### N-n-butyl-N'-[4-[(4,5-dimethoxy-2-ethoxycarbonylphenyl)-aminocarbonyl]phenyl]urea (compound number 107)

The title compound was synthesized in the same manner as in Example 4.

¹H-NMR (DMSO-d₆, 270 MHz) δ ppm : 0.90 (t, J = 6.7 Hz, 3H), 1.27 (m,4H), 1.34 (t, J = 7.2 Hz, 3H), 3.10 (q, J = 5.7 Hz, 2H), 3.80 (s, 3H), 3.87 (s, 3H), 4.37 (m, 4H), 6.45 (t, J =5.4 Hz, 1H), 7.47 (s, 1H), 7.59 (d, J = 8.9 Hz, 2H), 7.83 (d, J = 8.6 Hz, 1H), 8.45 (s, 1H), 8.98 (s, 1H), 11.72 (s, 1H)

### Example 15

### N-Phenyl-N'-[4-[(4,5-dimethoxy-2-ethoxycarbonylphenyl)aminocarbonyl]-phenyl]thiourea (compound number 315)

The title compound was synthesized in the same manner as in Example 4.

¹H-NMR (DMSO-d₆, 270 MHz) δ ppm : 1.35 (t, J = 7.2 Hz, 3H), 3.80 (s, 3H), 3.88 (s, 3H), 4.37 (q, J = 7.2 Hz, 2H), 7.14 (t, J = 6.8 Hz, 1H), 7.35 (m, 3H), 7.48 (m, 3H), 7.76 (d, J = 8.9 Hz, 2H), 7.91 (d, J = 8.9 Hz, 2H), 8.44 (s, 1H), 10.21 (s, br, 2H), 11.80 (s, 1H)

### Example 16

### N-Phenyl-N'-[3-[(4,5-dimethoxy-2-ethoxycarbonylphenyl)aminocarbonyl]-phenyl]urea (compound number 691)

The title compound was synthesized in the same manner as in Example 4.

¹H-NMR (DMSO-d₆, 270 MHz) δ ppm : 1.34 (t, J = 7.2 Hz, 3H), 3.81 (s, 3H), 3.89 (s, 3H), 4.36 (q, J = 7.2 Hz, 2H), 6.99 (t, J = 7.3 Hz, 1H), 7.29 (t, J = 8.3 Hz, 2H), 7.49 (m, 5H), 7.23 (m, 1H), 8.08 (s, 1H), 8.42 (s, 1H), 8.92 (s, 1H), 9.13 (s, 1H), 11.76 (s, 1H)

### Example 17

### N-Phenyl-N'-[2-[(4,5-dimethoxy-2-ethoxycarbonylphenyl)aminocarbonyl]-phenyl]urea (compound number 692)

The title compound was synthesized in the same manner as in Example 4.

¹H-NMR (DMSO-d₆, 270 MHz) δ ppm : 1.35 (t, J = 7.2 Hz, 3H), 3.80 (s, 3H), 3.88 (s, 3H), 4.37 (q, J = 7.2 Hz, 2H), 6.96 (t, J = 7.3 Hz, 1H), 7.16 (t, J = 7.8 Hz, 1H), 7.26 (t, J = 7.3 Hz, 2H), 7.51 (m, 4H), 7.80 (d, J = 7.0 Hz, 1H), 8.12 (s, 1H), 8.20 (d, J = 5.7 Hz, 2H), 9.61 (s, 1H), 9.79 (s, 1H), 11.47 (s, 1H)

### Example 18

### N-Phenyl-N'-[4-[(4,5-dimethoxy-2-carbamoylphenyl)aminocarbonyl]-phenyl]urea (compound number 158)

0.66 g of 4,5-dimethoxy-2-nitrobenzoic acid and 5 ml of thionyl chloride were added to 40 ml of chloroform. The mixture was stirred under reflux for 6 hours and concentrated. The residue was dissolved in 20 ml of dichloromethane and 20 ml of aqueous ammonia was then added thereto in an ice bath. The mixture was vigorously stirred at room temperature for 10 minutes, the organic layer was fractionated and concentrated, the residue and 0.20 g of 5% Pd/C were added to 50 ml of methanol, and the mixture was stirred under hydrogen atmosphere at room temperature for 19 hours. The reaction solution was filtered and the filtrate was concentrated. Thus, 0.55 g of 2-amino-4,5-dimethoxybenzamide was obtained as a white solid.

Subsequently, 0.55 g of this solid was dissolved in 50 ml of dichloromethane and 2.00 g of 4-nitrobenzoyl chloride and 2 ml of triethylamine were then added thereto. The resultant mixture was stirred at room temperature for 6 hours. The reaction solution was poured into saturated sodium bicarbonate water and extracted with dichloromethane, and the organic layer was dried with anhydrous magnesium sulfate, followed by concentration. The residue was washed with methanol and dried. Thus, 0.72 g of 2-(4-nitrophenyl)-carbonylamino-4,5-dimethoxybenzamide was obtained as a yellow ocher solid.

Thereafter, 0.68 g of this solid and 0.10 g of 5% Pd/C were added to 50 ml of methanol. Under hydrogen atmosphere, the obtained mixture was then stirred at room temperature for 40 hours. The reaction solution was filtered and the filtrate was concentrated. Thus, 0.35 g of 2-(4-aminophenyl)-carbonylamino-4,5-dimethoxybenzamide was obtained as a yellow solid.

Subsequently, 0.12 g of this solid, 0.14 g of phenyl isocyanate, and 0.10 g of 4-dimethylaminopyridine were added to 30 ml of tetrahydrofuran. The mixture was then stirred at 70°C for 4 hours. The reaction solution was poured into water and extracted with dichloromethane, and the organic layer was dried with anhydrous magnesium sulfate, followed by concentration. The residue was purified by column chromatography on silica gel (eluent, dichloromethane : ethyl acetate =20 : 1 → dichloromethane : methanol = 30 : 1) to obtain 80 mg of the title compound as a white solid.

¹H-NMR (DMSO-d₆, 270 MHz) δ ppm : 3.81 (s, 3H), 3.84 (s, 3H), 7.00 (t, J = 8.1 Hz, 1H), 7.30 (t, J = 8.4 Hz, 2H), 7.44 (s, 1H), 7.47 (d, J = 7.9 Hz, 2H), 7.64 (m, 3H), 7.87 (d, J = 8.6 Hz, 2H), 8.31 (s, 1H), 8.53 (s, 1H), 8.87 (s, 1H), 9.13 (s, 1H), 13.21 (s, 1H)

### Example 19

### N-Phenyl-N'-[4-[(4,5-dimethoxy-2-carbamoylphenyl)aminocarbonyl]-phenyl]-N'-methylurea (compound number 976)

40 mg of 2-(4-aminophenyl)-carbonylamino-4,5-dimethoxybenzamide, 60 mg of hydroxybenzotriazole (HOBt), 50 mg of triethylamine, and 70 mg of 4-methylaminobenzoic acid were added to DMF. The mixture was stirred for 30 minutes, and 80 mg of 1-ethyl-3-[3-(dimethylamino)propyl]-carbodiimide hydrochloride (WSCl) was added thereto in an ice bath and then returned to room temperature, followed by stirring for 50 hours. The reaction solution was poured into water and extracted with dichloromethane, and the organic layer was dried with anhydrous magnesium sulfate, followed by concentration. The residue was purified by column chromatography on silica gel (eluent, dichloromethane : ethyl acetate = 30 : 1 → dichloromethane : methanol = 50 : 1) to obtain 80 mg of white solid.

Subsequently, 30 mg of this solid, 60 mg of phenyl isocyanate, and 30 mg of 4-dimethylaminopyridine were added to 10 ml of tetrahydrofuran. The mixture was then stirred at 70°C for 6 hours. The reaction solution was poured into water and extracted with dichloromethane, and the organic layer was dried with anhydrous magnesium sulfate, followed by concentration. The residue was purified by column chromatography on silica gel (eluent, dichloromethane : ethyl acetate = 30 : 1 → dichloromethane : methanol = 30 : 1) to obtain 20 mg of the title compound as a white solid.

¹H-NMR (DMSO-d₆, 270 MHz) δ ppm : 3.35 (s, 3H), 3.81 (s, 3H), 3.85 (s, 3H), 6.97 (t, J = 8.5 Hz, 1H), 7.25 (t, J = 8.4 Hz, 2H), 7.45 (m, 5H), 7.68 (s, 1H), 7.94 (d, J = 8.1 Hz, 2H), 8.33 (s, 1H), 8.53 (s, 1H), 8.59 (s, 1H), 13.32 (s, 1H)

### Example 20

### N-Phenyl-N'-[3-[(4,5-dimethoxy-2-carbamoylphenyl)aminocarbonyl]-4-pyridyl]urea (compound number 971)

The title compound was synthesized in the same manner as in Example 19.

¹H-NMR (DMSO-d₆, 270 MHz) δ ppm : 3.82 (s, 3H), 3.85 (s, 3H), 7.02 (t, J = 7.3 Hz, 1H), 7.32 (m, 3H), 7.46 (s, 1H), 7.51 (d, J = 5.1 Hz, 2H), 7.69 (dd, J = 1.9, 5.1 Hz, 1H), 8.12 (s,1H), 8.25 (d, J = 2.4 Hz, 1H), 8.47 (d, J = 5.4 Hz, 1H), 8.57 (s, 1H), 9.33 (s, 1H), 9.83 (s, 1H), 13.33 (s, 1H)

### Example 21

### N-Phenyl-N'-[4-[(4,5-dimethoxy-2-carbamoylphenyl)aminocarbonyl]-2-pyridyl]urea (compound number 972)

The title compound was synthesized in the same manner as in Example 19.

¹H-NMR (DMSO-d₆, 270 MHz) δ ppm : 3.82 (s, 3H), 3.84 (s, 3H), 7.04 (t, J = 7.3 Hz, 1H), 7.33 (t, J = 7.8 Hz, 3H), 7.46 (s, 1H), 7.54 (d, J = 7.0 Hz, 2H ), 7.73 (s,1H), 7.79 (d, J = 8.9 Hz, 1H), 8.21 (dd, J = 2.4, 8.6Hz, 1H), 8.36 (s, 1H), 8.48 (s, 1H), 8.83 (d, J = 2.1 Hz, 1H), 9.86 (s, 1H), 10.20 (s, 1H), 13.35 (s, 1H)

### Example 22

### N-Phenyl-N'-[4-[(4,5-dimethoxy-2-carbamoylphenyl)aminocarbonyl]-3-methoxyphenyl]urea (compound number 726)

The title compound was synthesized in the same manner as in Example 19.

¹H-NMR (DMSO-d₆, 270 MHz) δ ppm : 3.81 (s, 3H), 3.84 (s, 3H), 3.99 (s, 3H), 6.99 (t, J = 7.3 Hz, 1H), 7.31 (t, J = 8.1 Hz, 2H), 7.46 (s, 1H), 7.49 (m, 2H), 7.58 (s, 1H), 7.73 (s,1H), 8.33 (s,1H), 8.36 (s,1H), 8.56 (d, J = 3.5 Hz, 2H), 9.49 (s, 1H), 13.29 (s, 1H)

### Example 23

### N-Phenyl-N'-[3-[(4,5-dimethoxy-2-carbamoylphenyl)aminocarbonyl]-4-methoxyphenyl]urea (compound number 727)

The title compound was synthesized in the same manner as in Example 19.

¹H-NMR (DMSO-d₆, 270 MHz) δ ppm : 3.81 (s, 3H), 3.84 (s, 3H), 3.98 (s, 3H), 6.98 (t, J = 7.3 Hz, 1H), 7.19 (d, J = 8.4 Hz, 1H), 7.30 (t, J = 7.8 Hz, 1H), 7.44 (s, 1H), 7.46 (t, J = 7.8 Hz, 2H), 7.60 (dd, J = 2.1, 8.1 Hz, 1H), 7.63 (s, 1H), 8.29 (s, 1H), 8.38 (s,1H), 8.53 (s,1H), 8.79 (d, J = 2.4 Hz, 2H), 9.37 (s, 1H), 13.14 (s, 1H)

### Example 24

### N-Phenyl-N'-[4-[(4,5-dimethoxy-2-carbamoylphenyl)aminocarbonylmethyl]-phenyl]urea (compound number 748)

The title compound was synthesized in the same manner as in Example 19.

¹H-NMR (DMSO-d₆, 270 MHz) δ ppm : 3.59 (s, 2H), 3.76 (s, 3H), 3.77 (s, 3H), 6.95 (t, J = 8.1 Hz, 1H), 7.24 (m, 9H), 7.56 (s, 1H), 8.16 (s, 1H), 8.28 (s, 1H), 8.76 (s, 2H), 12.13 (s, 1H)

### Example 25

### N-Phenyl-N'-[4-[(4,5-dimethoxy-2-carbamoylphenyl)aminocarbonylethyl]-phenyl]urea (compound number 751)

The title compound was synthesized in the same manner as in Example 19.

¹H-NMR (DMSO-d₆, 270 MHz) δ ppm : 2.61 (t, J = 7.6 Hz, 2H), 2.87 (t, J = 8.4 Hz, 2H), 3.78 (s, 6H), 6.94 (t, J = 7.6 Hz, 1H), 7.15 (d, J = 8.4 Hz, 2H), 7.26 (t, J = 8.4 Hz, 2H), 7.35 (s, 1H), 7.36 (d, J = 8.4 Hz, 2H), 7.44 (d, J =7.8 Hz, 2H), 7.56 (s, 1H), 8.17 (s, 1H), 8.29 (s, 1H), 8.73 (s, 1H), 8.77 (s, 1H), 12.12 (s, 1H)

### Example 26

### N-[4-[(4,5-Dimethoxy-2-carbamoylphenyl)aminocarbonyl]phenyl]-N'-methyl-N'-phenylurea (compound number 977)

0.11 g of 2-(4-aminophenyl)-carbonylamino-4,5-dimethoxybenzamide was dissolved in 10 ml of THF, 0.50 g of N-phenyl-N-methylcarbamoyl chloride and 1 ml of diisopropylethylamine were then added thereto. The mixture was stirred under reflux for 16 hours. The reaction solution was poured into water and extracted with dichloromethane, and then dried with anhydrous magnesium sulfate, followed by concentration. The residue was washed with methanol and dried to obtain 50 mg of white solid.

¹H-NMR (DMSO-d₆, 270 MHz) δ ppm : 3.29 (s, 3H), 3.81 (s, 3H), 3.83 (s, 3H), 7.27 (t, J = 6.8 Hz, 1H), 7.44 (m, 5H), 7.63 (m,3H), 7.80 (d, J = 8.9 Hz, 2H), 8.30 (s, 1H), 8.52 (s, 1H), 8.53 (s, 1H), 13.18 (s, 1H)

### Example 27

### N-[4-[(4,5-Dimethoxy-2-carbamoylphenyl)aminocarbonyl]phenyl]-N,N'-dimethyl-N'-phenylurea (compound number 978)

The title compound was synthesized in the same manner as in Example 26.

¹H-NMR (DMSO-d₆, 270 MHz) δ ppm : 3.12 (s, 3H), 3.18 (s, 3H), 3.81 (s, 3H), 3.83 (s, 3H), 7.00 (m, 3H), 7.12 (m, 4H), 7.44 (s,1H), 7.68 (m, 3H), 8.32 (s, 1H), 8.49 (s, 1H), 13.18 (s, 1H)

### Example 28

### N-(3,4,5-Trimethoxyphenyl)-N'-[4-[(4,5-dimethoxy-2-ethoxycarbonylphenyl)aminocarbonyl]-3-methoxyphenyl]urea (compound number 792)

The tide compound was synthesized in the same manner as in Example 18.

¹H-NMR (DMSO-d₆, 270 MHz) δ ppm : 3.61 (s, 3H), 3.76 (s, 6H), 3.81 (s, 3H), 3.84 (s, 3H), 3.98 (s, 3H), 6.81 (s, 2H), 7.53 (m, 3H), 7.74 (s, 1H), 8.33 (m, 2H), 8.51 (s, 1H), 8.55 (s, 1H), 9.49 (s, 1H), 13.28 (s, 1H)

### Example 29

### N-Phenyl-N'-[4-[(4-methyl-2-carbamoylphenyl)aminocarbonyl]phenyl]urea (compound number 633)

The title compound was synthesized in the same manner as in Example 18.

¹H-NMR (DMSO-d₆, 270 MHz) δ ppm : 2.32 (s, 3H), 6.99 (t, J = 8.1 Hz, 1H), 7.37 (m,3H), 7.48 (d, J = 7.3 Hz, 2H), 7.66 (m, 6H), 8.36 (s, 1H), 8.59 (d, J = 8.9 Hz, 2H), 9.00 (s, 1H), 9.26 (s, 1H), 12.73 (s, 1H)

### Example 30

### N-Phenyl-N'-[4-[(6-carbamoyl-3,4-methylenedioxyphenyl)aminocarbonyl]-phenyl]urea (compound number 652)

The title compound was synthesized in the same manner as in Example 18.

¹H-NMR (DMSO-d₆, 270 MHz) δ ppm : 6.12 (s,2H), 6.99 (t, J = 7.3 Hz, 1H), 7.30 (t, J = 7.3 Hz, 2H), 7.47 (d, J = 7.9 Hz, 2H), 7.50 (s, 1H), 7.63 (d, J = 8.9 Hz, 2H), 7.71 (s, 1H), 7.86 (d, J = 8.4 Hz, 2H), 8.21 (s, 1H), 8.36 (s, 1H), 8.91 (s, 1H), 9.18 (s, 1H), 13.28 (s, 1H)

### Example 31

### N-Phenyl-N'-[4-[(2-carbamoyl-4-methoxyphenyl)aminocarbonyl]-phenyl]urea (compound number 631)

The title compound was synthesized in the same manner as in Example 18.

¹H-NMR (DMSO-d₆, 270 MHz) δ ppm : 3.89 (s, 3H), 6.99 (t, J = 8.1 Hz, 1H), 7.30 (t, J = 7.8 Hz, 2H), 7.45 (m,4H), 7.64 (m, 3H), 7.97 (s, 1H), 8.13 (d, J = 8.7 Hz, 2H), 8.92 (s, 1H), 9.14 (s, 1H), 12.37 (s, 1H)

### Example 32

### N-(4-Ethoxycarbonylphenyl)-N'-[4-[(4,5-dimethoxy-2-carbamoylphenyl)-aminocarbonyl]phenyl]urea (compound number 171)

The title compound was synthesized in the same manner as in Example 18.

¹H-NMR (DMSO-d₆, 270 MHz) δ ppm : 1.32 (t, J = 7.3Hz, 3H), 3.81 (s, 3H), 3.84 (s, 3H), 4.33 (q, J = 7.3 Hz, 2H), 7.45 (s, 1H), 7.65 (m, 5H), 7.89 (m, 4H), 8.32 (s, 1H), 8.53 (s, 1H), 9.46 (s, 1H), 9.51 (s, 1H), 13.22 (s, 1H)

### Example 33

### N-Phenyl-N'-[3-[(2-carbamoylthienyl)aminocarbonyl]phenyl]urea (compound number 916)

The title compound was synthesized in the same manner as in Example 18.

¹H-NMR (DMSO-d₆, 270 MHz) δ ppm : 7.00 (t, J =7.3 Hz, 1H), 7.30 (t, J = 8.4 Hz, 2H), 7.48 (d, J = 7.8 Hz, 2H), 7.78 (m, 7H), 8.11 (d, J = 5.4 Hz, 1H), 8.93 (s, 1H), 9.23 (s, 1H), 12.31 (s, 1H)

### Example 34

### N-Phenyl-N'-[4-[(4,5-dimethoxy-2-carbamoylphenyl)aminocarbonyl]-3-methylphenyl]urea (compound number 744)

The title compound was synthesized in the same manner as in Example 19.

¹H-NMR (DMSO-d₆, 270 MHz) δ ppm : 2.34 (s, 3H), 3.81 (s, 3H), 3.84 (s, 3H), 6.99 (t, J = 7.3 Hz, 1H), 7.31 (t, J = 7.3 Hz, 2H), 7.44 (s, 1H), 7.49 (d, J = 7.6 Hz, 2H), 7.75 (m, 3H), 8.16 (d, J = 7.8 Hz, 1H), 8.33 (s, 2H), 8.54 (s, 1H), 9.38 (s, 1H), 13.22 (s, 1H)

### Example 35

### N-Phenyl-N'-[3-[(4,5-dimethoxy-2-carbamoylphenyl)aminocarbonyl]-4-methylphenyl]urea (compound number 745)

The title compound was synthesized in the same manner as in Example 19.

¹H-NMR (DMSO-d₆, 270 MHz) δ ppm : 2.33 (s, 3H), 3.81 (s, 3H), 3.84 (s, 3H), 6.97 (t, J = 7.3 Hz, 1H), 7.37 (m, 7H), 7.66 (s, 1H), 8.30 (s, 1H), 8.38 (s, 1H), 8.45 (s, 1H), 8.54 (s, 1H), 9.35 (s, 1H), 13.21 (s, 1H)

### Example 36

### N-Phenyl-N'-[4-chloro-3-[(4,5-dimethoxy-2-carbamoylphenyl)-aminocarbonyl]phenyl]urea (compound number 746)

The title compound was synthesized in the same manner as in Example 19.

¹H-NMR (DMSO-d₆, 270 MHz) δ ppm : 3.82 (s, 3H), 3.85 (s, 3H), 7.01 (t, J = 7.3 Hz, 1H), 7.31 (t, J = 8.1 Hz, 2H), 7.54 (m, 4H), 7.68 (d, J = 8.1 Hz, 2H), 8.34 (s, 1H), 8.50 (s, 1H), 8.69 (s, 1H), 8.78 (d, J = 1.8 Hz, 1H), 9.67 (s, 1H), 13.34 (s, 1H)

### Example 37

### N-Phenyl-N'-[3-[(4,5-dimethoxy-2-carbamoylphenyl)aminocarbonyl]-4-hydroxyphenyl]urea (compound number 728)

The title compound was synthesized in the same manner as in Example 19.

¹H-NMR (DMSO-d₆, 270 MHz) δ ppm : 3.81 (s, 3H), 3.84 (s, 3H), 6.97 (m, 2H), 7.29 (t, J = 7.8 Hz, 2H), 7.46 (m, 4H), 7.60 (s, 1H), 8.26 (s, 1H), 8.32 (s, 1H), 8.54 (s, 1H), 8.71 (d, J = 2.2 Hz, 1H), 9.34 (s, 1H), 13.22 (s, 1H)

### Example 38

### N-Phenyl-N'-[3-[(4,5-dimethoxy-2-carbamoylphenyl)aminocarbonyl]-4-(2-(N-morpholinyl)ethoxy)phenyl]urea (compound number 747)

The title compound was synthesized in the same manner as in Example 19.

¹H-NMR (DMSO-d₆, 270 MHz) δ ppm : 3.81 (s, 3H), 3.84 (s, 3H), 3.70 (m, 12H), 6.99 (t, J = 7.3 Hz, 1H), 7.27 (m, 3H), 7.50 (m, 4H), 7.64 (s, 1H), 8.23 (s, 1H), 8.29 (s, 1H), 8.53 (s, 1H), 8.75 (d, J = 2.4 Hz, 1H), 9.43 (s, 1H), 13.15 (s, 1H)

### Example 39

### N-Phenyl-N'-[4-[(4,5-dimethoxy-2-carbamoylphenyl)aminocarbonyl]-2-thienyl]urea (compound number 975)

The title compound was synthesized in the same manner as in Example 19.

¹H-NMR (DMSO-d₆, 270 MHz) δ ppm : 3.81 (s, 3H), 3.83 (s, 3H), 6.92 (s, 1H), 6.99 (t, J = 8.1 Hz, 1H), 7.30 (t, J = 8.1 Hz, 2H), 7.46 (m, 4H), 8.62 (s, 1H), 8.31 (s, 1H), 8.46 (s,1H), 9.00 (s, 1H), 10.28 (s, 1H), 13.02 (s, 1H)

### Example 40

### N-Toluyl-N'-[4-[(4,5-dimethoxy-2-ethoxycarbonylphenyl)aminocarbonyl]-phenyl]urea (compound number 2)

The title compound was synthesized in the same manner as in Example 1.

¹H-NMR (DMSO-d₆, 270 MHz) δ ppm : 1.35 (t, J = 7.0 Hz, 3H), 2.25 (s, 3H), 3.80 (s, 3H), 3.88 (s, 3H), 4.37 (q, J = 7.0 Hz, 2H), 7.10 (d, J = 8.4 Hz, 2H), 7.38 (d, J = 8.4 Hz, 2H), 7.48 (s, 1H), 7.67 (d, J = 8.9 Hz, 2H), 7.89 (d, J = 8.9 Hz, 2H), 8.45 (s, 1H), 9.09 (s, 1H), 9.43 (s, 1H), 11.75 (s, 1H)

### Example 41

### N-Phenyl-N'-[3-[(4,5-dimethoxy-2-carbamoylphenyl)-aminocarbonylmethoxy]phenyl]urea (compound number 994)

The title compound was synthesized in the same manner as in Example 19.

¹H-NMR (DMSO-d₆, 270 MHz) δ ppm : 3.79 (s, 3H), 3.80 (s, 3H), 4.63 (s, 2H), 6.67 (m, 1H), 6.96 (t, J = 7.0 Hz, 1H), 7.04 (d, J = 8.9 Hz, 1H), 7.24 (m, 4H), 7.38 (s, 1H), 7.47 (d, J = 7.8 Hz, 2H), 7.61 (s, 1H), 8.18 (s, 1H), 8.43 (s, 1H), 9.01 (s, 1H), 9.08 (s, 1H), 12.84 (s, 1H)

### Example 42

### N-(4-Acetoxyphenyl)-N'-[4-[(4,5-dimethoxy-2-carbamoylphenyl)-arninocarbonylethyl]phenyl]urea (compound number 1073)

The title compound was synthesized in the same manner as in Example 19.

¹H-NMR (DMSO-d₆, 270 MHz) δ ppm : 2.62 (t, J = 7.3 Hz, 2H), 2.88 (t, J = 7.3 Hz, 2H), 3.78 (s, 6H), 7.17 (d, J = 8.4 Hz, 2H), 7.36 (m,3H), 7.57 (m, 3H), 7.89 (d, J = 8.9 Hz, 2H), 8.18 (s, 1H), 8.29 (s, 1H), 8.86 (s, 1H), 9.21 (s, 1H), 12.13 (s, 1H)

### Example 43

### N-(3-Pyridyl)-N'-[4-[(4,5-dimethoxy-2-carbamoylphenyl)-aminocarbonylethyl]phenyl]urea (compound number 1071)

The title compound was synthesized in the same manner as in Example 19.

¹H-NMR (DMSO-d₆, 270 MHz) δ ppm : 2.62 (t, J = 7.3 Hz, 2H), 2.88 (t, J = 7.3 Hz, 2H), 3.78 (s, 6H), 7.17 (d, J = 8.4 Hz, 2H), 7.33 (m,4H), 7.56 (s, 1H), 7.91 (m, 1H), 8.17 (m, 2H), 8.29 (s, 1H), 8.59 (d, J = 2.4 Hz, 1H), 8.81 (s, 1H), 8.91 (s, 1H), 12.13 (s, 1H)

### Example 44

### N-(3-Pyridyl)-N'-[4-[(4,5-difluoro-2-ethoxycarbonylphenyl)aminocarbonyl]-phenyl]urea (compound number 1094)

The title compound was synthesized in the same manner as in Example 1.

¹H-NMR (DMSO-d₆, 270 MHz) δ ppm : 1.34 (t, J = 7.2 Hz, 3H), 4.37 (q, J = 7.2 Hz, 2H), 7.34 (m, 1H), 7.69 (d, J = 8.6 Hz, 2H), 7.97 (m, 4H), 8.21 (m, 1H), 8.64 (m, 2H), 9.31 (s, 1H), 9.55 (s, 1H), 11.59 (s, 1H)

### Example 45

### N-(4-Aminophenyl)-N'- {4-[(4,5-dimethoxy-2-ethoxycarbonylphenyl)-aminocarbonylethyl]phenyl}urea

Synthesis was carried out in the same manner as in Example 3.

¹H-NMR (DMSO-d₆, 270 MHz) δ ppm : 1.32 (t, J = 7.0 Hz, 3H), 2.67 (t, J = 7.3 Hz, 2H), 2.87 (t, J = 7.3 Hz, 2H), 3.77 (s, 3H), 3.81 (s, 3H), 4.31 (q, J = 7.3 Hz, 2H), 4.75 (s, 2H), 6.49 (d, J = 8.9 Hz, 2H), 7.05 (d, J = 8.6 Hz, 2H), 7.14 (d, J = 8.1 Hz, 2H), 7.32 (d, J = 8.4 Hz, 2H), 7.39 (s, 1H), 8.12 (s, 1H), 8.14 (s, 1H), 8.43 (s, 1H), 10.74 (s, 1H)

### Example 46

### N-(4-Nitrophenyl)-N'- (4-[(4,5-dimethoxy-2-ethoxycarbonylphenyl)-aminocarbonylethyl]phenyl} urea

The title compound was synthesized in the same manner as in Example 4.

¹H-NMR (DMSO-d₆, 270 MHz) δ ppm : 1.32 (t, J = 7.0 Hz, 3H), 2.69 (t, J = 7.3 Hz, 2H), 2.90 (t, J = 7.3 Hz, 2H), 3.77 (s, 3H), 3.82 (s, 3H), 4.31 (q, J = 7.0 Hz, 2H), 7.19 (d, J = 8.4 Hz, 2H), 7.38 (s, 1H), 7.40 (d, J = 8.9 Hz, 2H), 7.69 (d, J = 9.1 Hz, 2H), 8.14 (s, 1H), 8.18 (d, J = 9.1 Hz, 2H), 9.12 (s, 1H), 9.70 (s, 1H), 10.74 (s, 1H)

### Example 47

### N-(2-Aminophenyl)-N'- {4-[(4,5-dimethoxy-2-ethoxycarbonylphenyl)-aminocarbonylethyl]phenyl}urea

Synthesis was carried out in the same manner as in Example 3.

¹H-NMR (DMSO-d₆, 270 MHz) δ ppm : 1.32 (t, J = 7.3 Hz, 3H), 2.67 (t, J = 7.8 Hz, 2H), 2.87 (t, J = 7.8 Hz, 2H), 3.77 (s, 3H), 3.81 (s, 3H), 4.31 (q, J = 7.3 Hz, 2H), 4.78 (s, 2H), 6.56 (t, J =6.8 Hz, 1H), 6.71 (d, J = 6.8 Hz, 1H), 6.80 (t, J = 6.8 Hz, 1H), 7.39 (m, 4H), 7.95 (s, 1H), 8.14 (s, 1H), 8.94 (s, 1H), 10.74 (s, 1H)

### Example 48

### N-(2-Nitrophenyl)-N'- {4-[(4,5-dimethoxy-2-ethoxycarbonylphenyl)-aminocarbonylethyl]phenyl}urea

The title compound was synthesized in the same manner as in Example 4.

¹H-NMR (DMSO-d₆, 270 MHz) δ ppm : 1.32 (t, J = 7.3 Hz, 3H), 2.69 (t, J = 7.8 Hz, 2H), 2.89 (t, J = 7.8 Hz, 2H), 3.77 (s, 3H), 3.81 (s, 3H), 4.31 (q, J = 7.3 Hz, 2H), 7.20 (m, 3H), 7.39 (m, 3H), 7.69 (t, J = 7.3 Hz, 1H), 8.09 (dd, J = 1.1, 8.4 Hz, 1H), 8.14 (s, 1H), 8.28 (d, J = 8.4 Hz, 1H), 9.63 (s, 1H), 9.82 (s, 1H), 10.74 (s, 1H)

### Example 49

### N-(3-Aminophenyl)-N'-{4-[(4,5-dimethoxy-2-ethoxycarbonylphenyl)-aminocarbonylethyl]phenyl}urea

Synthesis was carried out in the same manner as in Example 3.

¹H-NMR (DMSO-d₆, 270 MHz) δ ppm : 1.32 (t, J = 7.0 Hz, 3H), 2.67 (t, J = 7.3 Hz, 2H), 2.88 (t, J = 7.3 Hz, 2H), 3.77 (s, 3H), 3.82 (s, 3H), 4.30 (q, J = 7.3 Hz, 2H), 5.01 (s, 2H), 6.17 (d, J =9.5 Hz, 1H), 6.54 (d, J = 8.6 Hz, 1H), 6.76 (s, 1H), 6.87 (t, J = 7.8 Hz, 1H), 7.15 (d, J = 8.1 Hz, 2H), 7.34 (d, J = 8.1 Hz, 2H), 7.39 (s, 1H), 8.14 (s, 1H), 8.40 (s, 1H), 8.55 (s, 1H), 10.74 (s, 1H)

### Example 50

### N-(3-Nitrophenyl)-N'- {4-[(4,5-dimethoxy-2-ethoxycarbonylphenyl)-aminocarbonylethyl]phenyl}urea

The title compound was synthesized in the same manner as in Example 4.

¹H-NMR (DMSO-d₆, 270 MHz) δ ppm : 1.32 (t, J = 7.0 Hz, 3H), 2.69 (t, J = 7.3 Hz, 2H), 2.90 (t, J = 7.3 Hz, 2H), 3.77 (s, 3H), 3.82 (s, 3H), 4.31 (q, J = 7.3 Hz, 2H), 7.18 (d, J = 8.4 Hz, 2H), 7.41 (m, 3H), 7.55 (t, J = 8.4 Hz, 1H), 7.72 (d, J = 9.2 Hz, 1H), 7.80 (dd, J = 1.9, 7.8 Hz, 1H), 8.14 (s, 1H), 8.56 (m, 1H), 9.04 (s, 1H), 9.48 (s, 1H), 10.74 (s, 1H)

### Example 51

### N-(4-Piperidino)-N'-{4-[(4,5-dimethoxy-2-ethoxycarbonylphenyl)-aminocarbonylethyl]phenyl}urea

60 mg of triphosgene was added to 10 ml of tetrahydrofuran. A solution of 110 mg of 4-amino-N-t-butyloxycarbonylpiperidine and 80 mg of diisopropylethylamine in THF was then added dropwise thereto under a nitrogen atmosphere at room temperature, followed by stirring at 60°C for 1 hour. To the reaction solution was added 110 mg of ethyl 2-(4-aminophenyl)ethylcarbonylamino-4,5-dimethoxybenzoate and 30 mg of 4-dimethylaminopyridine, and the mixture was then stirred at 60°C for 3 hours. The reaction solution was poured into water and extracted with dichloromethane, and the organic layer was dried with anhydrous magnesium sulfate, followed by concentration. The residue was purified by column chromatography on silica gel (eluent, dichloromethane : ethyl acetate =30:1 → dichloromethane : methanol = 50 : 1) to obtain 120 mg of white solid. Subsequently, the product was added to 20 ml of 4 N hydrogen chloride/dioxane solution and the mixture was stirred at room temperature for 3 hours. The precipitated solid was separated by filtration and subjected to vacuum drying. Thus, 90 mg of the title compound was obtained as a white solid.

¹H-NMR (DMSO-d₆, 270 MHz) δ ppm : 1.32 (t, J = 7.0 Hz, 3H), 1.52 (m, 2H), 1.92 (m, 2H), 3.00 (m, 8H), 3.51 (m, 1H), 3.76 (s, 3H), 3.81 (s, 3H), 4.30 (q, J = 7.0 Hz, 2H), 6.47 (d, J =7.3 Hz, 1H), 7.09 (t, J = 8.6 Hz, 2H), 7.27 (d, J = 8.6 Hz, 2H), 7.34 (s, 1H), 8.14 (s, 1H), 8.35 (s, 1H), 10.74 (s, 1H)

### Example 52

### N-Phenyl-N'- {4-[(2-ethoxycarbonyl-5-hydroxy-4-methoxyphenyl)-aminocarbonylethyl]phenyl}urea

2.00 g of vanillin was dissolved in 20 ml of DMF and 4.00 g of benzyl chloride, and 2.20 g of potassium carbonate was then added thereto, followed by stirring at 55°C for 7 hours. The reaction solution was poured into water and extracted with dichloromethane, and the organic layer was dried with anhydrous magnesium sulfate, followed by concentration. Thus, 3.82 g of colorless transparent liquid was obtained.

Subsequently, the resultant liquid was added to 60 ml of concentrated nitric acid in an ice bath over a period of 30 minutes. The mixture was then stirred at room temperature for 2 hours. The reaction solution was poured into ice, and the precipitated solid was separated by filtration and washed with water, followed by vacuum drying. Thus, 2.00 g of yellow solid was obtained.

Thereafter, the resultant solid was dissolved in 40 ml of acetone. The product was slowly added dropwise to a reaction solution comprising 1.80 g of potassium permanganate dissolved in 30 ml of water in an oil bath with a temperature of 80°C. The reaction solution was stirred in that state for 2 hours and filtered. The filtrate was concentrated, and the residue was poured into water and extracted with dichloromethane. The aqueous layer was then adjusted to pH 4 with the aid of hydrochloric acid. Extraction with dichloromethane was carried out, and the organic layer was dried with anhydrous magnesium sulfate, followed by concentration. Thus, 0.50 g of yellow liquid was obtained.

Next, the resultant liquid was added to 30 ml of chloroform and 5 ml of thionyl chloride was then added thereto, followed by stirring under reflux for 5 hours. The solvent was removed by distillation under reduced pressure. 20 ml of ethanol was added to the residue, and the mixture was stirred for 3 hours. The reaction solution was poured into water and extracted with dichloromethane, and the organic layer was dried with anhydrous magnesium sulfate, followed by concentration. Thus, the residue was purified by column chromatography on silica gel (eluent, dichloromethane) to obtain 0.12 g of white solid.

Subsequently, this solid and 30 mg of 5% Pd/C were added to 100 ml of methanol. The mixture was then stirred under hydrogen atmosphere at room temperature for 16 hours. The reaction solution was filtered and the filtrate was concentrated. The residue was purified by column chromatography on silica gel (eluent, dichloromethane) to obtain 0.05 g of white solid.

Thereafter, the resultant solid was dissolved in 10 ml of dichloromethane. 0.04 g of 4-nitrocinnamoyl chloride and 0.2 ml of diisopropylethylamine were then added thereto and the mixture was stirred at room temperature for 2 hours. The reaction solution was poured into saturated sodium bicarbonate water and extracted with dichloromethane, and the organic layer was dried with anhydrous magnesium sulfate, followed by concentration. The residue and 20 mg of 5% Pd/C were added to 20 ml of ethanol. The mixture was then stirred under hydrogen atmosphere at room temperature for 16 hours. The reaction solution was filtered and the filtrate was concentrated. Thus, 0.08 g of yellow solid was obtained.

Subsequently, the resultant solid, 0.08 g of phenyl isocyanate, and 0.03 g of dimethylaminopyridine were added to 10 ml of tetrahydrofuran. The mixture was then stirred under reflux for 8 hours. The reaction solution was poured into water and extracted with dichloromethane, and the organic layer was dried with anhydrous magnesium sulfate, followed by concentration. The residue was purified by column chromatography on silica gel (eluent, dichloromethane : methanol = 100 : 1 → 10 : 1 ) to obtain 90 mg of the title compound as a white solid.

¹H-NMR (DMSO-d₆, 270 MHz) δ ppm : 1.31 (t, J = 7.0 Hz, 3H), 2.64 (t, J = 7.3 Hz, 2H), 2.87 (t, J = 7.3 Hz, 2H), 3.75 (s, 3H), 4.28 (q, J = 7.3 Hz, 2H), 6.95 (t, J = 7.6 Hz, 1H), 7.15 (d, J = 8.4 Hz, 2H), 7.26 (t, J =7.8 Hz, 2H), 7.35 (d, J = 8.9 Hz, 2H), 7.43 (s, 1H), 7.44 (d, J = 7.8 Hz, 2H), 8.00 (s, 1H), 8.62 (m, 2H), 10.30 (s, 1H), 10.75 (s, 1H)

### Example 53

### N-Phenyl-N'-{4-[(2-ethoxycarbonyl-4-methoxy-5-(N-morpholino-2-ethoxy)-phenyl)aminocarbonylethyl]phenyl}urea

60 mg of the compound synthesized in Example 52 was dissolved in 10 ml of DMF. Thereafter, 0.44 g of potassium carbonate and 0.38 g of N-(2-chloroethyl)morpholine hydrochloride were added thereto, followed by stirring at room temperature for 16 hours. The solvent was removed by distillation under reduced pressure and the residue was then poured into water and extracted with dichloromethane. The organic layer was dried with anhydrous magnesium sulfate and concentrated. The residue was purified by column chromatography on silica gel ( eluent, dichloromethane : methanol = 100 : 1 → 30 : 1) to obtain 60 mg of the title compound as a white solid.

¹H-NMR (DMSO-d₆, 270 MHz) δ ppm : 1.32 (t, J = 7.0 Hz, 3H), 2.66 (m, 4H), 2.87 (t, J = 7.3 Hz, 2H), 3.30 (m, 2H), 3.57 (t, J = 4.3 Hz, 2H), 3.77 (s, 3H), 4.12 (t, J = 5.9 Hz, 2H), 4.30 (q, J = 7.3 Hz, 2H), 6.94 (t, J = 7.6 Hz, 1H), 7.15 (d, J = 8.6 Hz, 2H), 7.26 (t, J =8.1 Hz, 2H), 7.37 (d, J = 8.9 Hz, 2H), 7.39 (s, 1H), 7.45 (d, J = 7.8 Hz, 2H), 8.13 (s, 1H), 8.96 (s, 1H), 9.01 (s, 1H), 10.70 (s, 1H)

### Example 54

### N-(4-Aminophenyl)-N'-{4-[(2-ethoxycarbonyl-4-methoxy-5-(N-morpholino-2-ethoxy)-phenyl)aminocarbonylethyl]phenyl}urea

0.50 g of vanillin was dissolved in 20 ml of DMF, and then, 1.23 g of N-(2-chloroethyl)morpholine hydrochloride and 1.38 g of potassium carbonate were added thereto. The mixture was stirred at 69°C for 10 hours. The solvent was removed by distillation under reduced pressure and the residue was poured into water and extracted with dichloromethane. The organic layer was dried with anhydrous magnesium sulfate and concentrated to obtain 0.93 g of yellowish brown liquid.

Subsequently, the resultant liquid was added to 40 ml of concentrated nitric acid in an ice bath over a period of 30 minutes, followed by stirring for an additional 3 hours. The reaction solution was poured into ice and the precipitated solid was separated by filtration and washed with water, followed by vacuum drying. Thus, 0.51 g of yellow solid was obtained.

Thereafter, the resultant solid was dissolved in 20 ml of acetone, 10 ml of aqueous solution comprising 2.00 g of sulfamic acid and 2.00 g of chlorous acid dissolved therein was slowly added dropwise to the reaction solution at room temperature. The reaction solution was stirred in that state for 80 hours and then concentrated to half its initial volume. The residue was adjusted to pH 10 with the aid of an aqueous solution of sodium hydroxide, followed by extraction with dichloromethane. The aqueous layer was concentrated, and the residue, 5.00 g of potassium carbonate, and 7 ml of ethyl iodide were added to 50 ml of DMF, followed by stirring at room temperature for 14 hours. The solvent was removed by distillation under reduced pressure, and the residue was poured into water, followed by extraction with dichloromethane. The organic layer was dried with anhydrous magnesium sulfate and then concentrated. The residue was purified by column chromatography on silica gel (eluent, dichloromethane : methanol = 100 : 1 → 50 : 1) to obtain 0.40 g of yellow tar.

Next, the resultant tar and 0.24 g of 5% Pd/C were added to 30 ml of ethanol, followed by stirring under hydrogen atmosphere at room temperature for 86 hours. The reaction solution was filtered and the filtrate was concentrated. The residue was purified by column chromatography on silica gel (eluent, dichloromethane : methanol =100: 1 → 50 : 1) to obtain 0.19 g of white solid.

Subsequently, the resultant solid was dissolved in 10 ml of dichloromethane. 0.14 g of 4-nitrocinnamoyl chloride and 0.4 ml of diisopropylethylamine were then added thereto, followed by stirring at room temperature for 2 hours. The reaction solution was poured into saturated sodium bicarbonate water and extracted with dichloromethane. The organic layer was dried with anhydrous magnesium sulfate and concentrated. The residue was washed with methanol and subjected to vacuum drying. Thus, 0.28 g of yellow solid was obtained. This solid and 50 mg of 5% Pd/C were added to 50 ml of ethanol, followed by stirring under hydrogen atmosphere at room temperature for 18 hours. The reaction solution was filtered and the filtrate was concentrated. The residue was purified by column chromatography on silica gel (eluent, dichloromethane : methanol =50 : 1→30 : 1) to obtain 0.20 g of yellow tar.

Thereafter, 0.07 g of the resultant solid and 0.04 g of 4-nitrophenyl isocyanate were added to 10 ml of tetrahydrofuran, followed by stirring at 69°C for 30 minutes. The reaction solution was concentrated, and the residue was washed with methanol and subjected to vacuum drying to obtain 0.08 g of white solid. The resultant solid and 50 mg of 5% Pd/C were added to 30 ml of ethanol, followed by stirring under hydrogen atmosphere at room temperature for 14 hours. The reaction solution was filtered and the filtrate was concentrated. The residue was washed with methanol and vacuum dried to obtain 0.02 g of yellow tar.

20 g of the title compound was obtained as a red solid.

¹H-NMR (DMSO-d₆, 270 MHz) δ ppm : 1.32 (t, J = 7.0 Hz, 3H), 2.66 (m, 4H), 2.87 (t, J = 7.3 Hz, 2H), 3.30 (m, 2H), 3.57 (t, J = 4.3 Hz, 2H), 3.77 (s, 3H), 4.12 (t, J = 5.9 Hz, 2H), 4.30 (q, J = 7.3 Hz, 2H), 4.74 (s, 2H), 6.49 (d, J = 7.6 Hz, 2H), 7.05 (d, J = 8.4 Hz, 2H), 7.09 (d, J =8.1 Hz, 2H), 7.33 (d, J = 8.1 Hz, 2H), 7.39 (s, 1H), 8.13 (s, 1H), 8.27 (s, 1H), 8.57 (s, 1H), 10.70 (s, 1H)

### Example 55

### N-(2-Nitrophenyl)-N'- {4-[(2-carbamoyl-4,5-dimethoxyphenyl)-aminocarbonylethyl]phenyl}urea

The title compound was synthesized in the same manner as in Example 18.

¹H-NMR (DMSO-d₆, 270 MHz) δ ppm : 2.69 (t, J = 7.8 Hz, 2H), 2.89 (t, J = 7.8 Hz, 2H), 3.78 (s, 6H), 7.20 (m, 3H), 7.39 (m, 4H), 7.56 (s, 1H), 7.69 (t, J = 7.3 Hz, 1H), 8.09 (dd, J = 1.1, 8.4 Hz, 1H), 8.14 (s, 1H), 8.28 (d, J = 8.4 Hz, 1H), 9.63 (s, 1H), 9.82 (s, 1H), 10.74 (s, 1H)

### Example 56

### N-(3-Nitrophenyl)-N'- {4-[(2-carbamoyl-4,5-dimethoxyphenyl)-aminocarbonylethyl]phenyl}urea

The title compound was synthesized in the same manner as in Example 18.

¹H-NMR (DMSO-d₆, 270 MHz) δ ppm : 2.69 (t, J = 7.3 Hz, 2H), 2.90 (t, J = 7.3 Hz, 2H), 3.77 (s, 6H), 7.18 (d, J = 8.4 Hz, 2H), 7.41 (m, 4H), 7.55 (m, 2H), 7.72 (d, J = 9.2 Hz, 1H), 7.80 (dd, J = 1.9, 7.8 Hz, 1H), 8.14 (s, 1H), 8.56 (m, 1H), 9.04 (s, 1H), 9.48 (s, 1H), 10.74 (s, 1H)

### Example 57

### N-(3,4,5-Trimethoxyphenyl)-N'-{4-[(2-carbamoyl-4,5-dimethoxyphenyl)-aminocarbonyl]-3-methoxyphenyl}urea

The title compound was synthesized in the same manner as in Example 18.

¹H-NMR (DMSO-d₆, 270 MHz) δ ppm : 3.62 (s, 3H), 3.77 (s, 6H), 3.81 (s, 3H), 3.84 (s, 3H), 3.99 (s, 3H), 6.81 (s, 2H), 7.54 (m, 3H), 7.74 (s, 1H), 8.33 (d, J = 8.1 Hz, 1H), 8.51 (s, 1H), 8.55 (s, 1H), 9.49 (s, 1H), 13.28 (s, 1H)

### Example 58

### N-Phenyl-N'- {3-[(2-carbamoyl-4,5-difluorophenyl)aminocarbonyl]-phenyl}urea

The title compound was synthesized in the same manner as in Example 18.

¹H-NMR (DMSO-d₆, 270 MHz) δ ppm : 6.99 (t, J = 7.3 Hz, 1H), 7.48 (m, 4H), 7.73 (m,1H), 8.04 (m, 3H), 8.46 (s, 1H), 8.78 (m, 12H), 9.03 (s, 1H), 13.11 (s, 1H)

### Example 59

### N-Phenyl-N'-{3-[(6-carbamoyl-2-pyridyl)aminocarbonylmethoxy]phenyl}-urea

The title compound was synthesized in the same manner as in Example 18.

¹H-NMR (DMSO-d₆, 270 MHz) δ ppm : 4.80 (s, 2H), 6.63 (dd, J = 1.9, 8.4 Hz, 1H), 7.00 (m, 2H), 7.25 (m, 5H), 7.44 (d, J = 7.3 Hz, 2H), 7.86 (s, 1H), 8.19 (dd, J = 1.4, 7.8 Hz, 1H), 8.33 (s, 1H), 8.49 (dd, J = 1.9, 9.9 Hz, 1H), 8.70 (s, 1H), 8.77 (s, 1H), 11.98 (s, 1H)

### Example 60

### N-Phenyl-N'- {3-[(2-carbamoyl-4,5-diacetoxyphenyl)-aminocarbonylmethoxy]phenyl}urea

0.50 g of 2-amino-4,5-dimethoxyphenylcarboxamide was dissolved in 20 ml of dichloromethane. In an isopropanol/dry ice bath, a mixed solution of 2 ml of boron tribromide and 10 ml of dichloromethane was added dropwise thereto. Thereafter, the mixture was stirred at room temperature for 16 hours. The reaction solution was poured into water and extracted with ethyl acetate, and the organic layer was dried with anhydrous magnesium sulfate, followed by concentration. Thus, 0.54 g of black solid was obtained. The resultant solid was dissolved in 20 ml of DMF and 0.56 g of acetic anhydride and 0.56 g of triethylamine were then added thereto, followed by stirring at room temperature for 7 hours. The solvent was removed by distillation under reduced pressure, the residue was then poured into water, the precipitated solid was separated by filtration, and washed with water, followed by vacuum drying. Thus, 0.35 g of cream solid was obtained. The resultant solid and 0.08 g of 5% Pd/C were added to 50 ml of methanol and the mixture was stirred under hydrogen atmosphere at room temperature for 19 hours. The reaction solution was filtered and the filtrate was concentrated. The residue was purified by column chromatography on silica gel (eluent, dichloromethane: methanol = 100 : 1 → 50:1) to obtain 0.17 g of pale yellow solid.

Synthesis was thereafter carried out in the same manner as in Example 18.

¹H-NMR (DMSO-d₆, 270 MHz) δ ppm : 2.31 (s, 3H), 2.32 (s, 3H), 4.68 (s, 2H), 6.68 (dd, J =2.1, 8.1 Hz, 1H), 6.97 (t, J = 7.3 Hz, 1H), 7.05 (d, J = 7.8 Hz, 1H), 7.25 (m, 4H), 7.45 (d, J = 7.8 Hz, 2H), 7.79 (s, 1H), 7.88 (s, 1H), 8.29 (s, 1H), 8.56 (s, 1H), 8.76 (s, 1H), 8.83 (s, 1H), 12.66 (s, 1H)

### Example 61

### N-Phenyl-N'- {3-[(2-carbamoyl-4,5-dimethoxyphenyl)-aminocarbonylmethyl]phenyl}urea

Synthesis was carried out in the same manner as in Example 18.

¹H-NMR (DMSO-d₆, 270 MHz) δ ppm : 3.62 (s, 2H), 3.76 (s, 3H), 3.77 (s, 3H), 6.95 (m, 2H), 7.34 (m, 7H), 7.56 (s, 1H), 8.16 (s, 1H), 8.29 (s, 1H), 8.81 (s, 1H), 8.86 (s, 1H), 12.19 (s, 1H)

### Example 62

### N-Phenyl-N'- {3-[(5-carbamoyl-4-methyl-2-thienyl)aminocarbonyl]phenyl}-urea

Synthesis was carried out in the same manner as in Example 18.

¹H-NMR (DMSO-d₆, 270 MHz) δ ppm : 2.41 (s, 3H), 6.72 (s, 1H), 6.99 (t, J = 7.3 Hz, 1H), 7.30 (t, J = 8.1 Hz, 2H), 7.47 (m, 3H), 7.72 (m, 2H), 8.04 (s, 1H), 8.75 (s, 1H), 9.01 (s, 1H), 12.99 (s, 1H)

### Example 63

### Benzyl- {4-[(4,5-dimethoxy-2-ethoxycarbonylphenyl)aminocarbonyl]-phenyl}carbamate

60 mg of ethyl 2-(4-aminophenyl)carbonylamino-4,5-dimethoxybenzoate, 0.5 ml of benzyloxycarbonyl chloride, and 30 mg of 4-dimethylaminopyridine were added to 20 ml of tetrahydrofuran and the mixture was stirred at room temperature for 30 minutes. The reaction solution was poured into water and extracted with dichloromethane, and the organic layer was dried with anhydrous magnesium sulfate, followed by concentration. The residue was washed with ethanol and subjected to vacuum drying. Thus, 60 mg of the title compound was obtained as a white solid.

¹H-NMR (DMSO-d₆, 270 MHz) δ ppm : 1.33 (t, J = 7.0 Hz, 3H), 3.80 (s, 3H), 3.87 (s, 3H), 4.36 (q, J = 7.3 Hz, 4H), 5.19 (s, 2H), 7.41 (m, 6H), 7.67 (d, J = 8.6 Hz, 2H), 7.89 (d, J = 8.9 Hz, 2H), 8.41 (s, 1H), 10.20 (s, 1H), 11.72 (s, 1H)

### Example 64

### (4-Pyridylmethyl) {4-[(4,5-dimethoxy-2-ethoxycarbonylphenyl)-aminocarbonylethyl]phenyl) carbamate

0.32 g of 1,1-carbonyldiimidazole was dissolved in 3 ml of tetrahydrofuran (THF). 0.22 g of 4-pyridinemethanol was then added thereto and the mixture was stirred at room temperature for 1 hour. 0.35 g of 4-aminohydrocinnamic acid, 0.60 g of DBU (1,8-diazabicyclo[4.3.0]undec-7-ene), and 0.5 ml of triethylamine were added to 10 ml of tetrahydrofuran, followed by stirring at room temperature for 1 hour. The former solution was added to the latter solution and the obtained mixture was stirred in that state for 18 hours. The solvent was removed by distillation under reduced pressure. The residue was poured into water and adjusted to pH 6 with the aid of IN hydrochloric acid. The precipitated solid was separated by filtration and subjected to vacuum drying. Thus, 0.08 g of (4-pyridylmethyl) (4-hydroxycarbonylethylphenyl)carbamate was obtained as a pink solid. The resultant solid was added to 20 ml of toluene, and 0.1 ml of oxalyl chloride and 0.01 ml of DMF were added thereto, followed by stirring at room temperature for 5 hours. The precipitated solid was separated by filtration and washed with toluene and then with ether. 0.05 g of ethyl 2-amino-4,5-dimethoxybenzoate was dissolved in 10 ml of dichloromethane and the resultant solid and 0.5 ml of triethylamine were added thereto, followed by stirring at room temperature for 1 hour. The reaction solution was poured into saturated sodium bicarbonate water and extracted with dichloromethane. The organic layer was dried with anhydrous magnesium sulfate and concentrated. The residue was purified by column chromatography on silica gel (eluent, dichloromethane : methanol = 100 : 1 → 40 : 1) and was further washed with methanol, followed by drying. Thus, 30 mg of the title compound was obtained as a white solid.

¹H-NMR (DMSO-d₆, 270 MHz) δ ppm : 1.31 (t, J = 7.0 Hz, 3H), 2.67 (t, J = 7.5 Hz, 2H), 2.88 (t, J = 7.5 Hz, 2H), 3.76 (s, 3H), 3.81 (s, 3H), 4.29 (q, J = 7.3 Hz, 4H), 5.19 (s, 2H), 7.18 (d, J = 8.4 Hz, 2H), 7.37 (m, 5H), 8.12 (s, 1H), 8.57 (dd, J = 1.9, 4.3 Hz, 2H), 9.80 (s, 1H), 10.71 (s, 1H)

### Example 65

### N-Ethyl-N'- {4-[(2-acetyl-4,5-dimethoxyphenyl)aminocarbonylethyl]-phenyl}urea

0.60 g of 2-amino-4,5-dimethoxyacetophenone was dissolved in 30 ml of THF. 0.75 g of 4-nitrocinnamoyl chloride and 0.45 g of triethylamine were then added thereto and the mixture was stirred under reflux for 1.5 hours. The solvent of the reaction solution was removed by distillation under reduced pressure. The residue was washed with methanol and then dried. Thus, 1.22 g of yellow solid was obtained.

Subsequently, this solid and 90 mg of 5% Pd/C were added to a mixed solvent comprising 100 ml of ethanol and 30 ml of THF and the mixture was stirred under hydrogen atmosphere at room temperature for 32 hours. The reaction solution was filtered and the filtrate was concentrated. Thus, 0.92 g of 2-(4-aminophenyl)carbonylaminoethyl-4,5-dimethoxyacetophenone was obtained as a white solid.

Thereafter, 70 mg of the resultant solid, 0.11 g of ethyl isocyanate, and 20 mg of 4-dimethylaminopyridine were added to 20 ml of tetrahydrofuran and the mixture was stirred at 70°C for 5 hours. The reaction solution was concentrated and the residue was purified by column chromatography on silica gel ( eluent, dichloromethane : methanol = 100 : 1 → 30 : 1). The residue was washed with methanol and was vacuum dried to obtain 50 mg of the title compound as a white solid.

¹H-NMR (DMSO-d₆, 270 MHz) δ ppm : 1.03 (t, J = 7.0 Hz, 3H), 2.60 (s, 1H), 2.65 (t, J = 7.3 Hz, 2H), 2.85 (t, J = 7.3 Hz, 2H), 3.10 (5, J = 7.0 Hz, 2H), 3.82 (s, 1H), 6.03 (t, 1H), 7.09 (d, J = 8.4 Hz, 2H), 7.27 (d, J =8.4 Hz, 2H), 7.43 (s, 1H), 8.23 (s, 1H), 8.30 (s, 1H), 11.65 (s, 1H)

### Example 66

### N-Phenyl-N'- {4-[(2-acetyl-4,5-dimethoxyphenyl)aminocarbonylethyl]-phenyl}urea

Synthesis was carried out in the same manner as in Example 65.

¹H-NMR (DMSO-d₆, 270 MHz) δ ppm : 2.60 (s, 1H), 2.67 (t, J = 7.6 Hz, 2H), 2.89 (t, J = 7.6 Hz, 2H), 3.82 (s, 6H), 6.95 (t, J = 7.3 Hz, 1H), 7.16 (d, J = 8.4 Hz, 2H), 7.27 (t, J = 8.1 Hz, 2H), 7.35 (d, J = 8.4 Hz, 2H), 7.43 (s, 1H), 7.44 (d, J = 8.4 Hz, 2H), 8.24 (s, 1H), 8.59 (s, 1H), 8.64 (s, 1H), 11.67 (s, 1H)

### Example 67

### N-(4-Aminophenyl)-N'- {4-[(2-acetyl-4,5-dimethoxyphenyl)-aminocarbonylethyl]phenyl}urea

Synthesis was carried out in the same manner as in Example 3.

¹H-NMR (DMSO-d₆, 270 MHz) δ ppm : 2.60 (s, 3H), 2.66 (t, J = 7.6 Hz, 2H), 2.87 (t, J = 7.3 Hz, 2H), 3.82 (s, 6H), 4.57 (s, 2H), 6.49 (d, J =8.4 Hz, 2H), 7.05 (d, J =8.1 Hz, 2H), 7.13 (d, J =8.6 Hz, 2H), 7.32 (d, J =8.6 Hz, 2H), 7.43 (s, 1H), 8.10 (s, 1H), 8.24 (s, 1H), 9.40 (s, 1H), 11.67 (s, 1H)

### Example 68

### N-(4-Nitrophenyl)-N'-{4-[(2-acetyl-4,5-dimethoxyphenyl)-aminocarbonylethyl]phenyl}urea

The title compound was synthesized in the same manner as in Example 65.

¹H-NMR (DMSO-d₆, 270 MHz) δ ppm : 2.51 (s, 3H), 2.68 (t, J = 7.6 Hz, 2H), 2.90 (t, J = 7.3 Hz, 2H), 3.85 (s, 6H), 7.19 (d, J = 7.3 Hz, 2H), 7.40 (m, 3H), 7.68 (d, J = 9.5 Hz, 2H), 8.20 (m, 3H), 8.86 (s, 1H), 9.42 (s, 1H), 11.68 (s, 1H)

### Example 69

### N-(2-Aminophenyl)-N'- {4-[(2-acetyl-4,5-dimethoxyphenyl)-aminocarbonylethyl]phenyl}urea

Synthesis was carried out in the same manner as in Example 3.

¹H-NMR (DMSO-d₆, 270 MHz) δ ppm : 2.60 (s, 3H), 2.67 (t, J = 7.3 Hz, 2H), 2.88 (t, J = 7.3 Hz, 2H), 3.82 (s, 6H), 4.76 (s, 2H), 6.56 (dt, J =1.4, 7.3 Hz, 1H), 6.72 (dd, J = 1.4, 7.8 Hz, 1H), 6.83 (dt, J =1.4, 7.8 Hz, 1H), 7.15 (d, J =8.4 Hz, 2H), 7.36 (m, 3H), 7.43 (s, 1H), 7.71 (s, 1H), 8.24 (s, 1H), 8.69 (s, 1H), 11.66 (s, 1H)

### Example 70

### N-(2-Nitrophenyl)-N'- {4-[(2-acetyl-4,5-dimethoxyphenyl)-aminocarbonylethyl]phenyl}urea

The title compound was synthesized in the same manner as in Example 65.

¹H-NMR (DMSO-d₆, 270 MHz) δ ppm : 2.60 (s, 3H), 2.68 (t, J = 7.3 Hz, 2H), 2.90 (t, J = 7.3 Hz, 2H), 3.82 (s, 6H), 7.16 (m, 3H), 7.40 (m, 3H), 7.69 (dt, J =1.6, 8.4 Hz, 1H), 8.09 (dd, J =1.4, 8.4 Hz, 1H), 8.25 (s, 1H), 8.31 (d, J =8.4 Hz, 1H), 9.58 (s, 1H), 9.79 (s, 1H), 11.69 (s, 1H)

### Example 71

### N-(3-Aminophenyl)-N'-{4-[(2-acetyl-4,5-dimethoxyphenyl)-aminocarbonylethyl]phenyl}urea

### Synthesis was carried out in the same manner as in Example 3.

¹H-NMR (DMSO-d₆, 270 MHz) δ ppm : 2.60 (s, 3H), 2.67 (t, J = 7.3 Hz, 2H), 2.88 (t, J = 7.3 Hz, 2H), 3.82 (s, 6H), 5.01 (s, 2H), 6.17 (d, J =9.5 Hz, 1H), 6.54 (d, J = 8.6 Hz, 1H), 6.76 (s, 1H), 6.87 (t, J = 7.8 Hz, 1H), 7.15 (d, J = 8.1 Hz, 2H), 7.34 (d, J = 8.1 Hz, 2H), 7.39 (s, 1H), 8.14 (s, 1H), 8.40 (s, 1H), 8.55 (s, 1H), 11.67 (s, 1H)

### Example 72

### N-(3-Nitrophenyl)-N'- {4-[(2-acetyl-4,5-dimethoxyphenyl)-aminocarbonylethyl]phenyl}urea

The title compound was synthesized in the same manner as in Example 65.

¹H-NMR (DMSO-d₆, 270 MHz) δ ppm : 2.60 (s, 3H), 2.69 (t, J = 7.3 Hz, 2H), 2.90 (t, J = 7.3 Hz, 2H), 3.82 (s, 6H), 7.19 (d, J = 8.9 Hz, 2H), 7.40 (m, 3H), 7.56 (t, J = 8.1 Hz, 1H), 7.69 (d, J = 8.4 Hz, 1H), 7.80 (dd, J = 1.9, 8.4 Hz, 1H), 8.24 (s, 1H), 8.56 (m, 1H), 8.78 (s, 1H), 9.20 (s, 1H), 11.68 (s, 1H)

### Example 73

### N-(4-Piperidino)-N'-{4-[(2-acetyl-4,5-dimethoxyphenyl)-aminocarbonylethyl]phenyl}urea

The title compound was synthesized in the same manner as in Example 51.

¹H-NMR (DMSO-d₆, 270 MHz) δ ppm : 1.76 (m, 4H), 2.59 (s, 3H), 3.00 (m, 8H), 3.60 (m, 1H), 3.82 (s, 6H), 6.54 (d, J =7.3 Hz, 1H), 7.09 (d, J = 8.4 Hz, 2H), 7.27 (d, J = 8.9 Hz, 2H), 7.43 (s, 1H), 8.23 (s, 1H), 8.40 (s, 1H), 8.56 (s, 1H), 11.66 (s, 1H)

### Example 74

### N-(3,4,5-Trimethoxyphenyl)-N'-{4-[(2-acetyl-4,5-dimethoxyphenyl)-aminocarbonylethyl]phenyl}urea

The title compound was synthesized in the same manner as in Example 65.

¹H-NMR (DMSO-d₆, 270 MHz) δ ppm : 2.51 (s, 3H), 2.67 (t, J = 7.6 Hz, 2H), 2.89 (t, J = 7.6 Hz, 2H), 3.60 (s, 3H), 3.74 (s, 6H), 3.82 (s, 6H), 6.78 (s, 2H), 7.15 (d, J = 8.4 Hz, 2H), 7.35 (d, J = 8.4 Hz, 2H), 7.43 (s, 1H), 8.24 (s, 1H), 8.54 (s, 1H), 8.60 (s, 1H), 11.68 (s, 1H)

### Example 75

### N-(4-Pyridyl)-N'-{4-[(2-acetyl-4,5-dimethoxyphenyl)aminocarbonylethyl]-phenyl}urea

The title compound was synthesized in the same manner as in Example 65.

¹H-NMR (DMSO-d₆, 270 MHz) δ ppm : 2.60 (s, 3H), 2.68 (t, J = 7.3 Hz, 2H), 2.90 (t, J = 7.3 Hz, 2H), 3.82 (s, 6H), 7.19 (d, J = 8.4 Hz, 2H), 7.40 (m, 5H), 8.24 (s, 1H), 8.34 (d, J = 6.5 Hz, 2H), 8.83 (s, 1H), 9.11 (s, 1H), 11.66 (s, 1H)

### Example 76

### N-(4-Piperidinomethyl)-N'-{4-[(2-acetyl-4,5-dimethoxyphenyl)-aminocarbonylethyl]phenyl}urea

The title compound was synthesized in the same manner as in Example 51.

¹H-NMR (DMSO-d₆, 270 MHz) δ ppm : 1.77 (m, 5H), 2.60 (s, 3H), 3.00 (m, 8H), 3.60 (m,2H), 3.82 (s, 6H), 6.28 (t, J =7.3 Hz, 1H), 7.09 (d, J = 8.0 Hz, 2H), 7.27 (d, J = 7.8 Hz, 2H), 7.43 (s, 1H), 8.23 (s, 1H), 8.44 (s, 1H), 11.66 (s, 1H)

### Example 77

### N-Phenyl-N'- {2-[(2-acetyl-4,5-dimethoxyphenyl)aminocarbonylethyl]-phenyl}urea

Synthesis was carried out in the same manner as in Example 65.

¹H-NMR (DMSO-d₆, 270 MHz) δ ppm : 2.60 (s, 1H), 2.67 (t, J = 7.6 Hz, 2H), 2.89 (t, J = 7.6 Hz, 2H), 3.82 (s, 6H), 7.00 (m, 2H), 7.25 (m, 4H), 7.45 (m, 3H), 7.76 (d, J = 7.3 Hz, 1H), 8.02 (s, 1H), 8.25 (s, 1H), 8.99 (s, 1H), 11.71 (s, 1H)

### Example 78

### N-(4-Aminophenyl)-N'-{2-[(2-acetyl-4,5-dimethoxyphenyl)-aminocarbonylethyl]phenyl}urea

Synthesis was carried out in the same manner as in Example 3.

¹H-NMR (DMSO-d₆, 270 MHz) δ ppm : 2.60 (s, 3H), 2.70 (t, J = 7.6 Hz, 2H), 2.93 (t, J = 7.3 Hz, 2H), 3.82 (s, 6H), 4.76 (s, 2H), 6.49 (d, J =8.6 Hz, 2H), 6.97 (dt, J =1.1, 7.3 Hz, 2H), 7.14 (m, 4H), 7.43 (s, 1H), 7.80 (m, 2H), 8.25 (s, 1H), 8.50 (s, 1H), 11.70 (s, 1H)

### Example 79

### N-(4-Nitrophenyl)-N'- {2-[(2-acetyl-4,5-dimethoxyphenyl)-aminocarbonylethyl]phenyl}urea

The title compound was synthesized in the same manner as in Example 65.

¹H-NMR (DMSO-d₆, 270 MHz) δ ppm : 2.57 (s, 3H), 2.72 (t, J = 7.6 Hz, 2H), 2.96 (t, J = 7.6 Hz, 2H), 3.80 (s, 6H), 7.08 (dt, J = 0.8, 7.3 Hz, 1H), 7.20 (m, 2H), 7.29 (s, 1H), 7.68 (m, 3H), 8.19 (m, 3H), 8.34 (s, 1H), 9.77 (s, 1H), 11.70 (s, 1H)

### Example 80

### N-Phenyl-N'- {4-[(2-acetyl-4,5-dimethoxyphenyl)aminocarbonylpropyl]-phenyl}urea

Synthesis was carried out in the same manner as in Example 65.

¹H-NMR (DMSO-d₆, 270 MHz) δ ppm : 1.90 (m, 2H), 2.38 (t, J = 7.3 Hz, 2H), 2.51 (t, J = 7.3 Hz, 2H), 2.62 (s,3H), 3.82 (s, 6H), 6.95 (t, J = 7.3 Hz, 3H), 7.12 (d, J = 8.9 Hz, 2H), 7.26 (t, J = 7.8 Hz, 2H), 7.37 (d, J = 8.4 Hz, 2H), 7.44 (s, 1H), 7.45 (d, J = 8.4 Hz, 2H), 8.27 (s, 1H), 8.80 (s, 1H), 8.85 (s, 1H), 11.68 (s, 1H)

### Example 81

### N-(4-Aminophenyl)-N'- {4-[(2-acetyl-4,5-dimethoxyphenyl)-aminocarbonylpropyl]phenyl} urea

Synthesis was carried out in the same manner as in Example 3.

¹H-NMR (DMSO-d₆, 270 MHz) δ ppm : 1.89 (m, 2H), 2.37 (t, J = 7.3 Hz, 2H), 2.56 (t, J = 7.3 Hz, 2H), 2.62 (s,3H), 3.82 (s, 6H), 4.76 (s, 2H), 6.49 (d, J = 7.3 Hz, 2H), 7.11 (m,4H), 7.33 (d, J = 8.9 Hz, 2H), 7.44 (s, 1H), 8.13 (s, 1H), 8.27 (s, 1H), 8.43 (s, 1H), 11.68 (s, 1H)

### Example 82

### N-(4-Nitrophenyl)-N'- {4-[(2-acetyl-4,5-dimethoxyphenyl)-aminocarbonylpropyl]phenyl}urea

The title compound was synthesized in the same manner as in Example 65.

¹H-NMR (DMSO-d₆, 270 MHz) δ ppm : 1.91 (m, 2H), 2.38 (t, J = 7.3 Hz, 2H), 2.59 (t, J = 7.3 Hz, 2H), 2.61 (s,3H), 3.82 (s, 6H), 7.15 (d, J = 8.4 Hz, 2H), 7.42 (m, 3H), 7.69 (d, J = 9.2 Hz, 2H), 8.18 (d, J = 9.1 Hz, 2H), 8.27 (s, 1H), 9.02 (s, 1H), 9.60 (s, 1H), 11.68 (s, 1H)

### Example 83

### N-Phenyl-N'- {3-[(2-acetyl-4,5-dimethoxyphenyl)aminocarbonyl]-phenyl}urea

Synthesis was carried out in the same manner as in Example 65.

¹H-NMR (DMSO-d₆, 270 MHz) δ ppm : 2.70 (s, 3H), 3.87 (s, 3H), 3.90 (s, 3H), 6.99 (t, J = 7.3 Hz, 1H), 7.30 (t, J = 7.8 Hz, 2H), 7.51 (m, 5H), 7.75 (d, J = 7.3 Hz, 2H), 8.05 (s, 1H), 8.54 (s, 1H), 8.82 (s, 1H), 9.02 (s, 1H), 12.77 (s, 1H)

### Example 84

### (4-Pyridylmethyl) {4-[(2-acetyl-4,5-dimethoxyphenyl)aminocarbonylethyl]-phenyl}carbamate

The title compound was synthesized in the same manner as in Example 64.

¹H-NMR (DMSO-d₆, 270 MHz) δ ppm : 2.59 (s, 1H), 2.66 (t, J = 7.6 Hz, 2H), 2.88 (t, J = 7.6 Hz, 2H), 3.82 (s, 6H), 5.19 (s, 2H), 7.17 (d, J = 8.4 Hz, 2H), 7.39 (m, 5H), 8.22 (s, 1H), 8.57 (dd, J = 1.4, 4.3 Hz, 2H), 9.82 (s, 1H), 11.65 (s, 1H)

### Example 85

### (4-Pyridylmethyl) {4-[(2-acetyl-4,5-dimethoxyphenyl)aminocarbonylpropyl]-phenyl } carbamate

The title compound was synthesized in the same manner as in Example 64.

¹H-NMR (DMSO-d₆, 270 MHz) δ ppm : 1.89 (m, 2H), 2.37 (t, J = 7.3 Hz, 2H), 2.56 (t, J = 7.3 Hz, 2H), 2.62 (s,3H), 3.82 (s, 6H), 5.19 (s, 2H), 7.17 (d, J = 8.4 Hz, 2H), 7.39 (m, 5H), 8.22 (s, 1H), 8.57 (dd, J = 1.4, 4.3 Hz, 2H), 9.82 (s, 1H), 11.65 (s, 1H)

### Example 86

### (5-Hydroxypentyl) {4-[(2-acetyl-4,5-dimethoxyphenyl)aminocarbonylethyl]-phenyl}carbamate

0.04 g of triphosgene was dissolved in 5 ml of tetrahydrofuran (THF), and a solution of 0.08 g of 5-benzyloxypentyl alcohol and 0.06 g of diisopropylethylamine in 10 ml of THF was slowly added dropwise thereto at room temperature. Thereafter, the mixture was stirred at 60°C for 1 hour. 0.07 g of 2-(4-aminophenyl)carbonylaminoethyl-4,5-dimethoxyacetophenone and 30 mg of dimethylaminopyridine were added thereto and the mixture was stirred at 69°C for 2 hours. The reaction solution was poured into water and extracted with dichloromethane, and the organic layer was dried with anhydrous magnesium sulfate, followed by concentration. The residue was purified by column chromatography on silica gel (eluent, dichloromethane : methanol = 100 : 1 → 40 : 1) , was further washed with methanol, and was then dried. Thus, 0.13 g of pale yellow solid was obtained. Subsequently, the resultant solid and 50 mg of 5 % Pd/C were added to 40 ml of methanol and the mixture was stirred under hydrogen atmosphere at room temperature for 22 hours. The reaction solution was filtered and the filtrate was concentrated. The residue was washed with methanol and subjected to vacuum drying. Thus, 40 mg of the title compound was obtained as a white solid.

¹H-NMR (DMSO-d₆, 270 MHz) δ ppm : 1.42 (m, 4H), 1.61 (m, 2H), 2.51 (s, 1H), 2.63 (t, J = 7.3 Hz, 2H), 2.87 (t, J = 7.6 Hz, 2H), 3.40 (t, J = 5.7 Hz, 2H), 3.82 (s, 6H), 4.05 (t, J = 6.8 Hz, 2H), 4.37 (t, J = 5.1 Hz, 2H), 7.15 (d, J = 8.4 Hz, 2H), 7.39 (d, J = 8.4 Hz, 2H), 7.42 (s, 1H), 8.22 (s, 1H), 9.50 (s, 1H), 11.65 (s, 1H)

### Example 87

### {4-[(2-Acetyl-4,5-dimethoxyphenyl)aminocarbonylethyl]phenyl} phenylcarbamate

1.1 g of 3-(4-hydroxyphenyl)propionic acid was dissolved in 30 ml of THF and 0.82 g of acetic anhydride and 0.83 g of pyridine were then added thereto. The mixture was stirred at room temperature for 16 hours. The solvent was removed by distillation under reduced pressure and 5% citric acid was added to the residue. The precipitated solid was separated by filtration and washed with water and subjected to vacuum drying. Thus, 0.80 g of white solid was obtained. The resultant solid and 10 ml of thionyl chloride were added to 30 ml of chloroform and the mixture was stirred under reflux for 2 hours. The solvent was removed by distillation under reduced pressure and dissolved in 30 ml of THF. Thereafter, 0.59 g of 2-amino-4,5-dimethoxyacetophenone and 0.61 g of triethylamine were added thereto and the mixture was stirred under reflux for 3 hours. After the solvent was removed by distillation under reduced pressure, the residue and 0.20 g of sodium hydroxide were added to a mixed solvent comprising 10 ml of methanol and 30 ml of water and the mixture was stirred at room temperature for 16 hours. Half of the solvent was removed by distillation under reduced pressure and neutralized with hydrochloric acid. The precipitated solid was separated by filtration, washed with a mixed solvent of dichloromethane/methanol, and subjected to vacuum drying. Thus, 0.56 g of white solid was obtained. 0.11 g of the resultant solid, 90 mg of phenyl isocyanate, and 60 mg of triethylamine were added to 10 ml of THF and the mixture was stirred at room temperature for 16 hours. The precipitated solid was separated by filtration to obtain 58 mg of the title compound as a white solid.

¹H-NMR (DMSO-d₆, 270 MHz) δ ppm : 2.60 (s, 1H), 2.74 (t, J = 7.3 Hz, 2H), 2.96 (t, J = 7.3 Hz, 2H), 3.82 (s, 6H), 7.04 (t, J =7.3 Hz, 1H), 7.13 (d, J =8.6 Hz, 2H), 7.31 (m, 4H), 7.43 (s, 1H), 7.49 (d, J =7.8Hz, 2H), 8.24 (s, 1H), 10.18 (s, 1H), 11.69 (s, 1H)

### Example 88

### {4-[(2-acetyl-4,5-dimethoxyphenyl)aminocarbonylethyl]phenyl} 4-nitrophenylcarbamate

The title compound was synthesized in the same manner as in Example 87.

¹H-NMR (DMSO-d₆, 270 MHz) δ ppm : 2.60 (s, 3H), 2.77 (t, J = 7.3 Hz, 2H), 2.97 (t, J = 7.3 Hz, 2H), 3.82 (s, 6H), 7.18 (d, J = 8.4 Hz, 2H), 7.33 (d, J =8.9 Hz, 2H), 7.43 (s, 1H), 7.73 (d, J =9.1 Hz, 2H), 8.24 (s, 1H), 8.25 (d, J =9.1 Hz, 2H), 10.90 (s, 1H), 11.69 (s, 1H)

### Example 89

### {4-[(2-Acetyl-4,5-dimethoxyphenyl)aminocarbonylethyl]phenyl} 4-aminophenylcarbamate

Synthesis was carried out in the same manner as in Example 3.

¹H-NMR (DMSO-d₆, 270 MHz) δ ppm : 2.60 (s, 3H), 2.73 (t, J = 7.3 Hz, 2H), 2.95 (t, J = 7.3 Hz, 2H), 3.82 (s, 6H), 4.85 (s, 2H), 6.50 (d, J =8.1 Hz, 2H), 7.10 (m, 4H), 7.28 (d, J =8.4 Hz, 2H), 7.43 (s, 1H), 8.24 (s, 1H), 9.66 (s, 1H), 11.69 (s, 1H)

### Example 90

### (4-[(2-Acetyl-4,5-dimethoxyphenyl)aminocarbonylethyl]phenyl} 4-pyridylcarbamate

The title compound was synthesized in the same manner as in Example 87.

¹H-NMR (DMSO-d₆, 270 MHz) δ ppm : 2.60 (s, 3H), 2.65 (t, J = 7.3 Hz, 2H), 2.82 (t, J = 7.3 Hz, 2H), 3.82 (s, 6H), 7.19 (dd, J = 1.6, 5.9 Hz, 2H), 6.65 (d, J = 8.6 Hz, 2H), 7.03 (d, J = 8.4 Hz, 2H), 7.43 (s, 1H), 7.95 (d, J = 6.2 Hz, 2H), 8.40 (s, 1H), 9.16 (s, 1H), 11.65 (s, 1H)

### Test Example 1: Inhibition test against PDGF-BB-stimulated proliferation of smooth muscle cells

Human coronary vessel smooth muscle cells (primary culture) were spread on a 96-well microplate (50,000 cells/well) and cultured for 24 hours. After cells were confirmed to be confluent, the cells were cultured in a serum-free culture medium comprising 0.4 or 2 µM of compound added thereto (containing 20 ng/ml PDGF-BB) for 24 hours. ³H-thymidine (1 µCi/well) was added and was cultured for 4 hours. After cells were collected on a filter, Creasol (4 ml/vial) was added and the uptake quantity of ³H-thymidine was measured with a scintillation counter. The activity for inhibiting proliferation of the test compound was represented by a concentration (IC₅₀) indicating 50% inhibition based on the untreated group (no PDGF-BB added). As a control compound, tranilast and Reference Example 1 (compound of Compound 17 in Example 4 described in WO 97/09301) were employed. The result is as shown in Table 11.

**Table 11**

| Name of compound | Inhibition against PDGF-BB-stimulated proliferation of smooth muscle cell, IC₅₀ (µM) |
|---|---|
| Example 1 | 0.28 |
| Example 3 | 0.10 |
| Example 4 | 0.40 |
| Example 5 | 0.23 |
| Example 6 | 0.33 |
| Example 8 | 0.15 |
| Example 9 | 0.20 |
| Example 10 | 0.44 |
| Example 11 | 0.19 |
| Example 13 | 0.34 |
| Example 14 | 0.23 |
| Example 15 | 0.57 |
| Example 16 | 0.14 |
| Example 17 | 0.75 |
| Example 18 | 0.40 |
| Example 20 | 0.27 |
| Example 23 | 0.72 |
| Example 24 | 0.24 |
| Example 25 | 0.07 |
| Example 28 | 0.25 |
| Example 32 | 0.36 |
| Example 33 | 0.56 |
| Example 34 | 0.64 |
| Example 36 | 0.55 |
| Example 37 | 0.57 |
| Example 38 | 0.82 |
| Example 39 | 0.65 |
| Example 41 | 0.20 |
| Example 43 | 0.15 |
| Example 45 | 0.0001 |
| Example 46 | 0.057 |
| Example 47 | 0.011 |
| Example 48 | 0.008 |
| Example 49 | 0.015 |
| Example 50 | <0.08 |
| Example 53 | <0.0032 |
| Example 54 | 0.20 |
| Example 55 | 0.014 |
| Example 56 | 0.028 |
| Example 57 | 0.28 |
| Example 61 | 0.67 |
| Example 62 | 0.34 |
| Example 63 | 0.3 |
| Example 64 | <0.0032 |
| Example 66 | <0.016 |
| Example 67 | 0.020 |
| Example 68 | 0.026 |
| Example 69 | 0.061 |
| Example 70 | 0.045 |
| Example 71 | 0.061 |
| Example 72 | 0.039 |
| Example 74 | 0.31 |
| Example 75 | 0.16 |
| Example 77 | 0.20 |
| Example 80 | 0.05 |
| Example 81 | 0.06 |
| Example 82 | 0.002 |
| Example 83 | 0.31 |
| Example 84 | 0.044 |
| Example 85 | 0.079 |
| Example 86 | 0.49 |
| Example 87 | 0.083 |
| Example 88 | <0.016 |
| Example 89 | 0.31 |
| Example 91 | 0.22 |
| Example 92 | 0.39 |
| Example 93 | 0.011 |
| Example 94 | 0.037 |
| Example 96 | 0.17 |
| Tranilast | 24.5 |
| Reference Example 1 | 6.3 |

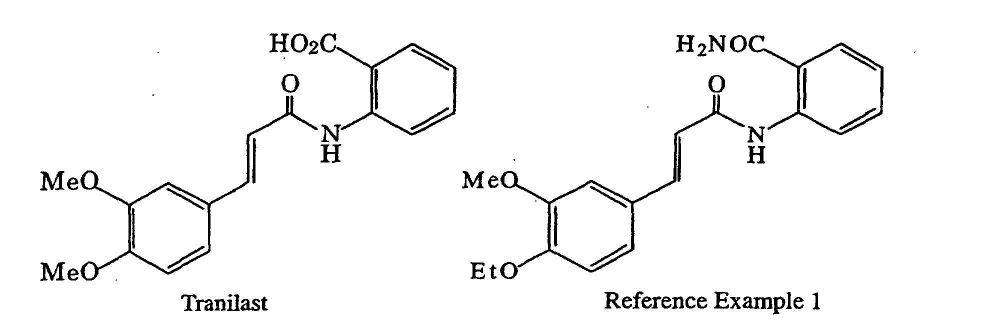

### Test Example 2: Inhibition test against PDGF-BB-stimulated proliferation of mesangial cells

Human mesangial cells (primary culture) were spread on a 96-well microplate (27,000 cells/well) and cultured for 24 hours. After cells were confirmed to be confluent, the cells were cultured in a serum-free culture medium comprising 0.016, 0.08, or 0.4 µM of test compound added thereto (containing 20 ng/ml PDGF-BB) for 24 hours. ³H-thymidine (1 µCi/well) was added and was cultured for 4 hours. Cells were then collected on a filter and the uptake quantity of ³H-thymidine was measured with a scintillation counter. The activity for inhibiting proliferation of the test compound was represented by a concentration (IC₅₀) for inhibiting 50% of the increase in the uptake quantity of ³H-thymidine by PDGF-BB stimulation (the value determined by subtracting a control without PDGF-BB added from a control with PDGF-BB added). As a control compound, tranilast was employed. The result is as shown in Table 12.

**Table 12**

| Name of compound Name of compound | Inhibition against PDGF-BB-stimulated proliferation of mesangial cell, IC₅₀ (µM) |
|---|---|
| Example 1 | 0.81 |
| Example 3 | 1.72 |
| Example 5 | 0.87 |
| Example 6 | 0.33 |
| Example 8 | 0.95 |
| Example 9 | 2.3 |
| Example 16 | 0.29 |
| Example 17 | 1.80 |
| Example 18 | 1.27 |
| Example 24 | 0.58 |
| Example 25 | 0.17 |
| Example 39 | 0.51 |
| Tranilast (78% inhibition concentration) : 10 µM | |

### Test Example 3: Proliferation of human funis venous endothelial cell (HUVEC)

HUVEC that was purchased from Clonetics (San Diego) was cultured in EGM-2 medium in the presence of 5% CO₂ at 37°C. HUVEC was wound into a U-bottomed 96-well plate (Falcon) to realize 3 × 10³ cells/100µl/well and was cultured at 37°C for 24 hours. Thereafter, 100 ì 1 of solution of a compound, which was prepared to a two-fold concentration in EGM-2 medium, was added and was cultured for an additional three days. [Methyl-³H] thymidine (1 µCi/20µl/well, Amersham) was added and four hours later cells were trapped into a 96-well glass filter (UniFilter-GF/C, Packard Japan) with the aid of a cell harvester for TopCount. A scintillation cocktail (MICROSCINT-20, Packard Japan) was added to bring the mixture to 20 µl/well, radioactivity was measured using TopCount (Packard Japan), and the inhibitive capacity against cell proliferation of various compounds was determined. As a control, as with Test Example 1, tranilast and a tranilast derivative (a compound of Compound 17, Example 4 described in WO 97/09301) were employed. The result is shown in Table 13.

**Table 13**

| Name of compound Name of compound | Inhibition against proliferation of human funis vascular endothelial cell, IC₅₀ (µM) |
|---|---|
| Example 4 | 1.2 |
| Example 6 | 3.6 |
| Example 15 | 2.1 |
| Example 18 | 0.5 |
| Example 45 | 0.0002 |
| Example 48 | 0.05 |
| Example 53 | 0.05 |
| Example 55 | 0.28 |
| Example 64 | 0.03 |
| Example 66 | 0.05 |
| Example 67 | 0.20 |
| Example 68 | 0.0006 |
| Example 74 | 1.3 |
| Example 75 | 0.16 |
| Example 77 | 1.7 |
| Example 82 | 0.0008 |
| Example 93 | 0.04 |
| Example 94 | 0.88 |
| Tranilast | 10.0 |
| Reference Example 1 | > 10 |

### Preparation Example

Tablets having the following formulation were prepared in accordance with the conventional method.

| | |
|---|---|
| Compound of Example 1 | 100 mg |
| Lactose | 120 mg |
| Potato starch | 30 mg |
| Hydroxypropylcellulose | 5 mg |
| Carboxymethylcellulose sodium | 7 mg |
| Magnesium stearate | 0.5 mg |

All publications, patents and patent applications cited herein are incorporated herein by reference in their entirety.

### INDUSTRIAL APPLICABILITY

The diarylamide derivative of the present invention has inhibitive activity against cell proliferation caused by PDGF and is useful for prevention or treatment of cell proliferative diseases such as arteriosclerosis, vascular reocclusion disease, and nephritis.

## Claims

1. A diarylamide derivative represented by general formula (1) or a pharmaceutically acceptable salt thereof: 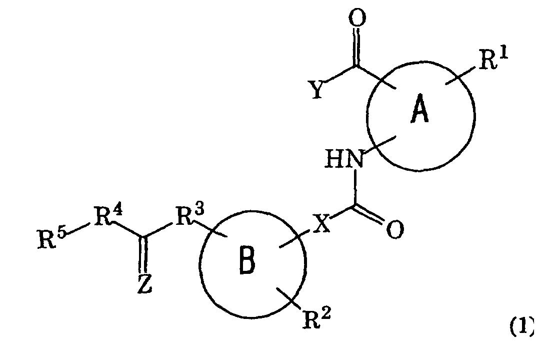 wherein,
A is an aromatic ring selected from the group consisting of a benzene ring, a pyridine ring, a thiophene ring, a furan ring, and a naphthalene ring;
a substituent represented by COY and a substituent represented by NHCOX are adjacent to each other and these substituents are linked to a carbon atom in the aromatic ring;
X denotes a C₁-C₄-alkylene group, a C₁-C₄-alkyleneoxy group, or a single bond;
Y is selected from the group consisting of a C₁-C₄-alkyl group, a C₁-C₄-alkoxy group, a hydroxyl group, and N(R⁶)(R⁷) in which each of R⁶ and R⁷, which can be the same or different, is selected from the group consisting of a hydrogen atom, a C₁-C₄-alkyl group, a C₁-C₄-alkoxy group, a C₃-C₉-cycloalkyl group, a C₄-C₉-cycloalkyl-alkyl group, a C₅-C₈-morpholino-N-alkoxy group, a C₃-C₉-alkenyl group, a phenyl group, a pyridyl group, and an aralkyl group, wherein the phenyl group and the pyridyl group and the aromatic ring of the aralkyl group are optionally substituted with 1 to 3 substituents selected from the group consisting of a C₁-C₄-alkyl group, a C₁-C₄-alkoxy group, and a halogen atom;
R¹ is selected from the group consisting of a hydrogen atom, a halogen atom, a hydroxyl group, a C₁-C₄-alkyl group, a C₃-C₉-cycloalkyl group, a C₄-C₉-cycloalkylalkyl group, a C₁-C₄-alkoxy group, a C₃-C₉-cycloalkyloxy group, a C₄-C₉-cycloalkylalkoxy group, an aralkyloxy group, a C₁-C₄-acyl group, and a nitro group and 1 to 4 R¹ₛ are present at a desired position in A, each of which can be the same or different, and when two R¹s are present, they may together form a C₁-C₄-alkylenedioxy group, provided that, when A is a benzene ring, R¹ does not denote a hydrogen atom;
B denotes a benzene, pyridine, or thiophene ring;
R² is a substituent selected from the group consisting of a hydrogen atom, a halogen atom, a hydroxyl group, a C₁-C₄-alkyl group, a C₁-C₄-alkoxy group, a C₁-C₄-alkylthio group, a C₁-C₄-hydroxyalkoxy group, a C₃-C₉-cycloalkyloxy group, a C₄-C₉-cycloalkyl-alkoxy group, an aralkyloxy group, a C₁-C₄-acyl group, a cyano group, a C₅-C₈-morpholino-N-alkoxy group, and an amino group which can be monosubstituted or disubstituted with a C₁-C₄-alkyl group, and 1 to 4 R²s, each of which can be the same or different, are present at a desired position;
R³ and R⁴ denote, when Y denotes other than a C₁-C₄-alkyl group, an oxygen atom or NR⁸ in which each R⁸ is selected from the group consisting of a hydrogen atom and a C₁-C₄-alkyl group, each of which can be the same or different, and when Y denotes a C₁-C₄-alkyl group, R³ denotes an oxygen atom or NR⁸ and R⁴ denotes an oxygen atom, NR⁸, or a single bond;
R⁵ is selected from the group consisting of a C₁-C₈-alkyl group, a C₂-C₄-alkenyl group, a C₃-C₉-cycloalkyl group, a C₄-C₉-cycloalkyl-alkyl group, a tetrahydropyranyl group, an aralkyl group, an indanyl group, an aromatic acyl group, a phenyl group, a pyridyl group, a furyl group, and a thienyl group wherein the aromatic rings of the aralkyl group, the indanyl group, and the aromatic acyl group, the phenyl group, the pyridyl group, the furyl group, and the thienyl group optionally have 1 to 5 substituents selected from the group consisting of a halogen atom, a hydroxyl group, a cyano group, a C₁-C₄-alkyl group, a C₁-C₄-alkoxy group, a C₁-C₄-alkylthio group, a C₂-C₅-alkoxycarbonyl group, a carboxyl group, a C₁-C₄-acyl group, an aromatic acyl group, a C₁-C₄-acyloxy group, a trifluoromethyl group, a phenyl group, a phenoxy group, a phenylthio group, a pyridyl group, a morpholino group, an aralkyloxy group, a nitro group, a methylsulfonyl group, an aminosulfonyl group, and an amino group that is optionally monosubstituted or disubstituted with a C₁-C₄-alkyl group or a C₁-C₄-acyl group, and wherein adjacent two substituents may together form a C₁-C₄-alkylenedioxy group to form a ring; and
Z denotes an oxygen or sulfur atom.

2. The compound according to claim 1 wherein, in general formula (1), X denotes a C₁-C₄-alkylene group.

3. The compound according to claim 1 wherein, in general formula (1), X denotes a single bond.

4. The compound according to claim 1 wherein, in general formula (1), each of A and B, which can be the same or different, denotes a benzene ring or a pyridine ring.

5. The compound according to claim 1 wherein, in general formula (1), A and B denote a benzene ring.

6. The compound according to claim 1 wherein, in general formula (1), Y denotes an unsubstituted amino group, a hydroxyl group, or a C₁-C₄-alkoxy group.

7. The compound according to claim 1 wherein, in general formula (1), Y denotes a C₁-C₄-alkyl group.

8. The compound according to claim 1 wherein, in general formula (1), R² denotes a hydrogen atom or a C₁-C₄-alkoxy group.

9. The compound according to claim 1 wherein, in general formula (1), R⁵ denotes a benzyl group, a phenyl group, a pyridyl group, or a pyridylmethyl group wherein the aromatic rings of the benzyl group and the pyridylmethyl group and the phenyl group and the pyridyl group optionally have 1 to 5 substituents selected from the group consisting of a halogen atom, a C₁-C₄-alkyl group, a C₁-C₄-alkoxy group, a C₂-C₅-alkoxycarbonyl group, a C₁-C₄-acyl group, a trifluoromethyl group, a C₁-C₄-alkylthio group, and an amino group which has been disubstituted with a C₁-C₄-alkyl group.

10. The compound according to claim 1 wherein, in general formula (1), R⁵ denotes a C₁-C₄-alkyl group, a C₂-C₄-alkenyl group, or a C₃-C₆-cycloalkyl group.

11. The compound according to claim 1 wherein, in general formula (1), R³ and R⁴ denote NH.

12. The compound according to claim 1 wherein, in general formula (1), R³ denotes NH and R⁴ denotes an oxygen atom.

13. A pharmaceutical composition comprising, as an active ingredient, the compound or pharmaceutically acceptable salt thereof according to claim 1.

14. A pharmaceutical composition comprising, as an active ingredient, the compound or pharmaceutically acceptable salt thereof according to claim 1, that is usable for prevention or treatment of diseases caused by abnormal proliferation of vascular smooth muscle cells.

15. A pharmaceutical composition comprising, as an active ingredient, the compound or pharmaceutically acceptable salt thereof according to claim 1, that is usable for prevention or treatment of restenosis or atherosclerosis after percutaneous transluminal coronary angioplasty or coronary artery bypass surgery.

16. A pharmaceutical composition comprising, as an active ingredient, the compound or pharmaceutically acceptable salt thereof according to claim 1, that is usable for prevention or treatment of diseases caused by abnormal proliferation of mesangial cells.

17. A pharmaceutical composition comprising, as an active ingredient, the compound or pharmaceutically acceptable salt thereof according to claim 1, that is usable for prevention or treatment of diseases caused by abnormal proliferation of vascular endothelial cells or epidermal cells.

18. A pharmaceutical composition comprising, as an active ingredient, the compound or pharmaceutically acceptable salt thereof according to claim 1, that is usable for prevention or treatment of psoriasis, diabetic retinopathy, or senile disciform macular degeneration.
